(19) Europäisches Patentamt
European Patent Office
Office européen des brevets

(11) EP 3 816 271 A1

(12) EUROPEAN PATENT APPLICATION

(43) Date of publication:
**05.05.2021 Bulletin 2021/18**

(51) Int Cl.:
***C11D 3/386** (2006.01)* ***C12N 9/88** (2006.01)*

(21) Application number: **20191348.0**

(22) Date of filing: **17.08.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.10.2019 EP 19206504**

(71) Applicant: **The Procter & Gamble Company**
**Cincinnati, OH 45202 (US)**

(72) Inventors:
- **BETTIOL, Jean-Luc Philippe**
  **1853 Strombeek-Bever (BE)**
- **GONZALES, Denis Alfred**
  **1853 Strombeek-Bever (BE)**
- **NAMANJA-MAGLIANO, Hilda Andamiche**
  **Cincinnati, Ohio 45202 (US)**
- **VELASQUEZ, Juan Esteban**
  **Cincinnati, Ohio 45202 (US)**

(74) Representative: **P&G Patent Belgium UK**
**N.V. Procter & Gamble Services Company S.A.**
**Temselaan 100**
**1853 Strombeek-Bever (BE)**

# (54) DETERGENT COMPOSITION

(57) The need for a hand-dishwashing composition which provides good sudsing and a good suds profile even in the presence of greasy stains comprising higher chain-length saturated and/or unsaturated fatty acid chains, as well as improved removal of such stains, is met by formulating the composition with a P450 fatty acid decarboxylase and a surfactant system.

EP 3 816 271 A1

## Description

REFERENCE TO A SEQUENCE LISTING

This application contains a Sequence Listing in computer readable form. The computer readable form is incorporated herein by reference.

FIELD OF THE INVENTION

The present invention relates to a hand dishwashing detergent composition comprising a surfactant system and at least one P450 fatty acid decarboxylase. The P450 fatty acid decarboxylases improve sudsing and grease removal by catalyzing the conversion of at least one fatty acid selected from the group consisting of: palmitic acid, stearic acid, oleic acid, linoleic acid, linolenic acid, and mixtures thereof.

BACKGROUND OF THE INVENTION

Hand-dishwashing detergent compositions should have a good suds profile, in particular a long lasting suds profile. Users typically connate the presence of suds with good residual cleaning, a lack of suds can lead to over-use of the detergent composition, especially in the presence of greasy soils. The appearance of the suds, such as its density and whiteness is also often seen as an indicator of the cleaning efficacy of the wash solution. However, greasy soils inhibit suds generation and promotes suds collapse, even when sufficient surfactancy is present to ensure good cleaning, including grease removal. It has now been found that greasy soils containing higher chain-length saturated and/or unsaturated fatty acid chains are particularly effective at inhibiting sudsing, especially inhibiting long lasting sudsing. In addition, such greasy soils containing higher chain-length saturated and/or unsaturated fatty acid chains are particularly hard to remove from dishes. Such greasy soils comprise long chain fatty acids, especially long chain saturated fatty acids such as palmitic acid and stearic acid, and long chain unsaturated fatty acids, such as oleic acid, linoleic acid, and linolenic acid, which can act as a suds suppressors. Conversion of these long chain saturated and/or unsaturated fatty acids into suds neutral or potentially suds boosting compounds is as such desired.

The use of two different classes fatty acid decarboxylases, OleT-like and UndA-like, to enhance the sudsing profile of detergent compositions have been previously reported (EP 3,243,896B1). However, these enzymes usually have a strong preference for medium chain length fatty acids (e.g. C12, C14), while longer fatty acids (e.g. oleic acid) are converted slowly or not converted. Thus, there remains a need for hand dishwashing compositions that have greater efficacy in transforming long chain fatty acids efficiently into components which are less suds-suppressing.

Moreover, it is often necessary to add more surfactant than necessary for cleaning, in order to provide the desired level of sudsing, as well as meet the users' need for sustained sudsing.

Hence, a need remains for a hand-dishwashing detergent which provides good sudsing and a good suds profile even in the presence of greasy stains comprising higher chain-length saturated and/or unsaturated fatty acid chains, as well as improved removal of such stains. A further need remains for a hand-dishwashing detergent which provides the desired level of sudsing, especially in the presence of such greasy stains, at lower surfactant levels.

EP3556834A relates to a detergent composition, preferably a manual dishwashing detergent composition and method of washing comprising a surfactant system and a fatty acid decarboxylase enzyme. EP3243896A relates to detergent compositions, especially manual dishwashing detergent compositions and method of washing comprising a surfactant system and a fatty acid decarboxylase enzyme. US 2009/0142821 A1 relates to novel variants of cytochrome P450 oxygenases. These variants have an improved ability to use peroxide as an oxygen donor as compared to the corresponding wild-type enzyme. These variants also have an improved thermostability as compared to the cytochrome P450 BM-3 F87 A mutant. Preferred variants include cytochrome P450 BM-3 heme domain mutants having I58V, F87A, H100R, F107L, A135S, M145A/V, N239H, S274T, L3241, 1366V, K434E, E442K, and/or V446I amino acid substitutions. S CHRISTOPHER DAVIS ET AL, "Oxidation of v-Oxo Fatty Acids by Cytochrome P450 BM-3 (CYP102)", ARCHIVES OF BIOCHEMISTRY AND BIOPHYSICS, (19960401), vol. 328, no. 1, pages 35 - 42 discusses the oxidation of aldehydes by cytochrome P450 enzymes either to the corresponding acid or, via a decarboxylation mechanism, to an olefin one carbon shorter than the parent substrate, and explores the factors that control partitioning between these two pathways. The authors have examined the cytochrome P450BM-3 (CYP102)-catalyzed oxidation of fatty acids with a terminal aldehyde group. P450BM-3 has been found to oxidize 18-oxooctadecanoic, 16-oxohexadecanoic, 14-oxotetradecanoic, and 12-oxododecanoic acids exclusively to the corresponding $\alpha,\omega$-diacids. The results demonstrated that aldehyde oxidation by cytochrome P450BM-3 is insensitive to changes in substrate structure expected to stabilize the transition state for decarboxylation. Decarboxylation, in contrast to the oxidation of aldehydes to acids, depends on specific substrate-protein interactions and is enzyme-specific. JAMES BELCHER ET AL., "Structure and Biochemical Properties of the Alkene Producing Cytochrome P450 OleTJE (CYP152L1) from the Jeotgalicoccus sp. 8456 Bacterium", JOURNAL

OF BIOLOGICAL CHEMISTRY, (20140307), vol. 289, no. 10, doi:10.1074/jbc.M113.527325, ISSN 0021-9258, pages 6535 - 6550, presents the biochemical characterization and crystal structures of a cytochrome P450 fatty acid peroxygenase: the terminal alkene forming $OleT_{JE}$ (CYP152L1) from *Jeotgalicoccus* sp. 8456. GIRVAN HAZEL M ET AL., "Applications of microbial cytochrome P450 enzymes in biotechnology and synthetic biology", CURRENT OPINION IN CHEMICAL BIOLOGY, (20160322), vol. 31, doi:10.1016/J.CBPA.2016.02.018, ISSN 1367-5931, pages 136 - 145, XP029536984 [A] 1-15 is a review focusing on the enzymatic properties and reaction mechanisms of P450 enzymes, and on recent studies that highlight their broad applications in the production of oxychemicals.

## SUMMARY OF THE INVENTION

The present invention relates to a hand-dishwashing composition comprising: a surfactant system comprising at least one anionic surfactant; and a P450 fatty acid decarboxylase;.

The present invention further relates to a method of manually washing dishware comprising the steps of delivering a detergent composition according to the invention into a volume of water to form a wash solution and immersing the dishware in the solution.

## DETAILED DESCRIPTION OF THE INVENTION

The need for compositions and methods which provide for good sudsing, including a good long-lasting suds-profile, even in the presence of greasy stains comprising higher chain-length saturated and/or unsaturated fatty acid chains, even at lower surfactant levels, can be met by formulating the hand-dishwashing composition with a P450 fatty acid decarboxylase, wherein said decarboxylase comprises a polypeptide sequence having at least about 80% identity to one or more sequences selected from the group consisting of: SEQ ID NO: 2, 22, 44, 60, 65, 71, 83, 117, 121, 122, 156, and their functional fragments thereof. Such P450 fatty acid decarboxylases catalyse the conversion of at least one fatty acid selected from the group consisting of: palmitic acid, stearic acid, oleic acid, linoleic acid, linolenic acid, and mixtures thereof, preferably stearic acid, oleic acid, and mixtures thereof. Such compositions are also particularly effective at removing grease stains comprising higher chain-length saturated and/or unsaturated fatty acid chains.

### Definitions

As used herein, "dishware" includes cookware and tableware.

As used herein, the articles "a" and "an" when used in a claim, are understood to mean one or more of what is claimed or described.

As used herein, the term "substantially free of" or "substantially free from" means that the indicated material is present in an amount of no more than about 5 wt%, preferably no more than about 2%, and more preferably no more than about 1 wt% by weight of the composition.

As used therein, the term "essentially free of" or "essentially free from" means that the indicated material is present in an amount of no more than about 0.1 wt% by weight of the composition, or preferably not present at an analytically detectible level in such composition. It may include compositions in which the indicated material is present only as an impurity of one or more of the materials deliberately added to such compositions.

All percentages and ratios used hereinafter are by weight of total composition, unless otherwise indicated. All percentages, ratios, and levels of ingredients referred to herein are based on the actual amount of the ingredient, and do not include solvents, fillers, or other materials with which the ingredient may be combined as a commercially available product, unless otherwise indicated.

As used herein the phrase "detergent composition" refers to compositions and formulations designed for cleaning soiled surfaces. Such compositions include dish-washing compositions.

As used herein the term "improved suds longevity" means an increase in the duration of visible suds in a washing process cleaning soiled articles using the composition comprising enzymes of use in the compositions of the present invention, compared with the suds longevity provided by the same composition and process in the absence of the enzyme.

As used herein, the term "soiled surfaces" refers to soiled dishware.

As used herein, the term "water hardness" or "hardness" means uncomplexed cation ions (*i.e.,* $Ca^{2+}$ or $Mg^{2+}$) present in water that have the potential to precipitate with anionic surfactants or any other anionically charged detergent actives under alkaline conditions, and thereby diminishing the surfactancy and cleaning capacity of surfactants. Further, the terms "high water hardness" and "elevated water hardness" can be used interchangeably and are relative terms for the purposes of the present invention, and are intended to include, but not limited to, a hardness level containing at least 12 grams of calcium ion per gallon water (gpg, "American grain hardness" units).

As used herein, the terms "protein," "polypeptide," and "peptide" are used interchangeably herein to denote a

polymer of at least two amino acids covalently linked by an amide bond, regardless of length or post-translational modification (e.g., glycosylation, phosphorylation, lipidation, myristilation, ubiquitination, etc.). Included within this definition are D- and L-amino acids, and mixtures of D- and L-amino acids.

As used herein, "polynucleotide" and "nucleic acid" refer to two or more nucleosides that are covalently linked together. The polynucleotide may be wholly comprised ribonucleosides (i.e., an RNA), wholly comprised of 2' deoxyribonucleotides (i.e., a DNA) or mixtures of ribo- and 2' deoxyribonucleosides. While the nucleosides will typically be linked together via standard phosphodiester linkages, the polynucleotides may include one or more non-standard linkages. The polynucleotide may be single-stranded or double-stranded, or may include both single-stranded regions and double-stranded regions. Moreover, while a polynucleotide will typically be composed of the naturally occurring encoding nucleobases (i.e., adenine, guanine, uracil, thymine, and cytosine), it may include one or more modified and/or synthetic nucleobases (e.g., inosine, xanthine, hypoxanthine, etc.). Such modified or synthetic nucleobases can be encoding nucleobases.

As used herein, "coding sequence" refers to that portion of a nucleic acid (e.g., a gene) that encodes an amino acid sequence of a protein.

As used herein, "naturally occurring," "wild-type," and "WT" refer to the form found in nature. For example, a naturally occurring or wild-type polypeptide or polynucleotide sequence is a sequence present in an organism that can be isolated from a source in nature and which has not been intentionally modified by human manipulation.

As used herein, "non-naturally occurring" or "engineered" or "recombinant" when used in the present invention with reference to (e.g., a cell, nucleic acid, or polypeptide), refers to a material, or a material corresponding to the natural or native form of the material, that has been modified in a manner that would not otherwise exist in nature, or is identical thereto but produced or derived from synthetic materials and/or by manipulation using recombinant techniques. Non-limiting examples include, among others, recombinant cells expressing genes that are not found within the native (non-recombinant) form of the cell or express native genes that are otherwise expressed at a different level.

As used herein the term "identity" means the identity between two or more sequences and is expressed in terms of the identity or similarity between the sequences as calculated over the entire length of a sequence aligned against the entire length of the reference sequence. Sequence identity can be measured in terms of percentage identity; the higher the percentage, the more identical the sequences are. The percentage identity is calculated over the length of comparison. For example, the identity is typically calculated over the entire length of a sequence aligned against the entire length of the reference sequence. Methods of alignment of sequences for comparison are well known in the art and identity can be calculated by many known methods. Various programs and alignment algorithms are described in the art. It should be noted that the terms 'sequence identity' and 'sequence similarity' can be used interchangeably.

As used herein, "percentage of sequence identity," "percent identity," and "percent identical" refer to comparisons between polynucleotide sequences or polypeptide sequences, and are determined by comparing two optimally aligned sequences over a comparison window, wherein the portion of the polynucleotide or polypeptide sequence in the comparison window may comprise additions or deletions (i.e., gaps) as compared to the reference sequence for optimal alignment of the two sequences. The percentage is calculated by determining the number of positions at which either the identical nucleic acid base or amino acid residue occurs in both sequences or a nucleic acid base or amino acid residue is aligned with a gap to yield the number of matched positions, dividing the number of matched positions by the total number of positions in the window of comparison and multiplying the result by 100 to yield the percentage of sequence identity.

As used herein, the term "variant" of P450 fatty acid decarboxylase enzyme means a modified P450 fatty acid decarboxylase enzyme amino acid sequence by or at one or more amino acids (for example 1, 2, 3, 4, 5, 6, 7, 8, 9 or 10 or more amino acid modifications) selected from substitutions, insertions, deletions and combinations thereof. The variant may have "conservative" substitutions, wherein a substituted amino acid has similar structural or chemical properties to the amino acid that replaces it, for example, replacement of leucine with isoleucine. A variant may have "non-conservative" changes, for example, replacement of a glycine with a tryptophan. Variants may also include sequences with amino acid deletions or insertions, or both. Guidance in determining which amino acid residues may be substituted, inserted, or deleted without abolishing the activity of the protein may be found using computer programs well known in the art. Variants may also include truncated forms derived from a wild-type P450 fatty acid decarboxylase enzyme, such as for example, a protein with a truncated N-terminus. Variants may also include forms derived by adding an extra amino acid sequence to a wild-type protein, such as for example, an N-terminal tag, a C-terminal tag or an insertion in the middle of the protein sequence.

As used herein, "reference sequence" refers to a defined sequence to which another sequence is compared. A reference sequence may be a subset of a larger sequence, for example, a segment of a full-length gene or polypeptide sequence. Generally, a reference sequence is at least 20 nucleotide or amino acid residues in length, at least 25 residues in length, at least 50 residues in length, or the full length of the nucleic acid or polypeptide. Since two polynucleotides or polypeptides may each (1) comprise a sequence (i.e., a portion of the complete sequence) that is similar between the two sequences, and (2) may further comprise a sequence that is divergent between the two sequences, sequence

comparisons between two (or more) polynucleotides or polypeptide are typically performed by comparing sequences of the two polynucleotides over a comparison window to identify and compare local regions of sequence similarity. The term "reference sequence" is not intended to be limited to wild-type sequences, and can include engineered or altered sequences. For example, a "reference sequence" can be a previously engineered or altered amino acid sequence.

As used herein, "comparison window" refers to a conceptual segment of at least about 20 contiguous nucleotide positions or amino acids residues wherein a sequence may be compared to a reference sequence of at least 20 contiguous nucleotides or amino acids and wherein the portion of the sequence in the comparison window may comprise additions or deletions (i.e., gaps) of 20 percent or less as compared to the reference sequence (which does not comprise additions or deletions) for optimal alignment of the two sequences. The comparison window can be longer than 20 contiguous residues, and includes, optionally 30, 40, 50, 100, or longer windows.

As used herein, "corresponding to", "reference to" or "relative to" when used in the context of the numbering of a given amino acid or polynucleotide sequence refers to the numbering of the residues of a specified reference sequence when the given amino acid or polynucleotide sequence is compared to the reference sequence. In other words, the residue number or residue position of a given polymer is designated with respect to the reference sequence rather than by the actual numerical position of the residue within the given amino acid or polynucleotide sequence. For example, a given amino acid sequence, such as that of an engineered P450 fatty acid decarboxylase, can be aligned to a reference sequence by introducing gaps to optimize residue matches between the two sequences. In these cases, although the gaps are present, the numbering of the residue in the given amino acid or polynucleotide sequence is made with respect to the reference sequence to which it has been aligned.

As used herein, "increased enzymatic activity" and "increased activity" refer to an improved property of a wild-type or an engineered enzyme, which can be represented by an increase in specific activity (e.g., product produced/time/weight protein) or an increase in percent conversion of the substrate to the product (e.g., percent conversion of starting amount of substrate to product in a specified time period using a specified amount of P450 fatty acid decarboxylase) as compared to a reference enzyme. Any property relating to enzyme activity may be affected, including the classical enzyme properties of Km, Vmax or kcat, changes of which can lead to increased enzymatic activity. The P450 fatty acid decarboxylase activity can be measured by any one of standard assays used for measuring P450 fatty acid decarboxylases, such as change in substrate or product concentration. Comparisons of enzyme activities are made using a defined preparation of enzyme, a defined assay under a set condition, and one or more defined substrates, as further described in detail herein. Generally, when enzymes in cell lysates are compared, the numbers of cells and the amount of protein assayed are determined as well as use of identical expression systems and identical host cells to minimize variations in amount of enzyme produced by the host cells and present in the lysates.

As used herein, "conversion" refers to the enzymatic transformation of a substrate to the corresponding product.

As used herein "percent conversion" refers to the percent of the substrate that is converted to the product within a period of time under specified conditions. Thus, for example, the "enzymatic activity" or "activity" of a P450 fatty acid decarboxylase polypeptide can be expressed as "percent conversion" of the substrate to the product.

As used herein, "amino acid difference" or "residue difference" refers to a difference in the amino acid residue at a position of a polypeptide sequence relative to the amino acid residue at a corresponding position in a reference sequence. The positions of amino acid differences generally are referred to herein as "Xn", where n refers to the corresponding position in the reference sequence upon which the residue difference is based. For example, a "residue difference at position X46 as compared to SEQ ID NO: 1" refers to a difference of the amino acid residue at the polypeptide position corresponding to position 46 of SEQ ID NO:1. Thus, if the reference polypeptide of SEQ ID NO:1 has a tyrosine at position 46, then a "residue difference at position X46 as compared to SEQ ID NO: 1" refers to an amino acid substitution of any residue other than tyrosine at the position of the polypeptide corresponding to position 46 of SEQ ID NO:1. In most instances herein, the specific amino acid residue difference at a position is indicated as "XnY" where "Xn" specified the corresponding position as described above, and "Y" is the single letter identifier of the amino acid found in the engineered polypeptide (i.e., the different residue than in the reference polypeptide). In some instances, the present invention also provides specific amino acid differences denoted by the conventional notation "AnB", where A is the single letter identifier of the residue in the reference sequence, "n" is the number of the residue position in the reference sequence, and B is the single letter identifier of the residue substitution in the sequence of the engineered polypeptide. In some instances, a polypeptide of the present invention can include at least one amino acid residue difference relative to a reference sequence, which is indicated by a list of the specified positions where residue differences are present relative to the reference sequence. In embodiments, where more than one amino acid can be used in a specific residue position of a polypeptide, the various amino acid residues that can be used are separated by a "/" (e.g., X46A/G). The present invention includes engineered polypeptide sequences comprising at least one amino acid difference that include either/or both conservative and non-conservative amino acid substitutions. The amino acid sequences of the specific recombinant P450 fatty acid decarboxylase polypeptides included in the Sequence Listing of the present invention include an initiating methionine (M) residue (i.e., M represents residue position 1). The skilled artisan, however, understands that this initiating methionine residue can be removed by biological processing machinery, such as in a host cell

or in vitro translation system, to generate a mature protein lacking the initiating methionine residue, but otherwise retaining the enzyme's properties. Consequently, the term "amino acid residue difference relative to SEQ ID NO:1 at position Xn" as used herein may refer to position "Xn" or to the corresponding position (e.g., position (X-1)n) in a reference sequence that has been processed so as to lack the starting methionine.

The term "amino acid substitution set" or "substitution set" refers to a group of amino acid substitutions in a polypeptide sequence, as compared to a reference sequence. A substitution set can have 2, 3, 4, 5, 6, 7, 8, 9, 10, 11, 12, 13, 14, 15, or more amino acid substitutions. In embodiments, a substitution set refers to the set of amino acid substitutions that is present in any of the variant P450 fatty acid decarboxylases.

As used herein, the phrase "conservative amino acid substitutions" refers to the interchangeability of residues having similar side chains, and thus typically involves substitution of the amino acid in the polypeptide with amino acids within the same or similar defined class of amino acids. As such, an amino acid with an aliphatic side chain can be substituted with another aliphatic amino acid (e.g., alanine, valine, leucine, and isoleucine); an amino acid with a hydroxyl side chain can be substituted with another amino acid with a hydroxyl side chain (e.g., serine and threonine); an amino acids having aromatic side chains can be substituted with another amino acid having an aromatic side chain (e.g., phenylalanine, tyrosine, tryptophan, and histidine); an amino acid with a basic side chain can be substituted with another amino acid with a basic side chain (e.g., lysine and arginine); an amino acid with an acidic side chain can be substituted with another amino acid with an acidic side chain (e.g., aspartic acid or glutamic acid); and/or a hydrophobic or hydrophilic amino acid can be replaced with another hydrophobic or hydrophilic amino acid, respectively. The appropriate classification of any amino acid or residue will be apparent to those of skill in the art, especially in light of the detailed invention provided herein.

As used herein, the phrase "non-conservative substitution" refers to substitution of an amino acid in the polypeptide with an amino acid with significantly differing side chain properties. Non-conservative substitutions may use amino acids between, rather than within, the defined groups and affects (a) the structure of the peptide backbone in the area of the substitution (e.g., proline for glycine) (b) the charge or hydrophobicity, or (c) the bulk of the side chain. By way of example and not limitation, an exemplary non-conservative substitution can be an acidic amino acid substituted with a basic or aliphatic amino acid; an aromatic amino acid substituted with a small amino acid; and a hydrophilic amino acid substituted with a hydrophobic amino acid.

As used herein, "deletion" refers to modification of the polypeptide by removal of one or more amino acids from the reference polypeptide. Deletions can comprise removal of 1 or more amino acids, 2 or more amino acids, 5 or more amino acids, 10 or more amino acids, 15 or more amino acids, or 20 or more amino acids, up to 10% of the total number of amino acids, or up to 20% of the total number of amino acids making up the polypeptide while retaining enzymatic activity and/or retaining the improved properties of an engineered enzyme. Deletions can be directed to the internal portions and/or terminal portions of the polypeptide. The deletion can comprise a continuous segment or can be discontinuous.

As used herein, "insertion" refers to modification of the polypeptide by addition of one or more amino acids to the reference polypeptide. In embodiments, the improved engineered P450 fatty acid decarboxylase enzymes comprise insertions of one or more amino acids to the naturally occurring P450 fatty acid decarboxylase polypeptide as well as insertions of one or more amino acids to engineered P450 fatty acid decarboxylase polypeptides. Insertions can be in the internal portions of the polypeptide, or to the carboxy or amino terminus. Insertions as used herein include fusion proteins as is known in the art. The insertion can be a contiguous segment of amino acids or separated by one or more of the amino acids in the naturally occurring polypeptide.

The term "amino acid substitution set" or "substitution set" refers to a group of amino acid substitutions in a polypeptide sequence, as compared to a reference sequence. A substitution set can have 2, 3, 4, 5, 6, 7, 8, 9, 10, 11, 12, 13, 14, 15, or more amino acid substitutions. A substitution set can refer to the set of amino acid substitutions that is present in any of the variant P450 fatty acid decarboxylases.

As used herein, "fragment" refers to a polypeptide that has an amino-terminal and/or carboxy-terminal deletion, but where the remaining amino acid sequence is identical to the corresponding positions in the sequence. Fragments can typically have about 80%, about 90%, about 95%, about 98%, or about 99% of the full-length P450 fatty acid decarboxylase polypeptide, for example, the polypeptide of SEQ ID NO: 1. In embodiments, the fragment is "biologically active" (i.e., it exhibits the same enzymatic activity as the full-length sequence).

A "functional fragment", or a "biologically active fragment", used interchangeably, herein refers to a polypeptide that has an amino-terminal and/or carboxy-terminal deletion(s) and/or internal deletions, but where the remaining amino acid sequence is identical to the corresponding positions in the sequence to which it is being compared and that retains substantially all of the activity of the full-length polypeptide.

As used herein, "isolated polypeptide" refers to a polypeptide which is substantially separated from other contaminants that naturally accompany it (e.g., protein, lipids, and polynucleotides). The term embraces polypeptides which have been removed or purified from their naturally-occurring environment or expression system (e.g., host cell or in vitro synthesis). The improved P450 fatty acid decarboxylase enzymes may be present within a cell, present in the

cellular medium, or prepared in various forms, such as lysates or isolated preparations. As such, in embodiments, the wild-type or engineered P450 fatty acid decarboxylase polypeptides of the present invention can be an isolated polypeptide.

As used herein, "substantially pure polypeptide" refers to a composition in which the polypeptide species is the predominant species present (i.e., on a molar or weight basis it is more abundant than any other individual macromolecular species in the composition), and is generally a substantially purified composition when the object species comprises at least about 50 percent of the macromolecular species present by mole or % weight. Generally, a substantially pure wild-type or engineered P450 fatty acid decarboxylase polypeptide composition will comprise about 60% or more, about 70% or more, about 80% or more, about 90% or more, about 91% or more, about 92% or more, about 93% or more, about 94% or more, about 95% or more, about 96% or more, about 97% or more, about 98% or more, or about 99% of all macromolecular species by mole or % weight present in the composition. Solvent species, small molecules (<500 Daltons), and elemental ion species are not considered macromolecular species. In embodiments, the isolated improved P450 fatty acid decarboxylase polypeptide is a substantially pure polypeptide composition.

As used herein, when used with reference to a nucleic acid or polypeptide, the term "heterologous" refers to a sequence that is not normally expressed and secreted by an organism (e.g., a wild-type organism). The term can encompass a sequence that comprises two or more subsequences which are not found in the same relationship to each other as normally found in nature, or is recombinantly engineered so that its level of expression, or physical relationship to other nucleic acids or other molecules in a cell, or structure, is not normally found in nature. For instance, a heterologous nucleic acid is typically recombinantly produced, having two or more sequences from unrelated genes arranged in a manner not found in nature (e.g., a nucleic acid open reading frame (ORF) of the invention operatively linked to a promoter sequence inserted into an expression cassette, such as a vector). "Heterologous polynucleotide" can refer to any polynucleotide that is introduced into a host cell by laboratory techniques, and includes polynucleotides that are removed from a host cell, subjected to laboratory manipulation, and then reintroduced into a host cell.

As used herein, "codon optimized" refers to changes in the codons of the polynucleotide encoding a protein to those preferentially used in a particular organism such that the encoded protein is efficiently expressed in the organism of interest. In embodiments, the polynucleotides encoding the P450 fatty acid decarboxylase enzymes may be codon optimized for optimal production from the host organism selected for expression.

As used herein, "suitable reaction conditions" refer to those conditions in the biocatalytic reaction solution (e.g., ranges of enzyme loading, substrate loading, temperature, pH, buffers, cosolvents, etc.) under which a P450 fatty acid decarboxylase polypeptide of the present invention is capable of converting a substrate compound to a product compound (e.g., conversion of one compound to another compound).

As used herein, "substrate" in the context of a biocatalyst mediated process refers to the compound or molecule acted on by the biocatalyst.

As used herein "product" in the context of a biocatalyst mediated process refers to the compound or molecule resulting from the action of the biocatalyst.

## Detergent Composition

The hand-dishwashing compositions of the present invention formulate a specific surfactant system with a specific P450 fatty acid decarboxylase, in order to provide improved sudsing, especially long-lasting sudsing, in the presence of greasy stains comprising higher chain length saturated and/or unsaturated fatty acids, and improved removal of such stains.

The hand-dishwashing composition is preferably in liquid form, more preferably is an aqueous cleaning composition. As such, the composition can comprise from 50% to 90%, preferably from 60% to 75%, by weight of the total composition of water.

Preferably the pH of the detergent composition of the invention, measured as a 10% product concentration in demineralized water at 20°C, is adjusted to between 3 and 14, more preferably between 4 and 13, more preferably between 6 and 12 and most preferably between 8 and 10. The pH of the detergent composition can be adjusted using pH modifying ingredients known in the art.

The composition of the present invention can be Newtonian or non-Newtonian, preferably Newtonian. Preferably, the composition has a viscosity of from 10 mPa·s to 10,000 mPa·s, preferably from 100 mPa·s to 5,000 mPa·s, more preferably from 300 mPa·s to 2,000 mPa·s, or most preferably from 500 mPa·s to 1,500 mPa·s, alternatively combinations thereof. The viscosity is measured at 20°C with a Brookfield RT Viscometer using spindle 31 with the RPM of the viscometer adjusted to achieve a torque of between 40% and 60%.

## Surfactant System

The cleaning composition comprises from 5% to 50%, preferably 8% to 45%, more preferably from 15% to

40%, by weight of the total composition of a surfactant system.

For improved sudsing, the surfactant system comprises anionic surfactant. The surfactant system preferably comprises from 60% to 90%, more preferably from 70% to 80% by weight of the surfactant system of the anionic surfactant. Alkyl sulphated anionic surfactants are preferred, particularly those selected from the group consisting of: alkyl sulphate, alkyl alkoxy sulphate, and mixtures thereof. More preferably, the anionic surfactant consists of alkyl sulphated anionic surfactant selected from the group consisting of: alkyl sulphate, alkyl alkoxy sulphate, and mixtures thereof.

For further improvements in sudsing, the surfactant system can comprise less than 30%, preferably less than 15%, more preferably less than 10% of further anionic surfactant, and most preferably the surfactant system comprises no further anionic surfactant. The alkyl sulphated anionic surfactant preferably has an average alkyl chain length of from 8 to 18, preferably from 10 to 14, more preferably from 12 to 14, most preferably from 12 to 13 carbon atoms. The alkyl sulphated anionic surfactant has an average degree of alkoxylation, of less than 5, preferably less than 3, more preferably from 0.5 to 2.0, most preferably from 0.5 to 0.9. Preferably, the alkyl sulphated anionic surfactant is ethoxylated. That is, the alkyl sulphated anionic surfactant has an average degree of ethoxylation, of less than 5, preferably less than 3, more preferably from 0.5 to 2.0, most preferably from 0.5 to 0.9.

The average degree of alkoxylation is the mol average degree of alkoxylation (*i.e.,* mol average alkoxylation degree) of all the alkyl sulphate anionic surfactant. Hence, when calculating the mol average alkoxylation degree, the mols of non-alkoxylated sulphate anionic surfactant are included:

$$\text{Mol average alkoxylation degree} = (x1 * \text{alkoxylation degree of surfactant 1} + x2 * \text{alkoxylation degree of surfactant 2} + \ldots) / (x1 + x2 + \ldots)$$

wherein x1, x2, ... are the number of moles of each alkyl (or alkoxy) sulphate anionic surfactant of the mixture and alkoxylation degree is the number of alkoxy groups in each alkyl sulphate anionic surfactant.

The alkyl sulphate anionic surfactant can have a weight average degree of branching of more than 10%, preferably more than 20%, more preferably more than 30%, even more preferably between 30% and 60%, most preferably between 30% and 50%. The alkyl sulphate anionic surfactant can comprise at least 5%, preferably at least 10%, most preferably at least 25%, by weight of the alkyl sulphate anionic surfactant, of branching on the C2 position (as measured counting carbon atoms from the sulphate group for non-alkoxylated alkyl sulphate anionic surfactants, and the counting from the alkoxy-group furthest from the sulphate group for alkoxylated alkyl sulphate anionic surfactants). More preferably, greater than 75%, even more preferably greater than 90%, by weight of the total branched alkyl content consists of C1-C5 alkyl moiety, preferably C1-C2 alkyl moiety. It has been found that formulating the inventive compositions using alkyl sulphate surfactants having the aforementioned degree of branching results in improved low temperature stability. Such compositions require less solvent in order to achieve good physical stability at low temperatures. As such, the compositions can comprise lower levels of organic solvent, of less than 5.0% by weight of the cleaning composition of organic solvent, while still having improved low temperature stability. Higher surfactant branching also provides faster initial suds generation, but typically less suds mileage. The weight average branching, described herein, has been found to provide improved low temperature stability, initial foam generation and suds longevity.

The weight average degree of branching for an anionic surfactant mixture can be calculated using the following formula:

$$\text{Weight average degree of branching (\%)} = [(x1 * \text{wt\% branched alcohol 1 in alcohol 1} + x2 * \text{wt\% branched alcohol 2 in alcohol 2} + \ldots) / (x1 + x2 + \ldots)] * 100$$

wherein x1, x2, ... are the weight in grams of each alcohol in the total alcohol mixture of the alcohols which were used as starting material before (alkoxylation and) sulphation to produce the alkyl (alkoxy) sulphate anionic surfactant. In the weight average degree of branching calculation, the weight of the alkyl alcohol used to form the alkyl sulphate anionic surfactant which is not branched is included.

The weight average degree of branching and the distribution of branching can typically be obtained from the technical data sheet for the surfactant or constituent alkyl alcohol. Alternatively, the branching can also be determined through analytical methods known in the art, including capillary gas chromatography with flame ionisation detection on medium polar capillary column, using hexane as the solvent. The weight average degree of branching and the distribution of branching is based on the starting alcohol used to produce the alkyl sulphate anionic surfactant.

The alkyl chain of the alkyl sulphated anionic surfactant preferably has a mol fraction of C12 and C13 chains

of at least 50%, preferably at least 65%, more preferably at least 80%, most preferably at least 90%. Suds mileage is particularly improved, especially in the presence of greasy soils, when the C13/C12 mol ratio of the alkyl chain is at least 50/50, preferably at least 57/43, preferably from 60/40 to 90/10, more preferably from 60/40 to 80/20, most preferably from 60/40 to 70/30, while not compromising suds mileage in the presence of particulate soils.

Suitable counterions include alkali metal cation earth alkali metal cation, alkanolammonium or ammonium or substituted ammonium, but preferably sodium.

Suitable examples of commercially available alkyl sulphate anionic surfactants include, those derived from alcohols sold under the Neodol® brand-name by Shell, or the Lial®, Isalchem®, and Safol® brand-names by Sasol, or some of the natural alcohols produced by The Procter & Gamble Chemicals company. The alcohols can be blended in order to achieve the desired mol fraction of C12 and C13 chains and the desired C13/C12 ratio, based on the relative fractions of C13 and C12 within the starting alcohols, as obtained from the technical data sheets from the suppliers or from analysis using methods known in the art.

In order to improve surfactant packing after dilution and hence improve suds mileage, the surfactant system preferably comprises a co-surfactant. Preferred co-surfactants are selected from the group consisting of an amphoteric surfactant, a zwitterionic surfactant, and mixtures thereof. The co-surfactant is preferably an amphoteric surfactant, more preferably an amine oxide surfactant. The co-surfactant is included as part of the surfactant system.

The composition preferably comprises from 0.1% to 20%, more preferably from 0.5% to 15% and especially from 2% to 10% by weight of the cleaning composition of the co-surfactant. The surfactant system of the cleaning composition of the present invention preferably comprises from 10% to 40%, preferably from 15% to 35%, more preferably from 20% to 30%, by weight of the surfactant system of a co-surfactant. The anionic surfactant to the co-surfactant weight ratio can be from 1:1 to 8:1, preferably from 2:1 to 5:1, more preferably from 2.5:1 to 4:1.

As mentioned earlier, amine oxide surfactants are preferred for use as a co-surfactant. The amine oxide surfactant can be linear or branched, though linear are preferred. Suitable linear amine oxides are typically water-soluble, and characterized by the formula R1 - N(R2)(R3) O wherein R1 is a C8-18 alkyl, and the R2 and R3 moieties are selected from the group consisting of C1-3 alkyl groups, C1-3 hydroxyalkyl groups, and mixtures thereof. For instance, R2 and R3 can be selected from the group consisting of: methyl, ethyl, propyl, isopropyl, 2-hydroxethyl, 2-hydroxypropyl and 3-hydroxypropyl, and mixtures thereof, though methyl is preferred for one or both of R2 and R3. The linear amine oxide surfactants in particular may include linear C10-C18 alkyl dimethyl amine oxides and linear C8-C12 alkoxy ethyl dihydroxy ethyl amine oxides.

Preferably, the amine oxide surfactant is selected from the group consisting of: alkyl dimethyl amine oxide, alkyl amido propyl dimethyl amine oxide, and mixtures thereof. Alkyl dimethyl amine oxides are preferred, such as C8-18 alkyl dimethyl amine oxides, or C10-16 alkyl dimethyl amine oxides (such as coco dimethyl amine oxide). Suitable alkyl dimethyl amine oxides include C10 alkyl dimethyl amine oxide surfactant, C10-12 alkyl dimethyl amine oxide surfactant, C12-C14 alkyl dimethyl amine oxide surfactant, and mixtures thereof. C12-C14 alkyl dimethyl amine oxide are particularly preferred.

Alternative suitable amine oxide surfactants include mid-branched amine oxide surfactants. As used herein, "mid-branched" means that the amine oxide has one alkyl moiety having n1 carbon atoms with one alkyl branch on the alkyl moiety having n2 carbon atoms. The alkyl branch is located on the $\alpha$ carbon from the nitrogen on the alkyl moiety. This type of branching for the amine oxide is also known in the art as an internal amine oxide. The total sum of n1 and n2 can be from 10 to 24 carbon atoms, preferably from 12 to 20, and more preferably from 10 to 16. The number of carbon atoms for the one alkyl moiety (n1) is preferably the same or similar to the number of carbon atoms as the one alkyl branch (n2) such that the one alkyl moiety and the one alkyl branch are symmetric. As used herein "symmetric" means that | n1 - n2 | is less than or equal to 5, preferably 4, most preferably from 0 to 4 carbon atoms in at least 50 wt%, more preferably at least 75 wt% to 100 wt% of the mid-branched amine oxides for use herein. The amine oxide further comprises two moieties, independently selected from a C1-3 alkyl, a C1-3 hydroxyalkyl group, or a polyethylene oxide group containing an average of from about 1 to about 3 ethylene oxide groups. Preferably, the two moieties are selected from a CI-3 alkyl, more preferably both are selected as C1 alkyl.

Alternatively, the amine oxide surfactant can be a mixture of amine oxides comprising a mixture of low-cut amine oxide and mid-cut amine oxide. The amine oxide of the composition of the invention can then comprises:

a) from about 10% to about 45% by weight of the amine oxide of low-cut amine oxide of formula R1R2R3AO wherein R1 and R2 are independently selected from hydrogen, C1-C4 alkyls or mixtures thereof, and R3 is selected from C10 alkyls and mixtures thereof; and

b) from 55% to 90% by weight of the amine oxide of mid-cut amine oxide of formula R4R5R6AO wherein R4 and R5 are independently selected from hydrogen, C1-C4 alkyls or mixtures thereof, and R6 is selected from C12-C16 alkyls or mixtures thereof

In a preferred low-cut amine oxide for use herein R3 is n-decyl, with preferably both R1 and R2 being methyl.

In the mid-cut amine oxide of formula R4R5R6AO, R4 and R5 are preferably both methyl.

Preferably, the amine oxide comprises less than about 5%, more preferably less than 3%, by weight of the amine oxide of an amine oxide of formula R7R8R9AO wherein R7 and R8 are selected from hydrogen, C1-C4 alkyls and mixtures thereof and wherein R9 is selected from C8 alkyls and mixtures thereof. Limiting the amount of amine oxides of formula R7R8R9AO improves both physical stability and suds mileage.

Suitable zwitterionic surfactants include betaine surfactants. Such betaine surfactants includes alkyl betaines, alkylamidobetaine, amidazoliniumbetaine, sulphobetaine (INCI Sultaines) as well as the Phosphobetaine, and preferably meets formula (II):

$$R^1\text{-}[CO\text{-}X(CH_2)_n]_x\text{-}N^+(R^2)(R_3)\text{-}(CH_2)_m\text{-}[CH(OH)\text{-}CH_2]_y\text{-}Y^-$$

wherein in formula (II),

R1 is selected from the group consisting of: a saturated or unsaturated C6-22 alkyl residue, preferably C8-18 alkyl residue, more preferably a saturated C10-16 alkyl residue, most preferably a saturated C12-14 alkyl residue;
X is selected from the group consisting of: NH, NR4 wherein R4 is a C1-4 alkyl residue, O, and S,
n is an integer from 1 to 10, preferably 2 to 5, more preferably 3,
x is 0 or 1, preferably 1,
R2 and R3 are independently selected from the group consisting of: a C1-4 alkyl residue, hydroxy substituted such as a hydroxyethyl, and mixtures thereof, preferably both R2 and R3 are methyl,
m is an integer from 1 to 4, preferably 1, 2 or 3,
y is 0 or 1, and
Y is selected from the group consisting of: COO, SO3, OPO(OR5)O or P(O)(OR5)O, wherein R5 is H or a C1-4 alkyl residue.

Preferred betaines are the alkyl betaines of formula (Ia), the alkyl amido propyl betaine of formula (Ib), the sulphobetaines of formula (Ic) and the amido sulphobetaine of formula (Id):

$$R^1\text{-}N(CH_3)_2\text{-}CH_2COO^- \quad \text{(IIa)}$$

$$R^1\text{-}CO\text{-}NH\text{-}(CH_2)_3\text{-}N^+(CH_3)_2\text{-}CH_2COO^- \quad \text{(IIb)}$$

$$R^1\text{-}N^+(CH_3)_2\text{-}CH_2CH(OH)CH_2SO_3^- \quad \text{(IIc)}$$

$$R^1\text{-}CO\text{-}NH\text{-}(CH_2)_3\text{-}N^+(CH_3)_2\text{-}CH_2CH(OH)CH_2SO_3^- \quad \text{(IId)}$$

in which R1 has the same meaning as in formula (II). Particularly preferred are the carbobetaines [i.e. wherein Y-=COO- in formula (II)] of formulae (Ia) and (Ib), more preferred are the alkylamidobetaine of formula (Ib).

Suitable betaines can be selected from the group consisting or [designated in accordance with INCI]: capryl/capramidopropyl betaine, cetyl betaine, cetyl amidopropyl betaine, cocamidoethyl betaine, cocamidopropyl betaine, cocobetaines, decyl betaine, decyl amidopropyl betaine, hydrogenated tallow betaine / amidopropyl betaine, isostearamidopropyl betaine, lauramidopropyl betaine, lauryl betaine, myristyl amidopropyl betaine, myristyl betaine, oleamidopropyl betaine, oleyl betaine, palmamidopropyl betaine, palmitamidopropyl betaine, palm-kernelamidopropyl betaine, stearamidopropyl betaine, stearyl betaine, tallowamidopropyl betaine, tallow betaine, undecylenamidopropyl betaine, undecyl betaine, and mixtures thereof. Preferred betaines are selected from the group consisting of: cocamidopropyl betaine, cocobetaines, lauramidopropyl betaine, lauryl betaine, myristyl amidopropyl betaine, myristyl betaine, and mixtures thereof. Cocamidopropyl betaine is particularly preferred.

Preferably, the surfactant system of the composition of the present invention further comprises from 1% to 25%, preferably from 1.25% to 20%, more preferably from 1.5% to 15%, most preferably from 1.5% to 5%, by weight of the surfactant system, of a non-ionic surfactant.

Suitable nonionic surfactants can be selected from the group consisting of: alkoxylated non-ionic surfactant, alkyl polyglucoside ("APG") surfactant, and mixtures thereof.

Suitable alkoxylated non-ionic surfactants can be linear or branched, primary or secondary alkyl alkoxylated non-ionic surfactants. Alkyl ethoxylated non-ionic surfactant are preferred. The ethoxylated non-ionic surfactant can comprise on average from 9 to 15, preferably from 10 to 14 carbon atoms in its alkyl chain and on average from 5 to 12, preferably from 6 to 10, most preferably from 7 to 8, units of ethylene oxide per mole of alcohol. Such alkyl ethoxylated nonionic surfactants can be derived from synthetic alcohols, such as OXO-alcohols and Fisher Tropsh alcohols, or from naturally derived alcohols, or from mixtures thereof. Suitable examples of commercially available alkyl ethoxylate nonionic

surfactants include, those derived from synthetic alcohols sold under the Neodol® brand-name by Shell, or the Lial®, Isalchem®, and Safol® brand-names by Sasol, or some of the natural alcohols produced by The Procter & Gamble Chemicals company.

The compositions of the present invention can comprise alkyl polyglucoside ("APG") surfactant. The addition of alkyl polyglucoside surfactants have been found to improve sudsing beyond that of comparative nonionic surfactants such as alkyl ethoxylated surfactants. Preferably the alkyl polyglucoside surfactant is a C8-C16 alkyl polyglucoside surfactant, preferably a C8-C14 alkyl polyglucoside surfactant. The alkyl polyglucoside preferably has an average degree of polymerization of between 0.1 and 3, more preferably between 0.5 and 2.5, even more preferably between 1 and 2. Most preferably, the alkyl polyglucoside surfactant has an average alkyl carbon chain length between 10 and 16, preferably between 10 and 14, most preferably between 12 and 14, with an average degree of polymerization of between 0.5 and 2.5 preferably between 1 and 2, most preferably between 1.2 and 1.6. C8-C16 alkyl polyglucosides are commercially available from several suppliers (e.g., Simusol® surfactants from Seppic Corporation; and Glucopon® 600 CSUP, Glucopon® 650 EC, Glucopon® 600 CSUP/MB, and Glucopon® 650 EC/MB, from BASF Corporation).

P450 FATTY ACID DECARBOXYLASES

P450 fatty acid decarboxylases are enzymes that belong to the cytochrome P450 family CYP152 and catalyze the decarboxylation of fatty acids to alkenes utilizing hydrogen peroxide as co-substrate and heme as a cofactor. The most well studied member of this family is OleTJE (SEQ ID NO: 1), an enzyme endogenous to Jeotgalicoccus sp. 8456 (J. Belcher et al., J. Biol. Chem. (2014), 289, 10: 6535-6550) and is classified as CYP152L1. Variants of OleTJE with fused domains are capable of using molecular oxygen as co-substrate in the presence of an additional co-substrate (Y. Liu et al., Biotechnol. Biofuels (2014) 7: 28). Examples of cytochrome P450 family CYP152 include SEQ ID 1 to 158, some of which are according to the invention and some of which are comparative.

The hand dish-washing compositions of the present invention comprise P450 fatty acid decarboxylases having increased enzymatic activity for long-chain fatty acid substrates, such as oleic acid or linoleic acid, as compared to the well-known naturally occurring wild-type fatty acid decarboxylases reported previously in the art (e.g. OleTJE, SEQ ID NO: 1). Such P450 fatty acid decarboxylases, which may comprise specific amino acid residues at certain positions (e.g. 40, 46, 74, 252, or 317), have been identified as having an increased enzymatic activity towards long chain fatty acids, such as oleic acid or linoleic acid in comparison to the well-known OleTJE (SEQ ID NO: 1) and as such, these decarboxylases provide improved sudsing in the presence of greasy soils comprising such long-chain fatty acids.

The hand dish-washing composition comprises a P450 fatty acid decarboxylase. The decarboxylase comprises a polypeptide sequence having at least 80% identity to one or more sequences selected from the group consisting of: SEQ ID NO: 2, 44, 60, 65, 71, 83, 117, 121, 122, 156, and their functional fragments thereof. The P450 fatty acid decarboxylase can comprise a polypeptide sequence having at least about 85%, 90%, 95%, 98%, 100% identity to one or more sequences selected from the group consisting of: SEQ ID NO: 2, 22, 44, 60, 65, 71, 83, 117, 121, 122, 156, and their functional fragments thereof, preferably SEQ ID NO: 2, 60, 65, 71, 83, 122, and their functional fragments, more preferably SEQ ID NO: 2, 60, 65, 71, 83, 122, and their functional fragments, more most preferably SEQ ID NO: 2, and 122, and most preferably SEQ ID NO: 2.

Said decarboxylase can comprise an amino acid selected from the group consisting of: a) valine, isoleucine, or leucine at position 40, b) alanine, glycine, or valine at position 46, c) valine at position 74, d) lysine at position 252, e) isoleucine, leucine, methionine, or valine at position 317, and combinations thereof; wherein said positions are numbered with reference to SEQ ID NO: 1.

The hand dish-washing composition can comprise a P450 fatty acid decarboxylase comprising an isoleucine, leucine, or valine, at position 40; wherein said positions are numbered with reference to SEQ ID NO: 1. A suitable example of P450 fatty acid decarboxylases comprising an isoleucine at position 40, wherein said position is numbered with reference to SEQ ID NO: 1, is SEQ ID NO: 22. Suitable examples of P450 fatty acid decarboxylases comprising a leucine at position 40, wherein said position is numbered with reference to SEQ ID NO: 1, are SEQ ID NO: 65, and 121. Suitable examples of P450 fatty acid decarboxylases comprising a valine at position 40, wherein said position is numbered with reference to SEQ ID NO: 1, are SEQ ID NO: 2, and 122.

The hand dish-washing composition can comprise a P450 fatty acid decarboxylase comprising an alanine, glycine, valine, or isoleucine at position 46; wherein said positions are numbered with reference to SEQ ID NO: 1. Suitable examples of P450 fatty acid decarboxylases comprising an alanine at position 46, wherein said position is numbered with reference to SEQ ID NO: 1, are SEQ ID NO: 2, and 22. Suitable examples of P450 fatty acid decarboxylases comprising an valine at position 46, wherein said position is numbered with reference to SEQ ID NO: 1, are SEQ ID NO: 44, 60, and 65.

The hand dish-washing composition can comprise a P450 fatty acid decarboxylase comprising a valine at position 74; wherein said positions are numbered with reference to SEQ ID NO: 1. Suitable examples of P450 fatty acid decarboxylases comprising a valine at position 74, wherein said position is numbered with reference to SEQ ID NO: 1,

are SEQ ID NO: 2, 71, and 83.

The hand dish-washing composition can comprise a P450 fatty acid decarboxylase comprising a lysine at position 252; wherein said positions are numbered with reference to SEQ ID NO: 1. A suitable example of a P450 fatty acid decarboxylase comprising a lysine at position 252, wherein said position is numbered with reference to SEQ ID NO: 1, is SEQ ID NO: 2.

The hand dish-washing composition can comprise a P450 fatty acid decarboxylase comprising an isoleucine, leucine, methionine, or valine at position 317; wherein said positions are numbered with reference to SEQ ID NO: 1. Suitable examples of P450 fatty acid decarboxylases comprising an isoleucine at position 317, wherein said position is numbered with reference to SEQ ID NO: 1, are SEQ ID NO:22, and 60. Suitable examples of P450 fatty acid decarboxylases comprising a leucine at position 317, wherein said position is numbered with reference to SEQ ID NO: 1, are SEQ ID NO: 2, 65, and 122. A suitable example of P450 fatty acid decarboxylases comprising a methionine at position 317, wherein said position is numbered with reference to SEQ ID NO: 1; is SEQ ID NO: 121. Suitable examples of P450 fatty acid decarboxylases comprising a valine at position 317, wherein said position is numbered with reference to SEQ ID NO: 1; are SEQ ID NO: 44, 71, 83, and 117.

The decarboxylases can have an increased enzymatic activity for oleic acid of at least about 2-fold, 3-fold, 4-fold, 5-fold, 10-fold, 20-fold, 30-fold, 40-fold, 50-fold, 150-fold, 500-fold or more relative to the activity of wild-type decarboxylase (SEQ ID NO: 1) under suitable reaction conditions.

The hand dish-washing composition can comprise a P450 fatty acid decarboxylase having at least about 80%, at least about 90%, at least about 95%, at least about 98%, at least about 100% identity to one or more sequences selected from the group consisting of SEQ ID NO: 2, 22, 44, 60, 65, 71, 83, 117, 121, 122, and 156, and their functional fragments thereof, preferably from the group consisting of SEQ ID NO: 2, 22, 44, 60, 65, 71, 117, 121, and 122. The hand dish-washing composition can comprise a P450 fatty acid decarboxylase having at least about 80%, at least about 90%, at least about 95%, at least about 98%, at least about 100% identity to one or more sequences selected from the group consisting of SEQ ID NO: 2, 22, 151, 156, and their functional fragments. The hand dish-washing composition can comprise a P450 fatty acid decarboxylase having at least about 80%, at least about 90%, at least about 95%, at least about 98%, at least about 100% identity to one or more sequences selected from the group consisting of SEQ ID NO: 2. Suitable examples of P450 fatty acid decarboxylases with at least about 80% identity to SEQ ID NO: 2 are SEQ ID NO: 3, 5, 4, 7, 6, 8, 9, 10, 11, 12, 13, 14, 15, and 151. Suitable examples of P450 fatty acid decarboxylases with at least about 80% identity to SEQ ID NO: 22 are SEQ ID NO: 23, 24, 129. Suitable examples of P450 fatty acid decarboxylases with at least about 80% identity to SEQ ID NO: 44 are SEQ ID NO: 39, 40, 41, 43, 44, 45, 46, 47, 48, 49, 50, 85, 126, 127, 128, 130, 131, 132, 133, 134, 135, 136, 137, 138, 139, 140, 141, 142, 143, 144, 145, and 146. Suitable examples of P450 fatty acid decarboxylases with at least about 80% identity to SEQ ID NO: 60 are SEQ ID NO: 54, 55, 56, 57, 58, 59, 61, 62, and 63. A suitable example of P450 fatty acid decarboxylases with at least about 80% identity to SEQ ID NO: 65 is SEQ ID NO: 64. Suitable examples of P450 fatty acid decarboxylases with at least about 80% identity to SEQ ID NO: 71 are SEQ ID NO: 67, 68, 69, 70, 72, 73, 74, 75, 76, 77, 78, 79, 80, 81, 123, 124, and 125. Suitable examples of P450 fatty acid decarboxylases with at least about 80% identity to SEQ ID NO: 117 are SEQ ID NO: 114, 115, 116, 118, and 119.

Identity, or homology, percentages as mentioned herein in respect of the present invention are those that can be calculated, for example, with AlignX obtainable from Thermo Fischer Scientific or with the alignment tool from Uniprot (https://www.uniprot.org/align/). Alternatively, a manual alignment can be performed. For enzyme sequence comparison the following settings can be used: Alignment algorithm: Needleman and Wunsch, J. Mol. Biol. 1970, 48: 443-453. As a comparison matrix for amino acid similarity the Blosum62 matrix is used (Henikoff S. and Henikoff J.G., P.N.A.S. USA 1992, 89: 10915-10919). The following gap scoring parameters are used: Gap penalty: 12, gap length penalty: 2, no penalty for end gaps.

A given sequence is typically compared against the full-length sequence or fragments of SEQ ID NO: 1, 2, 22, 44, 60, 65, 71, 83, 117, 121, 122, or 156 to obtain a score. Polypeptides of the present disclosure can include polypeptides containing an amino acid sequence having at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 98%, at least about 99%, or 100% identity to the amino acid sequence of any one of SEQ ID NO: 2, 22, 44, 60, 65, 71, 83, 117, 121, 122, and 156, preferably to the amino acid sequence of any one of SEQ ID NO: 2, 22, 44, 60, 65, 71, 83, 117, 121, 122, and 156. Polypeptides of the disclosure can also include polypeptides having at least about 10, at least about 12, at least about 14, at least about 16, at least about 18, at least about 20, at least about 30, at least about 40, at least about 50, at least about 60, at least about 70, or at least about 80 consecutive amino acids of the amino acid sequence of any one of SEQ ID NO: 2, 22, 44, 60, 65, 71, 83, 117, 121, 122, and 156, preferably of the amino acid sequence of any one of SEQ ID NO: 2, 22, 44, 60, 65, 71, 83, 117, 121, and 122.

The hand dish-washing composition can comprise variants of P450 fatty acid decarboxylases, as previously

described. Variants of P450 fatty acid decarboxylases include polypeptide sequences resulting from modification of a wild-type P450 fatty acid decarboxylase at one or more amino acids. A variant includes a "modified enzyme" or a "mutant enzyme" which encompasses proteins having at least one substitution, insertion, and/or deletion of an amino acid. A modified enzyme may have 1, 2, 3, 4, 5, 6, 7, 8, 9 or 10 or more amino acid modifications (selected from substitutions, insertions, deletions and combinations thereof).

The variants may have "conservative" substitutions. Suitable conservative substitutions include one conservative substitution in the enzyme, such as a conservative substitution in SEQ ID NO: 2, 22, 44, 60, 65, 71, 83, 117, 121, 122, 156, and their functional fragments thereof, preferably a conservative substitution in SEQ ID NO: 2, 22, 44, 60, 65, 71, 83, 117, 121, 122, and their functional fragments thereof. Other suitable substitutions include 10 or fewer conservative substitutions in the protein, such as five or fewer. An enzyme of use in the invention may therefore include 1, 2, 3, 4, 5, 6, 7, 8, 9, 10 or more conservative substitutions. An enzyme can be produced to contain one or more conservative substitutions by manipulating the nucleotide sequence that encodes that enzyme using, for example, standard procedures such as site-directed mutagenesis or PCR. Examples of amino acids which may be substituted for an original amino acid in an enzyme and which are regarded as conservative substitutions include: Ser for Ala; Lys for Arg; Gln or His for Asn; Glu for Asp; Asn for Gln; Asp for Glu; Pro for Gly; Asn or Gln for His; Leu or Val for Ile; Ile or Val for Leu; Arg or Gln for Lys; Leu or Ile for Met; Met, Leu or Tyr for Phe; Thr for Ser; Ser for Thr; Tyr for Trp; Trp or Phe for Tyr; and Ile or Leu for Val.

The hand dish-washing composition can comprise variants of the P450 fatty acid decarboxylase which comprise a polypeptide sequence comprising at least one amino acid substitution at positions selected from the group consisting of: 40, 46, 74, 79, and combinations thereof; wherein said positions are numbered with reference to SEQ ID NO: 1.

The variant of the P450 fatty acid decarboxylase can comprise a polypeptide sequence comprising at least one amino acid substitution selected from the group consisting of: F79A, R245P, P246R, K252Q, F253A, F256A, R286Q, and combinations thereof; wherein said positions are numbered with reference to SEQ ID NO: 1.

The variant of the P450 fatty acid decarboxylase can comprise a polypeptide sequence comprising at least one amino acid substitution at positions selected from the group consisting of: 77, 78, 79, 85, 166, 169, 170, 190, 193, 238, 240, 241, 242, 243, 244, 245, 246, 247, 248, 249, and combinations thereof; wherein said positions are numbered with reference to SEQ ID NO: 1; and wherein said amino acid substitution is for an amino acid selected from the group consisting of aspartate and glutamate.

The variant of the P450 fatty acid decarboxylase can comprise a polypeptide sequence comprising at least one amino acid substitution selected from the group consisting of: F80A, R246P, P247R, K253Q, F254A, F257A, R288Q, and combinations thereof; wherein said positions are numbered with reference to SEQ ID NO: 2.

It is important that variants of enzymes retain and preferably improve the ability of the wild-type protein to catalyze the conversion of the fatty acids. Some performance drop in a given property of variants may of course be tolerated, but the variants should retain and preferably improve suitable properties for the relevant application for which they are intended. Screening of variants of one of the wild-types can be used to identify whether they retain and preferably improve appropriate properties.

The decarboxylase polypeptides described herein are not restricted to the genetically encoded amino acids. Thus, in addition to the genetically encoded amino acids, the polypeptides described herein may be comprised, either in whole or in part, of naturally-occurring and/or synthetic non-encoded amino acids. Certain commonly encountered non-encoded amino acids of which the polypeptides described herein may be comprised include, but are not limited to: the D-stereoisomers of the genetically-encoded amino acids; 2,3-diaminopropionic acid (Dpr); $\alpha$-aminoisobutyric acid (Aib); $\varepsilon$-aminohexanoic acid (Aha); $\delta$-aminovaleric acid (Ava); N-methylglycine or sarcosine (MeGly or Sar); ornithine (Orn); citrulline (Cit); t-butylalanine (Bua); t-butylglycine (Bug); N-methylisoleucine (MeIle); phenylglycine (Phg); cyclohexylalanine (Cha); norleucine (Nle); naphthylalanine (Nal); 2-chlorophenylalanine (Oct); 3-chlorophenylalanine (Mcf); 4-chlorophenylalanine (Pcf); 2-fluorophenylalanine (Off); 3-fluorophenylalanine (Mff); 4-fluorophenylalanine (Pff); 2-bromophenylalanine (Obf); 3-bromophenylalanine (Mbf); 4-bromophenylalanine (Pbf); 2-methylphenylalanine (Omf); 3-methylphenylalanine (Mmf); 4-methylphenylalanine (Pmf); 2-nitrophenylalanine (Onf); 3-nitrophenylalanine (Mnf); 4-nitrophenylalanine (Pnf); 2-cyanophenylalanine (Ocf); 3-cyanophenylalanine (Mcf); 4-cyanophenylalanine (Pcf); 2-trifluoromethylphenylalanine (Otf); 3-trifluoromethylphenylalanine (Mtf); 4-trifluoromethylphenylalanine (Ptf); 4-aminophenylalanine (Paf); 4-iodophenylalanine (Pif); 4-aminomethylphenylalanine (Pamf); 2,4-dichlorophenylalanine (Opcf); 3,4-dichlorophenylalanine (Mpcf); 2,4-difluorophenylalanine (Opff); 3,4-difluorophenylalanine (Mpff); pyrid-2-ylalanine (2pAla); pyrid-3-ylalanine (3pAla); pyrid-4-ylalanine (4pAla); naphth-1-ylalanine (lnAla); naphth-2-ylalanine (2nAla); thiazolylalanine (taAla); benzothienylalanine (bAla); thienylalanine (tAla); furylalanine (fAla); homophenylalanine (hPhe); homotyrosine (hTyr); homotryptophan (hTrp); pentafluorophenylalanine (5ff); styrylkalanine (sAla); authrylalanine (aAla); 3,3-diphenylalanine (Dfa); 3-amino-5-phenylpentanoic acid (Afp); penicillamine (Pen); 1,2,3,4-tetrahydroisoquinoline-3-carboxylic acid (Tic); $\beta$-2-thienylalanine (Thi); methionine sulfoxide (Mso); N(w)-nitroarginine (nArg); homolysine (hLys); phosphonomethylphenylalanine (pmPhe); phosphoserine (pSer); phosphothreonine (pThr); homoaspartic acid (hAsp); homoglutamic acid (hGlu); 1-aminocyclopent-(2 or 3)-ene-4 carboxylic acid; pipecolic acid (PA), azetidine-3-carboxylic

acid (ACA); 1-aminocyclopentane-3-carboxylic acid; allylglycine (aOly); propargylglycine (pgGly); homoalanine (hAla); norvaline (nVal); homoleucine (hLeu), homovaline (hVal); homoisoleucine (hIle); homoarginine (hArg); N-acetyl lysine (AcLys); 2,4-diaminobutyric acid (Dbu); 2,3-diaminobutyric acid (Dab); N-methylvaline (MeVal); homocysteine (hCys); homoserine (hSer); hydroxyproline (Hyp) and homoproline (hPro). Additional non-encoded amino acids of which the polypeptides described herein may be comprised will be apparent to those of skill in the art. These amino acids may be in either the L- or D-configuration.

Suitable variants in the form of truncated forms or fragments can be derived from a wild-type enzyme, such as a protein with a truncated N-terminus or a truncated C-terminus. Suitable variants of decarboxylase enzymes can comprise a fragment of any of the decarboxylase polypeptides described herein that retain the functional decarboxylase activity and/or an improved property of an engineered decarboxylase polypeptide. Accordingly, the hand dish-washing composition can comprise a polypeptide fragment having decarboxylase activity (e.g., capable of converting substrate to product under suitable reaction conditions), wherein the fragment comprises at least about 80%, 90%, 95%, 98%, or 99% of a full-length amino acid sequence of an engineered polypeptide of use in the present invention.

Suitable decarboxylase enzymes can have an amino acid sequence comprising an insertion as compared to any one of the decarboxylase polypeptide sequences described herein. Thus, the insertions can comprise one or more amino acids, 2 or more amino acids, 3 or more amino acids, 4 or more amino acids, 5 or more amino acids, 6 or more amino acids, 8 or more amino acids, 10 or more amino acids, 15 or more amino acids, or 20 or more amino acids, where the associated functional activity and/or improved properties of the decarboxylase described herein is maintained. The insertions can be to amino or carboxy terminus, or internal portions of the decarboxylase polypeptide. The variants can be derived by adding an extra amino acid sequence, such as an N-terminal tag or a C-terminal tag. Non-limiting examples of tags are maltose binding protein (MBP) tag, glutathione S-transferase (GST) tag, thioredoxin (Trx) tag, His-tag, and any other tags known by those skilled in art. Tags can be used to improve solubility and expression levels during fermentation or as a handle for enzyme purification.

Enzymes can also be modified by a variety of chemical techniques to produce derivatives having essentially the same or preferably improved activity as the unmodified enzymes, and optionally having other desirable properties. For example, carboxylic acid groups of the protein, whether carboxyl-terminal or side chain, may be provided in the form of a salt of a pharmaceutically-acceptable cation or esterified, for example to form a C1-C6 alkyl ester, or converted to an amide, for example of formula CONR1R2 wherein R1 and R2 are each independently H or C1-C6 alkyl, or combined to form a heterocyclic ring, such as a 5- or 6-membered ring. Amino groups of the enzyme, whether amino-terminal or side chain, may be in the form of a pharmaceutically-acceptable acid addition salt, such as the HCl, HBr, acetic, benzoic, toluene sulfonic, maleic, tartaric and other organic salts, or may be modified to C1-C20 alkyl or dialkyl amino or further converted to an amide. Hydroxyl groups of the protein side chains may be converted to alkoxy or ester groups, for example C1-C20 alkoxy or C1-C20 alkyl ester, using well-recognized techniques. Phenyl and phenolic rings of the protein side chains may be substituted with one or more halogen atoms, such as F, Cl, Br or I, or with C1-C20 alkyl, C1-C20 alkoxy, carboxylic acids and esters thereof, or amides of such carboxylic acids. Methylene groups of the protein side chains can be extended to homologous C2-C4 alkylenes. Thiols can be protected with any one of a number of well-recognized protecting groups, such as acetamide groups. Those skilled in the art will also recognize methods for introducing cyclic structures into the proteins of this disclosure to select and provide conformational constraints to the structure that result in enhanced stability.

The enzymes can be provided on a solid support, such as a membrane, resin, solid carrier, or other solid phase material. A solid support can be composed of organic polymers such as polystyrene, polyethylene, polypropylene, polyfluoroethylene, polyethyleneoxy, and polyacrylamide, as well as co-polymers and grafts thereof. A solid support can also be inorganic, such as glass, silica, controlled pore glass (CPG), reverse phase silica or metal, such as gold or platinum. The configuration of a solid support can be in the form of beads, spheres, particles, granules, a gel, a membrane or a surface. Surfaces can be planar, substantially planar, or nonplanar. Solid supports can be porous or non-porous, and can have swelling or non-swelling characteristics. A solid support can be configured in the form of a well, depression, or other container, vessel, feature, or location.

The polypeptides having decarboxylase activity can be bound or immobilized on the solid support such that they retain at least a portion of their improved properties relative to a reference polypeptide (e.g., SEQ ID NO: 1). Accordingly, it is further contemplated that suitable methods of using the decarboxylase polypeptides can be carried out using the same decarboxylase polypeptides bound or immobilized on a solid support.

The decarboxylase polypeptide can be bound non-covalently or covalently. Various methods for conjugation and immobilization of enzymes to solid supports (e.g., resins, membranes, beads, glass, etc.) are well known in the art. Other methods for conjugation and immobilization of enzymes to solid supports (e.g., resins, membranes, beads, glass, etc.) are well known in the art (See, e.g., Yi et al., Proc. Biochem., 42: 895-898 [2007]; Martin et al., Appl. Microbiol. Biotechnol., 76: 843-851 [2007]; Koszelewski et al. J. Mol. Cat. B: Enz., 63: 39-44 [2010]; Truppo et al., Org. Proc. Res. Develop., published online: dx.doi.org/10.1021/op200157c; and Mateo et al., Biotechnol. Prog., 18:629-34 [2002], etc.). Solid supports useful for immobilizing the decarboxylase polypeptides of use in the present invention include, but are

not limited to, beads or resins comprising polymethacrylate with epoxide functional groups, polymethacrylate with amino epoxide functional groups, styrene/DVB copolymer or polymethacrylate with octadecyl functional groups.

The enzymes may be incorporated into the hand dish-washing compositions *via* an additive particle, such as an enzyme granule or in the form of an encapsulate or may be added in the form of a liquid formulation. Encapsulating the enzymes promote the stability of the enzymes in the composition and helps to counteract the effect of any hostile compounds present in the composition, such as bleach, protease, surfactant, chelant, etc. The P450 fatty acid decarboxylase enzymes may be the only enzymes in the additive particle or may be present in the additive particle in combination with one or more additional co-enzymes.

The hand dish-washing composition can comprise a P450 fatty acid decarboxylase, wherein said P450 fatty acid decarboxylase is present in an amount of from 0.0001 wt% to 1 wt%, preferably from 0.001 wt% to 0.2 wt%, by weight of the consumer product composition, based on active protein.

The hand dish-washing composition can comprise one or more co-enzymes selected from the group consisting of: fatty-acid peroxidases (EC 1.11.1.3), unspecific peroxygenases (EC 1.11.2.1), plant seed peroxygenases (EC 1.11.2.3), fatty acid peroxygenases (EC1.11.2.4), linoleate diol synthases (EC 1.13.11.44), 5,8-linoleate diol synthases (EC 1.13.11.60 and EC 5.4.4.5), 7,8-linoleate diol synthases (EC 1.13.11.60 and EC 5.4.4.6), 9,14-linoleate diol synthases (EC 1.13.11.B1), 8,11-linoleate diol synthases, oleate diol synthases, other linoleate diol synthases, unspecific monooxygenase (EC 1.14.14.1), alkane 1-monooxygenase (EC 1.14.15.3), oleate 12-hydroxylases (EC 1.14.18.4), fatty acid amide hydrolases (EC 3.5.1.99), fatty acid photodecarboxylases (EC 4.1.1.106), oleate hydratases (EC 4.2.1.53), linoleate isomerases (EC 5.2.1.5), linoleate (10E,12Z)-isomerases (EC 5.3.3.B2), non-heme fatty acid decarboxylases (UndA-like), alpha-dioxygenases, amylases, lipases, proteases, cellulases, and mixtures thereof; preferably fatty-acid peroxidases (EC 1.11.1.3), unspecific peroxygenases (EC 1.11.2.1), plant seed peroxygenases (EC 1.11.2.3), and fatty acid peroxygenases (EC 1.11.2.4), non-heme fatty acid decarboxylases (UndA-like), alpha-dioxygenases, and mixtures thereof.

Where necessary, the composition comprises, provides access to, or forms *in situ* any additional substrate necessary for the effective functioning of the enzyme. For example, molecular hydrogen peroxide can be provided as an additional substrate for P450 fatty acid decarboxylases. In embodiments, the consumer product composition may be supplemented with heme and/or a source of iron to enhance or facilitate the conversion of the fatty acids.

The P450 fatty acid decarboxylase can comprise a heme cofactor selected from the group comprising: heme a, heme b, heme c, heme d, heme i, heme m, heme o, heme s, their derivatives, and mixtures thereof; preferably heme b. The heme cofactor can be covalently attached to the P450 fatty acid decarboxylases.

The P450 fatty acid decarboxylase can comprise a heme cofactor comprising: a) a porphyrin group and b) a metal. Non-limiting examples of porphyrin groups are: protoporphyrin IX, N-methyl protoporphyrin IX, protoporphyrin IX monomethyl ester, protoporphyrin IX dimethyl ester, protoporphyrin IX diamide, protoporphyrin IX bis thiosulfate, porphin, phthalocyanine, octaethylporphoyrin, tetraphenylporphyrin, and their derivatives; preferably protoporphyrin IX. Non-limiting examples of metals are: Mg, Al, V, Cr, Mn, Fe, Co, Ni, Cu, Zn, Ga, Ge, Mo, Tc, Ru, Rh, Pd, Ag, Cd, In, Sn, Sb, W, Re, Os, Ir, Pt, Au, Hg, Tl, Pb, Bi, and mixtures thereof; preferably Fe. The P450 fatty acid decarboxylase can comprise a heme cofactor comprising a cation selected from the group comprising: $Mg^{2+}$, $Cr^{3+}$, $Mn^{3+}$, $Fe^{3+}$, $Co^{3+}$, $Ni^{2+}$, $Cu^{2+}$, $Zn^{2+}$, $Ga^{3+}$, $Rh^{2+}$, $Pd^{2+}$, $Ag^{2+}$, $In^{3+}$, $Sn^{4+}$, $VO^{2+}$, and mixtures thereof; preferably $Fe^{3+}$. The P450 fatty acid decarboxylase can comprise a heme cofactor comprising an axially bound ligand. Suitable ligands include: chloride, methyl group, carbonyl group, hydroxide group, and tetrahydrofuran.

Where necessary, the composition comprises, provides access to or forms *in situ* any additional substrate necessary for the effective functioning of the enzyme. For example, when molecular oxygen is an additional substrate, it can be obtained from the atmosphere or from a precursor that can be transformed to produce oxygen in situ. In many applications, oxygen from the atmosphere can be present in sufficient amounts.

Methods of Producing P450 fatty acid decarboxylase Polypeptides

Standard methods of culturing organisms such as, for example, bacteria and yeast, for production of enzymes are well-known in the art and are described herein. For example, host cells may be cultured in a standard growth media under standard temperature and pressure conditions, and in an aerobic environment. Standard growth media for various host cells are commercially available and well-known in the art, as are standard conditions for growing various host cells.

The P450 fatty acid decarboxylase enzymes expressed in a host cell can be recovered from the cells and or the culture medium using any one or more of the well-known techniques for protein purification, including, among others, lysozyme treatment, sonication, filtration, salting-out, ultra-centrifugation, and chromatography. Suitable solutions for lysing and the high efficiency extraction of proteins from bacteria, such as E. coli, are commercially available under the trade name CelLytic B (Sigma-Aldrich). Chromatographic techniques for isolation of the P450 fatty acid decarboxylase polypeptide include, among others, reverse phase chromatography high performance liquid chromatography (HPLC), ion exchange chromatography, gel electrophoresis, and affinity chromatography. Conditions for purifying a particular

enzyme will depend, in part, on factors such as net charge, hydrophobicity, hydrophilicity, molecular weight, molecular shape, etc., and will be apparent to those having skill in the art.

The P450 fatty acid decarboxylases may also be prepared and used in the form of cells expressing the enzymes, as crude extracts, or as isolated or purified preparations. The P450 fatty acid decarboxylases may be prepared as lyophilizates, in powder form (e.g., acetone powders), or prepared as enzyme solutions. The P450 fatty acid decarboxylases can be in the form of substantially pure preparations.

Adjunct Ingredients

The cleaning composition herein may optionally comprise a number of other adjunct ingredients such as additional enzymes, enzyme stabilisers, antioxidant compounds, free radical scavengers, source of hydrogen peroxide, organic solvents, polymers, cleaning amines, chelants, builders (*e.g.,* preferably citrate), structurants, emollients, humectants, skin rejuvenating actives, scrubbing particles, bleach and bleach activators, perfumes, malodor control agents, pigments, dyes, opacifiers, beads, pearlescent particles, capsules, inorganic cations such as alkaline earth metals such as Ca/Mg-ions, antibacterial agents, preservatives, viscosity adjusters (*e.g.,* salt such as NaCl, and other mono-, di- and trivalent salts) and pH adjusters and buffering means (*e.g.,* carboxylic acids such as citric acid, HCl, NaOH, KOH, alkanolamines, phosphoric and sulfonic acids, carbonates such as sodium carbonates, bicarbonates, sesquicarbonates, borates, silicates, phosphates, imidazole and alike).

Additional Enzymes

Preferred compositions of the invention comprise one or more enzymes selected from lipases, proteases, cellulases, amylases, and any combination thereof.

Each additional enzyme is typically present in an amount from 0.0001 wt% to 1 wt% (weight of active protein) more preferably from 0.0005 wt% to 0.5 wt%, most preferably 0.005-0.1% by weight of the detergent composition. It may be particularly preferred for the compositions of the present invention to additionally comprise a lipase enzyme. Lipases break down fatty ester soils into fatty acids which are then acted upon by the saturated and/or unsaturated fatty acid-transforming enzyme according to the invention into suds neutral or suds boosting agents.

It may be particularly preferred for the compositions of the present invention to additionally comprise a protease enzyme. Since oleic acid and other foam suppressing saturated and/or unsaturated fatty acids are present in body soils or even human skin, as protease enzyme acts as a skin care agent, or breaks down proteinaceous soils, fatty acids released are broken down, preventing suds suppression.

It may be particularly preferred for the compositions of the present invention to additionally comprise an amylase enzyme. Since oily soils are commonly entrapped in starchy soils, the amylase and saturated and/or unsaturated fatty acid transforming enzymes work synergistically together: fatty acid soils are released by breakdown of starchy soils with amylase, thus, the saturated and/or unsaturated fatty acid transforming enzyme according to the invention is particularly effective in ensuring there is no negative impact on suds in the wash liquor.

Enzyme Stabiliser

Preferably the composition of the invention comprises an enzyme stabilizer. Suitable enzyme stabilizers may be selected from the group consisting of (a) univalent, bivalent and/or trivalent cations preferably selected from the group of inorganic or organic salts of alkaline earth metals, alkali metals, aluminum, iron, copper and zinc, preferably alkali metals and alkaline earth metals, preferably alkali metal and alkaline earth metal salts with halides, sulfates, sulfites, carbonates, hydrogencarbonates, nitrates, nitrites, phosphates, formates, acetates, propionates, citrates, maleates, tartrates, succinates, oxalates, lactates, and mixtures thereof. The salt can be selected from the group consisting of sodium chloride, calcium chloride, potassium chloride, sodium sulfate, potassium sulfate, sodium acetate, potassium acetate, sodium formate, potassium formate, calcium lactate, calcium nitrate and mixtures thereof. Most preferred are salts selected from the group consisting of calcium chloride, potassium chloride, potassium sulfate, sodium acetate, potassium acetate, sodium formate, potassium formate, calcium lactate, calcium nitrate, and mixtures thereof, and in particular potassium salts selected from the group of potassium chloride, potassium sulfate, potassium acetate, potassium formate, potassium propionate, potassium lactate and mixtures thereof. Most preferred are potassium acetate and potassium chloride. Preferred calcium salts are calcium formate, calcium lactate and calcium nitrate including calcium nitrate tetrahydrate. Calcium and sodium formate salts may be preferred. These cations are present at at least 0.01 wt%, preferably at least 0.03 wt%, more preferably at least 0.05 wt%, most preferably at least 0.25 wt% up to 2 wt% or even up to 1 wt% by weight of the total composition. These salts are formulated from 0.1 wt% to 5 wt%, preferably from 0.2 wt% to 4 wt%, more preferably from 0.3 wt% to 3 wt%, most preferably from 0.5 wt% to 2 wt% relative to the total weight of the composition. Further enzyme stabilizers can be selected from the group (b) carbohydrates selected from

the group consisting of oligosaccharides, polysaccharides and mixtures thereof, such as a monosaccharide glycerate as described in WO201219844; (c) mass efficient reversible protease inhibitors selected from the group consisting of phenyl boronic acid and derivatives thereof, preferably 4-formyl phenylboronic acid; (d) alcohols such as 1,2-propane diol, propylene glycol; (e) peptide aldehyde stabilizers such as tripeptide aldehydes such as Cbz-Gly-Ala-Tyr-H, or disubstituted alaninamide; (f) carboxylic acids such as phenyl alkyl dicarboxylic acid as described in WO2012/19849 or multiply substituted benzyl carboxylic acid comprising a carboxyl group on at least two carbon atoms of the benzyl radical such as described in WO2012/19848, phthaloyl glutamine acid, phthaloyl asparagine acid, aminophthalic acid and/or an oligoamino-biphenyl-oligocarboxylic acid; and (g) mixtures thereof.

The composition of the present invention may optionally comprise from 0.01% to 3%, preferably from 0.05% to 2%, more preferably from 0.2% to 1.5%, or most preferably 0.5% to 1%, by weight of the total composition of a salt, preferably a monovalent, divalent inorganic salt or a mixture thereof, preferably sodium chloride. Most preferably the composition alternatively or further comprises a multivalent metal cation in the amount of from 0.01 wt% to 3 wt%, preferably from 0.05% to 2%, more preferably from 0.2% to 1.5%, or most preferably 0.5% to 1% by weight of said composition, preferably said multivalent metal cation is magnesium, aluminium, copper, calcium or iron, more preferably magnesium, most preferably said multivalent salt is magnesium chloride. Without wishing to be bound by theory, it is believed that use of a multivalent cation helps with the formation of protein/ protein, surfactant/ surfactant or hybrid protein/ surfactant network at the oil water and air water interface that is strengthening the suds.

Preferably the composition of the present invention comprises one or more carbohydrates selected from the group comprising O-glycan, N-glycan, and mixtures thereof. Preferably the cleaning composition further comprises one or more carbohydrates selected from the group comprising derivatives of glucose, mannose, lactose, galactose, allose, altrose, gulose, idose, talose, fucose, fructose, sorbose, tagatose, psicose, arabinose, ribose, xylose, lyxose, ribulose, and xylulose. More preferably the cleaning composition comprises one or more carbohydrates selected from the group of $\alpha$-glucans and $\beta$-glucans. Glucans are polysaccharides of D-glucose monomers, linked by glycosidic bonds. Suitable $\alpha$-glucans are dextran, starch, floridean starch, glycogen, pullulan, and their derivatives. Suitable $\beta$-glucans are cellulose, chrysolaminarin, curdlan, laminarin, lentinan, lichenin, oat beta-glucan, pleuran, zymosan, and their derivatives.

Hydrotrope

The composition of the present invention may optionally comprise from 1% to 10%, or preferably from 0.5% to 10%, more preferably from 1% to 6%, or most preferably from 0.1% to 3%, or combinations thereof, by weight of the total composition of a hydrotrope, preferably sodium cumene sulfonate. Other suitable hydrotropes for use herein include anionic-type hydrotropes, particularly sodium, potassium, and ammonium xylene sulfonate, sodium, potassium and ammonium toluene sulfonate, sodium potassium and ammonium cumene sulfonate, and mixtures thereof, as disclosed in U.S. Patent 3,915,903. Preferably the composition of the present invention is isotropic. An isotropic composition is distinguished from oil-in-water emulsions and lamellar phase compositions. Polarized light microscopy can assess whether the composition is isotropic. See e.g., The Aqueous Phase Behaviour of Surfactants, Robert Laughlin, Academic Press, 1994, pp. 538-542. Preferably an isotropic composition is provided. Preferably the composition comprises 0.1% to 3% by weight of the total composition of a hydrotrope, preferably wherein the hydrotrope is selected from sodium, potassium, and ammonium xylene sulfonate, sodium, potassium and ammonium toluene sulfonate, sodium potassium and ammonium cumene sulfonate, and mixtures thereof.

Antioxidant compounds and free radical scavengers

Antioxidant compounds and free radical scavengers can generally protect enzyme from degradation by preventing excessive generation of singlet oxygen and peroxy radicals that promote alteration of enzyme structure leading to short TON of Enzymes. Not to be limited by theory, a general discussion of the mode of action for antioxidants and free radical scavengers is disclosed in Kirk Othmer, The Encyclopedia of Chemical Technology, Volume 3, pages 128 - 148, Third Edition (1978).

The composition may optionally contain an anti-oxidant present from about 0.001% to about 2.0% by weight of the detergent composition. The antioxidant can be present at a concentration in the range 0.01 to 0.1% by weight of the detergent composition. Mixtures of anti-oxidants may be used and in some embodiments, may be preferred.

One class of anti-oxidants used in the present invention is alkylated phenols, having the general formula:

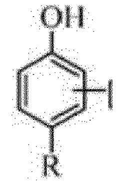

wherein R is C1-C22 linear or branched alkyl, preferably methyl or branched C3-C6 alkyl, Ci-$C_6$ alkoxy, preferably methoxy, or $CH_2CH_2C(0)0R'$, wherein R' is H, a charge balancing counterion or C1-C22 linear or branched alkyl; Ri is a C3-C6 branched alkyl, preferably tert-butyl; x is 1 or 2. Hindered phenolic compounds are a preferred type of alkylated phenols having this formula. A preferred hindered phenolic compound of this type is 3,5-di-tert-butyl-4-hydroxytoluene (BHT).

Furthermore, the anti-oxidant used in the composition may be selected from the group consisting of a-, b-, g-, d-tocopherol, ethoxyquin, 2, 2 4-trimethyl- 1 ,2-dihydroquinoline, 2,6-di- tert-butyl hydroquinone, tert-butyl hydroxyanisole, lignosulphonic acid and salts thereof, and mixtures thereof. It is noted that ethoxyquin (1,2-dihydro-6-ethoxy-2,2,4-trimethylquinoline) is marketed under the name Raluquin™ by the company Raschig™. Other types of anti-oxidants that may be used in the composition are 6-hydroxy-2, 5,7,8- tetramethylchroman-2-carboxylic acid (Trolox™) and 1,2-benzisothiazoline-3-one (Proxel GXL™).

A further class of anti-oxidants which may be suitable for use in the composition is a benzofuran or benzopyran derivative having the formula:

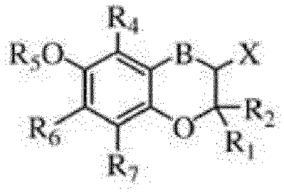

wherein Ri and $R_2$ are each independently alkyl or Ri and $R_2$ can be taken together to form a $C_5$-$C_6$ cyclic hydrocarbyl moiety; B is absent or $CH_2$; $R_4$ is Ci-Ce alkyl; $R_5$ is hydrogen or-C(O)$R_3$ wherein $R_3$ is hydrogen or $C_1$-$C_{19}$ alkyl; Re is Ci-Ce alkyl; $R_7$ is hydrogen or Ci-Ce alkyl; X is -$CH_2OH$, or -$CH_2A$ wherein A is a nitrogen comprising unit, phenyl, or substituted phenyl. Preferred nitrogen comprising A units include amino, pyrrolidino, piperidino, morpholino, piperazino, and mixtures thereof.

Anti-oxidants such as tocopherol sorbate, butylated hydroxyl benxoic acids and their salts, gallic acid and its alkyl esters, ascorbic, citric, tartric, uric acid and its salts, sorbic acid and its salts, and dihydroxyfumaric acid and its salts may also be used. Preferred types of anti-oxidant for use in the composition include 3,5-di-tert-butyl-4-hydroxytoluene (BHT), a-, b-, g-, d-tocopherol, 1,2-benzisothiazoline-3-one (Proxel GXL™), anthocyanins, carotene, catechins, flavonoids, lutein, lycopene and mixtures thereof. Other preferred types of anti-oxidant for use in the composition include hindered phenols, diarylamines (including phenoxazines with a maximum molar extinction coefficient in the wavelength range from 400 to 750 nm of less than 1,000 $M^{-1}ah^{-1}$), and mixtures thereof. The number of equivalents of hindered phenol initially formulated can normally be greater than or equal to the number of equivalents of diarylamine

Source of hydrogen peroxide

It may be preferred for the composition to comprise a source of hydrogen peroxide. Sources of hydrogen peroxide include, for example, hydrogen peroxide, inorganic perhydrate salts, including alkali metal salts such as sodium salts of perborate (usually mono- or tetra-hydrate), percarbonate, persulphate, perphosphate, persilicate salts and mixtures thereof. In one aspect of the invention the inorganic perhydrate salts are selected from the group consisting of sodium salts of perborate, percarbonate and mixtures thereof. Percarbonate salts can be preferred. When employed, inorganic perhydrate salts are typically present in amounts of from 0.05 to 40 wt%, or 1 to 30 wt% of the overall dish care product and are typically incorporated into such dish care products as a crystalline solid that may be coated. Suitable coatings include, inorganic salts such as alkali metal silicate, carbonate or borate salts or mixtures thereof, or organic materials such as water-soluble or dispersible polymers, waxes, oils or fatty soaps. These may be present in combination with bleach activators and/or bleach catalysts.

Suitable bleach activators are those having R-(C=O)-L wherein R is an alkyl group, optionally branched, having, when the bleach activator is hydrophobic, from 6 to 14 carbon atoms, or from 8 to 12 carbon atoms and, when the bleach activator is hydrophilic, less than 6 carbon atoms or even less than 4 carbon atoms; and L is leaving group. Examples

of suitable leaving groups are benzoic acid and derivatives thereof - especially benzene sulphonate. Suitable bleach activators include dodecanoyl oxybenzene sulphonate, decanoyl oxybenzene sulphonate, decanoyl oxybenzoic acid or salts thereof, 3,5,5-trimethyl hexanoyloxybenzene sulphonate, tetraacetyl ethylene diamine (TAED) and nonanoyloxybenzene sulphonate (NOBS). While any suitable bleach activator may be employed, it may be preferred if the subject composition comprises NOBS, TAED or mixtures thereof.

Suitable bleach catalysts include one or more bleach catalysts capable of accepting an oxygen atom from a peroxyacid and/or salt thereof and transferring the oxygen atom to an oxidizable substrate. Suitable bleach catalysts include, but are not limited to: iminium cations and polyions; iminium zwitterions; modified amines; modified amine oxides; N-sulphonyl imines; N-phosphonyl imines; N-acyl imines; thiadiazole dioxides; perfluoroimines; cyclic sugar ketones and alpha aminoketones and mixtures thereof.

Suitable bleach catalysts include oxaziridinium bleach catalysts, transition metal bleach catalysts, especially manganese and iron bleach catalysts. A suitable bleach catalyst has a structure corresponding to general formula below:

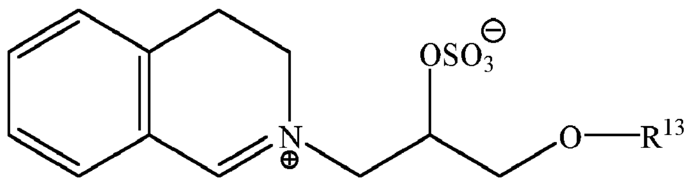

wherein $R^{13}$ is selected from the group consisting of 2-ethylhexyl, 2-propylheptyl, 2-butyloctyl, 2-pentylnonyl, 2-hexyldecyl, n-dodecyl, n-tetradecyl, n-hexadecyl, n-octadecyl, iso-nonyl, isodecyl, iso-tridecyl and iso-pentadecyl.

Another suitable source of hydrogen peroxide includes pre-formed peracids. Suitable preformed peracids include, but are not limited to compounds selected from the group consisting of pre-formed peroxyacids or salts thereof typically a percarboxylic acids and salts, percarbonic acids and salts, perimidic acids and salts, peroxymonosulfuric acids and salts, for example, Oxone ®, and mixtures thereof. Suitable examples include peroxycarboxylic acids or salts thereof, or peroxysulphonic acids or salts thereof. Typical peroxycarboxylic acid salts suitable for use herein have a chemical structure corresponding to the following chemical formula:

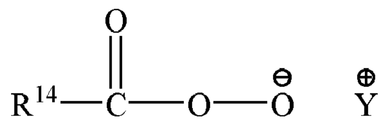

wherein: $R^{14}$ is selected from alkyl, aralkyl, cycloalkyl, aryl or heterocyclic groups; the $R^{14}$ group can be linear or branched, substituted or unsubstituted; having, when the peracid is hydrophobic, from 6 to 14 carbon atoms, or from 8 to 12 carbon atoms and, when the peracid is hydrophilic, less than 6 carbon atoms or even less than 4 carbon atoms and Y is any suitable counter-ion that achieves electric charge neutrality, preferably Y is selected from hydrogen, sodium or potassium. $R^{14}$ may be a linear or branched, substituted or unsubstituted $C_{6-9}$ alkyl. The peroxyacid or salt thereof may be selected from peroxyhexanoic acid, peroxyheptanoic acid, peroxyoctanoic acid, peroxynonanoic acid, peroxydecanoic acid, any salt thereof, or any combination thereof. Peroxyacids that mey be used include phthalimido-peroxy-alkanoic acids, in particular ε-phthalimido peroxy hexanoic acid (PAP). The peroxyacid or salt thereof may have a melting point in the range of from 30°C to 60°C.

The pre-formed peroxyacid or salt thereof can also be a peroxysulphonic acid or salt thereof, typically having a chemical structure corresponding to the following chemical formula:

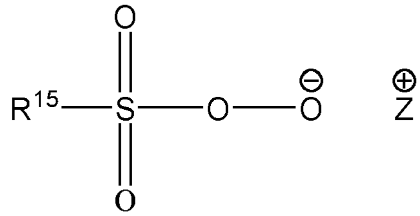

wherein: $R^{15}$ is selected from alkyl, aralkyl, cycloalkyl, aryl or heterocyclic groups; the $R^{15}$ group can be linear or branched, substituted or unsubstituted; and Z is any suitable counter-ion that achieves electric charge neutrality, preferably Z is selected from hydrogen, sodium or potassium. Preferably $R^{15}$ is a linear or branched, substituted or unsubstituted $C_{4-14}$, preferably $C_{6-14}$ alkyl. Preferably such bleach components may be present in the compositions of the invention in an

amount from 0.01 to 50%, most preferably from 0.1% to 20% by weight of the detergent composition.

Hydrogen peroxide may also be provided by the incorporation of one or more hydrogen peroxide producing enzymes such as alcohol oxidoreductases, aldehyde oxidoreductases, amino acid oxidoreductases, and monoamine oxidases. These enzymes can convert in situ (e.g. in the washing process) substrates such as carbohydrates, proteins, amino acids, alcohols, amines, or other substrates either from a soil or from a material also present in the composition, to generate hydrogen peroxide. Since this will tend to generate low levels of hydrogen peroxide this may be preferred. Non-limiting examples of hydrogen peroxide producing enzymes are: glycolate oxidases (EC 1.1.3.1), L-lactate oxidases (EC 1.1.3.2), malate oxidases (EC 1.1.3.3), glucose oxidases (EC 1.1.3.4), glycerol oxidases (EC 1.1.3.B4), hexose oxidases (EC 1.1.3.5), cholesterol oxidases (EC 1.1.3.6), aryl-alcohol oxidases (EC 1.1.3.7), L-gulonolactone oxidases (EC 1.1.3.8), galactose oxidases (EC 1.1.3.9), pyranose oxidases (EC 1.1.3.10), L-sorbose oxidases (EC 1.1.3.11), alcohol oxidases (EC 1.1.3.13), (S)-2-hydroxy-acid oxidases (EC 1.1.3.15), chlorine oxidases (EC 1.1.3.17), secondary-alcohol oxidases (EC 1.1.3.18), long-chain-alcohol oxidases (EC 1.1.3.20), thiamine oxidases (EC 1.1.3.23), nucleoside oxidases (EC 1.1.3.28, EC 1.1.3.39), polyvinyl-alcohol oxidases (EC 1.1.3.30), vanillyl-alcohol oxidases (EC 1.1.3.38), D-mannitol oxidase ((EC 1.1.3.40), alditol oxidases (EC 1.1.3.41), glucooligosaccharide oxidases (EC 1.1.99.B3), cellobiose dehydrogenase (EC 1.1.99.18), aldehyde oxidases (EC 1.2.3.1), pyruvate oxidases (EC 1.2.3.3), oxalate oxidases (EC 1.2.3.4), glyoxylate oxidases (EC 1.2.3.5), D-aspartate oxidases (EC 1.4.3.1), L-amino acid oxidases (EC 1.4.3.2), D-amino acid oxidases (EC 1.4.3.3), monoamine oxidases (EC 1.4.3.4), D-glutamate oxidases (EC 1.4.3.7), ethanolamine oxidases (EC 1.4.3.8), protein-lysine 6-oxidases (EC 1.4.3.13), L-lysine oxidases (EC 1.4.3.14), D-glutamate (D-aspartate) oxidases (EC 1.4.3.15), L-aspartate oxidases (EC 1.4.3.16), glycine oxidases (EC 1.4.3.19), L-lysine 6-oxidases (EC 1.4.3.20), primary-amine oxidases (EC 1.4.3.21), diamine oxidases (EC 1.4.3.22), L-arginine oxidases (EC 1.4.3.25), non-specific polyamine oxidases (EC 1.5.3.17), other alcohol oxidoreductases (EC 1.1.X.X), other aldehyde oxidoreductases (EC 1.2.X.X), other amino acid oxidoreductases or monoamine oxidases (EC 1.3.X.X), and other amine oxidoreductases (EC 1.5.X.X). The hydrogen peroxide producing enzyme can be fused to the P450 fatty acid decarboxylase to form a single polypeptide or can be independent enzymes.

The hydrogen peroxide source can be substituted by: a) a source of nicotinamide adenine dinucleotide (NADH) or nicotinamide adenine dinucleotide phosphate (NADPH) and b) an enzymatic redox system. Non-limiting examples of enzymatic redox systems are: the reductase domain of Bacillus megaterium CYP102A1 (P450BM3), the RhFred reductase domain from Rhodococcus sp. NCIMB 9784, the flavodoxin (Fld) / ferrodoxin reductase (FdR, EC 1.18.1.2 and EC 1.18.1.3) system, the putidaredoxin (Pd) / putidaredoxin reductase (PdR, EC 1.18.1.5) system, the rubredoxin / rubredoxin reductase (EC 1.18.1.1 and EC 1.18.1.4) system, and the adrenoxin / adrenodoxin reductase (EC 1.18.1.6) system. In some embodiments, the composition may also comprise a dehydrogenase-based NADH or NADPH regeneration system, such as the phosphonate / phosphonate dehydrogenase (EC 1.20.1.1) system.

Organic solvent

The composition of the present invention may optionally comprise an organic solvent. Suitable organic solvents include C4-14 ethers and diethers, polyols, glycols, alkoxylated glycols, C6-C16 glycol ethers, alkoxylated aromatic alcohols, aromatic alcohols, aliphatic linear or branched alcohols, alkoxylated aliphatic linear or branched alcohols, alkoxylated C1-C5 alcohols, C8-C14 alkyl and cycloalkyl hydrocarbons and halohydrocarbons, and mixtures thereof. Preferably the organic solvents include alcohols, glycols, and glycol ethers, alternatively alcohols and glycols. The composition comprises from 0% to less than 50%, preferably from 0.01% to 25%, more preferably from 0.1% to 10%, or most preferably from 0.5% to 5%, by weight of the total composition of an organic solvent, preferably an alcohol, more preferably an ethanol, a polyalkyleneglycol, more preferably polypropyleneglycol, and mixtures thereof.

Polymer:

The composition can comprise a polymer, preferably at a level of from 0.1% to 5%, more preferably from 0.2% to 3%, even more preferably from 0.3% to 2% by weight of the liquid composition. Suitable polymers can be selected from triblock copolymers, amphiphilic alkoxylated polyalkyleneimine, ethoxylated polyalkyleneimine, polyester soil release polymers, and mixtures thereof, preferably triblock copolymers, amphiphilic alkoxylated polyalkyleneimine, and mixtures thereof.

Suitable triblock copolymers comprise alkylene oxide moieties according to Formula (I): (EO)x(PO)y(EO)x, wherein EO represents ethylene oxide, and each x represents the number of EO units within the EO block. Each x is independently a number average between 3 and 50, preferably between 5 and 25, more preferably between 10 and 15. Preferably x is the same for both EO blocks, wherein the "same" means that the x between the two EO blocks varies within a maximum 2 units, preferably within a maximum of 1 unit, more preferably both x's are the same number of units. PO represents propylene oxide, and y represents the number of PO units in the PO block. Each y is a number average between 5 and 60, preferably between 10 and 40, more preferably between 25 and 35.

The triblock co-polymer can have a ratio of y to each x of from 0.8:1 to 5:1, preferably from 1:1 to 3:1, more preferably from 1.5:1 to 2.5:1. The triblock co-polymer can have an average weight percentage of total EO of between 30% and 50% by weight of the triblock co-polymer. As such, the triblock co-polymer can have an average weight percentage of total PO of between 50% and 70% by weight of the triblock copolymer. It is understood that the average total weight % of EO and PO for the triblock co-polymer adds up to 100%, excluding the end-caps. The end-caps are preferably hydrogen, hydroxyl, methyl, and mixtures thereof, more preferably hydrogen, methyl, and mixtures thereof, and most preferably hydrogen. The triblock co-polymer has a number average molecular weight of between 550 and 8000, preferably between 1000 and 4500, more preferably between 2000 and 3100. Number average molecular weight and compositional analysis of the co-polymer is determined using a 1H NMR spectroscopy (*see* Thermo scientific application note No. AN52907). It is an established tool for polymer characterization, including number-average molecular weight determination and co-polymer composition analysis.

EO-PO-EO triblock co-polymers are commercially available from BASF such as the Pluronic® PE series, and from the Dow Chemical Company such as Tergitol™ L series. Particularly preferred triblock co-polymer from BASF are sold under the tradenames Pluronic® L44 (MW ca 2200, ca 40wt% EO), Pluronic® PE6400 (MW ca 2900, ca 40wt% EO), Pluronic® PE4300 (MW ca 1600, ca 30wt% EO), and Pluronic® PE 9400 (MW ca 4600, 40 wt% EO). Particularly preferred triblock co-polymer from the Dow Chemical Company is sold under the tradename of Tergitol™ L64 (MW ca 2900, ca 40 wt% EO). The preparation method for such triblock co-polymers is well known to polymer manufacturers.

Suitable amphiphilic polymers can be selected from the group consisting of: amphiphilic alkoxylated polyalkyleneimine and mixtures thereof. Preferably, the amphiphilic alkoxylated polyalkyleneimine is an alkoxylated polyethyleneimine polymer comprising a polyethyleneimine backbone having a weight average molecular weight range of from 100 to 5,000, preferably from 400 to 2,000, more preferably from 400 to 1,000 Daltons. The polyethyleneimine backbone comprises the following modifications:

(i) one or two alkoxylation modifications per nitrogen atom, dependent on whether the modification occurs at an internal nitrogen atom or at an terminal nitrogen atom, in the polyethyleneimine backbone, the alkoxylation modification consisting of the replacement of a hydrogen atom on by a polyalkoxylene chain having an average of about 1 to about 50 alkoxy moieties per modification, wherein the terminal alkoxy moiety of the alkoxylation modification is capped with hydrogen, a C1-C4 alkyl or mixtures thereof;
(ii) a substitution of one C1-C4 alkyl moiety and one or two alkoxylation modifications per nitrogen atom, dependent on whether the substitution occurs at a internal nitrogen atom or at an terminal nitrogen atom, in the polyethyleneimine backbone, the alkoxylation modification consisting of the replacement of a hydrogen atom by a polyalkoxylene chain having an average of about 1 to about 50 alkoxy moieties per modification wherein the terminal alkoxy moiety is capped with hydrogen, a C1-C4 alkyl or mixtures thereof; or
(iii) a combination thereof.

A preferred amphiphilic alkoxylated polyethyleneimine polymer has the general structure of formula (II):

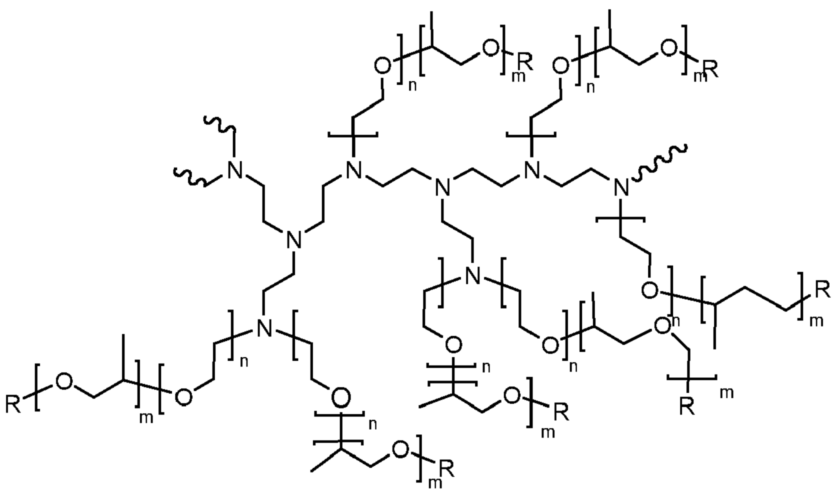

(II)

wherein the polyethyleneimine backbone has a weight average molecular weight of about 600, n of formula (II) has an average of about 10, m of formula (II) has an average of about 7 and R of formula (II) is selected from hydrogen, a $C_1$-$C_4$ alkyl and mixtures thereof, preferably hydrogen. The degree of permanent quaternization of formula (II) may be from

0% to about 22% of the polyethyleneimine backbone nitrogen atoms. The molecular weight of this amphiphilic alkoxylated polyethyleneimine polymer preferably is between 10,000 and 15,000 Da.

More preferably, the amphiphilic alkoxylated polyethyleneimine polymer has the general structure of formula (II) but wherein the polyethyleneimine backbone has a weight average molecular weight of about 600 Da, n of Formula (II) has an average of about 24, m of Formula (II) has an average of about 16 and R of Formula (II) is selected from hydrogen, a $C_1$-$C_4$ alkyl and mixtures thereof, preferably hydrogen. The degree of permanent quaternization of Formula (II) may be from 0% to about 22% of the polyethyleneimine backbone nitrogen atoms, and is preferably 0%. The molecular weight of this amphiphilic alkoxylated polyethyleneimine polymer preferably is between 25,000 and 30,000, most preferably 28,000 Da.

The amphiphilic alkoxylated polyethyleneimine polymers can be made by the methods described in more detail in PCT Publication No. WO 2007/135645.

Alternatively, the alkoxylated polyalkyleneimine polymer can be an ethoxylated polyalkyleneimine which comprises no further alkoxylation, and as such, is hydrophilic rather than amphiphilic. That is, the ethoxylated polyalkyleneimine comprises no further alkoxylation such as propoxylation or butoxylation. Preferred ethoxylated polyalkyleneimines consist of alkyleneimine monomer units and ethoxylation (-EO-) monomer units, with the exception of any end-caps, which are typically hydrogen. Ethyleneimine monomer units are highly preferred alkyleneimine monomer units. More preferably, the hydrophilic ethoxylated polyethyleneimine polymer has the general structure of formula (II) but wherein the polyethyleneimine backbone has a weight average molecular weight of about 600 Da, n of Formula (II) has an average of about 20, m of Formula (II) is zero and R of Formula (II) is selected from hydrogen, a $C_1$-$C_4$ alkyl and mixtures thereof, preferably hydrogen. The degree of permanent quaternization of Formula (II) may be from 0% to about 22% of the polyethyleneimine backbone nitrogen atoms, and is preferably 0%. The molecular weight of this ethoxylated polyethyleneimine polymer preferably is between 10,000 and 15,000, most preferably 12,600 Da.

Polyester soil release agents are also suitable polymers. Soil release agents are polymers having soil release properties, i.e. having the property to enhance the cleaning efficacy of the detergent composition by improving release of greasy and oil during the laundry process. See soil release agents' definition, p.278-279, "Liquid Detergents" by Kuo-Yann Lai.

Suitable polyester soil release agents can encompass simple copolymeric blocks of ethylene terephthalate or propylene terephthalate with polyethylene oxide or polypropylene oxide terephthalate (see US 3,959,230 and US 3,893,929). Other suitable polyester soil release agents can be polyesters with repeat units containing 10-15% by weight of ethylene terephthalate together with 90-80% by weight of polyoxyethylene terephthalate, derived from a polyoxyethylene glycol of average molecular weight 300-5,000. Commercial examples include ZELCON® 5126 from Dupont and MILEASE®T from ICI. Suitable polymeric soil release agents can be prepared by art-recognized methods. US 4, 702, 857 and US 4,711,730 describe the preferred method of synthesis for the block polyesters of use.

<u>Cyclic Polyamine</u>

The composition can comprise a cyclic polyamine having amine functionalities that helps cleaning. The composition of the invention preferably comprises from about 0.1% to about 3%, more preferably from about 0.2% to about 2%, and especially from about 0.5% to about 1%, by weight of the composition, of the cyclic polyamine.

The amine can be subjected to protonation depending on the pH of the cleaning medium in which it is used. Preferred cyclic polyamines have the following Formula (IV):

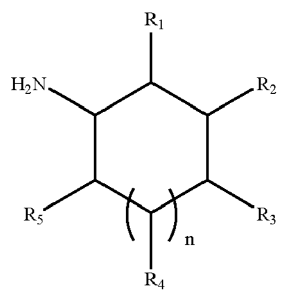

(IV)

wherein $R_1$, $R_2$, $R_3$, $R_4$ and $R_5$ are independently selected from the group consisting of NH2, -H, linear or branched alkyl having from about 1 to about 10 carbon atoms, and linear or branched alkenyl having from about 1 to about 10 carbon atoms, n is from about 1 to about 3, preferably n is 1, and wherein at least one of the Rs is NH2 and the remaining "Rs" are independently selected from the group consisting of NH2, -H, linear or branched alkyl having about 1 to about 10 carbon atoms, and linear or branched alkenyl having from about 1 to about 10 carbon atoms. Preferably, the cyclic

polyamine is a diamine, wherein n is 1, $R_2$ is NH2, and at least one of $R_1$, $R_3$, $R_4$ and $R_5$ is CH3 and the remaining Rs are H.

The cyclic polyamine has at least two primary amine functionalities. The primary amines can be in any position in the cyclic amine but it has been found that in terms of grease cleaning, better performance is obtained when the primary amines are in positions 1,3. It has also been found that cyclic amines in which one of the substituents is -CH3 and the rest are H provided for improved grease cleaning performance.

Accordingly, the most preferred cyclic polyamine for use with the detergent composition of the present invention are cyclic polyamine selected from the group consisting of: 2-methylcyclohexane-1,3-diamine, 4-methylcyclohexane-1,3-diamine and mixtures thereof. These specific cyclic polyamines work to improve suds and grease cleaning profile through-out the dishwashing process when formulated together with the surfactant system of the composition of the present invention.

Chelant

The detergent composition herein can comprise a chelant at a level of from 0.1% to 20%, preferably from 0.2% to 5%, more preferably from 0.2% to 3% by weight of total composition.

As commonly understood in the detergent field, chelation herein means the binding or complexation of a bi- or multidentate ligand. These ligands, which are often organic compounds, are called chelants, chelators, chelating agents, and/or sequestering agent. Chelating agents form multiple bonds with a single metal ion. Chelants, are chemicals that form soluble, complex molecules with certain metal ions, inactivating the ions so that they cannot normally react with other elements or ions to produce precipitates or scale, or forming encrustations on soils turning them harder to be removed. The ligand forms a chelate complex with the substrate. The term is reserved for complexes in which the metal ion is bound to two or more atoms of the chelant.

Preferably, the composition of the present invention comprises one or more chelant, preferably selected from the group comprising carboxylate chelants, amino carboxylate chelants, amino phosphonate chelants such as MGDA (methylglycine-N,N-diacetic acid), GLDA (glutamic-N,N- diacetic acid), and mixtures thereof.

Suitable chelating agents can be selected from the group consisting of amino carboxylates, amino phosphonates, polycarboxylate chelating agents and mixtures thereof.

Other chelants include homopolymers and copolymers of polycarboxylic acids and their partially or completely neutralized salts, monomeric polycarboxylic acids and hydroxycarboxylic acids and their salts. Suitable polycarboxylic acids are acyclic, alicyclic, heterocyclic and aromatic carboxylic acids, in which case they contain at least two carboxyl groups which are in each case separated from one another by, preferably, no more than two carbon atoms. A suitable hydroxycarboxylic acid is, for example, citric acid. Another suitable polycarboxylic acid is the homopolymer of acrylic acid. Preferred are the polycarboxylates end capped with sulfonates.

Method of washing

Other aspects of the invention are directed to methods of washing ware especially dishware with a composition of the present invention. Accordingly, there is provided a method of manually washing dishware comprising the steps of delivering a hand-dishwashing composition of the invention into a volume of water to form a wash solution and immersing the dishware in the solution. Preferably the P450 fatty acid decarboxylase is present at a concentration from 0.005 ppm to 15 ppm, preferably from 0.02 ppm to 0.5 ppm, in an aqueous wash liquor during the washing process. As such, the composition herein will be applied in its diluted form to the dishware. Soiled surfaces *e.g.* dishes are contacted with an effective amount, typically from 0.5 mL to 20 mL (per 25 dishes being treated), preferably from 3mL to 10 mL, of the detergent composition of the present invention, preferably in liquid form, diluted in water. The actual amount of detergent composition used will be based on the judgment of user, and will typically depend upon factors such as the particular product formulation of the composition, including the concentration of active ingredients in the composition, the number of soiled dishes to be cleaned, the degree of soiling on the dishes, and the like. Generally, from 0.01 mL to 150 mL, preferably from 3 mL to 40 mL of a liquid detergent composition of the invention is combined with from 2,000 mL to 20,000 mL, more typically from 5,000 mL to 15,000 mL of water in a sink having a volumetric capacity in the range of from 1,000 mL to 20,000 mL, more typically from 5,000 mL to 15,000 mL. The soiled dishes are immersed in the sink containing the diluted compositions then obtained, where contacting the soiled surface of the dish with a cloth, sponge, or similar article cleans them. The cloth, sponge, or similar article may be immersed in the detergent composition and water mixture prior to being contacted with the dish surface, and is typically contacted with the dish surface for a period of time ranged from 1 to 10 seconds, although the actual time will vary with each application and user. The contacting of cloth, sponge, or similar article to the surface is preferably accompanied by a concurrent scrubbing of the surface.

Alternatively, the dishwashing composition can be applied directly onto a cleaning implement or the dishes to be cleaned without any pre-dilution step, or with slight dissolutions as is the case when applied using a damp sponge or other implement.

TEST METHODS

The following assays set forth must be used in order that the invention described and claimed herein may be more fully understood.

Test Method 1 - Enzyme activity assay for P450 fatty acid decarboxylases

Enzymatic reactions with P450 fatty acid decarboxylases can be performed as follows. Aliquots of sodium salts of fatty acids (e.g. sodium palmitate, sodium stearate, sodium oleate, sodium linoleate, or sodium linolenate; final concentration 100 - 200 μM) and FAD (final concentration 200 μM) are resuspended in a suitable reaction buffer (pH 7 to 9). The reaction is started by addition of the enzyme (final concentration 1 μM) and the solutions are incubated for up to 240 minutes at a suitable temperature. Aliquots of 100 μL of the reaction solutions are collected at different time points and mixed with 900 μL of isopropyl alcohol to stop the reaction. Analysis of the samples is performed by reversed-phase LC/MS/MS or GC/MS using standard procedures known in the art to determine the concentrations of salts of fatty acid remaining in the solutions and the percent conversion is calculated. As used herein, a P450 fatty acid decarboxylase catalyzes the conversion of a fatty acid when the percent conversion of said fatty acid is at least 5% under optimal reaction conditions in 240 minutes or less time.

EXAMPLES

Hereinafter, the present invention is described in more detail based on examples. All percentages are by weight unless otherwise specified.

Comparative Example A - Production of *Jeotgalicoccus sp.* OleTJE

*Jeotgalicoccus sp.* OleTJE (SEQ ID NO: 1) is a P450 fatty acid decarboxylase that converts medium chain fatty acids (e.g. linoleic acid) into the corresponding terminal olefins and is included as a comparative example of the present invention. A codon optimized gene (SEQ ID NO: 152) encoding for a *Jeotgalicoccus sp.* OleTJE variant, including an N-terminal amino acid sequence containing a His-tag and a TEV protease cleavage site (SEQ ID NO: 153), was designed and synthesized. After gene synthesis, the protein was expressed and purified. In brief, the complete synthetic gene sequence was subcloned into a pET30a vector by GenScript (Piscataway, NJ). For heterologous expression. *Escherichia coli* BL21 Star™ (DE3) pLysS cells were transformed with the recombinant plasmid and a single colony was inoculated into LB medium containing kanamycin and chloramphenicol. Pre-starter cultures were then inoculated into a fermentor (BioFlo / CelliGen 310; NewBrunswick, Hamburg, Germany) containing LB medium supplemented with kanamycin and chloramphenicol and incubated at 25 °C. At an $OD_{600nm}$ = 0.4, isopropyl β-D-1-thiogalactopyranoside (IPTG) (final concentration 0.1 mM) and 5-aminolevulinic acid (final concentration 0.5 mM) were added to induce protein expression. Cells were harvested by centrifugation at 5000 rpm and 4°C and the pellets were lysed by a bacterial cell lysis buffer (B-PER - ThemoFisher, Waltham, MA). After centrifugation, the supernatant was collected, and the protein was purified by one-step purification using Ni-NTA agarose resin (Qiagen, Hilden, Germany; catalog # 30230) and standard protocols known in the art. The protein was dialyzed using a membrane with 10 kDa MW cutoff against a buffer containing 50 mM Tris-HCl and 10% Glycerol at pH 8.0. The final protein concentration was 1.1 mg/ mL as determined by Modified Lowry protein assay with BSA as a standard (ThermoFisher Scientific, Waltham, MA).

Example 1 - Production of *Micrococcus lylae* OleTML

*Micrococcus lylae* OleTML (SEQ ID NO: 2) is a P450 fatty acid decarboxylase that converts medium chain fatty acids (e.g. linoleic acid) into the corresponding terminal olefins and that is included as an example of the current invention. A codon optimized gene (SEQ ID NO: 154) encoding for a *Micrococcus lylae* OleTML variant (SEQ ID NO: 155), including an N-terminal amino acid sequence containing a His-tag and a TEV protease cleavage site, was designed and synthesized. After gene synthesis, the protein was expressed and purified by Genscript. In brief, the complete synthetic gene sequence was subcloned into a pET30a vector for heterologous expression. *Escherichia coli* C41 (DE3) cells were co-transformed with the recombinant plasmid and with plasmid pTf16. A single colony was inoculated into TB medium containing kanamycin and chloramphenicol. Cultures were incubated at 15 °C for 16 h at 200 rpm and L-arabinose (final concentration 0.1%), δ-aminolevulinic acid (final concentration 0.25 mM) and isopropyl β-D-1-thiogalactopyranoside (IPTG, final concentration 1 mM) were added to induce protein expression. Cells were harvested by centrifugation at 5000 rpm and 4°C and the pellets were lysed by sonication. After centrifugation, the supernatant was collected and the protein was purified by one-step purification using a nickel affinity column and standard protocols known in the art. The protein was stored in a buffer containing 50 mM Tris-HCl, 500 mM NaCl, and 10% Glycerol at pH 8.0. The final protein

concentration was 0.93 mg/ mL as determined by Bradford protein assay with BSA as a standard (ThermoFisher, catalog # 23236).

Example 2 - Production of *Macrococcus bovicus* OleTMB

*Macrococcus bovicus* OleTMB (SEQ ID NO: 22) is a predicted P450 fatty acid decarboxylase that converts medium chain fatty acids (e.g. linoleic acid) into the corresponding terminal olefins and that is included as an example of the current invention. A codon optimized gene (SEQ ID NO: 159) encoding for an OleTMB decarboxylase variant, including an N-terminal amino acid sequence containing a His-tag and a TEV protease cleavage site was designed and synthesized by Genscript. After gene synthesis, the protein was expressed and purified. In brief, the complete synthetic gene sequence was subcloned into a pET30a using the NdeI/XhoI cloning sites. For heterologous expression, *Escherichia coli* BL21 (DE3) cells were transformed with the recombinant plasmid and a single colony was inoculated into LB medium containing kanamycin (50 mg/L). Pre-starter cultures were then inoculated into a fermentor containing Magic Media (ThermoFisher, Catalog # K6803) supplemented with kanamycin (50 mg/L) and incubated at 16 °C for 72 h. At an $OD_{600nm}$ = 0.5 - 1.0, 5-aminolevulinic acid (final concentration 0.5 mM) was added. Cells were harvested by centrifugation at 5000 rpm and 4°C and the pellets were lysed using a bacterial cell lysis buffer (B-PER - ThemoFisher, Waltham, MA). After centrifugation, the supernatant was collected, and the protein was purified by one-step purification using a HisPur™ Ni-NTA Spin Columns (Thermo Scientific, Catalog # 88226) and standard protocols known in the art. The protein was concentrated using a 10 kDa MW cutoff Amicon Ultra Centrifugal Filter Unit (MilliporeSigm, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 3 - Production of *Staphylococcus delphini* OleTSD

*Staphylococcus delphini* OleTSD (SEQ ID NO: 44) is a predicted P450 fatty acid decarboxylase that converts medium chain fatty acids (e.g. linoleic acid) into the corresponding terminal olefins and that is included as an example of the current invention. A codon optimized gene (SEQ ID NO: 160) encoding for an OleTSD decarboxylase variant, including an N-terminal amino acid sequence containing a His-tag and a TEV protease cleavage site was designed and synthesized by Genscript. After gene synthesis, the protein was expressed and purified. In brief, the complete synthetic gene sequence was subcloned into a pET30a using the NdeI/XhoI cloning sites. For heterologous expression, *Escherichia coli* BL21 (DE3) cells were transformed with the recombinant plasmid and a single colony was inoculated into LB medium containing kanamycin (50 mg/L). Pre-starter cultures were then inoculated into a fermentor containing Magic Media (ThermoFisher, Catalog # K6803) supplemented with kanamycin (50 mg/L) and incubated at 16 °C for 72 h. At an $OD_{600nm}$ = 0.5 - 1.0, 5-aminolevulinic acid (final concentration 0.5 mM) was added. Cells were harvested by centrifugation at 5000 rpm and 4°C and the pellets were lysed using a bacterial cell lysis buffer (B-PER - ThemoFisher, Waltham, MA). After centrifugation, the supernatant was collected, and the protein was purified by one-step purification using a HisPur™ Ni-NTA Spin Columns (Thermo Scientific, Catalog # 88226) and standard protocols known in the art. The protein was concentrated using a 10 kDa MW cutoff Amicon Ultra Centrifugal Filter Unit (MilliporeSigma, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 4 - Production of *Staphylococcus felis* OleTSF

*Staphylococcus felis* OleTSF (SEQ ID NO: 60) is a predicted P450 fatty acid decarboxylase that converts medium chain fatty acids (e.g. palmitic acid) into the corresponding terminal olefins and that is included as an example of the current invention. A codon optimized gene (SEQ ID NO: 161) encoding for an OleTSF decarboxylase variant, including an N-terminal amino acid sequence containing a His-tag and a TEV protease cleavage site was designed and synthesized by Genscript. After gene synthesis, the protein was expressed and purified. In brief, the complete synthetic gene sequence was subcloned into a pET30a using the NdeI/XhoI cloning sites. For heterologous expression, *Escherichia coli* BL21 (DE3) cells were transformed with the recombinant plasmid and a single colony was inoculated into LB medium containing kanamycin (50 mg/L). Pre-starter cultures were then inoculated into a fermentor containing Magic Media (ThermoFisher, Catalog # K6803) supplemented with kanamycin (50 mg/L) and incubated at 16 °C for 72 h. At an $OD_{600nm}$ = 0.5 - 1.0, 5-aminolevulinic acid (final concentration 0.5 mM) was added. Cells were harvested by centrifugation at 5000 rpm and 4°C and the pellets were lysed using a bacterial cell lysis buffer (B-PER - ThemoFisher, Waltham, MA). After centrifugation, the supernatant was collected, and the protein was purified by one-step purification using a HisPur™ Ni-NTA Spin Columns (Thermo Scientific, Catalog # 88226) and standard protocols known in the art. The protein was

concentrated using a 10 kDa MW cutoff Amicon Ultra Centrifugal Filter Unit (MilliporeSigma, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 5 - Production of *Fictibacillus sp.* S7 OleTFS

*Fictibacillus sp. S7* OleTFS (SEQ ID NO: 65) is a predicted P450 fatty acid decarboxylase that converts medium chain fatty acids (e.g. palmitic acid) into the corresponding terminal olefins and that is included as an example of the current invention.v A codon optimized gene (SEQ ID NO: 162) encoding for an OleTFS decarboxylase variant, including an N-terminal amino acid sequence containing a His-tag and a TEV protease cleavage site was designed and synthesized by Genscript. After gene synthesis, the protein was expressed and purified. In brief, the complete synthetic gene sequence was subcloned into a pET30a using the NdeI/XhoI cloning sites. For heterologous expression, *Escherichia coli* BL21 (DE3) cells were transformed with the recombinant plasmid and a single colony was inoculated into LB medium containing kanamycin (50 mg/L). Pre-starter cultures were then inoculated into a fermentor containing Magic Media (ThermoFisher, Catalog # K6803) supplemented with kanamycin (50 mg/L) and incubated at 16 °C for 72 h. At an $OD_{600nm}$ = 0.5 - 1.0, 5-aminolevulinic acid (final concentration 0.5 mM) was added. Cells were harvested by centrifugation at 5000 rpm and 4°C and the pellets were lysed using a bacterial cell lysis buffer (B-PER - ThemoFisher, Waltham, MA). After centrifugation, the supernatant was collected, and the protein was purified by one-step purification using a HisPur™ Ni-NTA Spin Columns (Thermo Scientific, Catalog # 88226) and standard protocols known in the art. The protein was concentrated using a 10 kDa MW cutoff Amicon Ultra Centrifugal Filter Unit (MilliporeSigma, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 6 - Production of *Staphylococcus aureus* C0673 OleTSA

*Staphylococcus aureus C0673* OleTSA (SEQ ID NO: 71) is a predicted P450 fatty acid decarboxylase that converts medium chain fatty acids (e.g. palmitic acid) into the corresponding terminal olefins and that is included as an example of the current invention. A codon optimized gene (SEQ ID NO: 163) encoding for an OleTSA decarboxylase variant, including an N-terminal amino acid sequence containing a His-tag and a TEV protease cleavage site was designed and synthesized by Genscript. After gene synthesis, the protein was expressed and purified. In brief, the complete synthetic gene sequence was subcloned into a pET30a using the NdeI/XhoI cloning sites. For heterologous expression, *Escherichia coli* BL21 (DE3) cells were transformed with the recombinant plasmid and a single colony was inoculated into LB medium containing kanamycin (50 mg/L). Pre-starter cultures were then inoculated into a fermentor containing Magic Media (ThermoFisher, Catalog # K6803) supplemented with kanamycin (50 mg/L) and incubated at 16 °C for 72 h. At an $OD_{600nm}$ = 0.5 - 1.0, 5-aminolevulinic acid (final concentration 0.5 mM) was added. Cells were harvested by centrifugation at 5000 rpm and 4°C and the pellets were lysed using a bacterial cell lysis buffer (B-PER - ThemoFisher, Waltham, MA). After centrifugation, the supernatant was collected, and the protein was purified by one-step purification using a HisPur™ Ni-NTA Spin Columns (Thermo Scientific, Catalog # 88226) and standard protocols known in the art. The protein was concentrated using a 10 kDa MW cutoff Amicon Ultra Centrifugal Filter Unit (Millipore-Sigma, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 7 - Production of *Auricoccus indicus* OleTAI

*Auricoccus indicus* OleTAI (SEQ ID NO: 83) is a predicted P450 fatty acid decarboxylase that converts medium chain fatty acids (e.g. palmitic acid) into the corresponding terminal olefins and that is included as an example of the current invention. A codon optimized gene (SEQ ID NO: 164) encoding for an OleTAI decarboxylase variant, including an N-terminal amino acid sequence containing a His-tag and a TEV protease cleavage site was designed and synthesized by Genscript. After gene synthesis, the protein was expressed and purified. In brief, the complete synthetic gene sequence was subcloned into a pET30a using the NdeI/XhoI cloning sites. For heterologous expression, *Escherichia coli* BL21 (DE3) cells were transformed with the recombinant plasmid and a single colony was inoculated into LB medium containing kanamycin (50 mg/L). Pre-starter cultures were then inoculated into a fermentor containing Magic Media (ThermoFisher, Catalog # K6803) supplemented with kanamycin (50 mg/L) and incubated at 16 °C for 72 h. At an $OD_{600nm}$ = 0.5 - 1.0, 5-aminolevulinic acid (final concentration 0.5 mM) was added. Cells were harvested by centrifugation at 5000 rpm and 4°C and the pellets were lysed using a bacterial cell lysis buffer (B-PER - ThemoFisher, Waltham, MA). After centrifugation, the supernatant was collected, and the protein was purified by one-step purification using a HisPur™ Ni-NTA Spin

Columns (Thermo Scientific, Catalog # 88226) and standard protocols known in the art. The protein was concentrated using a 10 kDa MW cutoff Amicon Ultra Centrifugal Filter Unit (MilliporeSigma, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 8 - Production of *Nosocomiicoccus massiliensis* OleTNM

*Nosocomiicoccus massiliensis* OleTNM (SEQ ID NO: 117) is a predicted P450 fatty acid decarboxylase that converts medium chain fatty acids (e.g. palmitic acid) into the corresponding terminal olefins and that is included as an example of the current invention. A codon optimized gene (SEQ ID NO: 165) encoding for an OleTNM decarboxylase variant), including an N-terminal amino acid sequence containing a His-tag and a TEV protease cleavage site was designed and synthesized by Genscript. After gene synthesis, the protein was expressed and purified. In brief, the complete synthetic gene sequence was subcloned into a pET30a using the NdeI/XhoI cloning sites. For heterologous expression, *Escherichia coli* BL21 (DE3) cells were transformed with the recombinant plasmid and a single colony was inoculated into LB medium containing kanamycin (50 mg/L). Pre-starter cultures were then inoculated into a fermentor containing Magic Media (ThermoFisher, Catalog # K6803) supplemented with kanamycin (50 mg/L) and incubated at 16 °C for 72 h. At an $OD_{600nm}$ = 0.5 - 1.0, 5-aminolevulinic acid (final concentration 0.5 mM) was added. Cells were harvested by centrifugation at 5000 rpm and 4°C and the pellets were lysed using a bacterial cell lysis buffer (B-PER - ThemoFisher, Waltham, MA). After centrifugation, the supernatant was collected, and the protein was purified by one-step purification using a HisPur™ Ni-NTA Spin Columns (Thermo Scientific, Catalog # 88226) and standard protocols known in the art. The protein was concentrated using a 10 kDa MW cutoff Amicon Ultra Centrifugal Filter Unit (Millipore-Sigma, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 9 - Production of *Pontibacillus halophilus* JSM 076056 OleTPH

*Pontibacillus halophilus* JSM 076056 OleTPH (SEQ ID NO: 121) is a predicted P450 fatty acid decarboxylase that converts medium chain fatty acids (e.g. palmitic acid) into the corresponding terminal olefins and that is included as an example of the current invention. A codon optimized gene (SEQ ID NO: 166) encoding for an OleTPH decarboxylase variant, including an N-terminal amino acid sequence containing a His-tag and a TEV protease cleavage site was designed and synthesized by Genscript. After gene synthesis, the protein was expressed and purified. In brief, the complete synthetic gene sequence was subcloned into a pET30a using the NdeI/XhoI cloning sites. For heterologous expression, *Escherichia coli* BL21 (DE3) cells were transformed with the recombinant plasmid and a single colony was inoculated into LB medium containing kanamycin (50 mg/L). Pre-starter cultures were then inoculated into a fermentor containing Magic Media (ThermoFisher, Catalog # K6803) supplemented with kanamycin (50 mg/L) and incubated at 16 °C for 72 h. At an $OD_{600nm}$ = 0.5 - 1.0, 5-aminolevulinic acid (final concentration 0.5 mM) was added. Cells were harvested by centrifugation at 5000 rpm and 4°C and the pellets were lysed using a bacterial cell lysis buffer (B-PER - ThemoFisher, Waltham, MA). After centrifugation, the supernatant was collected, and the protein was purified by one-step purification using a HisPur™ Ni-NTA Spin Columns (Thermo Scientific, Catalog # 88226) and standard protocols known in the art. The protein was concentrated using a 10 kDa MW cutoff Amicon Ultra Centrifugal Filter Unit (Millipore-Sigma, Catalog# UFC901024), followed by desalting using a disposable PD-10 desalting column (GE Healthcare Life Sciences, Catalog# 17085101) and a buffer containing 50 mM Tris-HCl, 500 mMNaCl, and 10% Glycerol at pH 8.0. The purified enzyme was stored at -80 °C until use.

Example 10 - Production of *Macrococcus sp.* DPC7161 OleTMS

*Macrococcus sp.* DPC7161 OleTMS (SEQ ID NO: 122) is a predicted P450 fatty acid decarboxylase that converts medium chain fatty acids (e.g. palmitic acid) into the corresponding terminal olefins and that is included as an example of the current invention. A codon optimized gene (SEQ ID NO: 167) encoding for an OleTMS decarboxylase variant, including an N-terminal amino acid sequence containing a His-tag and a TEV protease cleavage site was designed and synthesized by Genscript. After gene synthesis, the protein was expressed and purified. In brief, the complete synthetic gene sequence was subcloned into a pET30a using the NdeI/XhoI cloning sites. For heterologous expression, *Escherichia coli* BL21 (DE3) cells were transformed with the recombinant plasmid and a single colony was inoculated into LB medium containing kanamycin (50 mg/L). Pre-starter cultures were then inoculated into a fermentor containing Magic Media (ThermoFisher, Catalog # K6803) supplemented with kanamycin (50 mg/L) and incubated at 16 °C for 72 h. At an $OD_{600nm}$ = 0.5 - 1.0, 5-aminolevulinic acid (final concentration 0.5 mM) was added. Cells were harvested by centrifugation at 5000 rpm and 4°C and the pellets were lysed using a bacterial cell lysis buffer (B-PER -

ThemoFisher, Waltham, MA). After centrifugation, the supernatant was collected, and the protein was purified by one-step purification using a HisPur™ Ni-NTA Spin Columns (Thermo Scientific, Catalog # 88226) and standard protocols known in the art. The protein was concentrated using a 10 kDa MW cutoff Amicon Ultra Centrifugal Filter Unit (Millipore-Sigma, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 11 - Production of *Staphylococcus massiliensis S46* OleTSM

*Staphylococcus massiliensis* S46 OleTSM (SEQ ID NO: 156) is a P450 fatty acid decarboxylase that converts medium chain fatty acids (e.g. palmitic acid) into the corresponding terminal olefins and that is included as an example of the current invention. A codon optimized gene (SEQ ID NO: 168) encoding for an OleTSM decarboxylase variant, without the initial N-terminal 29 amino acids including an N-terminal amino acid sequence containing a His-tag and a TEV protease cleavage site was designed and synthesized by Genscript.
In brief, the complete synthetic gene sequence was subcloned into a pET30a vector using the NdeI and HindIII cloning sites for heterologous expression. *Escherichia coli* C41 (DE3) cells were co-transformed with the recombinant plasmid and with plasmid pTf16. A single colony was inoculated into TB medium containing kanamycin and chloramphenicol. Cultures were incubated at 15 °C for 16 h at 200 rpm and L-arabinose (final concentration 0.1%), δ-aminolevulinic acid (final concentration 0.25 mM) and isopropyl β-D-1-thiogalactopyranoside (IPTG, final concentration 1 mM) were added to induce protein expression. Cells were harvested by centrifugation at 5000 rpm and 4°C and the pellets were lysed by sonication. After centrifugation, the supernatant was collected and the protein was purified by one-step purification using a nickel affinity column and standard protocols known in the art. The protein was stored in a buffer containing 50 mM Tris-HCl, 500 mM NaCl, and 10% Glycerol at pH 8.0. The final protein concentration was 1.65 mg/ mL as determined by Bradford protein assay with BSA as a standard (ThermoFisher, catalog # 23236).

Comparative Example B - Production of *Macrococcus goetzii* OleTMG

*Macrococcus goetzii* OleTMG (SEQ ID NO: 20) is a predicted P450 fatty acid decarboxylase that converts fatty acids into the corresponding terminal olefins and is included as a comparative example of the present invention. A codon optimized gene (SEQ ID NO: 169) encoding for an OleTMG decarboxylase variant, including an N-terminal amino acid sequence containing a His-tag and a TEV protease cleavage site was designed and synthesized by Genscript. After gene synthesis, the protein was expressed and purified. In brief, the complete synthetic gene sequence was subcloned into a pET30a using the NdeI/XhoI cloning sites. For heterologous expression, *Escherichia coli* BL21 (DE3) cells were transformed with the recombinant plasmid and a single colony was inoculated into LB medium containing kanamycin (50 mg/L). Pre-starter cultures were then inoculated into a fermentor containing Magic Media (ThermoFisher, Catalog # K6803) supplemented with kanamycin (50 mg/L) and incubated at 16 °C for 72 h. At an $OD_{600nm}$ = 0.5 - 1.0, 5-aminolevulinic acid (final concentration 0.5 mM) was added. Cells were harvested by centrifugation at 5000 rpm and 4°C and the pellets were lysed using a bacterial cell lysis buffer (B-PER - ThemoFisher, Waltham, MA). After centrifugation, the supernatant was collected, and the protein was purified by one-step purification using a HisPur™ Ni-NTA Spin Columns (Thermo Scientific, Catalog # 88226) and standard protocols known in the art. The protein was concentrated using a 10 kDa MW cutoff Amicon Ultra Centrifugal Filter Unit (MilliporeSigm, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Comparative Example C - Production of *Macrococcus lamae* OleTMA

*Macrococcus lamae* OleTMA (SEQ ID NO: 21) is a predicted P450 fatty acid decarboxylase that converts fatty acids into the corresponding terminal olefins and is included as a comparative example of the present invention. A codon optimized gene (SEQ ID NO: 170) encoding for an OleTMA decarboxylase variant, including an N-terminal amino acid sequence containing a His-tag and a TEV protease cleavage site was designed and synthesized by Genscript. After gene synthesis, the protein was expressed and purified. In brief, the complete synthetic gene sequence was subcloned into a pET30a using the NdeI/XhoI cloning sites. For heterologous expression, *Escherichia coli* BL21 (DE3) cells were transformed with the recombinant plasmid and a single colony was inoculated into LB medium containing kanamycin (50 mg/L). Pre-starter cultures were then inoculated into a fermentor containing Magic Media (ThermoFisher, Catalog # K6803) supplemented with kanamycin (50 mg/L) and incubated at 16 °C for 72 h. At an $OD_{600nm}$ = 0.5 - 1.0, 5-aminolevulinic acid (final concentration 0.5 mM) was added. Cells were harvested by centrifugation at 5000 rpm and 4°C and the pellets were lysed using a bacterial cell lysis buffer (B-PER - ThemoFisher, Waltham, MA). After centrifugation, the supernatant was collected, and the protein was purified by one-step purification using a HisPur™ Ni-NTA Spin

Columns (Thermo Scientific, Catalog # 88226) and standard protocols known in the art. The protein was concentrated using a 10 kDa MW cutoff Amicon Ultra Centrifugal Filter Unit (MilliporeSigm, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Comparative Example D - Production of *Salinicoccus sp. CT19* OleTSS

*Salinicoccus sp. CT19* OleTSS (SEQ ID NO: 42) is a predicted P450 fatty acid decarboxylase that converts fatty acids into the corresponding terminal olefins and is included as a comparative example of the present invention. A codon optimized gene (SEQ ID NO: 171) encoding for an OleTSS decarboxylase variant, including an N-terminal amino acid sequence containing a His-tag and a TEV protease cleavage site was designed and synthesized by Genscript. After gene synthesis, the protein was expressed and purified. In brief, the complete synthetic gene sequence was subcloned into a pET30a using the NdeI/XhoI cloning sites. For heterologous expression, *Escherichia coli* BL21 (DE3) cells were transformed with the recombinant plasmid and a single colony was inoculated into LB medium containing kanamycin (50 mg/L). Pre-starter cultures were then inoculated into a fermentor containing Magic Media (ThermoFisher, Catalog # K6803) supplemented with kanamycin (50 mg/L) and incubated at 16 °C for 72 h. At an $OD_{600nm}$ = 0.5 - 1.0, 5-aminolevulinic acid (final concentration 0.5 mM) was added. Cells were harvested by centrifugation at 5000 rpm and 4°C and the pellets were lysed using a bacterial cell lysis buffer (B-PER - ThemoFisher, Waltham, MA). After centrifugation, the supernatant was collected, and the protein was purified by one-step purification using a HisPur™ Ni-NTA Spin Columns (Thermo Scientific, Catalog # 88226) and standard protocols known in the art. The protein was concentrated using a 10 kDa MW cutoff Amicon Ultra Centrifugal Filter Unit (MilliporeSigm, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Comparative Example E - Production of *Aliicoccus persicus* OleTAP

*Aliicoccus persicus* OleTAP (SEQ ID NO: 84) is a predicted P450 fatty acid decarboxylase that converts fatty acids into the corresponding terminal olefins and is included as a comparative example of the present invention. A codon optimized gene (SEQ ID NO: 172) encoding for an OleTAP decarboxylase variant, including an N-terminal amino acid sequence containing a His-tag and a TEV protease cleavage site was designed and synthesized by Genscript. After gene synthesis, the protein was expressed and purified. In brief, the complete synthetic gene sequence was subcloned into a pET30a using the NdeI/XhoI cloning sites. For heterologous expression, *Escherichia coli* BL21 (DE3) cells were transformed with the recombinant plasmid and a single colony was inoculated into LB medium containing kanamycin (50 mg/L). Pre-starter cultures were then inoculated into a fermentor containing Magic Media (ThermoFisher, Catalog # K6803) supplemented with kanamycin (50 mg/L) and incubated at 16 °C for 72 h. At an $OD_{600nm}$ = 0.5 - 1.0, 5-aminolevulinic acid (final concentration 0.5 mM) was added. Cells were harvested by centrifugation at 5000 rpm and 4°C and the pellets were lysed using a bacterial cell lysis buffer (B-PER - ThemoFisher, Waltham, MA). After centrifugation, the supernatant was collected, and the protein was purified by one-step purification using a HisPur™ Ni-NTA Spin Columns (Thermo Scientific, Catalog # 88226) and standard protocols known in the art. The protein was concentrated using a 10 kDa MW cutoff Amicon Ultra Centrifugal Filter Unit (MilliporeSigm, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Comparative Example F - Production of *Salinicoccus qingdaonensis* OleTSQ

*Salinicoccus qingdaonensis* OleTSQ (SEQ ID NO: 100) is a predicted P450 fatty acid decarboxylase that converts fatty acids into the corresponding terminal olefins and is included as a comparative example of the present invention. A codon optimized gene (SEQ ID NO: 173) encoding for an OleTSQ decarboxylase variant, including an N-terminal amino acid sequence containing a His-tag and a TEV protease cleavage site was designed and synthesized by Genscript. After gene synthesis, the protein was expressed and purified. In brief, the complete synthetic gene sequence was subcloned into a pET30a using the NdeI/XhoI cloning sites. For heterologous expression, *Escherichia coli* BL21 (DE3) cells were transformed with the recombinant plasmid and a single colony was inoculated into LB medium containing kanamycin (50 mg/L). Pre-starter cultures were then inoculated into a fermentor containing Magic Media (ThermoFisher, Catalog # K6803) supplemented with kanamycin (50 mg/L) and incubated at 16 °C for 72 h. At an $OD_{600nm}$ = 0.5 - 1.0, 5-aminolevulinic acid (final concentration 0.5 mM) was added. Cells were harvested by centrifugation at 5000 rpm and 4°C and the pellets were lysed using a bacterial cell lysis buffer (B-PER - ThemoFisher, Waltham, MA). After centrifugation, the supernatant was collected, and the protein was purified by one-step purification using a HisPur™ Ni-NTA Spin Columns (Thermo Scientific, Catalog # 88226) and standard protocols known in the art. The protein was concentrated

using a 10 kDa MW cutoff Amicon Ultra Centrifugal Filter Unit (MilliporeSigm, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 12 - Enzyme activity assay

Reactions of fatty acids with the earlier described OleT enzymes produced as described in examples 1 to 11 and comparative example A, were performed as follows. Aliquots of sodium oleate or sodium linoleate (final concentration 100 μM) and enzyme (final concentration 6 ppm) were resuspended in buffer (50 mM phosphate, 500 mM NaCl, and 10% glycerol at pH 7.4). The reaction was started by addition of hydrogen peroxide (final concentration 220 μM) and the solutions were incubated at 30°C. Aliquots of 100 μL of the reaction solutions were collected at different time points and mixed with 900 μL of isopropyl alcohol to stop the reactions. Analysis of the samples was performed by reversed-phase LC/MS/MS to determine the concentrations of oleate remaining in the solutions. The TON numbers (in $s^{-1}$) were calculated as the ratio between the initial rate of substrate conversion (in μM/s) and the concentration of enzyme (in μM). Finally, the improvement factors were calculated as the ratio of the TON number for the specific enzyme and the TON number for *Jeotgalicoccus* sp. OleTJE (SEQ ID NO: 1). The results are summarized in Table 2.

Table 2. Conversion of sodium oleate by OleT decarboxylases at different time points.

| SEQ ID NO: | Organism | Linoleic - Improv. Factor | Oleic - Improv. Factor |
|---|---|---|---|
| 1* | Jeotgalicoccus sp. | 1.00 | 1.00 |
| 2 | Micrococcus lylae | 86.50 | 76.67 |
| 22 | Macrococcus bovicus | 0.00 | 2.00 |
| 44 | Staphylococcus delphini | 2.55 | 0.86 |
| 60 | Staphylococcus felis | 24.00 | 0.10 |
| 65 | Fictibacillus sp. S7 | 20.50 | 5.11 |
| 71 | Staphylococcus aureus C0673 | 10.35 | 3.22 |
| 83 | Auricoccus indicus | 12.00 | 0.33 |
| 117 | Nosocomiicoccus massiliensis | 5.00 | 0.77 |
| 121 | Pontibacillus halophilus JSM 076056 | 0.00 | 2.33 |
| 122 | Macrococcus sp. DPC7161 | 42.00 | 18.44 |
| 156 | Staphylococcus massiliensis S46 | 3.75 | 2.22 |
| 20* | Macrococcus goetzii | 0.00 | 1.44 |
| 21* | Macrococcus lamae | 0.00 | 1.39 |
| 42* | Salinicoccus sp. CT19 | 0.00 | 1.33 |
| 84* | Aliicoccus persicus | 0.00 | 1.11 |
| 100* | Salinicoccus qingdaonensis | 0.00 | 0.43 |
| * Comparative | | | |

Example 13. Exemplary Manual Dish-Washing Detergent Composition

| Ingredient (levels on active basis) | 2A | 2B | 2C |
|---|---|---|---|
| Sodium alkyl ethoxy sulfate (C1213E00.6S) | 22.91% | 22.91% | 22.91% |
| n-C12-14 Di Methyl Amine Oxide | 7.64% | 7.64% | 7.64% |
| Lutensol XP80 (non-ionic surfactant supplied by BASF) | 0.45% | 0.45% | 0.45% |
| Sodium Chloride | 1.2% | 1.2% | 1.2% |

(continued)

| Ingredient (levels on active basis) | 2A | 2B | 2C |
|---|---|---|---|
| Poly Propylene Glycol (weight average molecular wt. 2000) | 1% | 1% | 1% |
| Ethanol | 2% | 2% | 2% |
| Sodium Hydroxide | 0.24% | 0.24% | 0.24% |
| P450 fatty acid decarboxylase (SEQ ID NO: 2) | 0.1% | 0.1% | 0.1% |
| Hydrogen peroxide | 2.3% | 5.7% | 0.0% |
| Sodium percarbonate | 0.0% | 0.0% | 5.0% |
| Minors (perfume, preservative, dye) + water | To 100 % | To 100 % | To 100 % |

All percentages and ratios given for enzymes are based on active protein. All percentages and ratios herein are calculated by weight unless otherwise indicated. All percentages and ratios are calculated based on the total composition unless otherwise indicated.
It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

**1.** A hand-dishwashing composition which provides good sudsing and a good suds profile even in the presence of greasy stains comprising higher chain-length saturated and/or unsaturated fatty acid chains, the composition comprising:

a) a surfactant system comprising at least one anionic surfactant; and

b) a P450 fatty acid decarboxylase, wherein said decarboxylase comprises a polypeptide sequence having at least about 80% identity to one or more sequences selected from the group consisting of: SEQ ID NO: 2, 60, 65, 71, 83, 122, and their functional fragments thereof.

**2.** The composition according to claim 1, wherein said P450 fatty acid decarboxylase comprises a polypeptide sequence having at least about 85%, 90%, 95%, 98%, 100% identity to one or more sequences selected from the group consisting of: SEQ ID NO: 2, 60, 65, 71, 83, 122, and their functional fragments thereof, preferably SEQ ID NO: 2, 122, and their functional fragments, most preferably SEQ ID NO: 2,.

**3.** The composition according to any preceding claim, wherein the P450 fatty acid decarboxylase is present in an amount of from 0.0001 wt% to 1 wt%, preferably from 0.001 wt% to 0.2 wt%, by weight of the hand dish-washing composition, based on active protein.

**4.** The composition according to any preceding claim, wherein the P450 fatty acid decarboxylase comprises an amino acid selected from the group consisting of: a) valine at position 40, b) alanine, glycine, valine, or isoleucine at position 46, c) valine at position 74, d) lysine at position 252, e) leucine at position 317, and combinations thereof; wherein said positions are numbered with reference to SEQ ID NO: 1.

**5.** The composition according to any preceding claim, wherein the composition further comprises one or more co-enzymes selected from the group consisting of: fatty-acid peroxidases (EC 1.11.1.3), unspecific peroxygenases (EC 1.11.2.1), plant seed peroxygenases (EC 1.11.2.3), fatty acid peroxygenases (EC1.11.2.4), linoleate diol synthases (EC 1.13.11.44), 5,8-linoleate diol synthases (EC 1.13.11.60 and EC 5.4.4.5), 7,8-linoleate diol synthases (EC 1.13.11.60 and EC 5.4.4.6), 9,14-linoleate diol synthases (EC 1.13.11.B1), 8,11-linoleate diol synthases, oleate diol synthases, other linoleate diol synthases, unspecific monooxygenase (EC 1.14.14.1), alkane 1-monooxygenase (EC 1.14.15.3), oleate 12-hydroxylases (EC 1.14.18.4), fatty acid amide hydrolases (EC 3.5.1.99), fatty acid photodecarboxylases (EC 4.1.1.106), oleate hydratases (EC 4.2.1.53), linoleate isomerases (EC 5.2.1.5), linoleate (10E,12Z)-isomerases (EC 5.3.3.B2), non-heme fatty acid decarboxylases (UndA-like), alpha-dioxygenases, amylases, lipases, proteases, cellulases, and mixtures thereof; preferably fatty-acid peroxidases (EC 1.11.1.3), unspecific peroxygenases (EC 1.11.2.1), plant seed peroxygenases (EC 1.11.2.3), and fatty acid peroxygenases (EC 1.11.2.4), non-heme fatty acid decarboxylases (UndA-like), alpha-dioxygenases, and mixtures thereof.

**6.** The composition according to any preceding claims, wherein the composition comprises from 5% to 50%, preferably 8% to 45%, more preferably from 15% to 40%, by weight of the total composition of a surfactant system.

**7.** The composition according to any preceding claims, wherein the anionic surfactant comprises alkyl sulphated anionic surfactant selected from the group consisting of: alkyl sulphate, alkyl alkoxy sulphate, and mixtures thereof.

**8.** The composition according to claim 7, wherein the alkyl sulphated anionic surfactant has an average alkyl chain length of from 8 to 18, preferably from 10 to 14, more preferably from 12 to 14, most preferably from 12 to 13 carbon atoms.

**9.** The composition according to any of claims 7 or 8, wherein the alkyl sulphated anionic surfactant has an average degree of alkoxylation, of less than 5, preferably less than 3, more preferably from 0.5 to 2.0, most preferably from 0.5 to 0.9.

**10.** The composition according to any of claims 7 to 9, wherein the alkyl sulphated anionic surfactant has a weight average degree of branching of more than 10%, preferably more than 20%, more preferably more than 30%, even more preferably between 30% and 60%, most preferably between 30% and 50%.

**11.** The composition according to any preceding claims, wherein the surfactant system further comprises a co-surfactant, wherein the co-surfactant is selected from the group consisting of: an amphoteric surfactant, a zwitterionic surfactant, and mixtures thereof.

**12.** The composition according to claim 10, wherein the co-surfactant is an amphoteric surfactant, preferably an amphoteric surfactant selected from amine oxide surfactant, more preferably wherein the amine oxide surfactant is selected from the group consisting of: alkyl dimethyl amine oxide, alkyl amido propyl dimethyl amine oxide, and mixtures thereof.

**13.** The composition according to any preceding claims, wherein the composition comprises a source of hydrogen peroxide, preferably a source of hydrogen peroxide selected from the group consisting or: hydrogen peroxide, inorganic perhydrate salts, percarbonate, persulphate, perphosphate, persilicate salts, and mixtures thereof.

**14.** A method of manually washing dishware comprising the steps of delivering a detergent composition according to any preceding claims into a volume of water to form a wash solution and immersing the dishware in the solution.

**15.** The method according to claim 14, wherein the P450 fatty acid decarboxylase is present at a concentration of from 0.005 ppm to 15 ppm, preferably from 0.02 ppm to 0.5 ppm, in an aqueous wash liquor during the washing process.

Europäisches Patentamt
European Patent Office
Office européen des brevets

# EUROPEAN SEARCH REPORT

Application Number
EP 20 19 1348

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | EP 3 243 896 A1 (PROCTER & GAMBLE [US]) 15 November 2017 (2017-11-15)<br>* the whole document *<br>----- | 1-15 | INV.<br>C11D3/386<br>C12N9/88 |
| Y | WO 2016/097293 A1 (GLOBAL BIOENERGIES [FR]; SCIENTIST OF FORTUNE SA [LU]) 23 June 2016 (2016-06-23)<br>* sequence 8 *<br>----- | 1-15 | |
| Y | DATABASE UniProt [Online]<br><br>28 March 2018 (2018-03-28), "SubName: Full=Cytochrome P450 {ECO:0000313¦EMBL:AVP36873.1};", XP002802263, retrieved from EBI accession no. UNIPROT:A0A2K3ZEK3<br>Database accession no. A0A2K3ZEK3<br>* sequence *<br>----- | 1-15 | |
| Y | DATABASE UniProt [Online]<br><br>31 July 2019 (2019-07-31), "SubName: Full=Cytochrome P450 {ECO:0000313¦EMBL:RXZ02638.1};", XP002802264, retrieved from EBI accession no. UNIPROT:A0A4Q2I2R6<br>Database accession no. A0A4Q2I2R6<br>* sequence *<br>----- | 1-15 | TECHNICAL FIELDS SEARCHED (IPC)<br>C11D<br>C12N |
| Y | WO 2016/097289 A1 (GLOBAL BIOENERGIES [FR]; SCIENTIST OF FORTUNE SA [LU]) 23 June 2016 (2016-06-23)<br>* sequences 2,15 *<br>-----<br>-/-- | 1-15 | |

The present search report has been drawn up for all claims

1

| Place of search | Date of completion of the search | Examiner |
|---|---|---|
| The Hague | 4 March 2021 | Smalt, Rolf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches Patentamt
European Patent Office
Office européen des brevets

# EUROPEAN SEARCH REPORT

Application Number
EP 20 19 1348

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE UniProt [Online]<br><br>12 April 2017 (2017-04-12), "SubName: Full=Cytochrome {ECO:0000313¦EMBL:AQL56343.1};", XP002802265, retrieved from EBI accession no. UNIPROT:A0A1Q1G1V7 Database accession no. A0A1Q1G1V7<br>* sequence * | 1-15 | |
| Y | DATABASE UniProt [Online]<br><br>31 July 2019 (2019-07-31), "SubName: Full=Cytochrome P450 {ECO:0000313¦EMBL:RXK18941.1};", XP002802266, retrieved from EBI accession no. UNIPROT:A0A4Q1B2S8 Database accession no. A0A4Q1B2S8<br>* sequence * | 1-15 | TECHNICAL FIELDS SEARCHED (IPC) |
| A | CN 108 467 861 A (UNIV YANGZHOU) 31 August 2018 (2018-08-31) | 1 | |
| A | VIVEK S. BHARADWAJ ET AL: "Different Behaviors of a Substrate in P450 Decarboxylase and Hydroxylase Reveal Reactivity-Enabling Actors", SCIENTIFIC REPORTS, vol. 8, no. 1, 27 August 2018 (2018-08-27), XP055682022, DOI: 10.1038/s41598-018-31237-4<br>* the whole document * | 1 | |

-/--

The present search report has been drawn up for all claims

1

| Place of search | Date of completion of the search | Examiner |
|---|---|---|
| The Hague | 4 March 2021 | Smalt, Rolf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 19 1348

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HAMMERER LUCAS ET AL: "Regioselective Biocatalytic Hydroxylation of Fatty Acids by Cytochrome P450s", CATALYSIS LETTERS, J.C. BALTZER, NEW YORK, vol. 148, no. 3, 21 December 2017 (2017-12-21), pages 787-812, XP036440312, ISSN: 1011-372X, DOI: 10.1007/S10562-017-2273-4 [retrieved on 2017-12-21] * the whole document * | 1 | |
| A | HSIEH CHUN H ET AL: "Expanding the substrate scope and reactivity of cytochrome P450 OleT", BIOCHEMICAL AND BIOPHYSICAL RESEARCH COMMUNICATIONS, ELSEVIER, AMSTERDAM, NL, vol. 476, no. 4, 28 May 2016 (2016-05-28), pages 462-466, XP029629207, ISSN: 0006-291X, DOI: 10.1016/J.BBRC.2016.05.145 * the whole document * | 1 | TECHNICAL FIELDS SEARCHED (IPC) |
| A | SARAH MATTHEWS ET AL: "Catalytic Determinants of Alkene Production by the Cytochrome P450 Peroxygenase OleT JE", JOURNAL OF BIOLOGICAL CHEMISTRY, vol. 292, no. 12, 4 January 2017 (2017-01-04), pages 5128-5143, XP055532790, US ISSN: 0021-9258, DOI: 10.1074/jbc.M116.762336 * the whole document * | 1 | |
| T | WO 2020/020992 A1 (TOTAL RAFFINAGE CHIMIE [FR] ET AL.) 30 January 2020 (2020-01-30) * the whole document * | | |

The present search report has been drawn up for all claims

1

| Place of search | Date of completion of the search | Examiner |
|---|---|---|
| The Hague | 4 March 2021 | Smalt, Rolf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 19 1348

This annex lists the patent family members relating to the patent documents cited in the above-mentioned European search report. The members are as contained in the European Patent Office EDP file on
The European Patent Office is in no way liable for these particulars which are merely given for the purpose of information.

04-03-2021

| Patent document cited in search report | Publication date | Patent family member(s) | Publication date |
|---|---|---|---|
| EP 3243896 A1 | 15-11-2017 | CA 3020606 A1 | 16-11-2017 |
| | | CN 109072136 A | 21-12-2018 |
| | | EP 3243896 A1 | 15-11-2017 |
| | | EP 3556834 A1 | 23-10-2019 |
| | | EP 3556835 A1 | 23-10-2019 |
| | | ES 2746120 T3 | 04-03-2020 |
| | | JP 2019513878 A | 30-05-2019 |
| | | PL 3243896 T3 | 31-03-2020 |
| | | PL 3556834 T3 | 08-02-2021 |
| | | US 2017321162 A1 | 09-11-2017 |
| | | WO 2017196786 A1 | 16-11-2017 |
| WO 2016097293 A1 | 23-06-2016 | NONE | |
| WO 2016097289 A1 | 23-06-2016 | NONE | |
| CN 108467861 A | 31-08-2018 | NONE | |
| WO 2020020992 A1 | 30-01-2020 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3243896 B1
- EP 3556834 A
- EP 3243896 A
- US 20090142821 A1
- WO 201219844 A
- WO 201219849 A
- WO 201219848 A
- US 3915903 A
- WO 2007135645 PCT
- US 3959230 A
- US 3893929 A
- US 4702857 A
- US 4711730 A


### Non-patent literature cited in the description


- **S CHRISTOPHER DAVIS et al.** Oxidation of v-Oxo Fatty Acids by Cytochrome P450 BM-3 (CYP102). *ARCHIVES OF BIOCHEMISTRY AND BIOPHYSICS,* 01 April 1996, vol. 328 (1), 35-42
- **JAMES BELCHER et al.** Structure and Biochemical Properties of the Alkene Producing Cytochrome P450 OleTJE (CYP152L1) from the Jeotgalicoccus sp. 8456 Bacterium. *JOURNAL OF BIOLOGICAL CHEMISTRY,* 07 March 2014, vol. 289, ISSN 0021-9258, 6535-6550
- **GIRVAN HAZEL M et al.** Applications of microbial cytochrome P450 enzymes in biotechnology and synthetic biology. *CURRENT OPINION IN CHEMICAL BIOLOGY,* 22 March 2016, vol. 31, ISSN 1367-5931, 136-145
- **J. BELCHER et al.** *J. Biol. Chem.,* 2014, vol. 289 (10), 6535-6550
- **Y. LIU et al.** *Biotechnol. Biofuels,* 2014, vol. 7, 28
- **NEEDLEMAN ; WUNSCH.** *J. Mol. Biol.,* 1970, vol. 48, 443-453
- **HENIKOFF S. ; HENIKOFF J.G.** *P.N.A.S. USA,* 1992, vol. 89, 10915-10919
- **YI et al.** *Proc. Biochem.,* 2007, vol. 42, 895-898
- **MARTIN et al.** *Appl. Microbiol. Biotechnol,* 2007, vol. 76, 843-851
- **KOSZELEWSKI et al.** *J. Mol. Cat. B: Enz.,* 2010, vol. 63, 39-44
- **TRUPPO et al.** *Org. Proc. Res. Develop.*
- **MATEO et al.** *Biotechnol. Prog.,* 2002, vol. 18, 629-34
- **ROBERT LAUGHLIN.** The Aqueous Phase Behaviour of Surfactants. Academic Press, 1994, 538-542
- Kirk Othmer, The Encyclopedia of Chemical Technology. 1978, vol. 3, 128-148
- **KUO-YANN LAI.** *Liquid Detergents,* 278-279

SEQUENCE LISTING

<110>  The Procter & Gamble Company

<120>  DETERGENT COMPOSITION

<130>  CM05132FM

<160>  173

<170>  PatentIn version 3.5

<210>  1
<211>  422
<212>  PRT
<213>  Jeotgalicoccus sp.

<400>  1

```
Met Ala Thr Leu Lys Arg Asp Lys Gly Leu Asp Asn Thr Leu Lys Val
1               5                   10                  15

Leu Lys Gln Gly Tyr Leu Tyr Thr Thr Asn Gln Arg Asn Arg Leu Asn
            20                  25                  30

Thr Ser Val Phe Gln Thr Lys Ala Leu Gly Gly Lys Pro Phe Val Val
        35                  40                  45

Val Thr Gly Lys Glu Gly Ala Glu Met Phe Tyr Asn Asn Asp Val Val
    50                  55                  60

Gln Arg Glu Gly Met Leu Pro Lys Arg Ile Val Asn Thr Leu Phe Gly
65                  70                  75                  80

Lys Gly Ala Ile His Thr Val Asp Gly Lys Lys His Val Asp Arg Lys
                85                  90                  95

Ala Leu Phe Met Ser Leu Met Thr Glu Gly Asn Leu Asn Tyr Val Arg
            100                 105                 110

Glu Leu Thr Arg Thr Leu Trp His Ala Asn Thr Gln Arg Met Glu Ser
        115                 120                 125

Met Asp Glu Val Asn Ile Tyr Arg Glu Ser Ile Val Leu Leu Thr Lys
    130                 135                 140

Val Gly Thr Arg Trp Ala Gly Val Gln Ala Pro Pro Glu Asp Ile Glu
145                 150                 155                 160

Arg Ile Ala Thr Asp Met Asp Ile Met Ile Asp Ser Phe Arg Ala Leu
                165                 170                 175
```

Gly Gly Ala Phe Lys Gly Tyr Lys Ala Ser Lys Glu Ala Arg Arg Arg
180 185 190

Val Glu Asp Trp Leu Glu Glu Gln Ile Ile Glu Thr Arg Lys Gly Asn
195 200 205

Ile His Pro Pro Glu Gly Thr Ala Leu Tyr Glu Phe Ala His Trp Glu
210 215 220

Asp Tyr Leu Gly Asn Pro Met Asp Ser Arg Thr Cys Ala Ile Asp Leu
225 230 235 240

Met Asn Thr Phe Arg Pro Leu Ile Ala Ile Asn Arg Phe Val Ser Phe
245 250 255

Gly Leu His Ala Met Asn Glu Asn Pro Ile Thr Arg Glu Lys Ile Lys
260 265 270

Ser Glu Pro Asp Tyr Ala Tyr Lys Phe Ala Gln Glu Val Arg Arg Tyr
275 280 285

Tyr Pro Phe Val Pro Phe Leu Pro Gly Lys Ala Lys Val Asp Ile Asp
290 295 300

Phe Gln Gly Val Thr Ile Pro Ala Gly Val Gly Leu Ala Leu Asp Val
305 310 315 320

Tyr Gly Thr Thr His Asp Glu Ser Leu Trp Asp Asp Pro Asn Glu Phe
325 330 335

Arg Pro Glu Arg Phe Glu Thr Trp Asp Gly Ser Pro Phe Asp Leu Ile
340 345 350

Pro Gln Gly Gly Gly Asp Tyr Trp Thr Asn His Arg Cys Ala Gly Glu
355 360 365

Trp Ile Thr Val Ile Ile Met Glu Glu Thr Met Lys Tyr Phe Ala Glu
370 375 380

Lys Ile Thr Tyr Asp Val Pro Glu Gln Asp Leu Glu Val Asp Leu Asn
385 390 395 400

Ser Ile Pro Gly Tyr Val Lys Ser Gly Phe Val Ile Lys Asn Val Arg
405 410 415

Glu Val Val Asp Arg Thr
420

<210> 2
<211> 425
<212> PRT
<213> Micrococcus lylae

<400> 2

```
Met Ser Lys Arg Val Pro Lys Asp Arg Gly Ile Asp Asn Ser Leu Lys
1               5                   10                  15

Ile Met Lys Glu Gly Tyr Glu Tyr Val Pro Ala Arg Met Lys Lys Phe
            20                  25                  30

Asn Thr Asn Ile Phe Glu Thr Arg Val Leu Gly Gly Lys Thr Ala Val
        35                  40                  45

Val Ile Ser Gly Lys Asp Ala Ala Glu Leu Phe Tyr Asp Asn Asp Lys
    50                  55                  60

Thr Glu Arg Lys Gly Thr Leu Pro Lys Arg Val Val Lys Thr Leu Phe
65                  70                  75                  80

Gly Lys Gly Ala Ile His Thr Thr Thr Gly Lys Lys His Ile Asp Arg
                85                  90                  95

Lys Ala Leu Phe Met Ser Leu Met Thr Asp Glu Asn Leu Ala Tyr Leu
            100                 105                 110

Arg Lys Leu Thr Arg Ile Tyr Trp Phe Gln Asn Ile Glu His Met Gln
        115                 120                 125

Tyr Lys Gln Lys Val Asn Val Tyr Glu Glu Ala Thr Glu Leu Leu Thr
    130                 135                 140

Lys Val Gly Leu Arg Trp Ala Gly Ile Val Asp His Pro Glu Asn Ile
145                 150                 155                 160

Gln Lys Met Ala Asp Asp Met Asn Lys Met Ile Asp Ser Phe Ser Ala
                165                 170                 175

Ile Gly Ser Leu Tyr Gly Gly Tyr Arg Glu Ala Lys Lys Ala Arg Ala
            180                 185                 190

Arg Val Glu Gln Phe Leu Glu Asp Gln Ile Thr Ala Val Arg Lys Gly
        195                 200                 205

Lys Ile His Pro Glu Lys Gly Thr Ala Leu Tyr Glu Phe Ser His Trp
    210                 215                 220
```

Glu Asp Met Asn Gly Lys Pro Met Asp Ala Arg Leu Cys Ala Val Asp
225 230 235 240

Leu Met Asn Val Ile Arg Pro Leu Val Ala Ile Asn Lys Phe Val Ser
245 250 255

Phe Gly Val Leu Ala Leu His Glu Phe Pro Gly Glu Arg Val Arg Val
260 265 270

Ala Leu Asn Glu Gly Asp Tyr Ala Tyr Lys Phe Val Gln Glu Val Arg
275 280 285

Arg Tyr Tyr Pro Phe Val Pro Phe Leu Pro Gly Lys Ala Lys Glu Asn
290 295 300

Ile Thr Phe Asp Gly Tyr Lys Ile Gln Lys Asp Thr Met Met Leu Leu
305 310 315 320

Asp Ile Tyr Gly Thr Leu His Arg Asp Asp Leu Phe Ser Glu Pro Glu
325 330 335

Arg Phe Asn Pro Tyr Arg Phe Asp Asn Trp Asp Gly Ser Pro Phe Asp
340 345 350

Leu Ile Pro Gln Gly Gly Gly Asp Tyr Tyr Thr Asn His Arg Cys Ala
355 360 365

Gly Glu Trp Met Thr Ile Ile Ile Met Glu Glu Thr Met Lys Phe Phe
370 375 380

Ala Asn Glu Ile Ser Tyr Asp Val Pro Pro Gln Asp Phe Thr Val Asp
385 390 395 400

Thr Thr Lys Phe Pro Gly Lys Val Ala Ser Gly Met Asp Ile Glu Asn
405 410 415

Ile Arg Val Asn Ile Asp Arg Thr Lys
420 425

<210> 3
<211> 425
<212> PRT
<213> Unknown

<220>
<223> Codon optimized artificial DNA sequence

<400> 3

```
Met Ser Lys Arg Val Pro Lys Asp Arg Gly Ile Asp Asn Ser Leu Lys
1               5                   10                  15

Ile Met Lys Glu Gly Tyr Glu Tyr Val Pro Ala Arg Met Lys Lys Phe
            20                  25                  30

Asn Thr Asn Ile Phe Glu Thr Arg Val Leu Gly Gly Lys Thr Ala Val
        35                  40                  45

Val Ile Ser Gly Lys Asp Ala Ala Glu Leu Phe Tyr Asp Asn Asp Lys
    50                  55                  60

Thr Glu Arg Lys Gly Thr Leu Pro Lys Arg Val Val Lys Thr Leu Phe
65                  70                  75                  80

Gly Lys Gly Ala Ile His Thr Thr Thr Gly Lys Lys His Ile Asp Arg
                85                  90                  95

Lys Ala Leu Phe Met Ser Leu Met Thr Asp Glu Asn Leu Ala Tyr Leu
            100                 105                 110

Arg Lys Leu Thr Arg Ile Tyr Trp Phe Gln Asn Ile Glu His Met Gln
        115                 120                 125

Tyr Lys Gln Lys Val Asn Val Tyr Glu Glu Ala Thr Glu Leu Leu Thr
    130                 135                 140

Lys Val Gly Leu Arg Trp Ala Gly Ile Val Asp His Pro Glu Asn Ile
145                 150                 155                 160

Gln Lys Met Ala Asp Asp Met Asn Lys Met Ile Asp Ser Phe Ser Ala
                165                 170                 175

Ile Gly Ser Leu Tyr Gly Gly Tyr Arg Glu Ala Lys Lys Ala Arg Ala
            180                 185                 190

Arg Val Glu Gln Phe Leu Glu Asp Gln Ile Thr Ala Val Arg Lys Gly
        195                 200                 205

Lys Ile His Pro Glu Lys Gly Thr Ala Leu Tyr Glu Phe Ser His Trp
    210                 215                 220

Glu Asp Met Asn Gly Lys Pro Met Asp Ala Arg Leu Cys Ala Val Asp
225                 230                 235                 240

Leu Met Asn Val Ile Arg Pro Leu Val Ala Ile Asn Lys Phe Val Ser
                245                 250                 255
```

Phe Gly Val Leu Ala Leu His Glu Phe Pro Gly Glu Arg Val Arg Val
260 265 270

Ala Leu Asn Glu Gly Asp Tyr Ala Tyr Lys Phe Val Gln Glu Val Arg
275 280 285

Arg Tyr Tyr Pro Phe Val Pro Phe Leu Pro Gly Lys Ala Lys Glu Asn
290 295 300

Ile Thr Phe Asp Gly Tyr Lys Ile Gln Lys Asp Thr Met Met Leu Leu
305 310 315 320

Asp Ile Tyr Gly Thr Leu His Arg Asp Asp Leu Phe Ser Glu Pro Glu
325 330 335

Arg Phe Asn Pro Tyr Arg Phe Glu Asn Trp Asp Gly Ser Pro Phe Asp
340 345 350

Leu Ile Pro Gln Gly Gly Gly Asp Tyr Tyr Thr Asn His Arg Cys Ala
355 360 365

Gly Glu Trp Met Thr Ile Ile Ile Met Glu Glu Thr Met Lys Phe Phe
370 375 380

Ala Asn Glu Ile Ser Tyr Asp Val Pro Pro Gln Asp Phe Thr Val Asp
385 390 395 400

Thr Thr Lys Phe Pro Gly Lys Val Ala Ser Gly Met Asp Ile Glu Asn
405 410 415

Ile Arg Val Asn Ile Asp Arg Thr Lys
420 425

<210> 4
<211> 425
<212> PRT
<213> Macrococcus caseolyticus

<400> 4

Met Ser Lys Arg Val Pro Lys Asp Arg Gly Ile Asp Asn Ser Leu Lys
1 5 10 15

Ile Met Lys Glu Gly Tyr Glu Tyr Val Pro Ala Arg Met Lys Lys Phe
20 25 30

Asn Thr Asn Ile Phe Glu Thr Arg Val Leu Gly Gly Lys Thr Ala Val
35 40 45

```
Val Ile Ser Gly Lys Asp Ala Ala Glu Leu Phe Tyr Asp Asn Asp Lys
    50                  55                  60

Thr Glu Arg Lys Gly Thr Leu Pro Lys Arg Val Val Lys Thr Leu Phe
65                  70                  75                  80

Gly Lys Gly Ala Ile His Thr Thr Thr Gly Lys Lys His Ile Asp Arg
                85                  90                  95

Lys Val Leu Phe Met Ser Leu Met Thr Asp Glu Asn Leu Ala Tyr Leu
            100                 105                 110

Arg Lys Leu Thr Arg Ile Tyr Trp Phe Gln Asn Ile Glu His Met Gln
        115                 120                 125

Tyr Lys Gln Lys Val Asn Val Tyr Glu Glu Ala Thr Glu Leu Leu Thr
    130                 135                 140

Lys Val Gly Leu Arg Trp Ala Gly Ile Val Asp His Pro Glu Asn Ile
145                 150                 155                 160

Gln Lys Met Ala Asp Asp Met Asn Lys Met Ile Asp Ser Phe Ser Ala
                165                 170                 175

Ile Gly Ser Leu Tyr Gly Gly Tyr Arg Glu Ala Lys Lys Ala Arg Ala
            180                 185                 190

Arg Val Glu Gln Phe Leu Glu Asp Gln Ile Thr Ala Val Arg Lys Gly
        195                 200                 205

Lys Ile His Pro Glu Lys Gly Thr Ala Leu Tyr Glu Phe Ser His Trp
    210                 215                 220

Glu Asp Met Asn Gly Lys Pro Met Asp Ala Arg Leu Cys Ala Val Asp
225                 230                 235                 240

Leu Met Asn Val Ile Arg Pro Leu Val Ala Ile Asn Lys Phe Val Ser
                245                 250                 255

Phe Gly Val Leu Ala Leu His Glu Phe Pro Gly Glu Arg Val Arg Val
            260                 265                 270

Ala Leu Asn Glu Gly Asp Tyr Ala Tyr Lys Phe Val Gln Glu Val Arg
        275                 280                 285

Arg Tyr Tyr Pro Phe Val Pro Phe Leu Pro Gly Lys Ala Lys Glu Asn
    290                 295                 300
```

Ile Thr Phe Asp Gly Tyr Lys Ile Gln Lys Asp Thr Met Met Leu Leu
305 310 315 320

Asp Ile Tyr Gly Thr Leu His Arg Asp Asp Leu Phe Ser Glu Pro Glu
325 330 335

Arg Phe Asn Pro Tyr Arg Phe Glu Asn Trp Asp Gly Ser Pro Phe Asp
340 345 350

Leu Ile Pro Gln Gly Gly Gly Asp Tyr Tyr Thr Asn His Arg Cys Ala
355 360 365

Gly Glu Trp Met Thr Ile Ile Ile Met Glu Glu Thr Met Lys Phe Phe
370 375 380

Ala Asn Glu Ile Ser Tyr Asp Val Pro Pro Gln Asp Phe Thr Val Asp
385 390 395 400

Thr Thr Lys Phe Pro Gly Lys Val Ala Ser Gly Met Asp Ile Glu Asn
405 410 415

Ile Arg Val Asn Ile Asp Arg Thr Lys
420 425

<210> 5
<211> 425
<212> PRT
<213> Macrococcus caseolyticus

<400> 5

Met Ser Lys Arg Val Pro Lys Asp Arg Gly Ile Asp Asn Ser Leu Lys
1 5 10 15

Ile Met Lys Glu Gly Tyr Glu Tyr Val Pro Ala Arg Met Lys Lys Phe
20 25 30

Asn Thr Asn Ile Phe Glu Thr Arg Val Leu Gly Gly Lys Thr Ala Val
35 40 45

Val Ile Ser Gly Lys Glu Ala Ala Glu Leu Phe Tyr Asp Asn Asp Lys
50 55 60

Thr Glu Arg Lys Gly Thr Leu Pro Lys Arg Val Val Lys Thr Leu Phe
65 70 75 80

Gly Lys Gly Ala Ile His Thr Thr Thr Gly Lys Lys His Ile Asp Arg
85 90 95

Lys Ala Leu Phe Met Ser Leu Met Thr Asp Glu Asn Leu Ala Tyr Leu
100 105 110

Arg Lys Leu Thr Arg Ile Tyr Trp Phe Gln Asn Ile Glu His Met Gln
115 120 125

Tyr Lys Gln Lys Val Asn Val Tyr Glu Glu Ala Thr Glu Leu Leu Thr
130 135 140

Lys Val Gly Leu Arg Trp Ala Gly Ile Val Asp His Pro Glu Asn Ile
145 150 155 160

Gln Lys Met Ala Asp Asp Met Asn Lys Met Ile Asp Ser Phe Ser Ala
165 170 175

Ile Gly Ser Leu Tyr Gly Gly Tyr Arg Glu Ala Lys Lys Ala Arg Ala
180 185 190

Arg Val Glu Gln Phe Leu Glu Asp Gln Ile Thr Ala Val Arg Lys Gly
195 200 205

Lys Ile His Pro Glu Lys Gly Thr Ala Leu Tyr Glu Phe Ser His Trp
210 215 220

Glu Asp Met Asn Gly Lys Pro Met Asp Ala Arg Leu Cys Ala Val Asp
225 230 235 240

Leu Met Asn Val Ile Arg Pro Leu Val Ala Ile Asn Lys Phe Val Ser
245 250 255

Phe Gly Val Leu Ala Leu His Glu Phe Pro Gly Glu Arg Val Arg Val
260 265 270

Ala Leu Asn Glu Gly Asp Tyr Ala Tyr Lys Phe Val Gln Glu Val Arg
275 280 285

Arg Tyr Tyr Pro Phe Val Pro Phe Leu Pro Gly Lys Ala Lys Glu Asn
290 295 300

Ile Thr Phe Asp Gly Tyr Lys Ile Gln Lys Asp Thr Met Met Leu Leu
305 310 315 320

Asp Ile Tyr Gly Thr Leu His Arg Asp Asp Leu Phe Ser Glu Pro Glu
325 330 335

Arg Phe Asn Pro Tyr Arg Phe Glu Asn Trp Asp Gly Ser Pro Phe Asp

340 345 350

Leu Ile Pro Gln Gly Gly Gly Asp Tyr Tyr Thr Asn His Arg Cys Ala
355 360 365

Gly Glu Trp Met Thr Ile Ile Ile Met Glu Glu Thr Met Lys Phe Phe
370 375 380

Ala Asn Glu Ile Ser Tyr Asp Val Pro Pro Gln Asp Phe Thr Val Asp
385 390 395 400

Thr Thr Lys Phe Pro Gly Lys Val Ala Ser Gly Met Asp Ile Glu Asn
405 410 415

Ile Arg Val Asn Ile Asp Arg Thr Lys
420 425

<210> 6
<211> 425
<212> PRT
<213> Macrococcus caseolyticus

<400> 6

Met Ser Lys Arg Val Pro Lys Asp Arg Gly Ile Asp Asn Ser Leu Lys
1 5 10 15

Ile Met Lys Glu Gly Tyr Glu Tyr Val Pro Ala Arg Met Lys Lys Phe
20 25 30

Asn Thr Asn Ile Phe Glu Thr Arg Val Leu Gly Gly Lys Thr Ala Val
35 40 45

Val Ile Ser Gly Lys Asp Ala Ala Lys Leu Phe Tyr Asp Asn Asp Lys
50 55 60

Thr Glu Arg Lys Gly Thr Leu Pro Lys Arg Val Val Lys Thr Leu Phe
65 70 75 80

Gly Lys Gly Ala Ile His Thr Thr Thr Gly Lys Lys His Ile Asp Arg
85 90 95

Lys Ala Leu Phe Met Ser Leu Met Thr Asp Glu Asn Leu Ala Tyr Leu
100 105 110

Arg Lys Leu Thr Arg Ile Tyr Trp Phe Gln Asn Ile Glu His Met Gln
115 120 125

Tyr Lys Gln Lys Val Asn Val Tyr Glu Glu Ala Thr Glu Leu Leu Thr

```
            130                 135                 140

Lys Val Gly Leu Arg Trp Ala Gly Ile Val Asp His Pro Glu Asn Ile
145                 150                 155                 160

Gln Lys Met Ala Asp Asp Met Asn Lys Met Ile Asp Ser Phe Ser Ala
                165                 170                 175

Ile Gly Ser Leu Tyr Gly Gly Tyr Arg Glu Ala Lys Lys Ala Arg Ala
            180                 185                 190

Arg Val Glu Gln Phe Leu Glu Asp Gln Ile Thr Ala Val Arg Lys Gly
        195                 200                 205

Lys Ile His Pro Glu Lys Gly Thr Ala Leu Tyr Glu Phe Ser His Trp
    210                 215                 220

Glu Glu Met Asn Gly Lys Pro Met Asp Ala Arg Leu Cys Ala Val Asp
225                 230                 235                 240

Leu Met Asn Val Ile Arg Pro Leu Val Ala Ile Asn Lys Phe Val Ser
                245                 250                 255

Phe Gly Val Leu Ala Leu His Glu Phe Pro Gly Glu Arg Val Arg Val
            260                 265                 270

Ala Leu Asn Glu Gly Asp Tyr Ala Tyr Lys Phe Val Gln Glu Val Arg
        275                 280                 285

Arg Tyr Tyr Pro Phe Val Pro Phe Leu Pro Gly Lys Ala Lys Glu Asn
    290                 295                 300

Ile Thr Phe Asp Gly Tyr Lys Ile Gln Lys Asp Thr Met Met Leu Leu
305                 310                 315                 320

Asp Ile Tyr Gly Thr Leu His Arg Asp Asp Leu Phe Ser Glu Pro Glu
                325                 330                 335

Arg Phe Asn Pro Tyr Arg Phe Glu Asn Trp Asp Gly Ser Pro Phe Asp
            340                 345                 350

Leu Ile Pro Gln Gly Gly Gly Asp Tyr Tyr Thr Asn His Arg Cys Ala
        355                 360                 365

Gly Glu Trp Met Thr Ile Ile Ile Met Glu Glu Thr Met Lys Phe Phe
    370                 375                 380
```

Ala Asn Glu Ile Ser Tyr Asp Val Pro Pro Gln Asp Phe Thr Val Asp
385                 390                 395                 400

Thr Thr Lys Phe Pro Gly Lys Val Ala Ser Gly Met Asp Ile Glu Asn
                405                 410                 415

Ile Arg Val Asn Ile Asp Arg Thr Lys
            420                 425

<210>  7
<211>  425
<212>  PRT
<213>  Macrococcus caseolyticus

<400>  7

Met Ser Lys Arg Ile Pro Lys Asp Arg Gly Ile Asp Asn Ser Leu Lys
1               5                   10                  15

Ile Met Lys Glu Gly Tyr Glu Tyr Val Pro Ala Arg Met Lys Lys Phe
            20                  25                  30

Asn Thr Asn Ile Phe Glu Thr Arg Val Leu Gly Gly Lys Thr Ala Val
        35                  40                  45

Val Ile Ser Gly Lys Asp Ala Ala Glu Leu Phe Tyr Asp Asn Asp Lys
    50                  55                  60

Thr Glu Arg Lys Gly Thr Leu Pro Lys Arg Val Val Lys Thr Leu Phe
65                  70                  75                  80

Gly Lys Gly Ala Ile His Thr Thr Thr Gly Lys Lys His Ile Asp Arg
                85                  90                  95

Lys Val Leu Phe Met Ser Leu Met Thr Asp Glu Asn Leu Ala Tyr Leu
            100                 105                 110

Arg Lys Leu Thr Arg Ile Tyr Trp Phe Gln Asn Ile Glu His Met Gln
        115                 120                 125

Tyr Lys Gln Lys Val Asn Val Tyr Glu Glu Ala Thr Glu Leu Leu Thr
    130                 135                 140

Lys Val Gly Leu Arg Trp Ala Gly Ile Val Asp His Pro Glu Asn Ile
145                 150                 155                 160

Gln Lys Met Ala Asp Asp Met Asn Lys Met Ile Asp Ser Phe Ser Ala
                165                 170                 175

```
Ile Gly Ser Leu Tyr Gly Gly Tyr Arg Glu Ala Lys Lys Ala Arg Ala
            180                 185                 190

Arg Val Glu Gln Phe Leu Glu Asp Gln Ile Thr Ala Val Arg Lys Gly
        195                 200                 205

Lys Ile His Pro Glu Lys Gly Thr Ala Leu Tyr Glu Phe Ser His Trp
    210                 215                 220

Glu Asp Met Asn Gly Lys Pro Met Asp Ala Arg Leu Cys Ala Val Asp
225                 230                 235                 240

Leu Met Asn Val Ile Arg Pro Leu Val Ala Ile Asn Lys Phe Val Ser
                245                 250                 255

Phe Gly Val Leu Ala Leu His Glu Phe Pro Gly Glu Arg Val Arg Val
            260                 265                 270

Ala Leu Asn Glu Gly Asp Tyr Ala Tyr Lys Phe Val Gln Glu Val Arg
        275                 280                 285

Arg Tyr Tyr Pro Phe Val Pro Phe Leu Pro Gly Lys Ala Lys Glu Asn
    290                 295                 300

Ile Thr Phe Asp Gly Tyr Lys Ile Gln Lys Asp Thr Met Met Leu Leu
305                 310                 315                 320

Asp Ile Tyr Gly Thr Leu His Arg Asp Asp Leu Phe Ser Glu Pro Glu
                325                 330                 335

Arg Phe Asn Pro Tyr Arg Phe Glu Asn Trp Asp Gly Ser Pro Phe Asp
            340                 345                 350

Leu Ile Pro Gln Gly Gly Gly Asp Tyr Tyr Thr Asn His Arg Cys Ala
        355                 360                 365

Gly Glu Trp Met Thr Ile Ile Ile Met Glu Glu Thr Met Lys Phe Phe
    370                 375                 380

Ala Asn Glu Ile Ser Tyr Asp Val Pro Pro Gln Asp Phe Thr Val Asp
385                 390                 395                 400

Thr Thr Lys Phe Pro Gly Lys Val Ala Ser Gly Met Asp Ile Glu Asn
                405                 410                 415

Ile Arg Val Asn Ile Asp Arg Thr Lys
            420                 425
```

<210> 8
<211> 425
<212> PRT
<213> Macrococcus caseolyticus

<400> 8

Met Ser Lys Arg Val Pro Lys Asp Arg Gly Ile Asp Asn Ser Leu Lys
1 5 10 15

Ile Met Lys Glu Gly Tyr Glu Tyr Val Pro Ala Arg Met Lys Lys Phe
20 25 30

Asn Thr Asn Ile Phe Glu Thr Arg Val Leu Gly Gly Lys Thr Ala Val
35 40 45

Val Ile Ser Gly Lys Asp Ala Ala Glu Leu Phe Tyr Asp Asn Asp Lys
50 55 60

Thr Glu Arg Lys Gly Thr Leu Pro Lys Arg Val Val Lys Thr Leu Phe
65 70 75 80

Gly Lys Gly Ala Ile His Thr Thr Thr Gly Lys Lys His Ile Asp Arg
85 90 95

Lys Ala Leu Phe Met Ser Leu Met Thr Asp Glu Asn Leu Ala Tyr Leu
100 105 110

Arg Lys Leu Thr Arg Ile Tyr Trp Phe Gln Asn Ile Glu His Met Gln
115 120 125

Tyr Lys Gln Lys Val Asn Val Tyr Glu Glu Ala Thr Glu Leu Leu Thr
130 135 140

Lys Val Gly Leu Arg Trp Ala Gly Ile Val Asp His Pro Glu Asn Ile
145 150 155 160

Gln Lys Met Ala Asp Asp Met Asn Lys Met Ile Asp Ser Phe Ser Ala
165 170 175

Ile Gly Ser Leu Tyr Gly Gly Tyr Arg Glu Ala Lys Lys Ala Arg Ala
180 185 190

Arg Val Glu Gln Phe Leu Glu Asp Gln Ile Thr Ala Val Arg Lys Gly
195 200 205

Lys Ile His Pro Glu Lys Gly Thr Ala Leu Tyr Glu Phe Ser His Trp
210 215 220

Glu Asp Met Asn Gly Lys Pro Met Asp Ala Arg Leu Cys Ala Val Asp
225                 230                 235                 240

Leu Met Asn Val Ile Arg Pro Leu Val Ala Ile Asn Lys Phe Val Ser
                245                 250                 255

Phe Gly Val Leu Ala Leu His Glu Phe Pro Gly Glu Arg Val Arg Val
            260                 265                 270

Ala Leu Asn Glu Gly Asp Tyr Ala Tyr Lys Phe Val Gln Glu Val Arg
        275                 280                 285

Arg Tyr Tyr Pro Phe Val Pro Phe Leu Pro Gly Lys Ala Lys Glu Asn
    290                 295                 300

Ile Thr Phe Asn Gly Tyr Lys Ile Gln Lys Asp Thr Met Met Leu Leu
305                 310                 315                 320

Asp Ile Tyr Gly Thr Leu His Arg Asp Asp Leu Phe Ser Glu Pro Glu
                325                 330                 335

Arg Phe Asn Pro Tyr Arg Phe Glu Asn Trp Asp Gly Ser Pro Phe Asp
            340                 345                 350

Leu Ile Pro Gln Gly Gly Gly Asp Tyr Tyr Thr Asn His Arg Cys Ala
        355                 360                 365

Gly Glu Trp Met Thr Ile Ile Ile Met Glu Glu Thr Met Lys Phe Phe
    370                 375                 380

Ala Asn Glu Ile Ser Tyr Asp Val Pro Pro Gln Asp Phe Thr Val Asp
385                 390                 395                 400

Thr Thr Asn Phe Pro Gly Lys Val Ala Ser Gly Met Asp Ile Glu Asn
                405                 410                 415

Ile Arg Val Asn Ile Asp Arg Thr Lys
            420                 425

<210> 9
<211> 425
<212> PRT
<213> Macrococcus caseolyticus

<400> 9

Met Ser Lys Arg Val Pro Lys Asp Arg Gly Ile Asp Asn Ser Leu Lys
1               5                   10                  15

```
Ile Met Lys Glu Gly Tyr Glu Tyr Val Pro Ala Arg Met Lys Lys Phe
            20                  25                  30

Asn Thr Asn Ile Phe Glu Thr Arg Val Leu Gly Gly Lys Thr Ala Val
        35                  40                  45

Val Ile Ser Gly Lys Asp Ala Ala Glu Leu Phe Tyr Asp Asn Asp Lys
    50                  55                  60

Thr Glu Arg Lys Gly Thr Leu Pro Lys Arg Val Val Lys Thr Leu Phe
65                  70                  75                  80

Gly Lys Gly Ala Ile His Thr Thr Thr Gly Lys Lys His Ile Asp Arg
                85                  90                  95

Lys Ala Leu Phe Met Ser Leu Met Thr Asp Glu Asn Leu Ala Tyr Leu
            100                 105                 110

Arg Lys Leu Thr Arg Ile Tyr Trp Phe Gln Asn Ile Glu His Met Gln
        115                 120                 125

Tyr Lys Gln Lys Val Asn Val Tyr Glu Glu Ala Thr Glu Leu Leu Thr
    130                 135                 140

Lys Val Gly Leu Arg Trp Ala Gly Ile Thr Asp Asn Pro Asp Asn Ile
145                 150                 155                 160

Lys Lys Met Ala Asp Asp Met Asn Lys Met Ile Asp Ser Phe Ser Ala
                165                 170                 175

Ile Gly Ser Leu Tyr Gly Gly Tyr Arg Glu Ala Lys Lys Ala Arg Ala
            180                 185                 190

Arg Val Glu Gln Phe Leu Glu Asp Gln Ile Thr Ala Val Arg Lys Gly
        195                 200                 205

Lys Ile His Pro Glu Lys Gly Thr Ala Leu Tyr Glu Phe Ser His Trp
    210                 215                 220

Glu Asp Met Asn Glu Lys Pro Met Asp Ala Arg Leu Cys Ala Val Asp
225                 230                 235                 240

Leu Met Asn Val Ile Arg Pro Leu Val Ala Ile Asn Lys Phe Val Ser
                245                 250                 255

Phe Gly Val Leu Ala Leu His Glu Phe Pro Gly Glu Arg Val Arg Val
            260                 265                 270
```

Ala Leu Asn Glu Glu Asp Tyr Ala Tyr Lys Phe Val Gln Glu Val Arg
275 280 285

Arg Tyr Tyr Pro Phe Val Pro Phe Leu Pro Gly Lys Ala Lys Glu Asn
290 295 300

Ile Thr Phe Asp Gly Tyr Lys Ile Gln Lys Asp Thr Met Met Ile Leu
305 310 315 320

Asp Ile Tyr Gly Thr Leu His Arg Asp Asp Leu Phe Ser Glu Pro Glu
325 330 335

Arg Phe Asn Pro Tyr Arg Phe Glu Asn Trp Asp Gly Ser Pro Phe Asp
340 345 350

Leu Ile Pro Gln Gly Gly Gly Asp Tyr Tyr Thr Asn His Arg Cys Ala
355 360 365

Gly Glu Trp Met Thr Ile Ile Ile Met Glu Glu Thr Met Lys Phe Phe
370 375 380

Ala Asn Glu Ile Ser Tyr Asp Val Pro Pro Gln Asp Phe Thr Val Asp
385 390 395 400

Thr Thr Lys Phe Pro Gly Lys Val Ala Ser Gly Met Asp Ile Glu Asn
405 410 415

Ile Arg Val Asn Ile Asp Arg Thr Gln
420 425

<210> 10
<211> 425
<212> PRT
<213> Macrococcus caseolyticus

<400> 10

Met Ser Lys Arg Ile Pro Lys Asp Arg Gly Ile Asp Asn Ser Phe Lys
1 5 10 15

Ile Met Lys Glu Gly Tyr Glu Tyr Val Pro Ala Arg Met Lys Lys Phe
20 25 30

Asn Thr Asn Ile Phe Glu Thr Arg Val Leu Gly Gly Lys Ala Ala Val
35 40 45

Val Ile Ser Gly Lys Asp Ala Ala Glu Leu Phe Tyr Asp Asn Asp Lys
50 55 60

Thr Glu Arg Lys Gly Thr Leu Pro Lys Arg Val Val Lys Thr Leu Phe
65 70 75 80

Gly Lys Gly Ala Ile His Thr Thr Thr Gly Lys Lys His Ile Asp Arg
85 90 95

Lys Ala Leu Phe Met Ser Leu Met Thr Asp Glu Asn Leu Ala Tyr Leu
100 105 110

Arg Lys Leu Thr Arg Ile Tyr Trp Phe Gln Asn Ile Glu His Met Gln
115 120 125

Tyr Lys Gln Lys Val Asn Val Tyr Glu Glu Ala Thr Glu Leu Leu Thr
130 135 140

Lys Val Gly Leu Arg Trp Ala Gly Ile Thr Asp Asn Pro Asp Asn Ile
145 150 155 160

Lys Lys Met Ala Asp Asp Met Asn Lys Met Ile Asp Ser Phe Ser Ala
165 170 175

Ile Gly Ser Leu Tyr Gly Gly Tyr Arg Glu Ala Lys Lys Ala Arg Ala
180 185 190

Arg Val Glu Gln Phe Leu Glu Asp Gln Ile Thr Ala Val Arg Lys Gly
195 200 205

Lys Ile His Pro Glu Lys Gly Thr Ala Leu Tyr Glu Phe Ser His Trp
210 215 220

Glu Asp Met Asn Gly Lys Pro Met Asp Ala Arg Leu Cys Ala Val Asp
225 230 235 240

Leu Met Asn Val Ile Arg Pro Leu Val Ala Ile Asn Lys Phe Val Ser
245 250 255

Phe Gly Val Leu Ala Leu His Glu Phe Pro Gly Glu Arg Val Arg Val
260 265 270

Ala Leu Asn Glu Glu Asp Tyr Ala Tyr Lys Phe Val Gln Glu Val Arg
275 280 285

Arg Tyr Tyr Pro Phe Val Pro Phe Leu Pro Gly Lys Ala Lys Glu Asn
290 295 300

Ile Thr Phe Asp Gly Tyr Lys Ile Gln Lys Asp Thr Met Met Ile Leu

305 310 315 320

Asp Ile Tyr Gly Thr Leu His Arg Asp Asp Leu Phe Ser Glu Pro Glu
325 330 335

Arg Phe Asn Pro Tyr Arg Phe Glu Asn Trp Asp Gly Ser Pro Phe Asp
340 345 350

Leu Ile Pro Gln Gly Gly Gly Asp Tyr Tyr Thr Asn His Arg Cys Ala
355 360 365

Gly Glu Trp Met Thr Ile Ile Ile Met Glu Glu Thr Met Lys Phe Phe
370 375 380

Ala Asn Glu Ile Ser Tyr Asp Val Pro Pro Gln Asp Phe Thr Val Asp
385 390 395 400

Thr Thr Lys Phe Pro Gly Lys Val Ala Ser Gly Met Asp Ile Glu Asn
405 410 415

Ile Arg Val Asn Ile Asp Arg Thr Gln
420 425

<210> 11
<211> 425
<212> PRT
<213> Macrococcus canis

<400> 11

Met Ser Lys Arg Ile Pro Lys Asp Arg Gly Ile Asp Asn Ser Leu Lys
1 5 10 15

Ile Met Lys Glu Gly Tyr Glu Tyr Val Pro Ala Arg Met Lys Lys Phe
20 25 30

Asn Thr Asn Ile Phe Glu Thr His Val Leu Gly Gly Lys Thr Ala Val
35 40 45

Val Ile Ser Gly Lys Glu Ala Ala Glu Leu Phe Tyr Asp Asn Asp Lys
50 55 60

Thr Glu Arg Lys Gly Thr Leu Pro Lys Arg Val Val Lys Thr Leu Phe
65 70 75 80

Gly Lys Gly Ala Ile His Thr Thr Ala Gly Lys Thr His Ile Asp Arg
85 90 95

Lys Ala Leu Phe Met Ser Leu Met Thr Asp Glu Asn Leu Ala Tyr Leu

100 105 110

Arg Lys Leu Thr Arg Asn Tyr Trp Phe Gln Asn Ile Glu His Met Gln
115 120 125

Tyr Lys Gln Lys Val Asn Val Tyr Glu Glu Ala Thr Glu Leu Leu Thr
130 135 140

Lys Val Gly Leu Arg Trp Ala Gly Ile Val Asp Asn Pro Asp Asn Ile
145 150 155 160

Lys Lys Met Ala Asp Asp Met Asn Lys Met Ile Asp Ser Phe Ser Ala
165 170 175

Ile Gly Ser Leu Tyr Gly Gly Tyr Arg Glu Ala Lys Lys Ala Arg Ala
180 185 190

Arg Val Glu Gln Phe Leu Glu Asp Gln Ile Thr Ala Val Arg Lys Gly
195 200 205

Lys Ile His Pro Glu Lys Gly Thr Ala Leu Tyr Glu Phe Ser His Trp
210 215 220

Glu Asp Met Asn Gly Lys Pro Met Asp Ala Arg Leu Cys Ala Val Asp
225 230 235 240

Leu Met Asn Val Ile Arg Pro Leu Val Ala Ile Asn Lys Phe Val Ser
245 250 255

Phe Gly Val Leu Ala Leu His Glu Phe Pro Gly Glu Arg Val Arg Val
260 265 270

Ala Leu Asn Glu Glu Asp Tyr Ala Tyr Lys Phe Val Gln Glu Val Arg
275 280 285

Arg Tyr Tyr Pro Phe Val Pro Phe Leu Pro Gly Lys Ala Lys Glu Asn
290 295 300

Ile Thr Phe Asp Gly Tyr Lys Ile Lys Lys Asp Thr Met Met Ile Leu
305 310 315 320

Asp Ile Tyr Gly Thr Leu His Arg Asp Asp Leu Phe Ser Glu Pro Asp
325 330 335

Arg Phe Asn Pro Tyr Arg Phe Asp Asn Trp Asp Gly Ser Pro Phe Asp
340 345 350

```
Leu Ile Pro Gln Gly Gly Gly Asp Tyr Tyr Thr Asn His Arg Cys Ala
        355                 360                 365

Gly Glu Trp Met Thr Ile Ile Ile Met Glu Glu Thr Met Lys Phe Phe
    370                 375                 380

Ala Asn Glu Ile Ser Tyr Asp Val Pro Pro Gln Asp Phe Thr Val Asp
385                 390                 395                 400

Thr Thr Lys Phe Pro Gly Lys Val Ala Ser Gly Met Asp Ile Glu Asn
                405                 410                 415

Ile Arg Val Asn Ile Asp Arg Thr Lys
            420                 425


<210>  12
<211>  425
<212>  PRT
<213>  Macrococcus canis

<400>  12

Met Ser Lys Arg Ile Pro Lys Asp Arg Gly Ile Asp Asn Ser Leu Lys
1               5                   10                  15

Ile Met Lys Glu Gly Tyr Glu Tyr Val Pro Ala Arg Met Lys Lys Phe
            20                  25                  30

Asn Thr Asn Ile Phe Glu Thr His Val Leu Gly Gly Lys Thr Ala Val
        35                  40                  45

Val Ile Ser Gly Lys Glu Ala Ala Glu Leu Phe Tyr Asp Asn Asp Lys
    50                  55                  60

Thr Glu Arg Lys Gly Thr Leu Pro Lys Arg Val Val Lys Thr Leu Phe
65                  70                  75                  80

Gly Lys Gly Ala Ile His Thr Thr Ala Gly Lys Thr His Ile Asp Arg
                85                  90                  95

Lys Ala Leu Phe Met Ser Leu Met Thr Asp Glu Asn Leu Ala Tyr Leu
            100                 105                 110

Arg Lys Leu Thr Arg Asn Tyr Trp Phe Gln Asn Ile Glu His Met Gln
        115                 120                 125

Tyr Lys Gln Lys Val Asn Val Tyr Glu Glu Ala Thr Glu Leu Leu Thr
    130                 135                 140
```

```
Lys Val Gly Leu Arg Trp Ala Gly Ile Val Asp Asn Pro Asp Asn Ile
145                 150                 155                 160

Lys Lys Met Ala Asp Asp Met Asn Lys Met Ile Asp Ser Phe Ser Ala
                165                 170                 175

Ile Gly Ser Leu Tyr Gly Gly Tyr Arg Glu Ala Lys Lys Ala Arg Ala
            180                 185                 190

Arg Val Glu Gln Phe Leu Glu Asp Gln Ile Thr Ala Val Arg Lys Gly
        195                 200                 205

Lys Leu His Pro Glu Lys Gly Thr Ala Leu Tyr Glu Phe Ser His Trp
    210                 215                 220

Glu Asp Met Asn Gly Lys Pro Met Asp Ala Arg Leu Cys Ala Val Asp
225                 230                 235                 240

Leu Met Asn Val Ile Arg Pro Leu Val Ala Ile Asn Lys Phe Val Ser
                245                 250                 255

Phe Gly Val Leu Ala Leu His Glu Phe Pro Gly Glu Arg Val Arg Val
            260                 265                 270

Ala Leu Asn Glu Glu Asp Tyr Ala Tyr Lys Phe Val Gln Glu Val Arg
        275                 280                 285

Arg Tyr Tyr Pro Phe Val Pro Phe Leu Pro Gly Lys Ala Lys Glu Asn
    290                 295                 300

Ile Thr Phe Asp Gly Tyr Lys Ile Lys Lys Asp Thr Leu Met Ile Leu
305                 310                 315                 320

Asp Ile Tyr Gly Thr Leu His Arg Asp Asp Leu Phe Ser Glu Pro Glu
                325                 330                 335

Arg Phe Asn Pro Tyr Arg Phe Asp Asn Trp Asp Gly Ser Pro Phe Asp
            340                 345                 350

Leu Ile Pro Gln Gly Gly Gly Asp Tyr Tyr Thr Asn His Arg Cys Ala
        355                 360                 365

Gly Glu Trp Met Thr Ile Ile Ile Met Glu Glu Thr Met Lys Phe Phe
    370                 375                 380

Ala Asn Glu Ile Ser Tyr Asp Val Pro Pro Gln Asp Phe Thr Val Asp
385                 390                 395                 400
```

Thr Thr Lys Phe Pro Gly Lys Val Ala Ser Gly Met Asp Ile Glu Asn
405 410 415

Ile Arg Val Asn Ile Asp Arg Thr Lys
420 425

<210> 13
<211> 425
<212> PRT
<213> Macrococcus canis

<400> 13

Met Ser Lys Arg Ile Pro Lys Asp Arg Gly Ile Asp Asn Ser Leu Lys
1 5 10 15

Ile Met Lys Glu Gly Tyr Glu Tyr Val Pro Ala Arg Met Lys Lys Phe
20 25 30

Asn Thr Asn Ile Phe Glu Thr His Val Leu Gly Gly Lys Thr Ala Val
35 40 45

Val Ile Ser Gly Lys Glu Ala Ala Glu Leu Phe Tyr Asp Asn Asp Lys
50 55 60

Thr Glu Arg Lys Gly Thr Leu Pro Lys Arg Val Val Lys Thr Leu Phe
65 70 75 80

Gly Lys Gly Ala Ile His Thr Thr Ala Gly Lys Thr His Ile Asp Arg
85 90 95

Lys Ala Leu Phe Met Ser Leu Met Thr Asp Glu Asn Leu Ala Tyr Leu
100 105 110

Arg Lys Leu Thr Arg Asn Tyr Trp Phe Gln Asn Ile Glu His Met Gln
115 120 125

Tyr Lys Gln Lys Val Asn Val Tyr Glu Glu Ala Thr Glu Leu Leu Thr
130 135 140

Lys Val Gly Leu Arg Trp Ala Gly Ile Val Asp Asn Pro Asp Asn Ile
145 150 155 160

Lys Lys Met Ala Asp Asp Met Asn Lys Met Ile Asp Ser Phe Ser Ala
165 170 175

Ile Gly Ser Leu Tyr Gly Gly Tyr Arg Glu Ala Lys Lys Ala Arg Ala
180 185 190

Arg Val Glu Gln Phe Leu Glu Asp Gln Ile Thr Ala Val Arg Lys Gly
        195                 200                 205

Lys Leu His Pro Glu Lys Gly Thr Ala Leu Tyr Glu Phe Ser His Trp
    210                 215                 220

Glu Asp Met Asn Gly Lys Pro Met Asp Ala Arg Leu Cys Ala Val Asp
225                 230                 235                 240

Leu Met Asn Val Ile Arg Pro Leu Val Ala Ile Asn Lys Phe Val Ser
                245                 250                 255

Phe Gly Val Leu Ala Leu His Glu Phe Pro Gly Glu Arg Val Arg Val
            260                 265                 270

Ala Leu Asn Glu Glu Asp Tyr Ala Tyr Lys Phe Val Gln Glu Val Arg
        275                 280                 285

Arg Tyr Tyr Pro Phe Val Pro Phe Leu Pro Gly Lys Ala Lys Glu Asn
    290                 295                 300

Ile Thr Phe Asp Gly Tyr Lys Ile Lys Lys Asp Thr Leu Met Ile Leu
305                 310                 315                 320

Asp Ile Tyr Gly Thr Leu His Arg Asp Asp Leu Phe Gly Glu Pro Glu
                325                 330                 335

Arg Phe Asn Pro Tyr Arg Phe Asp Asn Trp Asp Gly Ser Pro Phe Asp
            340                 345                 350

Leu Ile Pro Gln Gly Gly Gly Asp Tyr Tyr Thr Asn His Arg Cys Ala
        355                 360                 365

Gly Glu Trp Met Thr Ile Ile Ile Met Glu Glu Thr Met Lys Phe Phe
    370                 375                 380

Ala Asn Glu Ile Ser Tyr Asp Val Pro Pro Gln Asp Phe Thr Val Asp
385                 390                 395                 400

Thr Thr Lys Phe Pro Gly Lys Val Ala Ser Gly Met Asp Ile Glu Asn
                405                 410                 415

Ile Arg Val Asn Ile Asp Arg Thr Lys
            420                 425

<210> 14
<211> 425

<212> PRT
<213> Macrococcus canis

<400> 14

Met Ser Lys Arg Ile Pro Lys Asp Arg Gly Ile Asp Asn Ser Leu Lys
1 5 10 15

Ile Met Lys Glu Gly Tyr Glu Tyr Val Pro Ala Arg Met Lys Lys Phe
20 25 30

Asn Thr Asn Ile Phe Glu Thr His Val Leu Gly Gly Lys Thr Ala Val
35 40 45

Val Ile Ser Gly Lys Glu Ala Ala Glu Leu Phe Tyr Asp Asn Asn Lys
50 55 60

Thr Glu Arg Lys Gly Thr Leu Pro Lys Arg Val Val Lys Thr Leu Phe
65 70 75 80

Gly Lys Gly Ala Ile His Thr Thr Ala Gly Lys Thr His Ile Asp Arg
85 90 95

Lys Ala Leu Phe Met Ser Leu Met Thr Asp Glu Asn Leu Ala Tyr Leu
100 105 110

Arg Lys Leu Thr Arg Asn Tyr Trp Phe Gln Asn Ile Glu His Met Gln
115 120 125

Tyr Lys Gln Lys Val Asn Val Tyr Glu Glu Ala Thr Glu Leu Leu Thr
130 135 140

Lys Val Gly Leu Arg Trp Ala Gly Ile Val Asp Asn Pro Asp Asn Ile
145 150 155 160

Lys Lys Met Ala Asp Asp Met Asn Lys Met Ile Asp Ser Phe Ser Ala
165 170 175

Ile Gly Ser Leu Tyr Gly Gly Tyr Arg Glu Ala Lys Lys Ala Arg Ala
180 185 190

Arg Val Glu Gln Phe Leu Glu Asp Gln Ile Thr Ala Val Arg Lys Gly
195 200 205

Lys Leu His Pro Glu Lys Gly Thr Ala Leu Tyr Glu Phe Ser His Trp
210 215 220

Glu Asp Met Asn Gly Lys Pro Met Asp Ala Arg Leu Cys Ala Val Asp
225 230 235 240

Leu Met Asn Val Ile Arg Pro Leu Val Ala Ile Asn Lys Phe Val Ser
245 250 255

Phe Gly Val Leu Ala Leu His Glu Phe Pro Gly Glu Arg Val Arg Val
260 265 270

Ala Leu Asn Glu Glu Asp Tyr Ala Tyr Lys Phe Val Gln Glu Val Arg
275 280 285

Arg Tyr Tyr Pro Phe Val Pro Phe Leu Pro Gly Lys Ala Lys Glu Asn
290 295 300

Ile Thr Phe Asp Gly Tyr Lys Ile Lys Lys Asp Thr Leu Met Ile Leu
305 310 315 320

Asp Ile Tyr Gly Thr Leu His Arg Asp Asp Leu Phe Gly Glu Pro Glu
325 330 335

Arg Phe Asn Pro Tyr Arg Phe Asp Asn Trp Asp Gly Ser Pro Phe Asp
340 345 350

Leu Ile Pro Gln Gly Gly Gly Asp Tyr Tyr Thr Asn His Arg Cys Ala
355 360 365

Gly Glu Trp Met Thr Ile Ile Ile Met Glu Glu Thr Met Lys Phe Phe
370 375 380

Ala Asn Glu Ile Ser Tyr Asp Val Pro Pro Gln Asp Phe Thr Val Asp
385 390 395 400

Thr Thr Lys Phe Pro Gly Lys Val Ala Ser Gly Met Asp Ile Glu Asn
405 410 415

Ile Arg Val Asn Ile Asp Arg Thr Lys
420 425

<210> 15
<211> 425
<212> PRT
<213> Macrococcus canis

<400> 15

Met Ser Lys Arg Ile Pro Lys Asp Arg Gly Ile Asp Asn Ser Leu Lys
1 5 10 15

Ile Met Lys Glu Gly Tyr Glu Tyr Val Pro Ala Arg Met Lys Lys Phe
20 25 30

Asn Thr Asn Ile Phe Glu Thr His Val Leu Gly Gly Lys Thr Ala Val
35 40 45

Val Ile Ser Gly Lys Glu Ala Ala Glu Leu Phe Tyr Asp Asn Asp Lys
50 55 60

Thr Glu Arg Lys Gly Thr Leu Pro Lys Arg Val Val Lys Thr Leu Phe
65 70 75 80

Gly Lys Gly Ala Ile His Thr Thr Ala Gly Lys Thr His Ile Asp Arg
85 90 95

Lys Ala Leu Phe Met Ser Leu Met Thr Asp Glu Asn Leu Ala Tyr Leu
100 105 110

Arg Lys Leu Thr Arg Asn Tyr Trp Phe Gln Asn Ile Glu His Met Gln
115 120 125

Tyr Lys Gln Lys Val Asn Val Tyr Glu Glu Ala Thr Glu Leu Leu Thr
130 135 140

Lys Val Gly Leu Arg Trp Ala Gly Ile Val Asp Asn Pro Asp Ser Ile
145 150 155 160

Lys Lys Met Ala Asp Asp Met Asn Lys Met Ile Asp Ser Phe Ser Ala
165 170 175

Ile Gly Ser Leu Tyr Gly Gly Tyr Arg Glu Ala Lys Lys Ala Arg Ala
180 185 190

Arg Val Glu Gln Phe Leu Glu Asp Gln Ile Thr Ala Val Arg Lys Gly
195 200 205

Lys Leu His Pro Glu Lys Gly Thr Ala Leu Tyr Glu Phe Ser His Trp
210 215 220

Glu Asp Met Asn Gly Lys Pro Met Asp Ala Arg Leu Cys Ala Val Asp
225 230 235 240

Leu Met Asn Val Ile Arg Pro Leu Val Ala Ile Asn Lys Phe Val Ser
245 250 255

Phe Gly Val Leu Ala Leu His Glu Phe Pro Gly Glu Arg Val Arg Val
260 265 270

Ala Leu Asn Glu Glu Asp Tyr Ala Tyr Lys Phe Val Gln Glu Val Arg

275 280 285

Arg Tyr Tyr Pro Phe Val Pro Phe Leu Pro Gly Lys Ala Lys Glu Asn
290 295 300

Ile Thr Phe Asp Gly Tyr Lys Ile Lys Lys Asp Thr Leu Met Ile Leu
305 310 315 320

Asp Ile Tyr Gly Thr Leu His Arg Asp Asp Leu Phe Gly Glu Pro Glu
325 330 335

Arg Phe Asn Pro Tyr Arg Phe Asp Asn Trp Asp Gly Ser Pro Phe Asp
340 345 350

Leu Ile Pro Gln Gly Gly Gly Asp Tyr Tyr Thr Asn His Arg Cys Ala
355 360 365

Gly Glu Trp Met Thr Ile Ile Ile Met Glu Glu Thr Met Lys Phe Phe
370 375 380

Ala Asn Glu Ile Ser Tyr Asp Val Pro Pro Gln Asp Phe Thr Val Asp
385 390 395 400

Thr Thr Lys Phe Pro Gly Lys Val Ala Ser Gly Met Asp Ile Glu Asn
405 410 415

Ile Arg Val Asn Ile Asp Arg Thr Lys
420 425

<210> 16
<211> 425
<212> PRT
<213> Macrococcus sp.

<400> 16

Met Ala Lys Arg Met Pro Lys Asp Arg Gly Leu Asp Asn Thr Leu Thr
1 5 10 15

Ile Met Lys Glu Gly Tyr Glu Tyr Ile Gln Asn Arg Thr Lys Lys Phe
20 25 30

Asn Ser Asn Val Phe Glu Thr Arg Val Leu Gly Gly Lys Gln Ala Val
35 40 45

Val Ile Ser Gly Lys Gln Ala Ala Glu Leu Phe Tyr Asp Asn Asp Lys
50 55 60

Ile Glu Arg Ser Gly Thr Leu Pro Pro Arg Val Val Lys Thr Leu Phe

65 70 75 80

Gly Lys Gly Ala Ile His Thr Thr Thr Gly Lys Lys His Ile Asp Arg
85 90 95

Lys Ala Leu Phe Met Ser Leu Met Thr Asp Glu Asn Leu Thr Tyr Leu
100 105 110

Arg Lys Leu Thr Arg Asn Tyr Trp Phe Gln Asn Thr Gln Arg Met Gln
115 120 125

Leu Met Asp Glu Val Asn Val Tyr Lys Glu Ser Ile Ile Leu Leu Thr
130 135 140

Lys Val Gly Phe Arg Trp Ala Gly Ile Ile Glu Thr Pro Glu Asn Ile
145 150 155 160

Glu Gln His Ala Met Asp Met Asp Lys Met Ile Asp Ser Phe Gly Ala
165 170 175

Ile Gly Ser Pro Tyr Lys Gly Tyr Arg Glu Ala Lys Lys Ala Arg Ala
180 185 190

Arg Val Glu Asp Phe Leu Glu Glu Gln Ile Ile Lys Thr Arg Lys Gly
195 200 205

Lys Ile His Pro Glu Pro Gly Ser Ala Leu Tyr Glu Phe Ser His Trp
210 215 220

Glu Asp Met Asn Gly Lys Pro Met Asp Ser Arg Leu Cys Ser Val Asp
225 230 235 240

Leu Met Asn Val Ile Arg Pro Leu Val Ala Ile Asn Arg Phe Val Ala
245 250 255

Tyr Gly Val Leu Ala Met His Glu Phe Pro Gly Glu Arg Thr Arg Val
260 265 270

Ala Arg Asp Asp Asn Asp Tyr Ala Tyr Lys Phe Val Gln Glu Val Arg
275 280 285

Arg Tyr Tyr Pro Phe Val Pro Phe Leu Pro Gly Lys Ala Ala Lys Asn
290 295 300

Ile Glu Phe Asp Gly Tyr Lys Ile Lys Lys Asp Thr Met Ile Ile Leu
305 310 315 320

Asp Val Phe Gly Thr Leu His Arg Glu Asp Thr Phe Ser Glu Pro Glu
325 330 335

Arg Phe Asn Pro Tyr Arg Phe Asp Asn Trp Asp Gly Ser Pro Phe Asp
340 345 350

Leu Ile Pro Gln Gly Gly Gly Asp Tyr Tyr Thr Asn His Arg Cys Ala
355 360 365

Gly Glu Trp Met Thr Ile Ile Ile Met Glu Glu Thr Met Lys Tyr Phe
370 375 380

Ala Gln Glu Ile Asp Tyr Asp Val Pro Pro Gln Asp Phe Thr Val Asp
385 390 395 400

Arg Thr Lys Leu Pro Gly Arg Val Lys Ser Gly Met Val Ile Asn Asn
405 410 415

Val Arg Leu Asn Ile Asp Arg Thr Lys
420 425

<210> 17
<211> 425
<212> PRT
<213> Macrococcus goetzii

<400> 17

Met Ala Lys Arg Met Pro Lys Asp Arg Gly Leu Asp Asn Thr Leu Thr
1 5 10 15

Ile Met Lys Glu Gly Tyr Glu Tyr Ile Gln Asn Arg Thr Lys Lys Phe
20 25 30

Asn Ser Asn Val Phe Glu Thr Arg Val Leu Gly Gly Lys Gln Ala Val
35 40 45

Val Ile Ser Gly Lys Gln Ala Ala Glu Leu Phe Tyr Asp Asn Asp Lys
50 55 60

Ile Glu Arg Ser Gly Thr Leu Pro Pro Arg Val Val Lys Thr Leu Phe
65 70 75 80

Gly Lys Gly Ala Ile His Thr Thr Thr Gly Lys Lys His Ile Asp Arg
85 90 95

Lys Ala Leu Phe Met Ser Leu Met Thr Asp Glu Asn Leu Thr Tyr Leu
100 105 110

Arg Lys Leu Thr Arg Asn Tyr Trp Phe Gln Asn Thr Gln Arg Met Gln
115 120 125

Leu Met Asp Glu Val Asn Val Tyr Lys Glu Ser Ile Ile Leu Leu Thr
130 135 140

Lys Val Gly Phe Arg Trp Ala Gly Ile Ile Glu Thr Pro Glu Asn Ile
145 150 155 160

Glu Gln His Ala Met Asp Met Asp Lys Met Ile Asp Ser Phe Gly Ala
165 170 175

Ile Gly Ser Pro Tyr Lys Gly Tyr Arg Glu Ala Lys Lys Ala Arg Ala
180 185 190

Arg Val Glu Asp Phe Leu Glu Glu Gln Ile Ile Lys Thr Arg Lys Gly
195 200 205

Lys Ile His Pro Glu Pro Gly Ser Ala Leu Tyr Glu Phe Ser His Trp
210 215 220

Glu Asp Met Asn Gly Lys Pro Met Asp Ser Arg Leu Cys Ser Val Asp
225 230 235 240

Leu Met Asn Val Ile Arg Pro Leu Val Ala Ile Asn Arg Phe Val Ala
245 250 255

Tyr Gly Val Leu Ala Met His Glu Phe Pro Gly Glu Arg Thr Arg Val
260 265 270

Ala Arg Asp Asp Asn Asp Tyr Ala Tyr Lys Phe Val Gln Glu Val Arg
275 280 285

Arg Tyr Tyr Pro Phe Val Pro Phe Leu Pro Gly Lys Ala Ala Lys Asn
290 295 300

Ile Glu Phe Asp Gly Tyr Lys Ile Lys Lys Asp Thr Met Ile Ile Leu
305 310 315 320

Asp Val Phe Gly Thr Leu His Arg Glu Asp Thr Phe Ser Glu Pro Glu
325 330 335

Arg Phe Asn Pro Tyr Arg Phe Asp Asn Trp Asp Gly Ser Pro Phe Asp
340 345 350

Leu Ile Pro Gln Gly Gly Gly Asp Tyr His Thr Asn His Arg Cys Ala
355 360 365

Gly Glu Trp Met Thr Ile Ile Ile Met Glu Glu Thr Met Lys Tyr Phe
    370                 375                 380

Ala Gln Glu Ile Asn Tyr Asp Val Pro Pro Gln Asp Phe Thr Val Asp
385                 390                 395                 400

Arg Thr Lys Leu Pro Gly Arg Val Lys Ser Gly Met Ile Ile Asn Asn
                405                 410                 415

Val Arg Leu Asn Ile Asp Arg Thr Lys
            420                 425

<210> 18
<211> 425
<212> PRT
<213> Macrococcus goetzii

<400> 18

Met Ala Lys Arg Met Pro Lys Asp Arg Gly Leu Asp Asn Thr Leu Thr
1               5                   10                  15

Ile Met Lys Glu Gly Tyr Glu Tyr Ile Gln Asn Arg Thr Lys Lys Phe
            20                  25                  30

Asn Ser Asn Val Phe Glu Thr Arg Val Leu Gly Gly Lys Gln Ala Val
        35                  40                  45

Val Ile Ser Gly Lys Gln Ala Ala Glu Leu Phe Tyr Asp Asn Asp Lys
    50                  55                  60

Ile Glu Arg Ser Gly Thr Leu Pro Pro Arg Val Val Lys Thr Leu Phe
65                  70                  75                  80

Gly Lys Gly Ala Ile His Thr Thr Thr Gly Lys Lys His Ile Asp Arg
                85                  90                  95

Lys Ala Leu Phe Met Ser Leu Met Thr Asp Glu Asn Leu Thr Tyr Leu
            100                 105                 110

Arg Lys Leu Thr Arg Asn Tyr Trp Phe Gln Asn Thr Gln Arg Met Gln
        115                 120                 125

Leu Met Asp Glu Val Asn Val Tyr Lys Glu Ser Ile Ile Leu Leu Thr
    130                 135                 140

Lys Val Gly Phe Arg Trp Ala Gly Ile Ile Glu Thr Pro Glu Asn Ile
145                 150                 155                 160

Glu Gln His Ala Met Asp Met Asp Lys Met Ile Asp Ser Phe Gly Ala
165 170 175

Ile Gly Ser Pro Tyr Lys Gly Tyr Arg Glu Ala Lys Lys Ala Arg Ala
180 185 190

Arg Val Glu Asp Phe Leu Glu Glu Gln Ile Ile Lys Thr Arg Lys Gly
195 200 205

Lys Ile His Pro Glu Pro Gly Ser Ala Leu Tyr Glu Phe Ser His Trp
210 215 220

Glu Asp Met Asn Gly Lys Pro Met Asp Ser Arg Leu Cys Ser Val Asp
225 230 235 240

Leu Met Asn Val Ile Arg Pro Leu Val Ala Ile Asn Arg Phe Val Ala
245 250 255

Tyr Gly Val Leu Ala Met His Glu Phe Pro Gly Glu Arg Thr Arg Val
260 265 270

Ala Arg Asp Asp Asp Asp Tyr Ala Tyr Lys Phe Val Gln Glu Val Arg
275 280 285

Arg Tyr Tyr Pro Phe Val Pro Phe Leu Pro Gly Lys Ala Ala Lys Asn
290 295 300

Ile Glu Phe Asp Gly Tyr Lys Ile Lys Lys Asp Thr Met Ile Ile Leu
305 310 315 320

Asp Val Phe Gly Thr Leu His Arg Glu Asp Thr Phe Ser Glu Pro Glu
325 330 335

Arg Phe Asn Pro Tyr Arg Phe Asp Asn Trp Asp Gly Ser Pro Phe Asp
340 345 350

Leu Ile Pro Gln Gly Gly Gly Asp Tyr His Thr Asn His Arg Cys Ala
355 360 365

Gly Glu Trp Met Thr Ile Ile Ile Met Glu Glu Thr Met Lys Tyr Phe
370 375 380

Ala Gln Glu Ile Asp Tyr Asp Val Pro Pro Gln Asp Phe Thr Val Asp
385 390 395 400

Arg Thr Lys Leu Pro Gly Arg Val Lys Ser Gly Met Ile Ile Asn Asn
405 410 415

Val Arg Leu Asn Ile Asp Arg Thr Lys
420 425

<210> 19
<211> 425
<212> PRT
<213> Macrococcus epidermidis

<400> 19

Met Ala Lys Arg Met Pro Lys Asp Arg Gly Leu Asp Asn Thr Leu Thr
1 5 10 15

Ile Met Lys Glu Gly Tyr Glu Tyr Ile Gln Asn Arg Thr Lys Lys Phe
20 25 30

Asn Ser Asn Val Phe Glu Thr Arg Val Leu Gly Gly Lys Gln Ala Val
35 40 45

Val Ile Ser Gly Lys Gln Ala Ala Glu Leu Phe Tyr Asp Asn Asp Lys
50 55 60

Ile Glu Arg Ser Gly Thr Leu Pro Pro Arg Val Val Lys Thr Leu Phe
65 70 75 80

Gly Lys Gly Ala Ile His Thr Thr Thr Gly Lys Lys His Ile Asp Arg
85 90 95

Lys Ala Leu Phe Met Ser Leu Met Thr Asp Glu Asn Leu Thr Tyr Leu
100 105 110

Arg Lys Leu Thr Arg Asn Tyr Trp Phe Gln Asn Thr Gln Arg Met Gln
115 120 125

Leu Met Asp Glu Val Asn Ile Tyr Lys Glu Ser Ile Ile Leu Leu Thr
130 135 140

Lys Val Gly Phe Arg Trp Ala Gly Ile Ile Glu Thr Pro Glu Asn Ile
145 150 155 160

Glu Gln His Ala Met Asp Met Asp Lys Met Ile Asp Ser Phe Gly Ala
165 170 175

Ile Gly Ser Pro Tyr Lys Gly Tyr Arg Glu Ala Lys Lys Ala Arg Ala
180 185 190

Arg Val Glu Asp Phe Leu Glu Glu Gln Ile Ile Lys Thr Arg Lys Gly
195 200 205

```
Lys Ile His Pro Glu Pro Gly Ser Ala Leu Tyr Glu Phe Ser His Trp
    210                 215                 220

Glu Asp Met Asn Gly Lys Pro Met Asp Ser Arg Leu Cys Ser Val Asp
225                 230                 235                 240

Leu Met Asn Val Ile Arg Pro Leu Val Ala Ile Asn Arg Phe Val Ala
                245                 250                 255

Tyr Gly Val Leu Ala Met His Glu Phe Pro Gly Glu Arg Thr Arg Val
            260                 265                 270

Ala Arg Asn Asp Asn Asp Tyr Ala Tyr Lys Phe Val Gln Glu Val Arg
        275                 280                 285

Arg Tyr Tyr Pro Phe Val Pro Phe Leu Pro Gly Lys Ala Ala Lys Asn
    290                 295                 300

Ile Glu Phe Asp Gly Tyr Lys Ile Lys Lys Asp Thr Met Ile Ile Leu
305                 310                 315                 320

Asp Val Phe Gly Thr Leu His Arg Glu Asp Thr Phe Ser Glu Pro Glu
                325                 330                 335

Arg Phe Asn Pro Tyr Arg Phe Asp Asn Trp Asp Gly Ser Pro Phe Asp
            340                 345                 350

Leu Ile Pro Gln Gly Gly Gly Asp Tyr His Thr Asn His Arg Cys Ala
        355                 360                 365

Gly Glu Trp Met Thr Ile Ile Ile Met Glu Glu Thr Met Lys Tyr Phe
    370                 375                 380

Ala Gln Glu Ile Asn Tyr Asp Val Pro Pro Gln Asp Phe Thr Val Asp
385                 390                 395                 400

Arg Thr Lys Leu Pro Gly Arg Val Lys Ser Gly Met Ile Ile Asn Asn
                405                 410                 415

Val Arg Leu Asn Ile Asp Arg Thr Lys
            420                 425


<210>  20
<211>  425
<212>  PRT
<213>  Macrococcus goetzii
```

<400> 20

```
Met Ala Lys Arg Met Pro Lys Asp Arg Gly Leu Asp Asn Thr Leu Thr
1               5                   10                  15

Ile Met Lys Glu Gly Tyr Glu Tyr Ile Gln Asn Arg Thr Lys Lys Phe
            20                  25                  30

Asn Ser Asn Val Phe Glu Thr Arg Val Leu Gly Gly Lys Gln Ala Val
        35                  40                  45

Val Ile Ser Gly Lys Gln Ala Ala Glu Leu Phe Tyr Asp Asn Asp Lys
    50                  55                  60

Ile Glu Arg Ser Gly Thr Leu Pro Pro Arg Val Val Lys Thr Leu Phe
65                  70                  75                  80

Gly Lys Gly Ala Ile His Thr Thr Thr Gly Lys Lys His Ile Asp Arg
                85                  90                  95

Lys Ala Leu Phe Met Ser Leu Met Thr Asp Glu Asn Leu Thr Tyr Leu
            100                 105                 110

Arg Lys Leu Thr Arg Asn Tyr Trp Phe Gln Asn Thr Gln Arg Met Gln
        115                 120                 125

Leu Met Asp Glu Val Asn Val Tyr Lys Glu Ser Ile Ile Leu Leu Thr
    130                 135                 140

Lys Ile Gly Phe Arg Trp Ala Gly Ile Ile Glu Thr Pro Glu Asn Ile
145                 150                 155                 160

Glu Gln His Ala Met Asp Met Asp Lys Met Ile Asp Ser Phe Gly Ala
                165                 170                 175

Ile Gly Ser Pro Tyr Lys Gly Tyr Arg Glu Ala Lys Lys Ala Arg Thr
            180                 185                 190

Arg Val Glu Asp Phe Leu Glu Glu Gln Ile Ile Lys Thr Arg Lys Gly
        195                 200                 205

Lys Ile His Pro Glu Pro Gly Ser Ala Leu Tyr Glu Phe Ser His Trp
    210                 215                 220

Glu Asp Met Asn Gly Gln Pro Met Asp Ser Arg Leu Cys Ser Val Asp
225                 230                 235                 240

Leu Met Asn Val Ile Arg Pro Leu Val Ala Ile Asn Arg Phe Val Ala
```

245 250 255

Tyr Gly Val Leu Ala Met His Glu Phe Pro Gly Glu Arg Thr Arg Val
260 265 270

Ala Arg Asp Asp Asp Asp Tyr Ala Tyr Lys Phe Val Gln Glu Val Arg
275 280 285

Arg Tyr Tyr Pro Phe Val Pro Phe Leu Pro Gly Lys Ala Ala Lys Asn
290 295 300

Ile Glu Phe Asp Gly Tyr Lys Ile Lys Lys Asp Thr Met Ile Ile Leu
305 310 315 320

Asp Val Phe Gly Thr Leu His Arg Glu Asp Thr Phe Ser Glu Pro Glu
325 330 335

Arg Phe Asn Pro Tyr Arg Phe Asp Asn Trp Asp Gly Ser Pro Phe Asp
340 345 350

Leu Ile Pro Gln Gly Gly Gly Asp Tyr His Thr Asn His Arg Cys Ala
355 360 365

Gly Glu Trp Met Thr Ile Ile Ile Met Glu Glu Thr Met Lys Tyr Phe
370 375 380

Ala Gln Glu Ile Asp Tyr Asp Val Pro Pro Gln Asp Phe Thr Val Asp
385 390 395 400

Arg Thr Lys Leu Pro Gly Arg Val Lys Ser Gly Met Ile Ile Asn Asn
405 410 415

Val Arg Leu Asn Ile Asp Arg Thr Lys
420 425

<210> 21
<211> 425
<212> PRT
<213> Macrococcus lamae

<400> 21

Met Ser Lys Pro Ile Pro Val Asp Lys Gly Ile Asp Lys Thr Leu Thr
1 5 10 15

Val Met Lys Glu Gly Tyr Glu Tyr Val Ser Asn Arg Thr Lys Lys Phe
20 25 30

Asn Ser Asp Ile Phe Glu Thr Arg Val Leu Gly Gly Lys Arg Ala Met

35 40 45

Val Ile Ser Gly Lys Glu Ala Ala Glu Leu Phe Tyr Asp Asn Asp Lys
50 55 60

Ile Glu Arg Lys Gly Thr Leu Pro Pro Arg Val Val Lys Thr Leu Phe
65 70 75 80

Gly Lys Gly Ala Ile His Thr Thr Thr Gly Lys Val His Ile Asp Arg
85 90 95

Lys Ser Leu Phe Met Ser Leu Met Thr Glu Asn Asn Leu Glu Tyr Leu
100 105 110

Arg Lys Leu Thr Arg Asn Phe Trp Tyr Gln Asn Thr Glu Arg Met Gln
115 120 125

Leu Met Gly Lys Val Asn Val Tyr Lys Glu Ser Ile Ile Leu Leu Thr
130 135 140

Lys Val Gly Phe Arg Trp Ala Gly Val Thr Glu Lys Pro Glu Lys Ile
145 150 155 160

Glu Gln His Ala Met Asp Met Asp Lys Met Ile Asp Ser Phe Gly Ser
165 170 175

Val Gly Thr Ala Tyr Lys Gly Tyr Arg Glu Ala Lys Lys Ala Arg Ala
180 185 190

Arg Val Glu Asp Phe Leu Glu Lys Gln Ile Ile Ala Thr Arg Lys Gly
195 200 205

Lys Ile His Pro Glu Glu Gly Thr Ala Leu His Ser Phe Ser His Trp
210 215 220

Arg Asp Phe Gln Gly Asn Tyr Met Asp Ser Arg Leu Ala Ala Val Asp
225 230 235 240

Leu Met Asn Val Ile Arg Pro Leu Val Ala Ile Asn Arg Phe Val Ala
245 250 255

Tyr Gly Val Leu Ala Leu His Glu Phe Pro Gly Glu Arg Glu Arg Val
260 265 270

Ser Ala Asp Glu Asn Asn Tyr Ala Tyr Lys Phe Thr Gln Glu Leu Arg
275 280 285

```
Arg Tyr Phe Pro Phe Val Pro Phe Leu Pro Ala Lys Ala Lys Val Asp
    290                 295                 300

Ile Asp Phe Gln Gly His Val Ile Pro Gln Asp Thr Met Ile Val Leu
305                 310                 315                 320

Asp Ile Phe Gly Thr Leu His Arg Glu Asp Leu Trp Glu Asp Pro Glu
                325                 330                 335

Arg Phe Tyr Pro Asp Arg Phe Glu Asn Trp Asp Gly Ser Pro Phe Asp
            340                 345                 350

Leu Ile Pro Gln Gly Gly Gly Asp Tyr His Thr Asn His Arg Cys Ala
        355                 360                 365

Gly Glu Trp Met Thr Ile Ile Ile Met Glu Glu Thr Met Lys Tyr Phe
    370                 375                 380

Ala Arg Glu Ile Thr Tyr Asp Val Pro Ala Gln Asp Phe Thr Val Asp
385                 390                 395                 400

Arg Thr Lys Leu Pro Gly Arg Val Lys Ser Gly Met Asp Ile Glu Asn
                405                 410                 415

Val Arg Leu Asn Phe Asp Arg Thr Leu
            420                 425


<210>  22
<211>  426
<212>  PRT
<213>  Macrococcus bovicus

<400>  22

Met Ala Lys Arg Met Pro Lys Val Lys Gly Leu Asp Gln Thr Leu Thr
1               5                   10                  15

Val Leu Lys Glu Gly Tyr Glu Phe Ile Pro Asn Arg Thr Arg Lys Leu
            20                  25                  30

Asn Thr Asn Val Phe Glu Ile Arg Ile Leu Gly Gly Lys Lys Ala Val
        35                  40                  45

Val Ile Ser Gly Lys Glu Ala Ala Glu Leu Phe Tyr Asp Asn Asp Lys
    50                  55                  60

Thr Glu Arg Ala Gly Thr Leu Pro Lys Arg Leu Val Lys Thr Leu Phe
65                  70                  75                  80
```

Gly Lys Gly Ala Ile His Thr Thr Thr Gly Asp Lys His Lys Asn Arg
85 90 95

Lys Ser Leu Phe Met Ser Leu Met Thr Asp Asn Asn Leu Glu Tyr Leu
100 105 110

Arg Lys Leu Thr Arg Asn Tyr Trp Phe Glu Asp Thr Ala Arg Leu Gln
115 120 125

Ala Met Asp Gln Thr Asn Val Tyr Gln Glu Ala Thr Leu Val Leu Leu
130 135 140

Lys Ile Gly Phe Arg Phe Ala Asp Val Pro Phe Asp Thr Pro Glu Arg
145 150 155 160

Met Glu Gln Tyr Ala Lys Asp Met Asn Ala Met Val Asp Ser Phe Gly
165 170 175

Ser Ile Gly Thr Ala Tyr Thr Gly Tyr Arg Arg Ala Leu Asn Ala Arg
180 185 190

Asp Arg Val Glu Asp Tyr Leu Glu Lys Gln Ile Ile Ala Thr Arg Lys
195 200 205

Gly Lys Leu Phe Pro Glu Glu Gly Ser Gly Leu Tyr Glu Phe Ser His
210 215 220

Trp Lys Asp Met Asn Gly Lys His Met Asp Ser Arg Leu Ala Ala Val
225 230 235 240

Asp Leu Met Asn Thr Ile Arg Pro Leu Val Ala Ile Asn Arg Phe Ile
245 250 255

Ser Phe Gly Val Leu Ala Met His Glu Tyr Pro Gly Glu Arg Ala Arg
260 265 270

Val Ala Gln Asn Asp Asn Ser Tyr Ala Tyr Lys Phe Ala Gln Glu Val
275 280 285

Arg Arg Tyr Tyr Pro Phe Val Pro Phe Leu Pro Gly Lys Ala Lys Gln
290 295 300

Asn Ile Thr Phe Glu Gly Tyr Lys Ile Asp Lys Asp Thr Met Leu Ile
305 310 315 320

Leu Asp Ile Phe Gly Thr Leu His Ser Glu Ser Leu Trp Glu Asn Ala
325 330 335

```
Glu Gln Phe Tyr Pro Glu Arg Phe Glu Asn Trp Asp Gly Ser Pro Phe
            340                 345                 350

Asp Leu Ile Pro Gln Gly Gly Gly Asp Tyr Tyr Thr Asn His Arg Cys
        355                 360                 365

Ala Gly Glu Trp Met Thr Ile Ile Ile Met Glu Glu Thr Met Lys Tyr
    370                 375                 380

Phe Ala Arg Glu Ile Gln Tyr Asp Val Pro Pro Gln Asp Phe Thr Val
385                 390                 395                 400

Asp Arg Thr Lys Leu Pro Gly Gln Ile Asn Ser Gly Met Ile Ile Glu
                405                 410                 415

Asn Ile Arg Asn Asn Phe Asp Arg Arg Arg
            420                 425


<210>  23
<211>  426
<212>  PRT
<213>  Macrococcus hajekii

<400>  23

Met Ala Lys Arg Ile Pro Lys Val Lys Gly Phe Asp Gln Thr Leu Asn
1               5                   10                  15

Val Ile Lys Glu Gly Tyr Glu Phe Ile Pro Asn Arg Thr Lys Lys Phe
            20                  25                  30

Asn Ser Asn Val Phe Glu Met Arg Val Leu Gly Gly Lys Arg Ala Ile
        35                  40                  45

Val Ile Ser Gly Lys Glu Ala Ala Glu Leu Phe Tyr Asp Asn Glu Lys
    50                  55                  60

Ile Glu Arg Gln Gly Thr Leu Pro Lys Arg Ile Val Lys Thr Leu Phe
65                  70                  75                  80

Gly Lys Gly Ala Ile His Thr Thr Thr Gly Ala Lys His Lys Asp Arg
                85                  90                  95

Lys Ser Leu Phe Met Ser Leu Met Thr Glu Asn Asn Leu Glu Tyr Leu
            100                 105                 110

Arg Lys Leu Thr Arg Asn Tyr Trp Phe Glu Asp Thr Ala Arg Leu Gln
        115                 120                 125
```

Ser Lys Glu Gln Val Asn Val Phe Glu Glu Ser Thr Leu Val Leu Leu
    130                 135                 140

Lys Val Ala Phe Arg Phe Ala Asp Val Pro Phe Asp Thr Pro Glu Arg
145                 150                 155                 160

Met Glu Gln Phe Ala Lys Asp Met Asn Ser Met Val Asp Ser Phe Gly
                165                 170                 175

Ser Ile Gly Thr Val Tyr Ser Gly Tyr Lys Lys Ala Arg Asp Ala Arg
            180                 185                 190

Thr Arg Val Glu Asp Tyr Leu Glu Lys Gln Ile Lys Leu Thr Arg Lys
        195                 200                 205

Gly Lys Leu Phe Pro Glu Glu Gly Ser Gly Leu Tyr Glu Phe Ser His
    210                 215                 220

Trp Lys Asp Met Asn Gly Lys Gln Met Asp Ser Arg Leu Ala Ala Ile
225                 230                 235                 240

Asp Leu Met Asn Thr Ile Arg Pro Leu Val Ala Ile Asn Arg Phe Ile
                245                 250                 255

Ser Phe Gly Val Leu Ala Met His Glu Phe Pro Gly Glu Arg Val Lys
            260                 265                 270

Val Ala Glu Asp Asp Asn Ser Tyr Ala Tyr Lys Phe Val Gln Glu Ile
        275                 280                 285

Arg Arg Tyr Tyr Pro Phe Val Pro Phe Leu Pro Gly Lys Val Lys Gln
    290                 295                 300

Asn Phe Thr Phe Asp Gly Tyr Lys Phe Asp Lys Glu Thr Met Val Ile
305                 310                 315                 320

Met Asp Ile Phe Gly Thr Leu His Ser Glu Ser Leu Trp Asn Asn Ala
                325                 330                 335

Glu Gln Phe Tyr Pro Glu Arg Phe Glu Asn Trp Asp Gly Ser Pro Phe
            340                 345                 350

Asp Leu Ile Pro Gln Gly Gly Gly Asp Tyr Tyr Thr Asn His Arg Cys
        355                 360                 365

Ala Gly Glu Trp Met Thr Ile Ile Ile Met Glu Glu Thr Met Lys Tyr
    370                 375                 380

Phe Ala Arg Glu Ile Gln Tyr Asp Val Pro Ala Gln Asp Phe Thr Val
385                 390                 395                 400

Asn Arg Gln Lys Leu Pro Gly Gln Val Lys Ser Gly Met Ile Ile Glu
                405                 410                 415

Asn Ile Arg Asn Asn Phe Asp Arg Arg Arg
            420                 425

<210>  24
<211>  426
<212>  PRT
<213>  Macrococcus sp.

<400>  24

Met Ser Lys Ser Ile Pro Lys Glu Gln Gly Leu Asp Gln Thr Leu Asn
1               5                   10                  15

Ile Leu Lys Glu Gly Tyr Glu Phe Ile Pro Asn Arg Thr Lys Lys Phe
            20                  25                  30

Asn Ser Asn Val Phe Glu Ile Arg Val Leu Gly Gly Lys Arg Ala Ile
        35                  40                  45

Val Ile Ser Gly Lys Glu Ala Ala Glu Leu Phe Tyr Asp Asn Glu Lys
    50                  55                  60

Ile Glu Arg Ala Gly Thr Leu Pro Lys Arg Leu Val Asn Thr Leu Phe
65                  70                  75                  80

Gly Lys Gly Ala Ile His Thr Thr Thr Gly Ala Thr His Lys Asp Arg
                85                  90                  95

Lys Ser Leu Phe Met Ser Leu Met Thr Glu Asn Asn Leu Glu Tyr Leu
            100                 105                 110

Arg Lys Leu Thr Arg Asn Tyr Trp Tyr Thr Asp Thr Glu Arg Leu Gln
        115                 120                 125

Met Lys Glu Gln Thr Asn Val Tyr Leu Glu Ser Thr Leu Val Leu Leu
    130                 135                 140

Lys Ile Gly Phe Arg Phe Ala Asp Val Pro Phe Asp Thr Pro Glu Arg
145                 150                 155                 160

Met Glu Gln Tyr Ala Lys Asp Met Asn Ala Met Val Asn Ser Phe Gly
                165                 170                 175

Ser Ile Gly Thr Val Tyr Ser Gly Tyr Lys Lys Ala Leu Asp Ala Arg
180 185 190

Ser Arg Val Glu Asp Tyr Leu Glu Lys Gln Ile Lys Leu Thr Arg Lys
195 200 205

Gly Lys Leu Asn Pro Glu Ala Gly Ser Gly Leu Tyr Glu Phe Ser His
210 215 220

Trp Lys Asp Met Asn Gly Asn His Met Asp Ala Arg Leu Ala Ala Val
225 230 235 240

Asp Leu Met Asn Thr Ile Arg Pro Leu Val Ala Ile Asn Arg Phe Ile
245 250 255

Ser Phe Gly Val Leu Ala Met His Glu Tyr Pro Gly Glu Arg Ala Lys
260 265 270

Val Ala Lys Asp Glu Asn Ser Tyr Ala Tyr Lys Phe Ala Gln Glu Val
275 280 285

Arg Arg Tyr Tyr Pro Phe Val Pro Phe Leu Pro Gly Lys Ala Lys Thr
290 295 300

Asp Phe Thr Phe Glu Gly Tyr Lys Phe Asp Lys Glu Thr Met Ile Ile
305 310 315 320

Leu Asp Ile Phe Gly Thr Leu His Ser Glu Ser Leu Trp Asn Asn Ala
325 330 335

Glu Gln Phe Tyr Pro Glu Arg Phe Glu His Trp Asp Gly Ser Pro Phe
340 345 350

Asp Leu Ile Pro Gln Gly Gly Gly Asp Tyr Tyr Thr Asn His Arg Cys
355 360 365

Ala Gly Glu Trp Met Thr Ile Ile Ile Met Glu Glu Thr Met Lys Tyr
370 375 380

Phe Ala Arg Glu Ile Gln Tyr Asp Val Pro Ala Gln Asp Phe Thr Val
385 390 395 400

Asp Arg Thr Lys Leu Pro Gly Gln Val Lys Ser Gly Met Ile Ile Glu
405 410 415

Asn Ile Arg Asn Asn Phe Asp Arg Arg Gln

420 425

<210> 25
<211> 423
<212> PRT
<213> Salinicoccus luteus

<400> 25

Met Ser Thr Ile Lys Lys His Lys Gly Leu Asp Asn Thr Leu Lys Val
1 5 10 15

Met Lys Glu Gly Tyr Leu Tyr Thr Thr Asn Gln Arg Arg Arg Leu Gly
20 25 30

Ser Asp Asn Ile Phe Glu Thr Arg Val Leu Gly Gly Lys Arg Ala Ile
35 40 45

Val Ile Ser Gly Lys Lys Ala Ala Glu Leu Phe Tyr Asp Asn Asp Lys
50 55 60

Thr Glu Arg Ser Gly Thr Leu Pro Lys Arg Leu Val Asn Thr Leu Phe
65 70 75 80

Gly Lys Gly Ala Ile His Thr Thr Thr Gly Lys Lys His Val Asp Arg
85 90 95

Lys Ala Leu Phe Met Ser Leu Met Thr Glu Gly Asn Leu Lys His Leu
100 105 110

Arg Glu Leu Thr Arg Asn His Trp Tyr Met Asn Thr His Arg Met Glu
115 120 125

Gln Met Asp Gln Ile Asn Ile Tyr Arg Glu Ser Ile Ile Leu Leu Thr
130 135 140

Lys Val Gly Thr Lys Trp Ala Gly Val Gln Ala Pro Glu Glu Lys Ile
145 150 155 160

Glu Glu Ile Ala Thr Asp Met Asp Ile Met Ile Asp Ser Phe Lys Gly
165 170 175

Leu Gly Thr Ala Tyr Lys Gly Tyr Lys Ala Ser Val Ala Ala Arg Arg
180 185 190

Arg Val Glu Asp Trp Leu Glu Asp Gln Ile Ile Gln Thr Arg Lys Gly
195 200 205

Lys Ile Phe Pro Pro Glu Gly Thr Ala Leu Tyr Glu Phe Ala His Trp

```
    210                 215                 220

Glu Asp Tyr Lys Gly Asn Pro Met Asp Ser Arg Leu Cys Ala Ile Asp
225                 230                 235                 240

Leu Met Asn Thr Phe Arg Pro Leu Ile Ala Ile Asn Arg Phe Val Ser
                245                 250                 255

Phe Gly Leu Leu Ala Met Tyr Glu His Pro Ile Ser Lys Glu Lys Ile
            260                 265                 270

Lys Asn Glu Pro Asp Tyr Ala Tyr Met Phe Ser Gln Glu Val Arg Arg
        275                 280                 285

Phe Tyr Pro Phe Val Pro Phe Leu Pro Gly Lys Ala Lys Thr Asp Ile
    290                 295                 300

Gln Phe Glu Gly His Asp Ile Glu Lys Asp Thr Met Leu Val Leu Asp
305                 310                 315                 320

Ile Tyr Gly Thr Met His Arg Asp Asp Val Phe Glu Asn Ala Asp Glu
                325                 330                 335

Phe Tyr Pro Glu Arg Phe Arg Asp Trp Asp Gly Ser Pro Phe Asp Leu
            340                 345                 350

Ile Pro Gln Gly Gly Gly Asp Tyr Tyr Thr Asn His Arg Cys Ala Gly
        355                 360                 365

Glu Trp Met Thr Ile Ile Ile Met Glu Glu Thr Met Lys Tyr Phe Ala
    370                 375                 380

Gly Lys Ile Ser Tyr Asp Val Pro Glu Gln Asp Leu Thr Val Asp Leu
385                 390                 395                 400

Asn Ser Ile Pro Gly Tyr Ile Lys Ser Gly Phe Ile Ile Glu Asn Val
                405                 410                 415

Gln Glu Asn Val Asp Arg Gly
            420

<210>  26
<211>  423
<212>  PRT
<213>  Salinicoccus roseus

<400>  26

Met Ser Thr Ile Lys Lys His Lys Gly Leu Asp Asn Thr Leu Lys Val
```

```
1               5                   10                  15

Met Lys Glu Gly Tyr Leu Tyr Thr Thr Asn Gln Arg Arg Arg Leu Gly
            20                  25                  30

Ala Glu Asn Ile Phe Glu Thr Arg Val Leu Gly Gly Lys Arg Ala Leu
        35                  40                  45

Val Ile Ser Gly Lys Lys Ala Ala Glu Leu Phe Tyr Asp Asn Asp Lys
    50                  55                  60

Thr Glu Arg Ser Gly Thr Leu Pro Lys Arg Leu Val Asn Thr Leu Phe
65                  70                  75                  80

Gly Lys Gly Ala Ile His Thr Thr Thr Gly Lys Lys His Ile Asp Arg
                85                  90                  95

Lys Ala Leu Phe Met Ser Leu Met Thr Glu Gly Asn Leu Lys His Leu
            100                 105                 110

Arg Glu Leu Thr Arg Asn His Trp Tyr Met Asn Thr His Arg Met Glu
        115                 120                 125

Gln Met Asp Gln Ile Asn Ile Tyr Arg Glu Ser Ile Ile Leu Leu Thr
    130                 135                 140

Lys Val Gly Thr Lys Trp Ala Gly Val Gln Ala Pro Glu Glu Lys Ile
145                 150                 155                 160

Glu Glu Ile Ala Thr Asp Met Asp Ile Met Ile Asp Ser Phe Arg Gly
                165                 170                 175

Leu Gly Thr Ala Phe Lys Gly Tyr Lys Glu Ser Val Ala Ala Arg Arg
            180                 185                 190

Arg Val Glu Asp Trp Leu Glu Asp Gln Ile Ile Gln Thr Arg Lys Gly
        195                 200                 205

Lys Ile Phe Pro Pro Glu Gly Thr Ala Leu Tyr Glu Phe Ala His Trp
    210                 215                 220

Glu Asp Tyr Lys Gly Asn Pro Met Asp Ser Arg Leu Cys Ala Ile Asp
225                 230                 235                 240

Leu Met Asn Thr Phe Arg Pro Leu Ile Ala Ile Asn Arg Phe Val Ser
                245                 250                 255
```

Phe Gly Leu Leu Ala Met His Glu His Pro Ile Ser Lys Glu Lys Ile
260 265 270

Asn Asn Asp Pro Asp Tyr Ala Tyr Met Phe Ser Gln Glu Val Arg Arg
275 280 285

Phe Tyr Pro Phe Val Pro Phe Leu Pro Gly Lys Ala Lys Thr Asp Ile
290 295 300

Gln Phe Glu Gly His Asp Ile Glu Lys Asp Thr Met Leu Val Leu Asp
305 310 315 320

Ile Tyr Gly Thr Met His Arg Asp Asp Val Phe Glu Asn Ala Asp Glu
325 330 335

Phe His Pro Glu Arg Phe Leu Asp Trp Asp Gly Ser Pro Phe Asp Leu
340 345 350

Ile Pro Gln Gly Gly Gly Asp Tyr Tyr Thr Asn His Arg Cys Ala Gly
355 360 365

Glu Trp Met Thr Ile Ile Ile Met Glu Glu Thr Met Lys Tyr Phe Ala
370 375 380

Gly Lys Ile Thr Tyr Asp Val Pro Glu Gln Asp Leu Thr Val Asp Leu
385 390 395 400

Asn Ser Ile Pro Gly Tyr Ile Lys Ser Gly Phe Ile Ile Glu Asn Val
405 410 415

Ala Glu Lys Val Asp Arg Lys
420

<210> 27
<211> 423
<212> PRT
<213> Salinicoccus roseus

<400> 27

Met Ser Thr Ile Lys Lys His Lys Gly Leu Asp Asn Thr Leu Lys Val
1 5 10 15

Met Lys Glu Gly Tyr Leu Tyr Thr Thr Asn Gln Arg Arg Arg Leu Gly
20 25 30

Ala Glu Asn Ile Phe Glu Thr Arg Val Leu Gly Gly Lys Arg Ala Leu
35 40 45

```
Val Ile Ser Gly Lys Lys Ala Ala Glu Leu Phe Tyr Asp Asn Asp Lys
    50                  55                  60

Thr Glu Arg Ser Gly Thr Leu Pro Lys Arg Leu Val Asn Thr Leu Phe
65                  70                  75                  80

Gly Lys Gly Ala Ile His Thr Thr Thr Gly Lys Lys His Ile Asp Arg
                85                  90                  95

Lys Ala Leu Phe Met Ser Leu Met Thr Glu Gly Asn Leu Lys His Leu
            100                 105                 110

Arg Glu Leu Thr Arg Asn His Trp Tyr Met Asn Thr His Arg Met Glu
        115                 120                 125

Gln Met Asp Gln Ile Asn Ile Tyr Arg Glu Ser Ile Ile Leu Leu Thr
    130                 135                 140

Lys Val Gly Thr Lys Trp Ala Gly Val Gln Ala Pro Glu Glu Lys Ile
145                 150                 155                 160

Glu Glu Ile Ala Thr Asp Met Asp Ile Met Ile Asp Ser Phe Arg Gly
                165                 170                 175

Leu Gly Thr Ala Phe Lys Gly Tyr Lys Ala Ser Val Ala Ala Arg Arg
            180                 185                 190

Arg Val Glu Asp Trp Leu Glu Asp Gln Ile Ile Gln Thr Arg Lys Gly
        195                 200                 205

Lys Ile Phe Pro Pro Glu Gly Thr Ala Leu Tyr Glu Phe Ala His Trp
    210                 215                 220

Glu Asp Tyr Lys Gly Asn Pro Met Asp Ser Arg Leu Cys Ala Ile Asp
225                 230                 235                 240

Leu Met Asn Thr Phe Arg Pro Leu Ile Ala Ile Asn Arg Phe Val Ser
                245                 250                 255

Phe Gly Leu Leu Ala Met His Glu His Pro Ile Ser Lys Glu Lys Ile
            260                 265                 270

Asn Asn Asp Pro Asp Tyr Ala Tyr Met Phe Ser Gln Glu Val Arg Arg
        275                 280                 285

Phe Tyr Pro Phe Val Pro Phe Leu Pro Gly Lys Ala Lys Thr Asp Ile
    290                 295                 300
```

Gln Phe Glu Gly His Asp Ile Glu Lys Asp Thr Met Met Val Leu Asp
305 310 315 320

Ile Tyr Gly Thr Met His Arg Asp Asp Val Phe Glu Asn Ala Asp Glu
325 330 335

Phe Tyr Pro Glu Arg Phe Leu Asp Trp Asp Gly Ser Pro Phe Asp Leu
340 345 350

Ile Pro Gln Gly Gly Gly Asp Tyr Tyr Thr Asn His Arg Cys Ala Gly
355 360 365

Glu Trp Met Thr Ile Ile Ile Met Glu Glu Thr Met Lys Tyr Phe Ala
370 375 380

Gly Lys Ile Thr Tyr Asp Val Pro Glu Gln Asp Leu Thr Val Asp Leu
385 390 395 400

Asn Ser Ile Pro Gly Tyr Ile Lys Ser Gly Phe Ile Ile Glu Asn Val
405 410 415

Ala Glu Lys Val Asp Arg Lys
420

<210> 28
<211> 423
<212> PRT
<213> Salinicoccus albus

<400> 28

Met Ser Thr Ile Lys Arg Asp Thr Gly Leu Asp Asn Thr Leu Lys Val
1 5 10 15

Met Lys Gln Gly Tyr Leu Tyr Thr Glu Asn Gln Arg Gln Arg Leu Gly
20 25 30

Ser Asp Asn Ile Phe Glu Thr Arg Val Leu Gly Gly Lys Arg Ala Leu
35 40 45

Val Ile Ser Gly Lys Arg Ala Ala Glu Leu Phe Tyr Asp Asn Asp Lys
50 55 60

Ile Glu Arg Ala Gly Thr Leu Pro Lys Arg Leu Val Asn Thr Leu Phe
65 70 75 80

Gly Lys Gly Ala Ile His Thr Thr Thr Gly Lys Gln His Ile Asp Arg
85 90 95

Lys Ala Leu Phe Met Ser Leu Met Thr Glu Gly Asn Leu Lys His Leu
100 105 110

Arg Glu Leu Thr Arg Asn His Trp Tyr Met Asn Thr His Arg Met Glu
115 120 125

Gln Met Asp Gln Ile Asn Ile Tyr Arg Glu Ser Ile Ile Leu Leu Thr
130 135 140

Lys Val Gly Thr Lys Trp Ala Gly Val Gln Ala Pro Glu Glu Lys Val
145 150 155 160

Glu Glu Ile Ala Thr Asp Met Asp Ile Met Ile Asp Ser Phe Arg Gly
165 170 175

Leu Gly Thr Ala Phe Lys Gly Tyr Lys Lys Ser Lys Asp Ala Arg Arg
180 185 190

Arg Val Glu Asp Trp Leu Glu Asp Gln Ile Ile Gln Thr Arg Lys Gly
195 200 205

Lys Ile Phe Pro Pro Glu Gly Thr Ala Leu Tyr Glu Phe Ala His Trp
210 215 220

Glu Asp Tyr Lys Gly Asn Pro Met Asp Ser Arg Leu Cys Ala Ile Asp
225 230 235 240

Leu Met Asn Thr Phe Arg Pro Leu Ile Ala Ile Asn Arg Phe Val Ser
245 250 255

Phe Gly Leu Leu Ala Met His Glu His Pro Ile Ser Lys Glu Lys Ile
260 265 270

Lys Ser Asp Asp Asp Tyr Ala Tyr Met Phe Ser Gln Glu Val Arg Arg
275 280 285

Tyr Tyr Pro Phe Val Pro Phe Leu Pro Gly Lys Ala Lys Thr Asp Ile
290 295 300

Glu Phe Asp Gly His Ala Ile Glu Lys Asp Thr Met Met Val Ile Asp
305 310 315 320

Ile Tyr Gly Thr Met His Arg Asp Asp Val Phe Glu Asn Ala Asn Glu
325 330 335

Phe Tyr Pro Glu Arg Phe Lys Glu Trp Asp Gly Ser Pro Phe Asp Leu
340 345 350

Ile Pro Gln Gly Gly Gly Asp Tyr Tyr Thr Asn His Arg Cys Ala Gly
355 360 365

Glu Trp Met Thr Ile Ile Ile Met Glu Glu Thr Met Lys Tyr Phe Ala
370 375 380

Asp Arg Ile Ser Tyr Asp Val Pro Gln Gln Asp Leu Thr Val Asp Leu
385 390 395 400

Asn Ser Ile Pro Gly Tyr Ile Lys Ser Gly Phe Val Ile Glu Asn Val
405 410 415

Ser Glu Asn Val Asp Arg Asn
420

<210> 29
<211> 435
<212> PRT
<213> Jeotgalibacillus alimentarius

<400> 29

Met Asp Val Lys Lys Pro Val Pro Arg Ala Lys Gly Ile Asp Asn Ser
1 5 10 15

Leu Asn Phe Ile Lys Glu Gly Phe His Phe Leu Pro Asn Gln Arg Gln
20 25 30

Glu Leu Gly Ser Asp Ile Phe Glu Thr Arg Leu Leu Gly Lys Lys Ala
35 40 45

Val Cys Ile Ala Gly Val Glu Ala Ser Glu Met Phe Tyr Asp Thr Glu
50 55 60

Lys Phe Lys Arg Glu Gly Ala Met Pro Lys Pro Phe Lys Met Ser Leu
65 70 75 80

Phe Gly Glu Gly Ser Leu His Gly Met Asp Gly Glu Ala His Leu Asn
85 90 95

Arg Lys Arg Thr Leu Leu Ser Met Phe Thr Pro Asp Arg Val Glu Asp
100 105 110

Leu Lys Arg Met Ala Ile Lys His Leu Asp Glu Lys Val Ser Glu Trp
115 120 125

Glu His Ala Asp Asp Val Val Ile Phe Asp Glu Met Gln Glu Ile Leu
130 135 140

Ala Arg Ala Gly Cys Glu Trp Ala Gly Val Pro Leu Glu Glu Glu Glu
145 150 155 160

Val Lys Gln Arg Thr Arg Glu Met Ile Asp Met Ile Asp Ser Phe Gly
165 170 175

Gly Ser Leu Ser Arg Phe Lys Asp Gly Lys Lys Ala Arg Asn Ser His
180 185 190

Glu Ala Trp Leu Gln Asp Ile Ile Lys Gly Val Arg Lys Gly Lys Met
195 200 205

Ser Pro Lys Pro Asn Thr Ala Ala Tyr Ile Met Ser His His Arg Glu
210 215 220

Asn Asn Gly Lys Arg Phe Asp Lys Glu Ile Ala Ala Val Glu Leu Ser
225 230 235 240

Asn Ala Phe Arg Pro Leu Leu Ala Thr Ala Tyr Phe Leu Thr Phe Gly
245 250 255

Val Leu Ala Met His Glu His Pro Glu Thr Val Glu Lys Leu Lys Asn
260 265 270

Asn Ala Lys Tyr Ser His Met Phe Ala Gln Glu Thr Arg Arg Tyr Tyr
275 280 285

Pro Phe Val Pro Ala Met Val Ala Lys Val Lys Lys Asn Phe Thr Phe
290 295 300

Lys Gly Tyr Lys Phe Lys Lys Asp Thr Leu Val Val Leu Asp Ile Phe
305 310 315 320

Gly Thr Asn Arg His Pro Asp Ser Trp Glu Asn Ala Asp Thr Phe Ile
325 330 335

Pro Glu Arg Phe Asp Gly Trp Lys Gly Ser Pro Phe Ser Phe Ile Pro
340 345 350

Gln Gly Gly Gly Asp His His Thr Gly His Arg Cys Ala Gly Glu Trp
355 360 365

Leu Thr Val Ile Val Met Ser Ser Phe Phe Asp Phe Phe Ala Lys Asn
370 375 380

Leu Thr Tyr Asp Val Pro Glu Gln Asp Leu Ser Phe Ser Met Glu Arg

385 390 395 400

Met Pro Thr Phe Pro Asn Ser Arg Leu Val Ile Lys Asn Val Arg Lys
405 410 415

Thr Asp Asp Tyr Glu Lys Asn Leu Ala Glu Leu Met Asp Gly Phe Ser
420 425 430

Ala Ile Arg
435

<210> 30
<211> 436
<212> PRT
<213> Jeotgalibacillus campisalis

<400> 30

Met Lys Val Lys Thr Pro Ile Pro Arg Ala Lys Gly Ile Asp Asn Ser
1 5 10 15

Leu Ser Leu Ile Lys Glu Gly Phe His Phe Leu Pro Asn Gln Arg Lys
20 25 30

Glu Met Gln Ser Asp Ile Phe Lys Thr Arg Ile Leu Gly Gln Lys Ala
35 40 45

Ile Cys Ile Ala Gly Glu Glu Ala Ala Glu Val Phe Tyr Asn Asn Asp
50 55 60

Leu Phe Lys Arg Lys Gly Ala Met Pro Lys Pro Leu Lys Leu Ser Leu
65 70 75 80

Phe Gly Glu Gly Ser Leu His Gly Leu Asp Gly Glu Pro His Lys Asn
85 90 95

Arg Lys Arg Met Phe Leu Ser Met Phe Thr Pro Glu Arg Val Glu Asp
100 105 110

Leu Val Asp Leu Ala Leu Lys His Leu Asp Ala Lys Ala Gly Glu Trp
115 120 125

Gln Ser Gln Asp Gly Val Ala Val Leu Asp Gln Met Gln Glu Ile Leu
130 135 140

Thr Arg Ala Gly Cys Glu Trp Ala Gly Val Pro Leu Ala Glu Gly Glu
145 150 155 160

Val Gln Gln Arg Thr Asp Glu Val Val Ala Met Val Asp Ser Phe Gly

165 170 175

Gly Ser Leu Ser Arg Phe Arg Asp Gly Lys Lys Ala Arg Asp Ser His
180 185 190

Glu Glu Trp Leu Gln Lys Ile Ile Lys Ser Val Arg Lys Gly Lys Tyr
195 200 205

Asn Pro Pro Glu Asn Thr Ala Val Tyr Thr Ile Ala His His Arg Asp
210 215 220

Glu Lys Gly Lys Pro Phe Asp Leu His Thr Ala Ala Val Glu Leu Ser
225 230 235 240

Asn Ala Phe Arg Pro Leu Ile Ala Thr Ala Asn Phe Leu Val Phe Gly
245 250 255

Ala Leu Ala Met His Glu His Pro Glu Thr Arg Gln Lys Ile Ala Ala
260 265 270

Asp Glu Asp Arg Tyr Ser His Met Phe Ser Gln Glu Val Arg Arg Tyr
275 280 285

Tyr Pro Phe Val Pro Ala Met Ala Ala Lys Val Lys Lys Asp Phe Thr
290 295 300

Trp His Asp Tyr Thr Phe Lys Lys Asp Thr Leu Val Ile Leu Asp Ile
305 310 315 320

Phe Gly Thr Asn Arg His Pro Asp Ser Trp Glu Gln Ala Glu Thr Phe
325 330 335

Leu Pro Glu Arg Phe Lys Asp Trp Gln Gly Ser Pro Phe Ala Phe Ile
340 345 350

Pro Gln Gly Gly Gly Asp His His Ile Gly His Arg Cys Ala Gly Glu
355 360 365

Trp Leu Thr Val Tyr Val Met Arg Ala Phe Phe Lys Tyr Phe Ala Gln
370 375 380

Asn Ile Ser Tyr Asp Val Pro Glu Gln Asp Leu Ser Tyr Ser Leu Glu
385 390 395 400

Arg Met Pro Thr Phe Pro Lys Ser Gly Phe Val Ile Thr Asn Val Lys
405 410 415

Lys Ala Ala Asp Ser Asp Glu Leu Leu His Ser Met Gln Lys Arg His
420 425 430

Thr Ala Ile Gln
435

<210> 31
<211> 435
<212> PRT
<213> Jeotgalibacillus malaysiensis

<400> 31

Met Asp Val Lys Lys Pro Val Pro Arg Ser Lys Gly Ile Asp Asn Ser
1 5 10 15

Leu Ser Leu Ile Lys Glu Gly Phe Tyr Phe Leu Pro Asn Gln Arg Gln
20 25 30

Lys Leu Gly Ser Asp Ile Phe Glu Thr Arg Leu Leu Gly Lys Lys Ala
35 40 45

Ile Cys Met Ala Gly Glu Glu Ala Ser Glu Leu Phe Tyr Asp Thr Glu
50 55 60

Lys Phe Lys Arg Glu Asp Ala Met Pro Lys Pro Leu Lys Met Ser Leu
65 70 75 80

Phe Gly Asp Gly Ser Leu His Gly Met Asp Gly Glu Ala His Arg Asn
85 90 95

Arg Lys Arg Ser Phe Leu Ser Met Phe Thr Pro Asp Arg Val Glu Asp
100 105 110

Leu Lys Arg Leu Ala Leu Glu His Leu Asp Ala Lys Val Ser His Trp
115 120 125

Glu Lys Ala Asp Glu Val Val Ile Phe Asp Glu Met Gln Glu Ile Leu
130 135 140

Ala Arg Ala Gly Cys Glu Trp Ala Gly Val Pro Leu Glu Glu Asn Glu
145 150 155 160

Val Gln Gln Arg Thr Gln Glu Met Ile Asp Met Ile Asp Ser Phe Gly
165 170 175

Gly Ser Leu Ser Arg Phe Lys Asp Gly Lys Lys Ala Arg Asp Ser His
180 185 190

Glu Ala Trp Leu Gln Glu Ile Ile Lys Gly Val Arg Lys Gly Lys Met
195 200 205

Ser Pro Pro Pro His Thr Ala Ala Tyr Val Met Ala His His Arg Glu
210 215 220

Asn Asn Gly Lys Arg Ile Asp Lys Gly Thr Ala Ala Val Glu Leu Ser
225 230 235 240

Asn Ser Phe Arg Pro Leu Leu Ala Thr Ala Tyr Phe Leu Thr Phe Gly
245 250 255

Ile Val Ala Met His Glu His Pro Leu Thr Val Ser Lys Leu Gln His
260 265 270

His Pro Arg Tyr Ser His Met Phe Ala Gln Glu Thr Arg Arg Phe Tyr
275 280 285

Pro Phe Val Pro Ala Met Ala Ala Lys Val Lys Lys Asn Phe Thr Phe
290 295 300

Lys Gly Tyr Lys Phe Lys Lys Asp Thr Leu Val Val Leu Asp Ile Phe
305 310 315 320

Gly Thr Asn Arg His Pro Asp Ser Trp Glu Asn Ala Asp Thr Phe Ile
325 330 335

Pro Glu Arg Phe Glu Gly Trp Lys Gly Ser Pro Phe Ser Phe Ile Pro
340 345 350

Gln Gly Gly Gly Asp His His Thr Gly His Arg Cys Ala Gly Glu Trp
355 360 365

Leu Thr Val Ile Val Met Ser Ser Phe Phe Glu Phe Phe Ala Lys Asn
370 375 380

Leu Thr Tyr Asp Val Pro Glu Gln Asp Leu Ser Tyr Ser Met Glu Arg
385 390 395 400

Met Pro Thr Phe Pro Asn Ser Arg Met Ile Ile Gln Asn Val Arg Lys
405 410 415

Ile Ala Asp Tyr Glu Lys Asn Leu Asn Glu Leu Met Asp Gly Phe Ser
420 425 430

Ala Ile Arg
435

<210> 32
<211> 436
<212> PRT
<213> Jeotgalibacillus proteolyticus

<400> 32

Met Lys Val Lys Thr Pro Ile Pro Arg Ala Lys Gly Ile Asp Asn Ser
1 5 10 15

Leu Ser Leu Ile Lys Glu Gly Phe His Phe Leu Pro Asn Gln Arg Lys
20 25 30

Glu Val Glu Ser Asp Ile Phe Lys Thr Arg Ile Leu Gly Gln Lys Ala
35 40 45

Ile Cys Met Cys Gly Glu Glu Ala Ala Glu Val Phe Tyr Asp Pro Asp
50 55 60

Lys Phe Lys Arg Lys Gly Ala Leu Pro Lys Pro Leu Lys Met Ser Leu
65 70 75 80

Phe Gly Asp Gly Ser Leu His Ala Leu Asp Gly Glu Asp His Arg Asn
85 90 95

Arg Lys Arg Met Phe Leu Ser Met Phe Thr Pro Glu Arg Val Glu Asp
100 105 110

Leu Lys Lys Ile Ala Leu Glu His Leu Ala Ala Lys Val Asp Val Trp
115 120 125

Glu Lys Ser Asp Gln Val Ile Phe Phe Gly Glu Met Gln Glu Ile Leu
130 135 140

Thr Arg Ala Gly Cys Glu Trp Ala Gly Phe Pro Leu Glu Glu Glu Glu
145 150 155 160

Val His Gln Arg Thr Glu Glu Ile Val Ala Met Ile Asp Ser Phe Gly
165 170 175

Gly Ser Leu Ser Arg Phe Arg Asp Gly Lys Lys Ala Arg Asp Ser His
180 185 190

Glu Glu Trp Leu Glu Gly Ile Ile Lys Ala Val Arg Lys Gly Lys Met
195 200 205

Asn Pro Pro Ala Tyr Thr Ala Ala Tyr Ile Val Ala His His Arg Glu
210 215 220

Asn Asn Gly Lys Arg Leu Asp Val Arg Thr Ala Ala Ile Glu Leu Ser
225                 230                 235                 240

Asn Ala Phe Arg Pro Leu Ile Ala Thr Ala Tyr Phe Leu Thr Phe Gly
                245                 250                 255

Ala Ala Ala Met His Glu His Pro Glu Thr Ala His Lys Leu Met Glu
            260                 265                 270

Asn Lys Asp His Tyr Ser His Met Phe Ser Gln Glu Val Arg Arg Tyr
        275                 280                 285

Tyr Pro Phe Val Pro Ala Met Ala Ala Lys Val Lys Glu Asp Phe Val
    290                 295                 300

Trp Gln Asp His Lys Phe Lys Lys Asn Thr Leu Val Ile Leu Asp Ile
305                 310                 315                 320

Phe Gly Thr Asn Arg His Pro Asp Leu Trp Ser Asp Ala Asp Ser Phe
                325                 330                 335

Ile Pro Glu Arg Phe Arg Asp Trp Lys Gly Ser Pro Phe Ser Phe Ile
            340                 345                 350

Pro Gln Gly Gly Gly Asp His His Ile Gly His Arg Cys Ala Gly Glu
        355                 360                 365

Trp Leu Thr Val Tyr Val Met Arg Ser Phe Phe Gln Phe Leu Ala Gln
    370                 375                 380

Asn Ile Thr Tyr Asp Val Pro Glu Gln Asp Leu Ser Tyr Ser Leu Glu
385                 390                 395                 400

Arg Met Pro Thr Phe Pro Val Ser Gly Leu Val Ile Thr Asn Val Arg
                405                 410                 415

Lys Gln Ala Asp Phe Asn Glu Lys Leu Asn Glu Leu Asp Lys Ser Tyr
            420                 425                 430

Thr Ala Ile Arg
        435

<210> 33
<211> 434
<212> PRT
<213> Halobacillus salinus

<400> 33

Met Ser Lys Pro Ile Pro Lys Ala Glu Gly Ala Asp Asn Thr Leu Ser
1               5                   10                  15

Leu Ile Gln Glu Gly Phe Pro Phe Phe Ile Lys Arg His Lys Glu Leu
            20                  25                  30

Gly Thr Asn Ile Phe Glu Thr Arg Leu Ile Gly Arg Lys Ala Ile Cys
        35                  40                  45

Ile Val Gly Glu Glu Ala Ser Lys Ile Phe Tyr Asp Thr Glu Lys Phe
    50                  55                  60

Lys Arg Glu Gly Ala Met Pro Lys Pro Leu Lys Met Ser Leu Phe Gly
65                  70                  75                  80

Glu Gly Ser Leu His Gly Met Asp Gly Glu Lys His Leu His Arg Lys
                85                  90                  95

Arg Thr Phe Leu Ser Met Phe Thr Pro Asp Arg Val Glu Asp Leu Lys
            100                 105                 110

Gln Asn Ala Leu Glu Glu Leu Asp Arg Lys Met Glu Gln Trp Gln Glu
        115                 120                 125

Arg Asp Lys Val Val Leu Tyr Glu Glu Phe Pro Glu Ile Leu Thr Arg
    130                 135                 140

Ala Val Cys Lys Trp Ala Gly Val Pro Leu Glu Glu His Glu Val Arg
145                 150                 155                 160

Gln Arg Thr Asp Glu Met Met Ser Met Val Glu Ser Phe Gly Gly Ser
                165                 170                 175

Leu Ser Arg Phe Gln Glu Gly Lys Lys Ser Arg Asp Ser His Glu Ala
            180                 185                 190

Trp Leu Lys Gln Ile Ile Lys Asp Val Arg Lys Gly Lys Leu Gln Pro
        195                 200                 205

Glu Pro Asn Thr Ala Ala Tyr Ile Met Ala His His Arg Glu Thr Asp
    210                 215                 220

Gly Lys Leu Trp Asp Val Gln Thr Ala Ala Val Glu Leu Ser Asn Ser
225                 230                 235                 240

Phe Arg Pro Leu Leu Ala Thr Val His Leu Leu Val Leu Gly Ala Val
                245                 250                 255

Ala Met His Glu His Pro Glu Thr Arg Thr Arg Leu Glu Lys Gly Glu
260 265 270

Pro Leu Tyr Ser His Trp Phe Ala Gln Glu Thr Arg Arg Phe Tyr Pro
275 280 285

Phe Val Pro Ala Met Ile Ala Lys Val Lys Thr Pro Phe Leu Trp Glu
290 295 300

Gly Tyr Glu Phe Lys Lys Asn Thr Arg Val Val Leu Asp Leu Tyr Gly
305 310 315 320

Thr Asn His His Ala Asp Ser Trp Val His Pro Asp Ala Phe Ile Pro
325 330 335

Glu Arg Phe Glu Asn Trp Gln Gly Ser Pro Phe Ser Phe Val Pro Gln
340 345 350

Gly Gly Gly Asp His His Thr Gly His Arg Cys Ala Gly Glu Trp Leu
355 360 365

Thr Val Ile Val Met Ser Ser Phe Phe Glu Phe Leu Ala Lys Asn Val
370 375 380

Ser Tyr Asp Val Pro Glu Gln Asn Leu Ser Phe Ser Leu Glu Arg Met
385 390 395 400

Pro Thr Arg Pro Glu Ser Gly Phe Val Ile Glu Asn Val Arg Lys Lys
405 410 415

Asp Ala Tyr Glu Glu Thr Phe Asp Glu Leu Asn Gln Gly Phe Ala Ala
420 425 430

Arg Arg

<210> 34
<211> 436
<212> PRT
<213> Bacillus clarkii

<400> 34

Met Lys Val Lys Thr Pro Ile Pro Lys Ala Lys Gly Ile Asp Asn Ser
1 5 10 15

Met Ser Leu Ile Lys Glu Gly Phe His Phe Leu Pro Asn Arg Arg Glu
20 25 30

Glu Leu Gly Ser Asp Ile Phe Glu Thr Arg Leu Met Gly Arg Lys Ala
35 40 45

Val Cys Met Ser Gly Glu Glu Ala Ala Glu Val Phe Tyr Asp Asn Asp
50 55 60

Lys Phe Lys Arg Lys Gly Ala Leu Pro Lys Pro Leu Gln Leu Ser Leu
65 70 75 80

Phe Gly Thr Gly Ser Val His Ala Leu Asp Gly Glu Ala His Lys Asn
85 90 95

Arg Lys Arg Met Phe Leu Ser Met Phe Thr Pro Glu Arg Val Glu Asp
100 105 110

Leu Asn Arg Ile Ala Leu Lys His Leu Asp Asn Lys Val Asn Gln Trp
115 120 125

Val Lys Arg Asp Lys Val Ile Leu Ile Asp Glu Met His Glu Ile Leu
130 135 140

Thr Arg Ala Gly Cys Glu Trp Ala Gly Val Pro Leu Lys Glu Glu Glu
145 150 155 160

Val Glu Gln Arg Thr Ala Glu Val Gly Gln Met Ile Asp Ser Phe Gly
165 170 175

Gly Ser Leu Ser Arg Trp Arg Asn Gly Lys Lys Ala Arg Asp Ser His
180 185 190

Glu Glu Trp Leu Glu Gly Ile Ile Lys Lys Ile Arg Lys Gly Lys Met
195 200 205

Glu Val Pro Ala Asn Thr Ala Ala Tyr Ile Val Ala His His Arg Glu
210 215 220

Leu Asn Gly Lys Arg Leu Asp Ser Arg Gln Ala Ala Ile Glu Leu Ser
225 230 235 240

Asn Ser Phe Arg Pro Leu Ile Ala Thr Ala Tyr Phe Leu Thr Leu Gly
245 250 255

Ala Val Ala Met His Glu His Pro Gly Thr Met Glu Lys Leu Lys Lys
260 265 270

Asp Glu Gly Asn Tyr Ser His Met Phe Ser Gln Glu Val Arg Arg Tyr

275 280 285

Tyr Pro Phe Val Pro Ala Met Ala Ala Lys Val Lys Lys Asp Phe Val
290 295 300

Trp His Asp Tyr Lys Phe Lys Lys Asp Thr Leu Val Val Leu Asp Ile
305 310 315 320

Phe Gly Thr Asn Arg His Pro Asp Asn Trp Glu Gln Ala Asp Glu Phe
325 330 335

Ile Pro Glu Arg Phe Lys Asp Trp Lys Gly Ser Pro Phe Ser Phe Ile
340 345 350

Pro Gln Gly Gly Gly Asp His His Ile Gly His Arg Cys Ala Gly Glu
355 360 365

Trp Leu Thr Val Met Val Met Arg Ser Phe Phe Lys Tyr Phe Val Asn
370 375 380

Asn Met Asn Tyr His Val Pro Glu Gln Asn Leu Ala Tyr Ser Leu Glu
385 390 395 400

Arg Met Pro Thr Phe Pro Lys Ser Gly Phe Val Ile Thr Asp Val Lys
405 410 415

Lys Ala Ser Asp Ser Gly Glu Lys Met Gln Glu Met Met His Asn Pro
420 425 430

Ile Thr Val Lys
435

<210> 35
<211> 436
<212> PRT
<213> Bacillus polygoni

<400> 35

Met Lys Val Lys Thr Pro Ile Pro Lys Ala Lys Gly Ile Asp Asn Ser
1 5 10 15

Met Ser Leu Ile Lys Glu Gly Phe His Phe Leu Pro Asn Arg Arg Glu
20 25 30

Glu Leu Gly Ser Asp Ile Phe Glu Thr Arg Leu Met Gly Arg Lys Ala
35 40 45

Val Cys Met Ser Gly Glu Glu Ala Ala Glu Val Phe Tyr Asp Asn Asp

50 55 60

Lys Phe Lys Arg Lys Gly Ala Leu Pro Lys Pro Leu Gln Leu Ser Leu
65 70 75 80

Phe Gly Thr Gly Ser Val His Ala Leu Asp Gly Glu Ala His Lys Asn
85 90 95

Arg Lys Arg Met Phe Leu Ser Met Phe Thr Pro Glu Arg Val Glu Asp
100 105 110

Leu Asn Arg Ile Ala Leu Lys His Leu Asp Asn Lys Val Asn Gln Trp
115 120 125

Val Lys Arg Asp Lys Val Ile Leu Ile Asp Glu Met His Glu Ile Leu
130 135 140

Thr Arg Ala Gly Cys Glu Trp Ala Gly Val Pro Leu Lys Glu Glu Glu
145 150 155 160

Val Glu Gln Arg Thr Ala Glu Val Gly Gln Met Ile Asp Ser Phe Gly
165 170 175

Gly Ser Leu Ser Arg Trp Arg Asn Gly Lys Lys Ala Arg Asp Ser His
180 185 190

Glu Glu Trp Leu Glu Gly Ile Ile Lys Lys Ile Arg Lys Gly Lys Met
195 200 205

Glu Val Pro Ala Asn Thr Ala Ala Tyr Ile Val Ala His His Arg Glu
210 215 220

Leu Asn Gly Lys Arg Leu Asp Ser Arg Gln Ala Ala Ile Glu Leu Ser
225 230 235 240

Asn Ser Phe Arg Pro Leu Ile Ala Thr Ala Tyr Phe Leu Thr Leu Gly
245 250 255

Ala Val Ala Met His Glu His Pro Gly Thr Met Glu Lys Leu Lys Lys
260 265 270

Asp Glu Gly Asn Tyr Ser His Met Phe Ser Gln Glu Val Arg Arg Tyr
275 280 285

Tyr Pro Phe Val Pro Ala Met Ala Ala Lys Val Lys Lys Asp Phe Val
290 295 300

Trp His Asp Tyr Lys Phe Lys Lys Asp Thr Leu Val Val Leu Asp Ile
305 310 315 320

Phe Gly Thr Asn Arg His Pro Asp Asn Trp Glu Gln Ala Asp Glu Phe
325 330 335

Ile Pro Glu Arg Phe Lys Asp Trp Lys Gly Ser Pro Phe Ser Phe Ile
340 345 350

Pro Gln Gly Gly Gly Asp His His Ile Gly His Arg Cys Ala Gly Glu
355 360 365

Trp Leu Thr Val Met Val Met Arg Ser Phe Phe Lys Tyr Phe Val Asn
370 375 380

Asn Met Thr Tyr His Val Pro Glu Gln Asn Leu Ala Tyr Ser Leu Glu
385 390 395 400

Arg Met Pro Thr Phe Pro Lys Ser Gly Phe Val Ile Thr Asp Val Lys
405 410 415

Lys Ala Ala Asp Ser Gly Glu Lys Met Gln Glu Met Met His Asn Pro
420 425 430

Ile Thr Val Lys
435

<210> 36
<211> 434
<212> PRT
<213> Halobacillus salinus

<400> 36

Met Ser Lys Pro Ile Pro Lys Ala Glu Gly Ala Asp Asn Thr Arg Ser
1 5 10 15

Leu Ile Gln Glu Gly Phe Pro Phe Phe Ile Lys Arg His Lys Glu Leu
20 25 30

Gly Thr Asn Ile Phe Glu Thr Arg Leu Ile Gly Arg Lys Ala Ile Cys
35 40 45

Ile Val Gly Glu Glu Ala Ser Lys Ile Phe Tyr Asp Thr Glu Lys Phe
50 55 60

Lys Arg Glu Gly Ala Met Pro Lys Pro Leu Lys Met Ser Leu Phe Gly
65 70 75 80

Glu Gly Ser Leu His Gly Met Asp Gly Glu Lys His Leu His Arg Lys
85 90 95

Arg Thr Phe Leu Ser Met Phe Thr Pro Asp Arg Val Glu Asp Leu Lys
100 105 110

Gln Asn Ala Leu Glu Glu Leu Asp Arg Lys Val Glu Gln Trp Gln Glu
115 120 125

Arg Asp Lys Val Val Leu Tyr Glu Glu Phe Pro Glu Ile Leu Thr Arg
130 135 140

Ala Val Cys Lys Trp Ala Gly Val Pro Leu Glu Glu His Glu Val Arg
145 150 155 160

Gln Arg Thr Asp Glu Met Met Ser Met Val Glu Ser Phe Gly Gly Ser
165 170 175

Leu Ser Arg Phe Gln Glu Gly Lys Lys Ser Arg Asp Ser His Glu Ala
180 185 190

Trp Leu Lys Gln Ile Ile Lys Asp Val Arg Lys Gly Lys Leu Gln Pro
195 200 205

Glu Pro Asn Thr Ala Ala Tyr Ile Met Ala His His Arg Glu Thr Asp
210 215 220

Gly Lys Leu Trp Asp Val Gln Thr Ala Ala Val Glu Leu Ser Asn Ser
225 230 235 240

Phe Arg Pro Leu Leu Ala Thr Val His Leu Leu Val Leu Gly Ala Val
245 250 255

Ala Met His Glu His Pro Glu Thr Arg Thr Arg Leu Glu Lys Gly Glu
260 265 270

Pro Leu Tyr Ser His Trp Phe Ala Gln Glu Thr Arg Arg Phe Tyr Pro
275 280 285

Phe Val Pro Ala Met Ile Ala Lys Val Lys Thr Pro Phe Leu Trp Glu
290 295 300

Gly Tyr Glu Phe Lys Lys Asn Thr Arg Val Val Leu Asp Leu Tyr Gly
305 310 315 320

Thr Asn His His Ala Asp Ser Trp Val His Pro Asp Ala Phe Ile Pro
325 330 335

Glu Arg Phe Glu Asn Trp Gln Gly Ser Pro Phe Ser Phe Val Pro Gln
340 345 350

Gly Gly Gly Asp His His Thr Gly His Arg Cys Ala Gly Glu Trp Leu
355 360 365

Thr Val Ile Val Met Ser Ser Phe Phe Glu Phe Leu Ala Lys Asn Val
370 375 380

Ser Tyr Asp Val Ser Glu Gln Asn Leu Ser Phe Ser Leu Glu Arg Met
385 390 395 400

Pro Thr Arg Pro Glu Ser Gly Phe Val Ile Glu Asn Val Arg Lys Lys
405 410 415

Asp Ala Tyr Glu Glu Thr Phe Asp Glu Leu Asn Gln Gly Phe Ala Ala
420 425 430

Arg Arg

<210> 37
<211> 435
<212> PRT
<213> Halobacillus sp.

<400> 37

Met Ile His Lys Asp Ile Pro Arg Ala Lys Gly Ile Asp Asn Thr Met
1 5 10 15

Asn Leu Ile Lys Glu Gly Phe His Phe Leu Pro Asn Gln Arg Gln Ala
20 25 30

Leu Asn Ser Asp Val Phe Glu Thr Arg Leu Ile Gly Lys Lys Ala Ile
35 40 45

Cys Ile Ala Gly Glu Glu Ala Ser Lys Leu Phe Tyr Asp Thr Glu Lys
50 55 60

Phe Lys Arg Glu Gly Ala Met Pro Lys Pro Leu Lys Met Ser Leu Phe
65 70 75 80

Gly Asp Gly Ser Leu His Gly Leu Asp Gly Glu Glu His Arg Asn Arg
85 90 95

Lys Arg Thr Phe Leu Ser Met Phe Thr Pro Asp Arg Val Glu Asp Leu
100 105 110

Lys Lys Leu Ala Leu Lys His Leu Asp Ala Lys Ala Thr Glu Trp Gln
115 120 125

Glu Gln Glu Glu Val Tyr Leu Phe Asp Glu Met Gln Glu Ile Leu Ala
130 135 140

Arg Ala Gly Cys Glu Trp Ala Gly Val Pro Leu Gln Glu Glu Glu Val
145 150 155 160

Lys Gln Arg Thr Asp Glu Met Ile Glu Met Ile Asp Ser Phe Gly Gly
165 170 175

Ser Leu Ser Arg Phe Arg Glu Gly Lys Gln Ala Arg Asn His His Glu
180 185 190

Lys Trp Leu Gly Gln Ile Ile Lys Asp Val Arg Lys Gly Lys Leu Asp
195 200 205

Pro Glu Pro Asn Thr Ala Ala Tyr Ile Met Ala His His Arg Glu Thr
210 215 220

Asn Gly Lys Lys Trp Asp Thr Arg Thr Ala Ala Val Glu Leu Ser Asn
225 230 235 240

Ser Phe Arg Pro Leu Leu Ala Thr Ala Tyr Phe Leu Thr Phe Gly Ala
245 250 255

Val Ala Met His Glu His Pro Glu Ser Lys Glu Lys Leu Lys Gln Asp
260 265 270

Thr Ser Arg Tyr Ser His Trp Phe Ala Gln Glu Thr Arg Arg Tyr Tyr
275 280 285

Pro Phe Val Pro Ala Met Val Ala Lys Val Lys Glu Asp Phe Ile Trp
290 295 300

Asn Asp Phe Leu Phe Lys Lys Asn Thr Leu Val Val Leu Asp Ile Phe
305 310 315 320

Gly Thr Asn Arg His Pro Asp Ser Trp Glu Glu Pro Asp Ala Phe Ile
325 330 335

Pro Glu Arg Phe Lys Asp Trp Glu Gly Ser Pro Phe Ser Phe Ile Pro
340 345 350

Gln Gly Gly Gly Asp His His Thr Gly His Arg Cys Ala Gly Glu Trp
355 360 365

Leu Thr Val Ile Val Met Ser Ser Phe Phe Glu Phe Phe Ala Lys Asn
370 375 380

Leu Ser Tyr Asp Val Pro Glu Gln Asp Leu Ser Phe Ser Met Glu Arg
385 390 395 400

Met Pro Ser Tyr Pro Asn Ser Arg Phe Lys Ile Gln Asn Val Lys Lys
405 410 415

Thr Gly Tyr Tyr Asp Glu His Leu Ser Asp Leu Asn Arg Gly Phe Ser
420 425 430

Ala Ile Arg
435

<210> 38
<211> 438
<212> PRT
<213> Planomicrobium flavidum

<400> 38

Met Arg Ile Arg Lys Pro Ile Pro Lys Ala Glu Gly Leu Asp Asn Thr
1 5 10 15

Leu Ala Leu Ile Lys Glu Gly Phe His Phe Ile Pro Ser Arg Arg Glu
20 25 30

Lys Leu Ala Ser Asp Ile Phe Glu Thr Arg Leu Ile Gly Arg Lys Ala
35 40 45

Val Cys Met Ala Gly Glu Glu Ala Ala Ala Ile Phe Tyr Asp Asn Asn
50 55 60

Leu Phe Lys Arg Ala Gly Ala Ala Pro Lys Pro Leu Lys Gln Gly Leu
65 70 75 80

Leu Gly Val Gly Gly Ile His Gly Arg Asp Gly Glu Asp His His Gln
85 90 95

Gln Lys Arg Met Phe Leu Ser Met Met Thr Pro Glu Arg Leu Glu Asp
100 105 110

Met Arg Arg Ile Ala Val Glu Glu Leu Asp Arg Lys Ala Ala Ala Trp
115 120 125

Gln Lys Glu Asp Ser Val Ala Met Leu Pro Glu Met Glu Glu Ile Met
130 135 140

Gly Leu Ala Gly Met Arg Trp Ala Gly Leu Pro Leu Gly Ser Glu Gln
145 150 155 160

Glu Ala Lys Arg Arg Ala Lys Glu Leu Ala Asp Met Val Asp Ser Phe
165 170 175

Gly Gly Ser Phe Ser Arg Tyr Gln Glu Gly Lys Arg Ala Arg Glu Ser
180 185 190

His Glu Glu Trp Leu Arg Asn Ile Ile Arg Asp Ile Arg Gln Glu Lys
195 200 205

Tyr Asn Pro Pro Pro Tyr Thr Ala Ala Tyr Ile Val Ala His His Arg
210 215 220

Gly Ala Asp Gly Lys Leu Leu Asp Leu Glu Thr Ala Ala Val Asp Leu
225 230 235 240

Asn Asn Ser Tyr Arg Pro Leu Val Ala Thr Ala Tyr Phe Ile Val Phe
245 250 255

Gly Met Leu Ala Met Tyr Glu His Pro Glu Thr Val Ala Lys Ile Arg
260 265 270

Ser Asn Glu Gly Asn Tyr Ser His Met Phe Ser Gln Glu Ile Arg Arg
275 280 285

Tyr Tyr Pro Phe Ala Pro Ala Met Val Ala Lys Ala Lys Lys Asp Phe
290 295 300

Ile Trp His Gly Tyr His Phe Lys Lys Asp Gln Thr Val Val Leu Asp
305 310 315 320

Leu Phe Gly Thr Asn Arg His Pro Asp Asn Trp Glu Glu Ala Asp Arg
325 330 335

Phe Ile Pro Glu Arg Phe His Asn Trp Ser Gly Ser Pro Phe Ala Phe
340 345 350

Val Pro Gln Gly Gly Gly Asp His Tyr Met Gly His Arg Cys Ala Gly
355 360 365

Glu Trp Met Thr Val Ile Val Met Gln Ala Phe Phe His Tyr Leu Thr
370 375 380

Ala Gly Val Ala Tyr Asp Val Pro Glu Gln Asp Leu Thr Tyr Asp Leu

385 390 395 400

Arg Arg Met Pro Thr Met Pro Lys Ser Gly Phe Val Ile Arg Asn Val
405 410 415

Arg Arg Ser Gly Asn Tyr Pro Leu Asp Ala Leu Met Glu Asp Gln Asn
420 425 430

His Thr Ala Val Lys Phe
435

<210> 39
<211> 422
<212> PRT
<213> Staphylococcus delphini

<400> 39

Met Ala Lys Lys Leu Pro Lys Asp Thr Gly Leu Asp Asn Thr Leu Lys
1 5 10 15

Ile Ile Asn Glu Ala Tyr Thr Tyr Val Pro Lys Arg Leu Glu Lys Phe
20 25 30

Gly Thr Lys Ala Phe Glu Thr Arg Ala Leu Gly Met Lys Pro Val Val
35 40 45

Val Ile Ser Gly Lys Ala Ala Ala Glu Leu Phe Tyr Asp Asn Asp Lys
50 55 60

Ile Ser Arg Lys Gly Thr Leu Pro Lys Arg Ile Val His Thr Leu Phe
65 70 75 80

Gly Lys Gly Ala Ile His Thr Thr Glu Gly Lys Val His Val Asp Arg
85 90 95

Lys Ala Leu Phe Met Ser Leu Met Thr Glu Lys Asn Leu Lys Tyr Leu
100 105 110

Arg Glu Leu Thr Arg Asn Tyr Trp Phe Met His Thr Glu Arg Met Gln
115 120 125

Asn Lys Asp Lys Val Asn Val Tyr Gln Glu Ala Gly Leu Ile Leu Thr
130 135 140

Lys Val Gly Phe Arg Trp Ala Gly Leu Lys Gln Thr Asp Glu Gln Ala
145 150 155 160

Ala Gln Asn Ala Glu Asp Met Asn Thr Met Ile Asp Ser Phe Ser Gly

165 170 175

Leu Gly Gln Ser Leu Lys Gly Tyr Arg Glu Ala Lys Lys Ala Arg Ala
180 185 190

Arg Val Glu Gln Phe Leu Gln Asp Gln Ile Glu Ala Val Arg Glu Gly
195 200 205

Gln Gln Tyr Ala Glu Pro Gly Thr Ala Leu Tyr Glu Phe Ala His Trp
210 215 220

Lys Asp Leu Asn Asp Gln Pro Met Asp Pro Tyr Leu Cys Ala Val Asp
225 230 235 240

Leu Met Asn Ile Val Arg Pro Leu Val Ala Val Asn Arg Phe Val Ser
245 250 255

Tyr Gly Val Lys Ala Leu Ile Glu Phe Asp Gln Glu Arg Lys Lys Leu
260 265 270

Gln Val Thr Asn Asp Pro Asn Tyr Ala Tyr Lys Phe Ala Gln Glu Val
275 280 285

Arg Arg Ile Phe Pro Phe Val Pro Phe Leu Pro Gly Arg Leu Lys Lys
290 295 300

Thr Val Glu Phe Asp Gly Phe Lys Leu Lys Lys Gly Thr Leu Thr Val
305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Glu Ala Pro Ala Gln Asp Leu Ser Val
385 390 395 400

Lys Leu Asp Gln Phe Pro Gly Lys Val Thr Ser Gly Thr Ile Ile Lys
405 410 415

Ser Val Tyr Pro Arg Ile
420

<210> 40
<211> 422
<212> PRT
<213> Staphylococcus delphini

<400> 40

Met Ala Lys Lys Leu Pro Lys Asp Thr Gly Leu Asp Asn Thr Leu Lys
1 5 10 15

Ile Ile Asn Glu Ala Tyr Thr Tyr Val Pro Lys Arg Leu Glu Lys Phe
20 25 30

Gly Thr Lys Ala Phe Glu Thr Arg Ala Leu Gly Met Lys Pro Val Val
35 40 45

Val Ile Ser Gly Lys Ala Ala Ala Glu Leu Phe Tyr Asp Asn Asp Lys
50 55 60

Ile Ser Arg Lys Gly Thr Leu Pro Lys Arg Ile Val His Thr Leu Phe
65 70 75 80

Gly Lys Gly Ala Ile His Thr Thr Glu Gly Lys Val His Val Asp Arg
85 90 95

Lys Ala Leu Phe Met Ser Leu Met Thr Glu Lys Asn Leu Lys Tyr Leu
100 105 110

Arg Glu Leu Thr Arg Asn Tyr Trp Phe Met His Thr Glu Arg Met Gln
115 120 125

Asn Lys Asp Glu Val Asn Val Tyr Gln Glu Ala Gly Leu Ile Leu Thr
130 135 140

Lys Val Gly Phe Arg Trp Ala Gly Leu Lys Gln Thr Asp Glu Gln Ala
145 150 155 160

Ala Gln Asn Ala Glu Asp Met Asn Thr Met Ile Asp Ser Phe Ser Gly
165 170 175

Leu Gly Gln Ser Leu Lys Gly Tyr Arg Glu Ala Lys Lys Ala Arg Ala
180 185 190

Arg Val Glu Gln Phe Leu Gln Asp Gln Ile Glu Ala Val Arg Glu Gly
195 200 205

Gln Gln Tyr Ala Glu Pro Gly Thr Ala Leu Tyr Glu Phe Ala His Trp
    210                 215                 220

Lys Asp Leu Asn Asp Gln Pro Met Asp Pro His Leu Cys Ala Val Asp
225                 230                 235                 240

Leu Met Asn Ile Val Arg Pro Leu Val Ala Val Asn Arg Phe Val Ser
                245                 250                 255

Tyr Gly Val Lys Ala Leu Ile Glu Phe Asp Gln Glu Arg Lys Lys Leu
            260                 265                 270

Gln Val Thr Asn Asp Pro Asn Tyr Ala Tyr Lys Phe Ala Gln Glu Val
        275                 280                 285

Arg Arg Ile Phe Pro Phe Val Pro Phe Leu Pro Gly Arg Leu Lys Lys
    290                 295                 300

Thr Val Glu Phe Asp Gly Phe Lys Leu Lys Lys Gly Thr Leu Thr Val
305                 310                 315                 320

Leu Asp Ile Phe Gly Thr Thr His Asp Pro Glu Leu Phe Glu Asn Pro
                325                 330                 335

Tyr Gln Phe Asn Pro Asp Arg Phe Asp Asn Trp Asp Gly Ser Pro Phe
            340                 345                 350

Asp Leu Ile Pro Gln Gly Gly Gly Asp Phe Tyr Thr Asn His Arg Cys
        355                 360                 365

Ala Gly Glu Trp Met Thr Val Ile Val Met Glu Glu Thr Ile Gln Tyr
    370                 375                 380

Phe Ala Asn Lys Ile Asp Phe Glu Ala Pro Ala Gln Asp Leu Ser Val
385                 390                 395                 400

Lys Leu Asp Gln Phe Pro Gly Lys Val Thr Ser Gly Thr Ile Ile Lys
                405                 410                 415

Ser Val Tyr Pro Arg Ile
            420

<210> 41
<211> 422
<212> PRT
<213> Staphylococcus delphini

<400> 41

```
Met Ala Lys Lys Leu Pro Lys Asp Thr Gly Leu Asp Asn Thr Leu Lys
1               5                   10                  15

Ile Ile Asn Asp Ala Tyr Thr Tyr Val Pro Lys Arg Leu Glu Lys Phe
            20                  25                  30

Gly Thr Lys Ala Phe Glu Thr Arg Ala Leu Gly Met Lys Pro Val Val
        35                  40                  45

Val Ile Ser Gly Lys Ala Ala Ala Glu Leu Phe Tyr Asp Asn Asp Lys
    50                  55                  60

Ile Ser Arg Lys Gly Thr Leu Pro Lys Arg Ile Val His Thr Leu Phe
65                  70                  75                  80

Gly Lys Gly Ala Ile His Thr Thr Glu Gly Lys Val His Val Asp Arg
                85                  90                  95

Lys Ala Leu Phe Met Ser Leu Met Thr Glu Lys Asn Leu Lys Tyr Leu
            100                 105                 110

Arg Glu Leu Thr Arg Asn Tyr Trp Phe Met His Thr Glu Arg Met Gln
        115                 120                 125

Asn Lys Asp Glu Val Asn Val Tyr Gln Glu Ala Gly Leu Ile Leu Thr
    130                 135                 140

Lys Val Gly Phe Arg Trp Ala Gly Leu Lys Gln Thr Asp Glu Gln Ala
145                 150                 155                 160

Ala Gln Asn Ala Glu Asp Met Asn Thr Met Ile Asp Ser Phe Ser Gly
                165                 170                 175

Leu Gly Gln Ser Leu Lys Gly Tyr Arg Glu Ala Lys Lys Ala Arg Ala
            180                 185                 190

Arg Val Glu Gln Phe Leu Gln Asp Gln Ile Glu Ala Val Arg Glu Gly
        195                 200                 205

Gln Gln Tyr Ala Glu Pro Gly Thr Ala Leu Tyr Glu Phe Ala His Trp
    210                 215                 220

Lys Asp Leu Asn Asp Gln Pro Met Asp Pro His Leu Cys Ala Val Asp
225                 230                 235                 240

Leu Met Asn Ile Ile Arg Pro Leu Val Ala Val Asn Arg Phe Val Ser
                245                 250                 255
```

Tyr Gly Val Lys Ala Leu Ile Glu Phe Asp Gln Glu Arg Lys Lys Leu
260 265 270

Gln Val Thr Asn Asp Pro Asn Tyr Ala Tyr Lys Phe Ala Gln Glu Val
275 280 285

Arg Arg Ile Phe Pro Phe Val Pro Phe Leu Pro Gly Arg Leu Lys Lys
290 295 300

Thr Val Glu Phe Asp Gly Phe Lys Leu Lys Lys Gly Thr Leu Thr Val
305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Glu Ala Pro Ala Gln Asp Leu Ser Val
385 390 395 400

Lys Leu Asp Gln Phe Pro Gly Lys Val Thr Ser Gly Thr Ile Ile Lys
405 410 415

Ser Val Tyr Pro Arg Ile
420

<210> 42
<211> 423
<212> PRT
<213> Salinicoccus sp. CT19

<400> 42

Met Ser Thr Ile Lys Arg Asp Lys Gly Leu Asp Asn Thr Leu Lys Val
1 5 10 15

Met Lys Glu Gly Tyr Leu Tyr Thr Thr Asn Gln Arg Asn Arg Leu Gly
20 25 30

Ala Asp Asn Ile Phe Glu Thr Arg Gly Leu Gly Gly Lys Arg Val Leu
35 40 45

```
Val Leu Ser Gly Lys Lys Ala Ala Glu Thr Phe Tyr Asp Asn Asp Lys
    50                  55                  60

Ile Glu Arg Lys Gly Thr Leu Pro Lys Arg Val Val Asn Thr Leu Phe
65                  70                  75                  80

Gly Lys Gly Ala Ile His Thr Thr Thr Gly Lys Lys His Ile Asp Arg
                85                  90                  95

Lys Ala Leu Phe Met Ser Leu Met Thr Glu Gly Asn Leu Lys His Leu
            100                 105                 110

Arg Glu Leu Thr Arg Asn His Trp Tyr Met Asn Thr His Arg Met Glu
        115                 120                 125

Gln Met Asp Gln Ile Asn Ile Tyr Arg Glu Ser Ile Ile Leu Leu Thr
    130                 135                 140

Lys Val Gly Thr Lys Trp Ala Gly Val Gln Ala Pro Glu Glu Lys Ile
145                 150                 155                 160

Glu Glu Ile Ala Thr Asp Met Asp Ile Met Ile Asp Ser Phe Arg Gly
                165                 170                 175

Leu Gly Thr Ala Phe Lys Gly Tyr Arg Ser Ser Val Lys Ala Arg Arg
            180                 185                 190

Arg Val Glu Asp Trp Leu Glu Asp Gln Ile Ile Gln Thr Arg Lys Gly
        195                 200                 205

Lys Ile Phe Pro Pro Glu Gly Thr Ala Leu Tyr Glu Phe Ala His Trp
    210                 215                 220

Glu Asp Tyr Leu Gly Asn Pro Met Asp Ser Arg Leu Cys Ala Ile Asp
225                 230                 235                 240

Leu Met Asn Thr Phe Arg Pro Leu Ile Ala Ile Asn Arg Phe Val Ser
                245                 250                 255

Phe Gly Leu Leu Ala Met His Glu Phe Pro Val Ser Arg Glu Lys Ile
            260                 265                 270

Asn Asn Glu Asp Asp Tyr Ala Tyr Met Phe Ser Gln Glu Val Arg Arg
        275                 280                 285

Phe Tyr Pro Phe Val Pro Phe Leu Pro Ala Lys Ala Lys Thr Asp Phe
    290                 295                 300
```

His Phe Asp Gly His Lys Val Asp Lys Asp Thr Met Leu Leu Leu Asp
305 310 315 320

Ile Tyr Gly Thr Met His Arg Glu Asp Val Phe Glu Asn Pro Asn Glu
325 330 335

Phe Arg Pro Glu Arg Phe Leu Asp Trp Asp Gly Ser Pro Phe Asp Leu
340 345 350

Ile Pro Gln Gly Gly Gly Asp Tyr Tyr Thr Asn His Arg Cys Ala Gly
355 360 365

Glu Trp Met Thr Val Ile Ile Met Glu Glu Thr Met Lys Tyr Phe Ala
370 375 380

Gly Arg Ile Thr Tyr Asp Val Pro Glu Gln Asp Leu Arg Val Asp Leu
385 390 395 400

Asn Ser Ile Pro Gly Tyr Val Lys Ser Gly Phe Val Ile Glu Asn Val
405 410 415

Lys Glu Asn Leu Asp Arg Arg
420

<210> 43
<211> 422
<212> PRT
<213> Staphylococcus delphini

<400> 43

Met Ala Lys Lys Leu Pro Lys Asp Thr Gly Leu Asp Asn Thr Leu Lys
1 5 10 15

Ile Ile Asn Glu Ala Tyr Thr Tyr Val Pro Lys Arg Leu Glu Lys Phe
20 25 30

Gly Thr Lys Ala Phe Glu Thr Arg Ala Leu Gly Met Lys Pro Val Val
35 40 45

Val Ile Ser Gly Lys Ala Ala Ala Glu Leu Phe Tyr Asp Asn Asp Lys
50 55 60

Ile Ser Arg Lys Gly Thr Leu Pro Lys Arg Ile Val His Thr Leu Phe
65 70 75 80

Gly Lys Gly Ala Ile His Thr Thr Glu Gly Lys Val His Val Asp Arg
85 90 95

```
Lys Ala Leu Phe Met Ser Leu Met Thr Glu Lys Asn Leu Lys Tyr Leu
            100                 105                 110

Arg Glu Leu Thr Arg Asn Tyr Trp Phe Met His Thr Glu Arg Met Gln
        115                 120                 125

Asn Lys Asp Glu Val Asn Val Tyr Gln Glu Ala Gly Leu Ile Leu Thr
    130                 135                 140

Lys Val Gly Phe Arg Trp Ala Gly Leu Lys Gln Thr Asp Glu Gln Ala
145                 150                 155                 160

Ala Gln Asn Ala Glu Asp Met Asn Thr Met Ile Asp Ser Phe Ser Gly
                165                 170                 175

Leu Gly Gln Ser Leu Lys Gly Tyr Arg Glu Ala Lys Lys Ala Arg Ala
            180                 185                 190

Arg Val Glu Gln Phe Leu Gln Asp Gln Ile Glu Ala Val Arg Glu Gly
        195                 200                 205

Gln Gln Tyr Ala Glu Pro Gly Thr Ala Leu Tyr Glu Phe Ala His Trp
    210                 215                 220

Lys Asp Leu Asn Asp Gln Pro Met Asp Pro His Leu Cys Ala Val Asp
225                 230                 235                 240

Leu Met Asn Ile Val Arg Pro Leu Val Ala Val Asn Arg Phe Val Ser
                245                 250                 255

Tyr Gly Val Lys Ala Leu Ile Glu Phe Asp Gln Glu Arg Lys Lys Leu
            260                 265                 270

Gln Val Thr Asn Asp Pro Asn Tyr Ala Tyr Lys Phe Ala Gln Glu Val
        275                 280                 285

Arg Arg Ile Phe Pro Phe Val Pro Phe Val Pro Gly Arg Leu Lys Lys
    290                 295                 300

Thr Val Glu Phe Asp Gly Phe Lys Leu Lys Lys Gly Thr Leu Thr Val
305                 310                 315                 320

Leu Asp Ile Phe Gly Thr Thr His Asp Pro Glu Leu Phe Glu Asn Pro
                325                 330                 335

Tyr Gln Phe Asn Pro Asp Arg Phe Asp Asn Trp Asp Gly Ser Pro Phe
```

340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Glu Ala Pro Ala Gln Asp Leu Ser Val
385 390 395 400

Lys Leu Asp Gln Phe Pro Gly Lys Val Thr Ser Gly Thr Ile Ile Lys
405 410 415

Ser Val Tyr Pro Arg Ile
420

<210> 44
<211> 422
<212> PRT
<213> Staphylococcus delphini

<400> 44

Met Ala Lys Lys Leu Pro Lys Asp Thr Gly Leu Asp Asn Thr Leu Lys
1 5 10 15

Ile Ile Asn Glu Ala Tyr Thr Tyr Val Pro Lys Arg Leu Glu Lys Phe
20 25 30

Gly Thr Lys Ala Phe Glu Thr Arg Ala Leu Gly Met Lys Pro Val Val
35 40 45

Val Ile Ser Gly Lys Ala Ala Ala Glu Leu Phe Tyr Asp Asn Asp Lys
50 55 60

Ile Ser Arg Lys Gly Thr Leu Pro Lys Arg Ile Val His Thr Leu Phe
65 70 75 80

Gly Lys Gly Ala Ile His Thr Thr Glu Gly Lys Val His Val Asp Arg
85 90 95

Lys Ala Leu Phe Met Ser Leu Met Thr Glu Lys Asn Leu Lys Tyr Leu
100 105 110

Arg Glu Leu Thr Arg Asn Tyr Trp Phe Met His Thr Glu Arg Met Gln
115 120 125

Asn Lys Asp Glu Val Asn Val Tyr Gln Glu Ala Gly Leu Ile Leu Thr

130 135 140

Lys Val Gly Phe Arg Trp Ala Gly Leu Lys Gln Thr Asp Glu Gln Ala
145 150 155 160

Ala Gln Asn Ala Glu Asp Met Asn Thr Met Ile Asp Ser Phe Ser Gly
165 170 175

Leu Gly Gln Ser Leu Lys Gly Tyr Arg Glu Ala Lys Lys Ala Arg Ala
180 185 190

Arg Val Glu Gln Phe Leu Gln Asp Gln Ile Glu Ala Val Arg Ala Gly
195 200 205

Gln Gln Tyr Ala Glu Pro Gly Thr Ala Leu Tyr Glu Phe Ala His Trp
210 215 220

Lys Asp Leu Asn Asp Gln Pro Met Asp Pro His Leu Cys Ala Val Asp
225 230 235 240

Leu Met Asn Ile Val Arg Pro Leu Val Ala Val Asn Arg Phe Val Ser
245 250 255

Tyr Gly Val Lys Ala Leu Ile Glu Phe Asp Gln Glu Arg Lys Lys Leu
260 265 270

Gln Val Thr His Asp Pro Asn Tyr Ala Tyr Lys Phe Ala Gln Glu Val
275 280 285

Arg Arg Ile Phe Pro Phe Val Pro Phe Leu Pro Gly Arg Leu Lys Gln
290 295 300

Thr Val Glu Phe Asp Gly Phe Lys Leu Lys Lys Gly Thr Leu Thr Val
305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Glu Ala Pro Ala Gln Asp Leu Ser Val
385                 390                 395                 400

Lys Leu Asp Gln Phe Pro Gly Lys Val Thr Ser Gly Thr Ile Ile Lys
                405                 410                 415

Ser Val Tyr Pro Arg Ile
            420

<210>  45
<211>  422
<212>  PRT
<213>  Staphylococcus delphini

<400>  45

Met Ala Lys Gln Leu Pro Lys Asp Thr Gly Leu Asp Asn Thr Leu Lys
1               5                   10                  15

Ile Ile Asn Glu Ala Tyr Thr Tyr Val Pro Lys Arg Leu Glu Lys Phe
            20                  25                  30

Gly Thr Lys Ala Phe Glu Thr Arg Ala Leu Gly Met Lys Pro Val Val
        35                  40                  45

Val Ile Ser Gly Lys Ala Ala Ala Glu Leu Phe Tyr Asp Asn Asp Lys
    50                  55                  60

Ile Ser Arg Lys Gly Thr Leu Pro Lys Arg Ile Val His Thr Leu Phe
65                  70                  75                  80

Gly Lys Gly Ala Ile His Thr Thr Glu Gly Lys Val His Val Asp Arg
                85                  90                  95

Lys Ala Leu Phe Met Ser Leu Met Thr Glu Lys Asn Leu Lys Tyr Leu
            100                 105                 110

Arg Glu Leu Thr Arg Asn Tyr Trp Phe Met His Thr Glu Arg Met Gln
        115                 120                 125

Asn Lys Asp Glu Val Asn Val Tyr Gln Glu Ala Gly Leu Ile Leu Thr
    130                 135                 140

Lys Val Gly Phe Arg Trp Ala Gly Leu Lys Gln Thr Asp Glu Gln Ala
145                 150                 155                 160

Ala Gln Asn Ala Glu Asp Met Asn Thr Met Ile Asp Ser Phe Ser Gly
                165                 170                 175

```
Leu Gly Gln Ser Leu Lys Gly Tyr Arg Glu Ala Lys Lys Ala Arg Ala
            180                 185                 190

Arg Val Glu Gln Phe Leu Gln Asp Gln Ile Glu Ala Val Arg Ala Gly
        195                 200                 205

Gln Gln Tyr Ala Glu Pro Gly Thr Ala Leu Tyr Glu Phe Ala His Trp
    210                 215                 220

Lys Asp Leu Asn Asp Gln Pro Met Asp Pro His Leu Cys Ala Val Asp
225                 230                 235                 240

Leu Met Asn Ile Val Arg Pro Leu Val Ala Val Asn Arg Phe Val Ser
                245                 250                 255

Tyr Gly Val Lys Ala Leu Ile Glu Phe Asp Gln Glu Arg Lys Lys Leu
            260                 265                 270

Gln Val Thr His Asp Pro Asn Tyr Ala Tyr Lys Phe Ala Gln Glu Val
        275                 280                 285

Arg Arg Ile Phe Pro Phe Val Pro Phe Leu Pro Gly Arg Leu Lys Lys
    290                 295                 300

Thr Val Glu Phe Asp Gly Phe Lys Leu Lys Lys Gly Thr Leu Thr Val
305                 310                 315                 320

Leu Asp Ile Phe Gly Thr Thr His Asp Pro Glu Leu Phe Lys Asn Pro
                325                 330                 335

Tyr Gln Phe Asn Pro Asp Arg Phe Asp Asn Trp Asp Gly Ser Pro Phe
            340                 345                 350

Asp Leu Ile Pro Gln Gly Gly Gly Asp Phe Tyr Thr Asn His Arg Cys
        355                 360                 365

Ala Gly Glu Trp Met Thr Val Ile Val Met Glu Glu Thr Ile Gln Tyr
    370                 375                 380

Phe Ala Asn Lys Ile Asp Phe Glu Ala Pro Ala Gln Asp Leu Ser Val
385                 390                 395                 400

Lys Leu Asp Gln Phe Pro Gly Lys Val Thr Ser Gly Thr Ile Ile Lys
                405                 410                 415

Ser Val Tyr Pro Arg Ile
            420
```

<210> 46
<211> 422
<212> PRT
<213> Staphylococcus delphini

<400> 46

Met Ala Lys Lys Leu Pro Lys Asp Thr Gly Leu Asp Asn Thr Leu Lys
1               5                   10                  15

Ile Ile Asn Glu Ala Tyr Thr Tyr Val Pro Lys Arg Leu Glu Asn Phe
            20                  25                  30

Gly Thr Lys Ala Phe Glu Thr Arg Ala Leu Gly Met Lys Pro Val Val
        35                  40                  45

Val Ile Ser Gly Lys Ala Ala Ala Glu Leu Phe Tyr Asp Asn Asp Lys
    50                  55                  60

Ile Ser Arg Lys Gly Thr Leu Pro Lys Arg Ile Val His Thr Leu Phe
65                  70                  75                  80

Gly Lys Gly Ala Ile His Thr Thr Glu Gly Lys Val His Val Asp Arg
                85                  90                  95

Lys Ala Leu Phe Met Ser Leu Met Thr Glu Lys Asn Leu Lys Tyr Leu
            100                 105                 110

Arg Glu Leu Thr Arg Asn Tyr Trp Phe Met His Thr Glu Arg Met Gln
        115                 120                 125

Asn Lys Asp Glu Val Asn Val Tyr Gln Glu Ala Gly Leu Ile Leu Thr
    130                 135                 140

Lys Val Gly Phe Arg Trp Ala Gly Leu Lys Gln Thr Asp Glu Gln Ala
145                 150                 155                 160

Ala Gln Asn Ala Glu Asp Met Asn Thr Met Ile Asp Ser Phe Ser Gly
                165                 170                 175

Leu Gly Gln Ser Leu Lys Gly Tyr Arg Glu Ala Lys Lys Ala Arg Ala
            180                 185                 190

Arg Val Glu Gln Phe Leu Gln Asp Gln Ile Glu Ala Val Arg Glu Gly
        195                 200                 205

Gln Gln Tyr Ala Glu Pro Gly Thr Ala Leu Tyr Glu Phe Ala His Trp
    210                 215                 220

Lys Asp Leu Asn Asp Gln Pro Met Asp Pro His Leu Cys Ala Val Asp
225 230 235 240

Leu Met Asn Ile Val Arg Pro Leu Val Ala Val Asn Arg Phe Val Ser
245 250 255

Tyr Gly Val Lys Ala Leu Ile Glu Phe Asp Gln Glu Arg Lys Lys Leu
260 265 270

Gln Val Thr Asn Asp Pro Asn Tyr Ala Tyr Lys Phe Ala Gln Glu Val
275 280 285

Arg Arg Ile Phe Pro Phe Val Pro Phe Leu Pro Gly Arg Leu Lys Lys
290 295 300

Thr Val Glu Phe Asp Gly Phe Lys Leu Lys Lys Gly Thr Leu Thr Val
305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Glu Ala Pro Ala Gln Asp Leu Ser Val
385 390 395 400

Lys Leu Asp Gln Phe Pro Gly Lys Val Thr Ser Gly Thr Ile Ile Lys
405 410 415

Ser Val Tyr Pro Arg Ile
420

<210> 47
<211> 422
<212> PRT
<213> Staphylococcus delphini

<400> 47

Met Ala Lys Lys Leu Pro Arg Asp Thr Gly Leu Asp Asn Thr Leu Lys
1 5 10 15

```
Ile Ile Asn Glu Ala Tyr Thr Tyr Val Pro Lys Arg Leu Glu Lys Phe
            20                  25                  30

Gly Thr Lys Ala Phe Glu Thr Arg Ala Leu Gly Met Lys Pro Val Val
        35                  40                  45

Val Ile Ser Gly Lys Ala Ala Ala Glu Leu Phe Tyr Asp Asn Asp Lys
    50                  55                  60

Ile Ser Arg Lys Gly Thr Leu Pro Lys Arg Ile Val His Thr Leu Phe
65                  70                  75                  80

Gly Lys Gly Ala Ile His Thr Thr Glu Gly Lys Val His Val Asp Arg
                85                  90                  95

Lys Ala Leu Phe Met Ser Leu Met Thr Glu Lys Asn Leu Lys Tyr Leu
            100                 105                 110

Arg Glu Leu Thr Arg Asn Tyr Trp Phe Met His Thr Glu Arg Met Gln
        115                 120                 125

Asn Lys Asp Glu Val Asn Val Tyr Gln Glu Ala Gly Leu Ile Leu Thr
    130                 135                 140

Lys Val Gly Phe Arg Trp Ala Gly Leu Lys Gln Thr Asp Glu Gln Ala
145                 150                 155                 160

Ala Gln Asn Ala Glu Asp Met Asn Leu Met Ile Asp Ser Phe Ser Gly
                165                 170                 175

Leu Gly Gln Ser Leu Lys Gly Tyr Arg Glu Ala Lys Lys Ala Arg Ala
            180                 185                 190

Arg Val Glu Gln Phe Leu Gln Asp Gln Ile Glu Ala Val Arg Ala Gly
        195                 200                 205

Gln Gln Tyr Ala Glu Pro Gly Thr Ala Leu Tyr Glu Phe Ala His Trp
    210                 215                 220

Lys Asp Leu Asn Asp Gln Pro Met Asp Pro His Leu Cys Ala Val Asp
225                 230                 235                 240

Leu Met Asn Ile Val Arg Pro Leu Val Ala Val Asn Arg Phe Val Ser
                245                 250                 255

Tyr Gly Val Lys Ala Leu Ile Glu Phe Asp Gln Glu Arg Lys Lys Leu
            260                 265                 270
```

Gln Val Thr Asn Asp Pro Asn Tyr Ala Tyr Lys Phe Ala Gln Glu Val
275 280 285

Arg Arg Ile Phe Pro Phe Val Pro Phe Leu Pro Gly Arg Leu Lys Gln
290 295 300

Thr Val Glu Phe Asp Gly Phe Lys Leu Lys Lys Gly Thr Leu Thr Val
305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Glu Ala Pro Val Gln Asp Leu Ser Val
385 390 395 400

Lys Leu Asp Gln Phe Pro Gly Lys Val Thr Ser Gly Thr Ile Ile Lys
405 410 415

Ser Val Tyr Pro Arg Ile
420

<210> 48
<211> 422
<212> PRT
<213> Staphylococcus cornubiensis

<400> 48

Met Ala Lys Lys Leu Pro Lys Asp Thr Gly Leu Asp Asn Thr Leu Lys
1 5 10 15

Ile Ile Asn Glu Ala Tyr Thr Tyr Val Pro Lys Arg Leu Glu Gln Phe
20 25 30

Gly Thr Lys Ala Phe Glu Thr Arg Ala Leu Gly Met Lys Pro Val Val
35 40 45

Val Ile Ser Gly Lys Glu Ala Ala Lys Leu Phe Tyr Asp Asn Asp Lys
50 55 60

Ile Ser Arg Lys Gly Thr Leu Pro Lys Arg Ile Val His Thr Leu Phe
65 70 75 80

Gly Lys Gly Ala Ile His Thr Thr Glu Gly Lys Val His Val Asp Arg
85 90 95

Lys Ala Leu Phe Met Ser Leu Met Thr Glu Lys Asn Leu Lys Tyr Leu
100 105 110

Arg Glu Leu Thr Arg Asn Tyr Trp Phe Met Asn Thr Glu Arg Met Gln
115 120 125

Asn Met Asp Glu Val Asn Val Tyr Gln Glu Ala Gly Leu Ile Leu Thr
130 135 140

Gln Val Gly Phe Arg Trp Ala Gly Leu Lys Gln Thr Asp Glu Glu Ala
145 150 155 160

Ala Lys Asn Ala Glu Asp Met Asn Ile Met Ile Asp Ser Phe Ser Gly
165 170 175

Leu Gly Gln Ser Leu Lys Gly Tyr Arg Glu Ala Lys Lys Ala Arg Ala
180 185 190

Arg Val Glu Gln Phe Leu Gln Asp Gln Ile Glu Ala Val Arg Ala Gly
195 200 205

Glu Gln Tyr Ala Glu Pro Gly Thr Ala Leu Tyr Glu Phe Ala His Trp
210 215 220

Lys Asp Leu Asn Asp Gln Pro Met Asp Pro His Leu Cys Val Val Asp
225 230 235 240

Leu Met Asn Ile Val Arg Pro Leu Val Ala Val Asn Arg Phe Val Ser
245 250 255

Tyr Gly Val Lys Ala Leu Ile Glu Phe Asp Gln Glu Arg Lys Lys Leu
260 265 270

Gln Val Thr Asn Asp Ala Asn Tyr Ala Tyr Lys Phe Ala Gln Glu Val
275 280 285

Arg Arg Ile Phe Pro Phe Val Pro Phe Leu Pro Gly Arg Leu Thr Lys
290 295 300

Thr Val Glu Phe Glu Gly Phe Lys Ile Lys Lys Asp Thr Leu Val Ala

305 310 315 320

Leu Asp Ile Phe Gly Thr Thr His Asp Pro Glu Leu Phe Glu Asn Pro
325 330 335

Tyr Gln Phe Asn Pro Asp Arg Phe Glu Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Met Gln Tyr
370 375 380

Phe Ala Asn Lys Ile Asp Phe Asp Ala Pro Ala Gln Asp Leu Ser Val
385 390 395 400

Lys Leu Asp Gln Phe Pro Gly Lys Val Thr Ser Gly Thr Ile Ile Lys
405 410 415

Asn Val Tyr Pro Arg Ile
420

<210> 49
<211> 422
<212> PRT
<213> Staphylococcus delphini

<400> 49

Met Ala Lys Lys Leu Pro Arg Asp Thr Gly Leu Asp Asn Thr Leu Lys
1 5 10 15

Ile Ile Asn Glu Ala Tyr Thr Tyr Val Pro Lys Arg Leu Glu Lys Phe
20 25 30

Gly Thr Lys Ala Phe Glu Thr Arg Ala Leu Gly Met Lys Pro Val Val
35 40 45

Val Ile Ser Gly Lys Ala Ala Ala Glu Leu Phe Tyr Asp Asn Asp Lys
50 55 60

Ile Ser Arg Lys Gly Thr Leu Pro Lys Arg Ile Val His Thr Leu Phe
65 70 75 80

Gly Lys Gly Ala Ile His Thr Thr Glu Gly Lys Val His Val Asp Arg
85 90 95

Lys Ala Leu Phe Met Ser Leu Met Thr Glu Lys Asn Leu Lys Tyr Leu

100 105 110

Arg Glu Leu Thr Arg Asn Tyr Trp Phe Met His Thr Glu Arg Met Gln
115 120 125

Asn Lys Asp Glu Val Asn Val Tyr Gln Glu Ala Gly Leu Ile Leu Thr
130 135 140

Lys Val Gly Phe Arg Trp Ala Gly Leu Lys Gln Thr Asp Glu Gln Ala
145 150 155 160

Ala Gln Asn Ala Glu Asp Met Asn Thr Met Ile Asp Ser Phe Ser Gly
165 170 175

Leu Gly Gln Ser Leu Lys Gly Tyr Arg Glu Ala Lys Lys Ala Arg Ala
180 185 190

Arg Val Glu Gln Phe Leu Gln Asp Gln Ile Glu Ala Val Arg Ala Gly
195 200 205

Gln Gln Tyr Ala Glu Pro Gly Thr Ala Leu Tyr Glu Phe Ala His Trp
210 215 220

Lys Asp Leu Asn Asp Gln Pro Met Asp Pro His Leu Cys Ala Val Asp
225 230 235 240

Leu Met Asn Ile Val Arg Pro Leu Val Ala Val Asn Arg Phe Val Ser
245 250 255

Tyr Gly Val Lys Ala Leu Ile Glu Phe Asp Gln Glu Arg Lys Lys Leu
260 265 270

Gln Val Thr His Asp Pro Asn Tyr Ala Tyr Lys Phe Ala Gln Glu Val
275 280 285

Arg Arg Ile Phe Pro Phe Val Pro Phe Leu Pro Gly Arg Leu Lys Gln
290 295 300

Thr Val Glu Phe Asp Gly Phe Lys Leu Lys Lys Gly Thr Leu Thr Val
305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
        355                 360                 365

Ala Gly Glu Trp Met Thr Val Ile Val Met Glu Glu Thr Ile Gln Tyr
    370                 375                 380

Phe Ala Asn Lys Ile Asp Phe Glu Ala Pro Ala Gln Asp Leu Ser Val
385                 390                 395                 400

Lys Leu Asp Gln Phe Pro Gly Lys Val Thr Ser Gly Thr Ile Ile Lys
                405                 410                 415

Ser Val Tyr Pro Arg Ile
            420

<210>  50
<211>  422
<212>  PRT
<213>  Staphylococcus intermedius

<400>  50

Met Ala Lys Lys Leu Pro Lys Asp Thr Gly Leu Asp Asn Thr Phe Lys
1               5                   10                  15

Ile Ile Asn Glu Ala Tyr Thr Tyr Val Pro Lys Arg Leu Glu Gln Phe
            20                  25                  30

Gly Thr Lys Ala Phe Glu Thr Arg Ala Leu Gly Met Lys Pro Val Val
        35                  40                  45

Val Ile Ser Gly Lys Ala Ala Ala Lys Leu Phe Tyr Asp Asn Asp Lys
    50                  55                  60

Ile Ser Arg Lys Gly Thr Leu Pro Lys Arg Ile Val His Thr Leu Phe
65                  70                  75                  80

Gly Lys Gly Ala Ile His Thr Thr Glu Gly Lys Val His Val Asp Arg
                85                  90                  95

Lys Ala Leu Phe Met Ser Leu Met Thr Glu Lys Asn Leu Lys Tyr Leu
            100                 105                 110

Arg Glu Leu Thr Arg Asn Tyr Trp Phe Met His Thr Glu Arg Met Gln
        115                 120                 125

Asn Met Asp Glu Val Asn Val Tyr Lys Glu Ala Gly Leu Ile Leu Thr
    130                 135                 140

Gln Val Gly Phe Arg Trp Ala Gly Leu Lys Gln Thr Asp Glu Glu Ala
145 150 155 160

Ala Lys Asn Ala Glu Asp Met Asn Ile Met Ile Asp Ser Phe Ser Gly
165 170 175

Leu Gly Gln Ser Leu Lys Gly Tyr Arg Gln Ala Lys Lys Ala Arg Ala
180 185 190

Arg Val Glu Gln Phe Leu Gln Asp Gln Ile Glu Ala Val Arg Ala Gly
195 200 205

Lys Gln Tyr Ala Glu Pro Gly Thr Ala Leu Tyr Glu Phe Ala His Trp
210 215 220

Lys Asp Leu Asn Asn Gln Pro Met Asp Ser His Leu Cys Ala Val Asp
225 230 235 240

Leu Met Asn Ile Val Arg Pro Leu Val Ala Val Asn Arg Phe Val Ser
245 250 255

Tyr Gly Val Lys Ala Leu Ile Glu Phe Asp Gln Glu Arg Lys Lys Leu
260 265 270

Gln Val Thr Asn Asp Pro Asn Tyr Ala Tyr Lys Phe Ala Gln Glu Val
275 280 285

Arg Arg Ile Phe Pro Phe Val Pro Phe Leu Pro Gly Arg Leu Thr Lys
290 295 300

Thr Val Glu Phe Asp Gly Phe Lys Ile Lys Lys Gly Thr Leu Val Ala
305 310 315 320

Leu Asp Ile Phe Gly Thr Thr His Asp Pro Glu Leu Phe Glu Asn Pro
325 330 335

Tyr Gln Phe Asn Pro Asp Arg Phe Glu Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Asp Ala Pro Ala Gln Asp Leu Ser Val
385 390 395 400

Lys Leu Asp Gln Phe Pro Gly Lys Val Thr Ser Gly Thr Val Ile Lys
405 410 415

Asn Val Tyr Pro Arg Ile
420

<210> 51
<211> 423
<212> PRT
<213> Salinicoccus roseus

<400> 51

Met Ser Thr Ile Lys Lys His Lys Gly Leu Asp Asn Thr Leu Lys Val
1 5 10 15

Met Lys Glu Gly Tyr Leu Tyr Thr Thr Asn Gln Arg Arg Arg Leu Gly
20 25 30

Ala Glu Asn Ile Phe Glu Thr Arg Ala Leu Gly Gly Lys Arg Val Leu
35 40 45

Val Ile Ser Gly Lys Lys Ala Ala Glu Leu Phe Tyr Asp Asn Asp Lys
50 55 60

Thr Glu Arg Ser Gly Thr Leu Pro Lys Arg Leu Val Asn Thr Leu Phe
65 70 75 80

Gly Lys Gly Ala Ile His Thr Thr Thr Gly Lys Lys His Ile Asp Arg
85 90 95

Lys Ala Leu Phe Met Ser Leu Met Thr Glu Gly Asn Leu Lys His Leu
100 105 110

Arg Glu Leu Thr Arg Asn His Trp Tyr Met Asn Thr His Arg Met Glu
115 120 125

Gln Met Asp Gln Ile Asn Ile Tyr Arg Glu Ser Ile Ile Leu Leu Thr
130 135 140

Lys Val Gly Thr Lys Trp Ala Gly Val Gln Ala Pro Glu Glu Lys Ile
145 150 155 160

Glu Glu Ile Ala Thr Asp Met Asp Ile Met Ile Asp Ser Phe Lys Gly
165 170 175

Leu Gly Thr Ala Phe Lys Gly Tyr Lys Glu Ser Val Ala Ala Arg Arg
180 185 190

Arg Val Glu Asp Trp Leu Glu Asp Gln Ile Ile Gln Thr Arg Lys Gly
        195                 200                 205

Lys Ile Phe Pro Pro Glu Gly Thr Ala Leu Tyr Glu Phe Ala His Trp
    210                 215                 220

Glu Asp Tyr Lys Gly Asn Pro Met Asp Ser Arg Leu Cys Ala Ile Asp
225                 230                 235                 240

Leu Met Asn Thr Phe Arg Pro Leu Ile Ala Ile Asn Arg Phe Val Ser
                245                 250                 255

Phe Gly Leu Leu Ala Met His Glu Tyr Pro Ile Ser Lys Glu Lys Ile
            260                 265                 270

Asn Asn Asp Pro Asp Tyr Ala Tyr Met Phe Ser Gln Glu Val Arg Arg
        275                 280                 285

Phe Tyr Pro Phe Val Pro Phe Leu Pro Gly Lys Ala Lys Thr Asp Ile
    290                 295                 300

Gln Phe Glu Gly His Asp Ile Glu Lys Asp Thr Met Leu Val Leu Asp
305                 310                 315                 320

Ile Tyr Gly Thr Met His Gln Asp Asp Val Phe Glu Asn Ala Asp Glu
                325                 330                 335

Phe Tyr Pro Glu Arg Phe Leu Asp Trp Asp Gly Ser Pro Phe Asp Leu
            340                 345                 350

Ile Pro Gln Gly Gly Gly Asp Tyr Tyr Thr Asn His Arg Cys Ala Gly
        355                 360                 365

Glu Trp Met Thr Ile Ile Ile Met Glu Glu Thr Met Lys Tyr Phe Ala
    370                 375                 380

Gly Lys Ile Thr Tyr Asp Val Pro Glu Gln Asp Leu Thr Val Asp Leu
385                 390                 395                 400

Asn Ser Ile Pro Gly Tyr Ile Lys Ser Gly Phe Ile Ile Glu Asn Val
                405                 410                 415

Ala Glu Lys Val Asp Arg Lys
            420

<210>  52
<211>  423

<212> PRT
<213> Salinicoccus halodurans

<400> 52

```
Met Gly Thr Ile Lys Lys Asp Lys Gly Leu Asp Asn Thr Leu Lys Val
1               5                   10                  15

Met Lys Gln Gly Tyr Leu Tyr Thr Ala Asn Gln Arg Gln Arg Leu Gly
            20                  25                  30

Ala Asp Asn Ile Phe Glu Thr Arg Ala Leu Gly Gly Lys Arg Val Val
        35                  40                  45

Val Ile Asn Gly Lys Glu Ala Ala Glu Leu Phe Tyr Asp Asn Asp Lys
    50                  55                  60

Thr Glu Arg Ser Gly Thr Leu Pro Lys Arg Leu Val Asn Thr Leu Phe
65                  70                  75                  80

Gly Lys Gly Ala Ile His Thr Thr Thr Gly Lys Lys His Ile Asp Arg
                85                  90                  95

Lys Ala Leu Phe Met Ser Leu Met Thr Glu Asn Asn Leu Gln His Leu
            100                 105                 110

Arg Glu Leu Thr Arg Asn His Trp Tyr Met Asn Thr His Lys Met Glu
        115                 120                 125

Gln Met Asp Gln Ile Asn Ile Tyr Arg Glu Ser Ile Val Gln Leu Thr
    130                 135                 140

Lys Ile Gly Thr Lys Trp Ala Gly Val Gln Ala Pro Glu Glu Lys Ile
145                 150                 155                 160

Glu Asp Ile Ala Thr Asp Met Asp Ile Met Ile Asp Ser Phe Lys Gly
                165                 170                 175

Leu Gly Ser Ala Phe Lys Gly Tyr Lys Glu Ser Val Asn Ala Arg Lys
            180                 185                 190

Arg Val Glu Asp Trp Leu Glu Glu Gln Ile Ile Glu Thr Arg Lys Gly
        195                 200                 205

Lys Ile Phe Pro Pro Glu Gly Thr Ala Leu Tyr Glu Phe Ala His Trp
    210                 215                 220

Glu Asp Tyr Gln Gly Asn Pro Met Asp Ser Arg Leu Cys Ala Ile Asp
225                 230                 235                 240
```

Leu Met Asn Thr Phe Arg Pro Leu Ile Ala Ile Asn Arg Phe Val Ser
245 250 255

Phe Gly Leu Leu Ala Met His Glu His Pro Ile Ser Lys Glu Lys Ile
260 265 270

Lys Thr Asp Asp Asp Tyr Ala Tyr Met Phe Ser Gln Glu Val Arg Arg
275 280 285

Phe Tyr Pro Phe Val Pro Phe Leu Pro Gly Lys Ala Lys Thr Asp Ile
290 295 300

Glu Phe Lys Gly His Lys Ile Glu Lys Asp Thr Met Met Val Ile Asp
305 310 315 320

Ile Tyr Gly Thr Met His Asp Glu Lys Val Phe Glu Asn Pro Asp Glu
325 330 335

Phe Tyr Pro Glu Arg Phe Lys Glu Trp Asp Gly Ser Pro Phe Asp Leu
340 345 350

Ile Pro Gln Gly Gly Gly Asp Tyr Tyr Thr Asn His Arg Cys Ala Gly
355 360 365

Glu Trp Met Thr Ile Ile Ile Met Glu Glu Thr Met Lys Tyr Phe Ala
370 375 380

Glu Arg Ile Thr Tyr Asp Val Pro Glu Gln Asp Leu Thr Val Asp Leu
385 390 395 400

Asn Ser Ile Pro Gly Tyr Val Asn Ser Gly Phe Ile Ile Glu Asn Val
405 410 415

Gln Glu Asn Ile Asp Arg Lys
420

<210> 53
<211> 424
<212> PRT
<213> Salinicoccus kekensis

<400> 53

Met Ala Thr Ile Lys Lys Asp Lys Gly Ile Asp Asn Thr Ala Lys Ile
1 5 10 15

Met Lys Gln Gly Tyr Leu Tyr Thr Thr Asn Gln Arg Glu Arg Leu Gly
20 25 30

Val Lys Asp Gly Val Phe Glu Thr Arg Ala Leu Gly Gly Lys Arg Val
35 40 45

Ile Ile Leu Ser Gly Lys Asp Gly Ala Glu Leu Phe Tyr Asp Asn Asp
50 55 60

Lys Ile Glu Arg Ser Gly Thr Leu Pro Lys Arg Val Val Asn Thr Leu
65 70 75 80

Phe Gly Lys Gly Ala Ile His Thr Thr Thr Gly Lys Val His Ile Asp
85 90 95

Arg Lys Ala Leu Phe Met Ser Leu Met Thr Glu Gly Asn Leu Gln Tyr
100 105 110

Leu Arg Glu Leu Thr Arg Asn His Trp Gln Met Asn Thr Gln Arg Met
115 120 125

Glu Asn Met Asp Gln Val Asn Val Tyr Arg Glu Ser Ile Ile Leu Leu
130 135 140

Thr Lys Val Gly Thr Arg Trp Ala Gly Val Gln Ala Pro Glu Lys Glu
145 150 155 160

Ile Glu Asn Ile Ala Thr Asp Met Asp Ile Met Ile Asp Ser Phe Lys
165 170 175

Ala Ile Gly Ser Ala Phe Lys Gly Tyr Arg Ala Ser Lys Ala Ala Arg
180 185 190

Arg Arg Val Glu Asp Trp Leu Glu Asp Gln Ile Ile Gln Thr Arg Lys
195 200 205

Gly Lys Ile His Pro Pro Glu Gly Thr Ala Leu Tyr Glu Phe Ala His
210 215 220

Trp Lys Asp Tyr Lys Gly Glu Pro Met Asp Ser Arg Leu Cys Gly Ile
225 230 235 240

Asp Leu Met Asn Thr Phe Arg Pro Leu Ile Ala Ile Asn Arg Phe Ile
245 250 255

Ala Phe Gly Ala Leu Ala Met Tyr Glu Asn Pro Val Ala Arg Glu Lys
260 265 270

Ile Lys Gln Asp Asp Asp Tyr Ala Tyr Met Phe Ala Gln Glu Val Arg

275 280 285

Arg Phe Tyr Pro Phe Val Pro Phe Leu Pro Gly Lys Val Lys Glu Asp
290 295 300

Phe Gln Tyr Lys Gly Tyr Asp Phe Glu Lys Asp Thr Met Leu Ala Ile
305 310 315 320

Asp Ile Tyr Gly Thr Met His Asp Pro Thr Val Trp Glu Asn Pro Asn
325 330 335

Glu Phe Tyr Pro Glu Arg Phe Lys Asp Trp Asp Gly Ser Pro Phe Asp
340 345 350

Leu Ile Pro Gln Gly Gly Gly Asp Tyr Tyr Thr Asn His Arg Cys Ala
355 360 365

Gly Glu Trp Met Thr Val Ile Ile Met Gln Glu Thr Met Lys Tyr Phe
370 375 380

Ala Ser Arg Ile Thr Tyr Asp Val Pro Glu Gln Asp Leu Thr Val Asp
385 390 395 400

Leu Asn Ser Leu Pro Gly Tyr Val Lys Ser Gly Phe Ile Ile Glu Asn
405 410 415

Val Arg Glu Val Val Asp Arg Gly
420

<210> 54
<211> 421
<212> PRT
<213> Staphylococcus felis

<400> 54

Met Asn Lys Ile Pro Lys Asp Lys Gly Leu Asp Asn Thr Leu Lys Ile
1 5 10 15

Val Lys Glu Ala Tyr Thr Tyr Val Pro Arg Arg Leu Glu Ala Phe Asp
20 25 30

Thr Asn Val Phe Glu Thr Arg Ala Leu Gly Met Lys Lys Val Leu Val
35 40 45

Val Ser Gly Lys Lys Ala Ala Glu Leu Phe Tyr Asp Asn Glu Lys Ile
50 55 60

Ser Arg Lys Gly Ala Leu Pro Lys Arg Ile Val Asn Thr Leu Phe Gly

65 70 75 80

Gln Gly Ala Ile His Thr Thr Glu Gly Lys Gln His Val Asp Arg Lys
85 90 95

Ala Leu Phe Met Ser Leu Met Thr Thr Lys Asn Leu Lys Tyr Leu Arg
100 105 110

Glu Leu Thr Arg Asn Tyr Trp Phe Ala His Thr Gln Arg Met Glu Asp
115 120 125

Met Asp Glu Val Asn Val Tyr Glu Glu Ala Thr Leu Leu Leu Thr Lys
130 135 140

Ile Gly Phe Arg Trp Ala Gly Leu Lys Gln Thr His Glu Glu Ala Val
145 150 155 160

Gln Asn Ala Lys Asp Met Asp Leu Leu Ile Asp Ser Phe Ser Ala Leu
165 170 175

Gly Gln Ser Pro Ala Gly Tyr Lys Lys Ala Lys Lys Ala Arg Glu Arg
180 185 190

Val Glu Lys Phe Leu Glu Arg Gln Ile Lys Ala Val Arg Glu Gly Lys
195 200 205

Gln Phe Ala Ala Pro Asn Thr Ala Leu Tyr Glu Phe Ala His Trp Lys
210 215 220

Asp Leu Asn Asp Lys Pro Met Asp Leu His Leu Cys Ala Val Asp Leu
225 230 235 240

Met Asn Val Val Arg Pro Leu Val Ala Val Asn Arg Phe Val Ser Tyr
245 250 255

Ala Val Lys Ala Leu Ile Glu Phe Asp Gln Glu Arg Leu Lys Leu Gln
260 265 270

Val Thr Lys Asp Gln Asn Tyr Ala Tyr Lys Phe Ala Gln Glu Val Arg
275 280 285

Arg Ile Phe Pro Phe Val Pro Phe Leu Pro Gly Arg Leu Lys Gln Asn
290 295 300

Ile Glu Phe Asp Gly Tyr Ser Leu Lys Lys Asn Thr Leu Ile Ile Leu
305 310 315 320

Asp Val Phe Gly Thr Thr His Asp Pro Lys Leu Phe Glu Asn Pro Tyr
325 330 335

Gln Phe Asn Pro Asn Arg Phe Glu Asp Trp Asp Gly Ser Pro Phe Asp
340 345 350

Leu Ile Pro Gln Gly Gly Gly Asp Phe Tyr Thr Asn His Arg Cys Ala
355 360 365

Gly Glu Trp Met Thr Val Ile Val Met Glu Glu Thr Ile Asn Tyr Phe
370 375 380

Ala Asn Lys Ile Asp Phe Ser Val Pro Thr Gln Asp Leu Ser Val Lys
385 390 395 400

Leu Asp Gln Phe Pro Gly Lys Val Ala Ser Gly Thr Val Ile Arg Asn
405 410 415

Val Arg Pro Arg Arg
420

<210> 55
<211> 421
<212> PRT
<213> Staphylococcus felis

<400> 55

Met Asn Lys Ile Pro Lys Asp Lys Gly Leu Asp Asn Thr Leu Lys Ile
1 5 10 15

Val Lys Glu Ala Tyr Thr Tyr Val Pro Arg Arg Leu Glu Ala Phe Asp
20 25 30

Thr Asn Val Phe Glu Thr Arg Ala Leu Gly Met Lys Lys Val Leu Val
35 40 45

Val Ser Gly Lys Lys Ala Ala Glu Leu Phe Tyr Asp Asn Glu Lys Ile
50 55 60

Ser Arg Lys Gly Ala Leu Pro Lys Arg Ile Val Asn Thr Leu Phe Gly
65 70 75 80

Gln Gly Ala Ile His Thr Thr Glu Gly Lys Gln His Val Asp Arg Lys
85 90 95

Ala Leu Phe Met Ser Leu Met Thr Thr Lys Asn Leu Lys Tyr Leu Arg
100 105 110

Glu Leu Thr Arg Asn Tyr Trp Phe Ala His Thr Gln Arg Met Glu Asp
        115                 120                 125

Met Asp Glu Val Asn Val Tyr Glu Glu Ala Thr Leu Leu Leu Thr Lys
    130                 135                 140

Ile Gly Phe Arg Trp Ala Gly Leu Lys Gln Thr His Glu Glu Ala Val
145                 150                 155                 160

Gln Asn Ala Lys Asp Met Asp Leu Leu Ile Asp Ser Phe Ser Ala Leu
                165                 170                 175

Gly Gln Ser Pro Ala Gly Tyr Lys Lys Ala Lys Lys Ala Arg Glu Arg
            180                 185                 190

Val Glu Lys Phe Leu Glu Arg Gln Ile Lys Ala Val Arg Glu Gly Lys
        195                 200                 205

Gln Phe Ala Ala Pro Asn Thr Ala Leu Tyr Glu Phe Ala His Trp Lys
    210                 215                 220

Asp Leu Asn Asp Lys Pro Met Asp Leu His Leu Cys Ala Val Asp Leu
225                 230                 235                 240

Met Asn Val Val Arg Pro Leu Val Ala Val Asn Arg Phe Val Ser Tyr
                245                 250                 255

Ala Val Lys Ala Leu Ile Glu Phe Asp Gln Glu Arg Leu Lys Leu Gln
            260                 265                 270

Val Thr Lys Asp Gln Asn Tyr Ala Tyr Lys Phe Ala Gln Glu Val Arg
        275                 280                 285

Arg Ile Phe Pro Phe Val Pro Phe Leu Pro Gly Arg Leu Lys Gln Asn
    290                 295                 300

Ile Glu Phe Asp Gly Tyr Ser Leu Lys Lys Asn Thr Leu Ile Ile Leu
305                 310                 315                 320

Asp Val Phe Gly Thr Thr His Asp Pro Lys Leu Phe Glu Asn Pro Tyr
                325                 330                 335

Gln Phe Asn Pro Asn Arg Phe Glu Asp Trp Asp Gly Ser Pro Phe Asp
            340                 345                 350

Leu Ile Pro Gln Gly Gly Gly Asp Phe Tyr Thr Asn His Arg Cys Ala
        355                 360                 365

Gly Glu Trp Met Thr Val Ile Val Met Glu Glu Thr Ile Asn Tyr Phe
    370                 375                 380

Ala Asn Lys Ile Asp Phe Ser Val Pro Thr Gln Asp Leu Ser Val Lys
385                 390                 395                 400

Leu Asp Gln Phe Pro Gly Lys Val Thr Ser Gly Thr Val Ile Arg Asn
                405                 410                 415

Val Arg Pro Arg Arg
            420

<210> 56
<211> 421
<212> PRT
<213> Staphylococcus felis

<400> 56

Met Asn Lys Ile Pro Lys Asp Lys Gly Leu Asp Asn Thr Leu Lys Ile
1               5                   10                  15

Val Lys Glu Ala Tyr Thr Tyr Val Pro Arg Arg Leu Glu Ala Phe Asp
            20                  25                  30

Thr Asn Ile Phe Glu Thr Arg Ala Leu Gly Met Lys Lys Val Leu Val
        35                  40                  45

Val Ser Gly Lys Lys Ala Ala Glu Leu Phe Tyr Asp Asn Glu Lys Ile
    50                  55                  60

Ser Arg Lys Gly Ala Leu Pro Lys Arg Ile Val Asn Thr Leu Phe Gly
65                  70                  75                  80

Gln Gly Ala Ile His Thr Thr Glu Gly Lys Gln His Val Asp Arg Lys
                85                  90                  95

Ala Leu Phe Met Ser Leu Met Thr Thr Lys Asn Leu Lys Tyr Leu Arg
            100                 105                 110

Glu Leu Thr Arg Asn Tyr Trp Phe Ala His Thr Gln Arg Met Glu Asp
        115                 120                 125

Met Asp Glu Val Asn Val Tyr Glu Glu Ala Thr Leu Leu Leu Thr Lys
    130                 135                 140

Ile Gly Phe Arg Trp Ala Gly Leu Lys Gln Thr His Glu Glu Ala Val
145                 150                 155                 160

Gln Asn Ala Lys Asp Met Asp Leu Leu Ile Asp Ser Phe Ser Ala Leu
165 170 175

Gly Gln Ser Pro Ala Gly Tyr Lys Lys Ala Lys Lys Ala Arg Glu Arg
180 185 190

Val Glu Lys Phe Leu Glu His Gln Ile Lys Ala Val Arg Glu Gly Glu
195 200 205

Gln Tyr Ala Ala Pro Asn Thr Ala Leu Tyr Glu Phe Ala His Trp Lys
210 215 220

Asp Leu Asn Asp Lys Pro Met Asp Leu His Leu Cys Ala Val Asp Leu
225 230 235 240

Met Asn Val Val Arg Pro Leu Val Ala Val Asn Arg Phe Val Ser Tyr
245 250 255

Ala Val Lys Ala Leu Ile Glu Phe Asp Gln Glu Arg Leu Lys Leu Gln
260 265 270

Val Thr Lys Asp Gln Asn Tyr Ala Tyr Lys Phe Ala Gln Glu Val Arg
275 280 285

Arg Ile Phe Pro Phe Val Pro Phe Leu Pro Gly Arg Leu Lys Gln Asn
290 295 300

Ile Glu Phe Asp Gly Tyr Ser Leu Lys Lys Asn Thr Leu Ile Ile Leu
305 310 315 320

Asp Val Phe Gly Thr Thr His Asp Pro Lys Leu Phe Glu Asn Pro Tyr
325 330 335

Gln Phe Asn Pro Asn Arg Phe Glu Asp Trp Asp Gly Ser Pro Phe Asp
340 345 350

Leu Ile Pro Gln Gly Gly Gly Asp Phe Tyr Thr Asn His Arg Cys Ala
355 360 365

Gly Glu Trp Met Thr Val Ile Val Met Glu Glu Thr Ile Asn Tyr Phe
370 375 380

Ala Asn Lys Ile Asp Phe Ser Val Pro Thr Gln Asp Leu Ser Val Lys
385 390 395 400

Leu Asp Gln Phe Pro Gly Lys Val Thr Ser Gly Thr Val Ile Arg Asn
405 410 415

Val Arg Pro Arg Arg
420

<210> 57
<211> 421
<212> PRT
<213> Staphylococcus felis

<400> 57

Met Asn Lys Ile Pro Lys Asp Lys Gly Leu Asp Asn Thr Leu Lys Ile
1 5 10 15

Val Lys Glu Ala Tyr Thr Tyr Val Pro Arg Arg Leu Glu Ala Phe Asp
20 25 30

Thr Asn Ile Phe Glu Thr Arg Ala Leu Gly Met Lys Lys Val Leu Val
35 40 45

Val Ser Gly Lys Lys Ala Ala Glu Leu Phe Tyr Asp Asn Glu Lys Ile
50 55 60

Ser Arg Lys Gly Ala Leu Pro Lys Arg Ile Val Asn Thr Leu Phe Gly
65 70 75 80

Gln Gly Ala Ile His Thr Thr Glu Gly Lys Gln His Val Asp Arg Lys
85 90 95

Ala Leu Phe Met Ser Leu Met Thr Thr Lys Asn Leu Lys Tyr Leu Arg
100 105 110

Glu Leu Thr Arg Asn Tyr Trp Phe Ala His Thr Gln Arg Met Glu Asp
115 120 125

Met Asp Glu Val Asn Val Tyr Glu Glu Ala Thr Leu Leu Leu Thr Lys
130 135 140

Ile Gly Phe Arg Trp Ala Gly Leu Lys Gln Thr His Glu Glu Ala Val
145 150 155 160

Gln Asn Ala Lys Asp Met Asp Leu Leu Ile Asp Ser Phe Ser Ala Leu
165 170 175

Gly Gln Ser Pro Ala Gly Tyr Lys Lys Ala Lys Lys Ala Arg Glu Arg
180 185 190

Val Glu Lys Phe Leu Glu His Gln Ile Lys Ala Val Arg Glu Gly Glu
195 200 205

Gln Phe Ala Ala Pro Asn Thr Ala Leu Tyr Glu Phe Ala His Trp Lys
    210                 215                 220

Asp Leu Asn Asp Lys Pro Met Asp Leu His Leu Cys Ala Val Asp Leu
225                 230                 235                 240

Met Asn Val Val Arg Pro Leu Val Ala Val Asn Arg Phe Val Ser Tyr
                245                 250                 255

Ala Val Lys Ala Leu Ile Glu Phe Asp Gln Glu Arg Leu Lys Leu Gln
            260                 265                 270

Val Thr Lys Asp Gln Asn Tyr Ala Tyr Lys Phe Ala Gln Glu Val Arg
        275                 280                 285

Arg Ile Phe Pro Phe Val Pro Phe Leu Pro Gly Arg Leu Lys Gln Asn
    290                 295                 300

Ile Glu Phe Asp Gly Tyr Ser Leu Lys Lys Asn Thr Leu Ile Ile Leu
305                 310                 315                 320

Asp Val Phe Gly Thr Thr His Asp Pro Lys Leu Phe Glu Asn Pro Tyr
                325                 330                 335

Gln Phe Asn Pro Asn Arg Phe Glu Asp Trp Asp Gly Ser Pro Phe Asp
            340                 345                 350

Leu Ile Pro Gln Gly Gly Gly Asp Phe Tyr Thr Asn His Arg Cys Ala
        355                 360                 365

Gly Glu Trp Met Thr Val Ile Val Met Glu Glu Thr Ile Asn Tyr Phe
    370                 375                 380

Ala Asn Lys Ile Asp Phe Ser Val Pro Thr Gln Asp Leu Ser Val Lys
385                 390                 395                 400

Leu Asp Gln Phe Pro Gly Lys Val Thr Ser Gly Thr Val Ile Arg Asn
                405                 410                 415

Val Arg Pro Arg Arg
            420

<210>  58
<211>  421
<212>  PRT
<213>  Staphylococcus felis

<400> 58

Met Asn Lys Ile Pro Lys Asp Lys Gly Leu Asp Asn Thr Leu Lys Ile
1 5 10 15

Val Lys Glu Ala Tyr Thr Tyr Val Pro Arg Arg Leu Glu Ala Phe Asp
20 25 30

Thr Asn Ile Phe Glu Thr Arg Ala Leu Gly Met Lys Lys Val Leu Val
35 40 45

Val Ser Gly Lys Lys Ala Ala Glu Leu Phe Tyr Asp Asn Glu Lys Ile
50 55 60

Ser Arg Lys Gly Ala Leu Pro Lys Arg Ile Val Asn Thr Leu Phe Gly
65 70 75 80

Gln Gly Ala Ile His Thr Thr Glu Gly Lys Gln His Val Asp Arg Lys
85 90 95

Ala Leu Phe Met Ser Leu Met Thr Thr Lys Asn Leu Lys Tyr Leu Arg
100 105 110

Glu Leu Thr Arg Asn Tyr Trp Phe Ala His Thr Gln Arg Met Glu Asp
115 120 125

Met Asp Glu Val Asn Val Tyr Glu Glu Ala Thr Leu Leu Leu Thr Lys
130 135 140

Ile Gly Phe Arg Trp Ala Gly Leu Lys Gln Thr His Glu Glu Ala Val
145 150 155 160

Gln Asn Ala Lys Asp Met Asp Leu Leu Ile Asp Ser Phe Ser Ala Leu
165 170 175

Gly Gln Ser Pro Ala Gly Tyr Lys Lys Ala Lys Lys Ala Arg Glu Arg
180 185 190

Val Glu Lys Phe Leu Glu His Gln Ile Lys Ala Val Arg Glu Gly Glu
195 200 205

Gln Tyr Ala Ala Pro Asn Thr Ala Leu Tyr Glu Phe Ala His Trp Lys
210 215 220

Asp Leu Asn Asp Lys Pro Met Asp Leu His Leu Cys Ala Val Asp Leu
225 230 235 240

Met Asn Val Val Arg Pro Leu Val Ala Val Asn Arg Phe Val Ser Tyr

245 250 255

Ala Val Lys Ala Leu Ile Glu Phe Asp Gln Glu Arg Leu Lys Leu Gln
260 265 270

Val Thr Lys Asp Gln Asn Tyr Ala Tyr Lys Phe Ala Gln Glu Val Arg
275 280 285

Arg Ile Phe Pro Phe Val Pro Phe Leu Pro Gly Arg Leu Lys Gln Asn
290 295 300

Ile Glu Phe Asp Gly Tyr Ser Leu Lys Lys Asn Thr Leu Ile Ile Leu
305 310 315 320

Asp Val Phe Gly Thr Thr His Asp Pro Lys Leu Phe Glu Asn Pro Tyr
325 330 335

Gln Phe Asn Pro Asn Arg Phe Glu Asp Trp Asp Gly Ser Pro Phe Asp
340 345 350

Leu Ile Pro Gln Gly Gly Gly Asp Phe Tyr Thr Asn His Arg Cys Ala
355 360 365

Gly Glu Trp Met Thr Val Ile Val Met Glu Glu Thr Ile Asn Tyr Phe
370 375 380

Ala Asn Lys Ile Asp Phe Ser Val Pro Thr Gln Asp Leu Ser Val Lys
385 390 395 400

Leu Asp Gln Phe Pro Gly Lys Val Thr Ser Gly Thr Val Ile Arg Asn
405 410 415

Val Arg Leu Arg Arg
420

<210> 59
<211> 421
<212> PRT
<213> Staphylococcus felis

<400> 59

Met Asn Lys Ile Pro Lys Asp Lys Gly Leu Asp Asn Thr Leu Lys Ile
1 5 10 15

Val Lys Glu Ala Tyr Thr Tyr Val Pro Arg Arg Leu Glu Ala Phe Asp
20 25 30

Thr Asn Val Phe Glu Thr Arg Ala Leu Gly Met Lys Lys Val Leu Val

35 40 45

Val Ser Gly Lys Lys Ala Ala Glu Leu Phe Tyr Asp Asn Glu Lys Ile
50 55 60

Ser Arg Lys Gly Ala Leu Pro Lys Arg Ile Val Asn Thr Leu Phe Gly
65 70 75 80

Gln Gly Ala Ile His Thr Thr Glu Gly Lys Gln His Val Asp Arg Lys
85 90 95

Ala Leu Phe Met Ser Leu Met Thr Thr Lys Asn Leu Lys Tyr Leu Arg
100 105 110

Glu Leu Thr Arg Asn Tyr Trp Phe Ala His Thr Gln Arg Met Glu Asp
115 120 125

Met Asn Glu Val Asn Val Tyr Glu Glu Ala Thr Leu Leu Leu Thr Lys
130 135 140

Ile Gly Phe Arg Trp Ala Gly Leu Lys Gln Thr His Glu Glu Ala Val
145 150 155 160

Gln Asn Ala Lys Asp Met Asp Leu Leu Ile Asp Ser Phe Ser Ala Leu
165 170 175

Gly Gln Ser Pro Ala Gly Tyr Lys Lys Ala Lys Lys Ala Arg Glu Arg
180 185 190

Val Glu Lys Phe Leu Glu His Gln Ile Lys Ala Val Arg Glu Gly Glu
195 200 205

Gln Phe Ala Ala Pro Asn Thr Ala Leu Tyr Glu Phe Ala His Trp Lys
210 215 220

Asp Leu Asn Asp Lys Pro Met Asp Ile His Leu Cys Ala Val Asp Leu
225 230 235 240

Met Asn Val Val Arg Pro Leu Val Ala Val Asn Arg Phe Val Ser Tyr
245 250 255

Ala Val Lys Ala Leu Ile Glu Phe Asp Gln Glu Arg Leu Lys Leu Gln
260 265 270

Val Thr Lys Asp Gln Asn Tyr Ala Tyr Lys Phe Ala Gln Glu Val Arg
275 280 285

```
Arg Ile Phe Pro Phe Val Pro Phe Leu Pro Gly Arg Leu Lys Gln Asn
    290                 295                 300

Ile Glu Phe Asp Gly Tyr Ser Leu Lys Lys Asn Thr Leu Ile Ile Leu
305                 310                 315                 320

Asp Val Phe Gly Thr Thr His Asp Pro Lys Leu Phe Glu Asn Pro Tyr
                325                 330                 335

Gln Phe Asn Pro Asn Arg Phe Glu Asp Trp Asp Gly Ser Pro Phe Asp
            340                 345                 350

Leu Ile Pro Gln Gly Gly Gly Asp Phe Tyr Thr Asn His Arg Cys Ala
        355                 360                 365

Gly Glu Trp Met Thr Val Ile Val Met Glu Glu Thr Ile Asn Tyr Phe
    370                 375                 380

Ala Asn Lys Ile Asp Phe Ser Val Pro Thr Gln Asp Leu Ser Val Lys
385                 390                 395                 400

Leu Asp Gln Phe Pro Gly Lys Val Thr Ser Gly Thr Val Ile Arg Asn
                405                 410                 415

Val Arg Pro Arg Arg
            420


<210>  60
<211>  421
<212>  PRT
<213>  Staphylococcus felis

<400>  60

Met Asn Lys Ile Pro Lys Asp Lys Gly Leu Asp Asn Thr Leu Lys Ile
1               5                   10                  15

Val Lys Glu Ala Tyr Thr Tyr Val Pro Arg Arg Leu Glu Ala Phe Asp
            20                  25                  30

Thr Asn Val Phe Glu Thr Arg Ala Leu Gly Met Lys Lys Val Leu Val
        35                  40                  45

Val Ser Gly Lys Lys Ala Ala Glu Leu Phe Tyr Asp Asn Glu Lys Ile
    50                  55                  60

Ser Arg Lys Gly Ala Leu Pro Lys Arg Ile Val Asn Thr Leu Phe Gly
65                  70                  75                  80
```

Gln Gly Ala Ile His Thr Thr Glu Gly Lys Gln His Val Asp Arg Lys
85 90 95

Ala Leu Phe Met Ser Leu Met Thr Thr Lys Asn Leu Lys Tyr Leu Arg
100 105 110

Glu Leu Thr Arg Asn Tyr Trp Phe Ala His Thr Gln Arg Met Glu Asp
115 120 125

Met Asp Glu Val Asn Val Tyr Glu Glu Ala Thr Leu Leu Leu Thr Lys
130 135 140

Ile Gly Phe Arg Trp Ala Gly Leu Lys Gln Thr His Glu Glu Ala Val
145 150 155 160

Gln Asn Ala Lys Asp Met Asp Leu Leu Ile Asp Ser Phe Ser Ala Leu
165 170 175

Gly Gln Ser Pro Ala Gly Tyr Lys Lys Ala Lys Lys Ala Arg Glu Arg
180 185 190

Val Glu Lys Phe Leu Glu His Gln Ile Lys Ala Val Arg Glu Gly Glu
195 200 205

Gln Tyr Ala Ala Pro Asn Thr Ala Leu Tyr Glu Phe Ala His Trp Lys
210 215 220

Asp Leu Asn Asp Lys Pro Met Asp Leu His Leu Cys Ala Val Asp Leu
225 230 235 240

Met Asn Val Val Arg Pro Leu Val Ala Val Asn Arg Phe Val Ser Tyr
245 250 255

Ala Val Lys Ala Leu Ile Glu Phe Asp Gln Glu Arg Leu Lys Leu Gln
260 265 270

Val Thr Lys Asp Gln Asn Tyr Ala Tyr Lys Phe Ala Gln Glu Val Arg
275 280 285

Arg Ile Phe Pro Phe Val Pro Phe Leu Pro Gly Arg Leu Lys Gln Asn
290 295 300

Ile Glu Phe Asp Gly Tyr Ser Leu Lys Lys Asn Thr Leu Ile Ile Leu
305 310 315 320

Asp Val Phe Gly Thr Thr His Asp Pro Lys Leu Phe Glu Asn Pro Tyr
325 330 335

Gln Phe Asn Pro Asn Arg Phe Glu Asp Trp Asp Gly Ser Pro Phe Asp
340 345 350

Leu Ile Pro Gln Gly Gly Gly Asp Phe Tyr Thr Asn His Arg Cys Ala
355 360 365

Gly Glu Trp Met Thr Val Ile Val Met Glu Glu Thr Ile Asn Tyr Phe
370 375 380

Ala Asn Lys Ile Asp Phe Ser Val Pro Thr Gln Asp Leu Ser Val Lys
385 390 395 400

Leu Asp Gln Phe Pro Gly Lys Val Thr Ser Gly Thr Val Ile Arg Asn
405 410 415

Val Arg Pro Arg Arg
420

<210> 61
<211> 421
<212> PRT
<213> Staphylococcus felis

<400> 61

Met Asn Lys Ile Pro Lys Asp Lys Gly Leu Asp Asn Thr Leu Lys Ile
1 5 10 15

Val Lys Glu Ala Tyr Thr Tyr Val Pro Arg Arg Leu Glu Ala Phe Asp
20 25 30

Thr Asn Val Phe Glu Thr Arg Ala Leu Gly Met Lys Lys Val Leu Val
35 40 45

Val Ser Gly Lys Lys Ala Ala Glu Leu Phe Tyr Asp Asn Glu Lys Ile
50 55 60

Ser Arg Lys Gly Ala Leu Pro Lys Arg Ile Val Asn Thr Leu Phe Gly
65 70 75 80

Gln Gly Ala Ile His Thr Thr Glu Gly Lys Gln His Val Asp Arg Lys
85 90 95

Ala Leu Phe Met Ser Leu Met Thr Thr Lys Asn Leu Lys Tyr Leu Arg
100 105 110

Glu Leu Thr Arg Asn Tyr Trp Phe Ala His Thr Gln Arg Met Glu Asp
115 120 125

Met Asp Glu Val Asn Val Tyr Glu Glu Ala Thr Leu Leu Leu Thr Lys
    130                 135                 140

Ile Gly Phe Arg Trp Ala Gly Leu Lys Gln Thr His Glu Val Ala Val
145                 150                 155                 160

Gln Asn Ala Lys Asp Met Asp Leu Leu Ile Asp Ser Phe Ser Ala Leu
                165                 170                 175

Gly Gln Ser Pro Ala Gly Tyr Lys Lys Ala Lys Lys Ala Arg Glu Arg
            180                 185                 190

Val Glu Lys Phe Leu Glu His Gln Ile Lys Ala Val Arg Glu Gly Glu
        195                 200                 205

Gln Tyr Ala Ala Pro Asn Thr Ala Leu Tyr Glu Phe Ala His Trp Lys
    210                 215                 220

Asp Leu Asn Asp Lys Pro Met Asp Leu His Leu Cys Ala Val Asp Leu
225                 230                 235                 240

Met Asn Val Val Arg Pro Leu Val Ala Val Asn Arg Phe Val Ser Tyr
                245                 250                 255

Ala Val Lys Ala Leu Ile Glu Phe Asp Gln Glu Arg Leu Lys Leu Gln
            260                 265                 270

Val Thr Lys Asp Gln Asn Tyr Ala Tyr Lys Phe Ala Gln Glu Val Arg
        275                 280                 285

Arg Ile Phe Pro Phe Val Pro Phe Leu Pro Gly Arg Leu Lys Gln Asn
    290                 295                 300

Ile Glu Phe Asp Gly Tyr Ser Leu Lys Lys Asn Thr Leu Ile Ile Leu
305                 310                 315                 320

Asp Val Phe Gly Thr Thr His Asp Ser Lys Leu Phe Glu Asn Pro Tyr
                325                 330                 335

Gln Phe Asn Pro Asn Arg Phe Glu Asp Trp Asp Gly Ser Pro Phe Asp
            340                 345                 350

Leu Ile Pro Gln Gly Gly Gly Asp Phe Tyr Thr Asn His Arg Cys Ala
        355                 360                 365

Gly Glu Trp Met Thr Val Ile Val Met Glu Glu Thr Ile Asn Tyr Phe
    370                 375                 380

Ala Asn Lys Ile Asp Phe Ser Val Pro Thr Gln Asp Leu Ser Val Lys
385 390 395 400

Leu Asp Gln Phe Pro Gly Lys Val Thr Ser Gly Thr Val Ile Arg Asn
405 410 415

Val Arg Pro Arg Arg
420

<210> 62
<211> 421
<212> PRT
<213> Staphylococcus felis

<400> 62

Met Asn Lys Ile Pro Lys Asp Lys Gly Leu Asp Asn Thr Leu Lys Ile
1 5 10 15

Val Lys Glu Ala Tyr Thr Tyr Val Pro Arg Arg Leu Glu Ala Phe Asp
20 25 30

Thr Asn Val Phe Glu Thr Arg Ala Leu Gly Met Lys Lys Val Leu Val
35 40 45

Val Ser Gly Lys Lys Ala Ala Glu Leu Phe Tyr Asp Asn Glu Lys Ile
50 55 60

Ser Arg Lys Gly Ala Leu Pro Lys Arg Ile Val Asn Thr Leu Phe Gly
65 70 75 80

Gln Gly Ala Ile His Thr Thr Glu Gly Lys Gln His Val Asp Arg Lys
85 90 95

Ala Leu Phe Met Ser Leu Met Thr Thr Lys Asn Leu Lys Tyr Leu Arg
100 105 110

Glu Leu Thr Arg Asn Tyr Trp Phe Ala His Thr Gln Leu Met Glu Asp
115 120 125

Met Asp Glu Val Asn Val Tyr Glu Glu Ala Thr Leu Leu Leu Thr Lys
130 135 140

Ile Gly Phe Arg Trp Ala Gly Leu Lys Gln Thr His Glu Glu Ala Val
145 150 155 160

Gln Asn Ala Lys Asp Met Asp Leu Leu Ile Asp Ser Phe Ser Ala Leu
165 170 175

Gly Gln Ser Pro Ala Gly Tyr Lys Lys Ala Lys Lys Ala Arg Glu Arg
180 185 190

Val Glu Lys Phe Leu Glu His Gln Ile Lys Ala Val Arg Glu Gly Glu
195 200 205

Gln Tyr Ala Ala Pro Asn Thr Ala Leu Tyr Glu Phe Ala His Trp Lys
210 215 220

Asp Leu Asn Asp Lys Pro Met Asn Leu His Leu Cys Ala Val Asp Leu
225 230 235 240

Met Asn Val Val Arg Pro Leu Val Ala Val Asn Arg Phe Val Ser Tyr
245 250 255

Ala Val Lys Ala Leu Ile Glu Phe Asp Gln Glu Arg Leu Lys Leu Gln
260 265 270

Val Thr Lys Asp Gln Asn Tyr Ala Tyr Lys Phe Ala Gln Glu Val Arg
275 280 285

Arg Ile Phe Pro Phe Val Pro Phe Leu Pro Gly Arg Leu Lys Gln Asn
290 295 300

Ile Glu Phe Asp Gly Tyr Ser Leu Lys Lys Asn Thr Leu Ile Ile Leu
305 310 315 320

Asp Val Phe Gly Thr Thr His Asp Pro Lys Leu Phe Glu Asn Pro Tyr
325 330 335

Gln Phe Asn Pro Asn Arg Phe Glu Asp Trp Asp Gly Ser Pro Phe Asp
340 345 350

Leu Ile Pro Gln Gly Gly Gly Asp Phe Tyr Thr Asn His Arg Cys Ala
355 360 365

Gly Glu Trp Met Thr Val Ile Val Met Glu Glu Thr Ile Asn Tyr Phe
370 375 380

Ala Asn Lys Ile Asp Phe Ser Val Pro Thr Gln Asp Leu Ser Val Lys
385 390 395 400

Leu Asp Gln Phe Pro Gly Lys Val Thr Ser Gly Thr Val Ile Arg Asn
405 410 415

Val Arg Pro Arg Arg

420

<210> 63
<211> 421
<212> PRT
<213> Staphylococcus felis

<400> 63

Met Asn Lys Ile Pro Lys Asp Lys Gly Leu Asp Asn Thr Leu Lys Ile
1 5 10 15

Val Lys Glu Ala Tyr Thr Tyr Val Pro Arg Arg Leu Glu Ala Phe Asp
20 25 30

Thr Asn Val Phe Glu Thr Arg Ala Leu Gly Met Lys Lys Val Leu Val
35 40 45

Val Ser Gly Lys Lys Ala Ala Glu Leu Phe Tyr Asp Asn Glu Lys Ile
50 55 60

Ser Arg Lys Gly Ala Leu Pro Lys Arg Ile Val Asn Thr Leu Phe Gly
65 70 75 80

Gln Gly Ala Ile His Thr Thr Glu Gly Lys Gln His Val Asp Arg Lys
85 90 95

Ala Leu Phe Met Ser Leu Met Thr Thr Lys Asn Leu Lys Tyr Leu Arg
100 105 110

Glu Leu Thr Arg Asn Tyr Trp Phe Ala His Thr Gln Arg Met Glu Asp
115 120 125

Met Asp Glu Val Asn Val Tyr Glu Glu Ala Thr Leu Leu Leu Thr Lys
130 135 140

Ile Gly Phe Arg Trp Ala Gly Leu Lys Gln Thr His Glu Glu Ala Val
145 150 155 160

Gln Asn Ala Lys Asp Met Asp Leu Leu Ile Asp Ser Phe Ser Ala Leu
165 170 175

Gly Gln Ser Pro Ala Gly Tyr Lys Lys Ala Lys Lys Ala Arg Glu Arg
180 185 190

Val Glu Lys Phe Leu Glu His Gln Ile Lys Ala Val Arg Glu Gly Glu
195 200 205

Gln Tyr Ala Ala Pro Asn Thr Ala Leu Tyr Glu Phe Ala His Trp Lys

210 215 220

Asp Leu Asn Asp Lys Pro Met Asn Leu His Leu Cys Ala Val Asp Leu
225 230 235 240

Met Asn Val Val Arg Pro Leu Val Ala Val Asn Arg Phe Val Ser Tyr
245 250 255

Ala Val Lys Ala Leu Ile Glu Phe Asp Gln Glu Arg Leu Lys Leu Gln
260 265 270

Val Thr Lys Asp Gln Asn Tyr Ala Tyr Lys Phe Ala Gln Glu Val Arg
275 280 285

Arg Ile Phe Pro Phe Val Pro Phe Leu Pro Gly Arg Leu Lys Gln Asn
290 295 300

Ile Glu Phe Asp Gly Tyr Ser Leu Lys Lys Asn Thr Leu Ile Ile Leu
305 310 315 320

Asp Val Phe Gly Thr Thr His Asp Pro Lys Leu Phe Glu Asn Pro Tyr
325 330 335

Gln Phe Asn Pro Asn Arg Phe Glu Asp Trp Asp Gly Ser Pro Phe Asp
340 345 350

Leu Ile Pro Gln Gly Gly Gly Asp Phe Tyr Thr Asn His Arg Cys Ala
355 360 365

Gly Glu Trp Met Thr Val Ile Val Met Glu Glu Thr Ile Asn Tyr Phe
370 375 380

Ala Asn Lys Ile Asp Phe Ser Val Pro Thr Gln Asp Leu Ser Val Lys
385 390 395 400

Leu Asp Gln Phe Pro Gly Lys Val Thr Ser Gly Thr Val Ile Arg Asn
405 410 415

Val Arg Pro Arg Arg
420

<210> 64
<211> 424
<212> PRT
<213> Fictibacillus enclensis

<400> 64

Met Ser Lys Gln Leu Asn Ile Pro Lys Glu Glu Gly Leu Asp His Ser

```
1               5                   10                  15

Leu Ala Leu Ile Gln Glu Gly Tyr Gln Tyr Ile Thr Ser Arg Arg Thr
            20                  25                  30

Lys Phe Lys Ser Asp Leu Phe Glu Thr Arg Leu Leu Gly Gln Lys Val
        35                  40                  45

Val Cys Ile Gly Gly Gln Glu Gly Ala Glu Leu Phe Tyr Asp Asn Glu
    50                  55                  60

Lys Phe Glu Arg Lys Gly Ala Val Pro Lys Arg Ile Gln Lys Ser Leu
65                  70                  75                  80

Phe Gly Glu Asn Ala Ile His Thr Leu Asp Gly Glu Ala His Glu His
                85                  90                  95

Arg Lys Leu Leu Phe Met Ser Leu Met Thr Glu Glu Arg Leu Glu Leu
            100                 105                 110

Leu Ala Glu Leu Thr Arg Glu Glu Trp Arg Leu Ala Ala Ala Glu Trp
        115                 120                 125

Ala Lys Met Glu Gln Val Val Leu Phe Asp Glu Val Gln Gln Ile Leu
    130                 135                 140

Cys Arg Val Ala Cys Arg Trp Ala Gly Val Pro Ile Asp Ala Ile Glu
145                 150                 155                 160

Val Lys Pro Leu Ala Arg Asp Phe Ile Ala Met Val Asp Ala Phe Gly
                165                 170                 175

Ala Val Gly Pro Arg His Gln Lys Gly Arg Lys Ala Arg Lys Lys Met
            180                 185                 190

Glu Asp Tyr Phe Lys Gly Leu Ile Ile Glu Ile Arg His Arg Arg Ile
        195                 200                 205

Glu Pro Ile Glu Gly Thr Ala Leu His Lys Ile Ala Trp His Arg Asp
    210                 215                 220

His Lys Glu Lys His Leu Asp Glu His Thr Ala Ala Val Glu Leu Leu
225                 230                 235                 240

Asn Val Leu Arg Pro Ile Val Ala Ile Thr Tyr Leu Val Thr Phe Gly
                245                 250                 255
```

Ala Leu Ala Leu Phe Glu His Asn Val Trp Lys Glu Lys Leu Gln Ser
            260                 265                 270

Gly Asp Glu Glu Ser Val Gln Met Phe Ala Gln Glu Val Arg Arg Phe
        275                 280                 285

Tyr Pro Phe Ala Pro Phe Leu Gly Thr Lys Val Lys Asn Gly Phe Glu
    290                 295                 300

Trp Lys Gly His Thr Phe Glu Lys Gly Gln Leu Val Leu Leu Asp Val
305                 310                 315                 320

Tyr Gly Thr Asn His Asp Asn Arg Leu Trp Glu Glu Pro Tyr Val Phe
                325                 330                 335

Asn Pro Glu Arg Phe Arg Asn Trp Asn Gly Gly Met Phe Asp Leu Ile
            340                 345                 350

Pro Gln Gly Gly Gly Asp Glu Tyr Thr Gly His Arg Cys Pro Gly Glu
        355                 360                 365

Met Ala Thr Ile Gln Val Met Lys Thr Ser Phe Ser Phe Leu Thr Asn
    370                 375                 380

Glu Met Ile Tyr Asn Val Pro Val Gly Gln Asp Leu Ser Tyr Ser Leu
385                 390                 395                 400

Ser Arg Met Pro Thr Tyr Pro Glu Ser Gly Phe Ile Ile Gln Gly Val
                405                 410                 415

Ser Ile Lys Ser Gln Thr Thr Val
            420

<210> 65
<211> 424
<212> PRT
<213> Fictibacillus sp. S7

<400> 65

Met Ser Lys Gln Leu Asn Ile Pro Lys Glu Glu Gly Leu Asp His Ser
1               5                   10                  15

Leu Ala Leu Ile Gln Glu Gly Tyr Gln Tyr Ile Thr Ser Arg Arg Thr
            20                  25                  30

Lys Phe Lys Ser Asp Leu Phe Glu Thr Arg Leu Leu Gly Gln Lys Val
        35                  40                  45

Val Cys Ile Gly Gly Gln Glu Gly Ala Glu Leu Phe Tyr Asp Asn Glu
    50                  55                  60

Lys Phe Glu Arg Lys Gly Ala Val Pro Lys Arg Ile Gln Lys Ser Leu
65                  70                  75                  80

Phe Gly Glu Asn Ala Ile His Thr Leu Asp Gly Glu Ala His Glu His
                85                  90                  95

Arg Lys Leu Leu Phe Met Ser Leu Met Thr Glu Glu Arg Leu Glu Leu
            100                 105                 110

Leu Ala Glu Leu Thr Arg Glu Glu Trp Arg Leu Ala Ala Ala Glu Trp
        115                 120                 125

Ala Lys Met Glu Gln Val Val Leu Phe Asp Glu Val Gln Gln Ile Leu
    130                 135                 140

Cys Arg Val Ala Cys Arg Trp Ala Gly Val Pro Ile Asp Ala Ile Glu
145                 150                 155                 160

Val Lys Pro Leu Ala Arg Asp Phe Ile Ala Met Val Asp Ala Phe Gly
                165                 170                 175

Ala Val Gly Pro Arg His Gln Lys Gly Arg Lys Ala Arg Lys Lys Met
            180                 185                 190

Glu Asp Tyr Phe Lys Gly Leu Ile Ile Glu Ile Arg His Arg Arg Ile
        195                 200                 205

Glu Pro Ile Glu Gly Thr Ala Leu His Lys Ile Ala Trp His Arg Asp
    210                 215                 220

His Lys Glu Lys His Leu Asp Glu His Thr Ala Ala Val Glu Leu Leu
225                 230                 235                 240

Asn Val Leu Arg Pro Ile Val Ala Ile Thr Tyr Leu Val Thr Phe Gly
                245                 250                 255

Ala Leu Ala Leu Phe Glu His Asn Val Trp Lys Glu Lys Leu Gln Ser
            260                 265                 270

Gly Asp Glu Glu Ser Val Gln Met Phe Ala Gln Glu Val Arg Arg Phe
        275                 280                 285

Tyr Pro Phe Ala Pro Phe Leu Gly Ala Lys Val Lys Asn Gly Phe Glu
    290                 295                 300

Trp Lys Gly His Thr Phe Glu Lys Gly Gln Leu Val Leu Leu Asp Val
305 310 315 320

Tyr Gly Thr Asn His Asp Asn Arg Leu Trp Glu Glu Pro Tyr Val Phe
325 330 335

Asn Pro Glu Arg Phe Arg Asn Trp Asn Gly Gly Met Phe Asp Leu Ile
340 345 350

Pro Gln Gly Gly Gly Asp Glu Tyr Thr Gly His Arg Cys Pro Gly Glu
355 360 365

Met Ala Thr Ile Gln Val Met Lys Thr Ser Phe Ser Phe Leu Thr Asn
370 375 380

Glu Met Ile Tyr Asn Val Pro Val Gly Gln Asp Leu Ser Tyr Ser Leu
385 390 395 400

Ser Arg Met Pro Thr Tyr Pro Glu Ser Gly Phe Ile Ile Gln Gly Val
405 410 415

Ser Ile Lys Ser Gln Thr Thr Val
420

<210> 66
<211> 420
<212> PRT
<213> Lentibacillus salicampi

<400> 66

Met Lys Gly Val Ala Pro Val Ala Ile Pro Arg Glu Asn Val Leu Asp
1 5 10 15

Asn Thr Leu Thr Leu Leu Arg Glu Gly Tyr His Tyr Ile Pro Gly Arg
20 25 30

Arg Arg His Phe Lys Ser Asp Ile Phe Gln Thr Arg Leu Leu Gly Gln
35 40 45

Lys Val Ile Cys Ile Gly Gly Lys Glu Ala Ala Glu Val Phe Tyr Asp
50 55 60

Glu Asp Lys Phe Thr Arg Ile Gly Ala Ile Pro Asn Arg Met Lys Glu
65 70 75 80

Ser Leu Phe Gly Lys Asn Gly Val His Val Leu Glu Gly Asn Ala His
85 90 95

Ser His Arg Lys Thr Met Phe Met Ser Leu Met Thr Ser Glu Arg Leu
100 105 110

Ser Glu Leu Thr Ala Leu Thr Glu Lys Gln Trp Glu Val Ala Ile Ala
115 120 125

Asp Trp Gln Gln Lys Arg Arg Val Ile Leu Phe Pro Glu Ala Glu Lys
130 135 140

Ile Met Cys Arg Ile Ala Cys Glu Trp Ala Gly Val Pro Leu Trp Ala
145 150 155 160

Asn Glu Leu Ser Gln Arg Thr Arg Asp Leu Ser Ala Met Ile Asp Ala
165 170 175

Phe Gly Ala Ile Gly Pro Arg His Trp Gln Gly Arg Ile Ala Arg Asn
180 185 190

Arg Ser Glu Arg Trp Ile Arg Arg Ile Ile Ala Gln Val Arg Ser Gly
195 200 205

Glu Leu Lys Pro Arg Glu Glu Thr Ala Leu Tyr Thr Phe Ala Trp His
210 215 220

Arg Asp Leu Gln Asp Arg Leu Leu Asp Leu Gln Thr Ala Ala Val Glu
225 230 235 240

Leu Leu Asn Ile Val Arg Pro Ile Val Ala Val Gly Arg Phe Val Thr
245 250 255

Phe Gly Ala Leu Ala Ile Asn Asp Phe Pro Glu Thr Lys Glu Lys Leu
260 265 270

Arg Val Gly Asp Asp Asp Tyr Ile Arg Met Phe Val Gln Glu Val Arg
275 280 285

Arg Tyr Tyr Pro Phe Gly Pro Phe Leu Gly Ala Lys Val Arg Ser Asn
290 295 300

Phe Lys Trp Thr Gly His Gln Phe Lys Lys Gly Thr Leu Val Leu Leu
305 310 315 320

Asp Ile Tyr Gly Thr Asn His Asp Pro Asp Leu Trp Asp Lys Pro Asp
325 330 335

Glu Phe Arg Pro Glu Arg Phe Asp Glu Trp Glu Gly Ser Pro Phe Asp
340 345 350

Phe Ile Pro Gln Gly Gly Gly Tyr Asp Met Gly His Arg Cys Pro Gly
355 360 365

Glu Trp Ile Thr Ile Asp Ile Met Lys Val Ser Leu Ala Phe Leu Ala
370 375 380

Asn Arg Ile Asp Tyr Asn Val Pro Lys Gln Asn Leu Asp Tyr Ser Met
385 390 395 400

Val Arg Met Pro Ser Ile Pro Lys Ser Arg Phe Ile Met Arg Asp Val
405 410 415

Ala Ile Ser Ser
420

<210> 67
<211> 426
<212> PRT
<213> Staphylococcus vitulinus

<400> 67

Met Gly Lys Gln Ile Pro Lys Asp Arg Gly Leu Asp Ser Thr Leu Lys
1 5 10 15

Val Leu Lys Glu Gly Tyr Lys Tyr Val Pro Asn Arg Leu Glu Lys Phe
20 25 30

Asp Thr Asn Ile Phe Glu Leu Arg Ala Leu Gly Gly Arg Arg Thr Val
35 40 45

Val Ile Ser Gly Lys Glu Ala Ala Glu Leu Phe Tyr Asn Asn Glu Leu
50 55 60

Thr Glu Arg Gln Gly Thr Leu Pro Lys Arg Val Val Asn Thr Leu Phe
65 70 75 80

Gly Lys Gly Ala Ile His Thr Thr Gly Gly Lys Lys His Ile Asp Arg
85 90 95

Lys Ala Leu Phe Met Ser Leu Met Thr Glu Glu Asn Leu Asp Tyr Leu
100 105 110

Arg Glu Leu Thr Arg Ser Thr Trp Phe Met Asn Thr Glu Arg Met Glu
115 120 125

Arg Met Asp Glu Val Asn Val Tyr Lys Glu Ser Ile Ile Leu Leu Thr
130 135 140

Lys Val Gly Phe Arg Trp Ala Gly Ile Ile Ala Ser Pro Glu Glu Ile
145 150 155 160

Glu Ser Cys Ala Gln Asp Met Asp Thr Met Ile Asp Ser Phe Lys Asn
165 170 175

Ile Gly Thr Val Phe Lys Gly Tyr Arg Glu Ala Lys Gln Ala Arg Ala
180 185 190

Arg Val Glu Thr Phe Leu Glu Asn Gln Ile Ile Ala Val Arg Glu Gly
195 200 205

Lys Leu Thr Pro Pro Gln Gly Thr Ala Leu His Glu Phe Ser His Trp
210 215 220

Glu Asp Phe Glu Gly Asn Leu Met Asp Ser Arg Leu Cys Ala Ile Asp
225 230 235 240

Leu Met Asn Val Val Arg Pro Leu Val Ala Ile Asn Arg Phe Ile Ser
245 250 255

Phe Gly Val Lys Ala Leu His Asp Tyr Pro Gly Glu Ala Glu Lys Val
260 265 270

Phe Asn Asn Glu Asp Asp Tyr Ala Tyr Lys Phe Val Gln Glu Ile Arg
275 280 285

Arg Phe Tyr Pro Phe Val Pro Phe Leu Pro Gly Lys Ala Ala Val Asp
290 295 300

Ile Glu Phe Glu Gly Tyr Thr Ile Glu Lys Asp Thr Phe Leu Val Leu
305 310 315 320

Asp Ile Tyr Gly Thr Leu His Arg Glu Gly Leu Trp Glu Asn Pro Glu
325 330 335

Arg Phe Tyr Pro Asn Arg Phe Ser Asp Trp Asp Gly Ser Pro Phe Asp
340 345 350

Leu Ile Pro Gln Gly Gly Gly Asp Tyr His Thr Asn His Arg Cys Ala
355 360 365

Gly Glu Trp Met Thr Leu Ile Ile Met Glu Glu Ser Met Lys Tyr Phe
370 375 380

Ala Arg Asn Ile Ser Tyr Asp Val Lys Lys Asp Gln Asp Leu Ser Val

385 390 395 400

Asn Leu Asn Lys Leu Pro Gly Arg Val Val Ser Gly Thr Ile Ile Glu
405 410 415

Asn Val Asn Ala Leu Val Asn Arg Asn Val
420 425

<210> 68
<211> 429
<212> PRT
<213> Staphylococcus sciuri

<400> 68

Met Gly Lys Gln Ile Pro Lys Asp Arg Gly Leu Asp Ser Thr Phe Lys
1 5 10 15

Val Leu Lys Glu Gly Tyr Lys Tyr Val Pro Asn Arg Leu Glu Lys Phe
20 25 30

Asp Thr Asn Ile Phe Glu Leu Arg Ala Leu Gly Gly Arg Arg Thr Val
35 40 45

Val Ile Ser Gly Lys Glu Ala Ala Glu Leu Phe Tyr Asn Asn Asp Leu
50 55 60

Ile Glu Arg Gln Gly Thr Leu Pro Lys Arg Val Val Asn Thr Leu Phe
65 70 75 80

Gly Lys Gly Ala Ile His Thr Thr Gly Gly Lys Lys His Ile Asp Arg
85 90 95

Lys Ala Leu Phe Met Ser Leu Met Thr Glu Glu Asn Leu Glu Tyr Leu
100 105 110

Arg Glu Leu Thr Arg Ser Thr Trp Phe Met Asn Thr Glu Arg Met Glu
115 120 125

Arg Met Asp Glu Val Asn Val Tyr Lys Glu Ser Ile Ile Leu Leu Thr
130 135 140

Lys Val Gly Phe Arg Trp Ala Gly Val Ile Ala Ser Pro Glu Glu Ile
145 150 155 160

Glu Ser Cys Ala Gln Asp Met Asp Thr Met Ile Asp Ser Phe Lys Asn
165 170 175

Ile Gly Thr Ala Phe Lys Gly Tyr Arg Glu Ala Lys Lys Ala Arg Asp

```
            180                 185                 190

Arg Val Glu Thr Phe Leu Glu Asn Gln Ile Ile Ala Val Arg Glu Gly
        195                 200                 205

Lys Leu Thr Pro Pro Gln Gly Thr Ala Leu His Glu Phe Ser His Trp
    210                 215                 220

Glu Asp Phe Glu Gly Asn Leu Met Asp Ser Arg Leu Cys Ala Ile Asp
225                 230                 235                 240

Leu Met Asn Val Val Arg Pro Leu Val Ala Ile Asn Arg Phe Ile Ser
                245                 250                 255

Phe Gly Val Lys Ala Leu His Asp Tyr Pro Gly Glu Ala Glu Lys Val
            260                 265                 270

Phe Asn Asn Glu Asn Asp Tyr Ala Tyr Lys Phe Val Gln Glu Val Arg
        275                 280                 285

Arg Phe Tyr Pro Phe Val Pro Tyr Leu Pro Gly Lys Ala Ala Val Asp
    290                 295                 300

Ile Glu Phe Asp Gly Tyr Thr Ile Glu Lys Asp Thr Phe Leu Val Leu
305                 310                 315                 320

Asp Ile Tyr Gly Thr Leu His Arg Asp Gly Leu Trp Glu Asn Pro Glu
                325                 330                 335

Arg Phe Tyr Pro Asn Arg Phe Ser Asp Trp Asp Gly Ser Pro Phe Asp
            340                 345                 350

Leu Ile Pro Gln Gly Gly Gly Asp Tyr Tyr Thr Asn His Arg Cys Ala
        355                 360                 365

Gly Glu Trp Met Thr Ile Ile Ile Met Glu Glu Ser Met Lys Tyr Phe
    370                 375                 380

Ala Arg Asn Ile Ser Tyr Asp Val Lys Lys Asp Gln Asp Leu Ser Val
385                 390                 395                 400

Asn Leu Asn Lys Leu Pro Gly Arg Val Val Ser Gly Thr Ile Ile Glu
                405                 410                 415

Asn Val Asn Ala Leu Val Asn Arg Asn Val Glu Ser Val
            420                 425
```

<210> 69
<211> 429
<212> PRT
<213> Staphylococcus sciuri

<400> 69

Met Gly Lys Gln Ile Pro Lys Asp Arg Gly Leu Asp Ser Thr Leu Lys
1 5 10 15

Val Leu Lys Glu Gly Tyr Lys Tyr Val Pro Asn Arg Leu Glu Lys Phe
20 25 30

Asp Thr Asn Ile Phe Glu Leu Arg Ala Leu Gly Gly Arg Arg Thr Val
35 40 45

Val Phe Ser Gly Lys Glu Ala Ala Glu Ile Phe Tyr Asn Asn Glu Leu
50 55 60

Ile Glu Arg Gln Gly Thr Leu Pro Lys Arg Val Val Asn Thr Leu Phe
65 70 75 80

Gly Lys Gly Ala Ile His Thr Thr Gly Gly Lys Lys His Ile Asp Arg
85 90 95

Lys Ala Leu Phe Met Ser Leu Met Thr Glu Glu Asn Leu Glu Tyr Leu
100 105 110

Arg Glu Leu Thr Arg Ser Thr Trp Phe Met Asn Thr Glu Arg Met Glu
115 120 125

Arg Met Asp Glu Val Asn Val Tyr Lys Glu Ser Ile Ile Leu Leu Thr
130 135 140

Lys Val Gly Phe Arg Trp Ala Gly Ile Ile Ala Ser Pro Glu Glu Ile
145 150 155 160

Glu Ser Cys Ala Lys Asp Met Asp Thr Met Ile Asp Ser Phe Lys Asn
165 170 175

Ile Gly Thr Ala Phe Lys Gly Tyr Arg Glu Ala Lys Lys Ala Arg Asp
180 185 190

Arg Val Glu Thr Phe Leu Glu Asn Gln Ile Ile Ala Val Arg Glu Gly
195 200 205

Lys Leu Thr Pro Pro Gln Gly Thr Ala Leu His Glu Phe Ser His Trp
210 215 220

Glu Asp Phe Glu Gly Asn Leu Met Asp Ser Arg Leu Cys Ala Ile Asp
225                 230                 235                 240

Leu Met Asn Val Val Arg Pro Leu Val Ala Ile Asn Arg Phe Val Ser
                245                 250                 255

Phe Gly Val Lys Ala Leu His Asp Tyr Pro Gly Glu Ala Glu Lys Val
            260                 265                 270

Phe Asn Asn Glu Asn Asp Tyr Ala Tyr Lys Phe Val Gln Glu Val Arg
        275                 280                 285

Arg Phe Tyr Pro Phe Val Pro Phe Leu Pro Gly Lys Ala Ala Val Asp
    290                 295                 300

Ile Glu Phe Asp Gly Tyr Lys Ile Glu Lys Asp Thr Phe Leu Val Leu
305                 310                 315                 320

Asp Ile Tyr Gly Thr Leu His Arg Glu Gly Leu Trp Glu Asn Pro Glu
                325                 330                 335

Arg Phe Tyr Pro Asn Arg Phe Ser Asp Trp Asp Gly Ser Pro Phe Asp
            340                 345                 350

Leu Ile Pro Gln Gly Gly Gly Asp Tyr Tyr Thr Asn His Arg Cys Ala
        355                 360                 365

Gly Glu Trp Met Thr Ile Ile Ile Met Glu Glu Ser Met Lys Tyr Phe
    370                 375                 380

Ala Arg Asn Ile Ser Tyr Asp Met Lys Lys Asp Gln Asp Leu Ser Val
385                 390                 395                 400

Asn Leu Asn Lys Leu Pro Gly Arg Val Val Ser Gly Thr Ile Ile Glu
                405                 410                 415

Asn Val Asn Ala Leu Val Asn Arg Asn Val Glu Ser Val
            420                 425

<210>  70
<211>  429
<212>  PRT
<213>  Staphylococcus sciuri

<400>  70

Met Gly Lys Gln Ile Pro Lys Asp Arg Gly Leu Asp Ser Thr Phe Lys
1               5                   10                  15

```
Val Leu Lys Glu Gly Tyr Lys Tyr Val Pro Asn Arg Leu Glu Lys Phe
            20                  25                  30

Asp Thr Asn Ile Phe Glu Leu Arg Ala Leu Gly Gly Arg Arg Thr Val
        35                  40                  45

Val Ile Ser Gly Lys Glu Ala Ala Glu Leu Phe Tyr Asn Asn Asp Leu
    50                  55                  60

Ile Glu Arg Gln Gly Thr Leu Pro Lys Arg Val Val Asn Thr Leu Phe
65                  70                  75                  80

Gly Lys Gly Ala Ile His Thr Thr Gly Gly Lys Lys His Ile Asp Arg
                85                  90                  95

Lys Ala Leu Phe Met Ser Leu Met Thr Glu Glu Asn Leu Glu Tyr Leu
            100                 105                 110

Arg Glu Leu Thr Arg Ser Thr Trp Phe Met Asn Thr Glu Arg Met Glu
        115                 120                 125

Arg Met Asp Glu Val Asn Val Tyr Lys Glu Ser Ile Ile Leu Leu Thr
    130                 135                 140

Lys Val Gly Phe Arg Trp Ala Gly Val Ile Ala Ser Pro Glu Glu Ile
145                 150                 155                 160

Glu Ser Cys Ala Gln Asp Met Asp Thr Met Ile Asp Ser Phe Lys Asn
                165                 170                 175

Ile Gly Thr Ala Phe Lys Gly Tyr Arg Glu Ala Lys Lys Ala Arg Asp
            180                 185                 190

Arg Val Glu Thr Phe Leu Glu Asn Gln Ile Ile Ala Val Arg Glu Gly
        195                 200                 205

Lys Leu Thr Pro Pro Gln Gly Thr Ala Leu His Glu Phe Ser His Trp
    210                 215                 220

Glu Asp Phe Glu Gly Asn Leu Met Asp Ser Arg Leu Cys Ala Ile Asp
225                 230                 235                 240

Leu Met Asn Val Val Arg Pro Leu Val Ala Ile Asn Arg Phe Ile Ser
                245                 250                 255

Phe Gly Val Lys Ala Leu His Asp Tyr Pro Gly Glu Ala Glu Lys Val
            260                 265                 270
```

Phe Asn Asn Glu Asn Asp Tyr Ala Tyr Lys Phe Val Gln Glu Val Arg
275 280 285

Arg Phe Tyr Pro Phe Val Pro Tyr Leu Pro Gly Lys Ala Ala Val Asn
290 295 300

Ile Glu Phe Asp Gly Tyr Thr Ile Glu Lys Asp Thr Phe Leu Val Leu
305 310 315 320

Asp Ile Tyr Gly Thr Leu His Arg Asp Gly Leu Trp Glu Asn Pro Glu
325 330 335

Arg Phe Tyr Pro Asn Arg Phe Ser Asp Trp Asp Gly Ser Pro Phe Asp
340 345 350

Leu Ile Pro Gln Gly Gly Gly Asp Tyr Tyr Thr Asn His Arg Cys Ala
355 360 365

Gly Glu Trp Met Thr Ile Ile Ile Met Glu Glu Ser Met Lys Tyr Phe
370 375 380

Ala Arg Asn Ile Ser Tyr Asp Met Lys Lys Asp Gln Asp Leu Ser Val
385 390 395 400

Asn Leu Asn Lys Leu Pro Gly Arg Val Val Ser Gly Thr Ile Ile Glu
405 410 415

Asn Val Asn Ala Leu Val Asn Arg Asn Val Glu Ser Val
420 425

<210> 71
<211> 429
<212> PRT
<213> Staphylococcus sp.

<400> 71

Met Gly Lys Gln Ile Pro Lys Asp Arg Gly Leu Asp Ser Thr Leu Lys
1 5 10 15

Val Leu Lys Glu Gly Tyr Lys Tyr Val Pro Asn Arg Leu Glu Lys Phe
20 25 30

Asp Thr Asn Ile Phe Glu Leu Arg Ala Leu Gly Gly Arg Arg Thr Val
35 40 45

Val Phe Ser Gly Lys Glu Ala Ala Glu Ile Phe Tyr Asn Asn Glu Leu
50 55 60

```
Ile Glu Arg Gln Gly Thr Leu Pro Lys Arg Val Val Asn Thr Leu Phe
65                  70                  75                  80

Gly Lys Gly Ala Ile His Thr Thr Gly Gly Lys Lys His Ile Asp Arg
                85                  90                  95

Lys Ala Leu Phe Met Ser Leu Met Thr Glu Glu Asn Leu Glu Tyr Leu
            100                 105                 110

Arg Glu Leu Thr Arg Ser Thr Trp Phe Met Asn Thr Glu Arg Met Glu
        115                 120                 125

Arg Met Asp Glu Val Asn Val Tyr Lys Glu Ser Ile Ile Leu Leu Thr
    130                 135                 140

Lys Val Gly Phe Arg Trp Ala Gly Ile Ile Ala Ser Pro Glu Glu Ile
145                 150                 155                 160

Glu Ser Cys Ala Lys Asp Met Asp Thr Met Ile Asp Ser Phe Lys Asn
                165                 170                 175

Ile Gly Thr Ala Phe Lys Gly Tyr Arg Glu Ala Lys Lys Ala Arg Asp
            180                 185                 190

Arg Val Glu Thr Phe Leu Glu Asn Gln Ile Ile Ala Val Arg Glu Gly
        195                 200                 205

Lys Leu Thr Pro Pro Gln Gly Thr Ala Leu His Glu Phe Ser His Trp
    210                 215                 220

Glu Asp Phe Glu Gly Asn Leu Met Asp Ser Arg Leu Cys Ala Ile Asp
225                 230                 235                 240

Leu Met Asn Val Val Arg Pro Leu Val Ala Ile Asn Arg Phe Val Ser
                245                 250                 255

Phe Gly Val Lys Ala Leu His Asp Tyr Pro Gly Glu Ala Glu Lys Val
            260                 265                 270

Phe Asn Asn Glu Asn Asp Tyr Ala Tyr Lys Phe Val Gln Glu Val Arg
        275                 280                 285

Arg Phe Tyr Pro Phe Val Pro Phe Leu Pro Gly Lys Ala Ala Val Asp
    290                 295                 300

Ile Glu Phe Asp Gly Tyr Thr Ile Glu Lys Asp Thr Phe Leu Val Leu
305                 310                 315                 320
```

Asp Ile Tyr Gly Thr Leu His Arg Glu Gly Leu Trp Glu Asn Pro Glu
325 330 335

Arg Phe Tyr Pro Asn Arg Phe Ser Asp Trp Asp Gly Ser Pro Phe Asp
340 345 350

Leu Ile Pro Gln Gly Gly Gly Asp Tyr Tyr Thr Asn His Arg Cys Ala
355 360 365

Gly Glu Trp Met Thr Ile Ile Ile Met Glu Glu Ser Met Lys Tyr Phe
370 375 380

Ala Arg Asn Ile Ser Tyr Asp Met Lys Lys Asp Gln Asp Leu Ser Val
385 390 395 400

Asn Leu Asn Lys Leu Pro Gly Arg Val Val Ser Gly Thr Ile Ile Glu
405 410 415

Asn Val Asn Ala Leu Val Asn Arg Asn Val Glu Ser Val
420 425

<210> 72
<211> 429
<212> PRT
<213> Staphylococcus sciuri

<400> 72

Met Gly Lys Gln Ile Pro Lys Asp Arg Gly Leu Asp Ser Thr Phe Lys
1 5 10 15

Val Leu Lys Glu Gly Tyr Lys Tyr Val Pro Asn Arg Leu Glu Lys Phe
20 25 30

Asp Thr Asn Ile Phe Glu Leu Arg Ala Leu Gly Gly Arg Arg Thr Val
35 40 45

Val Ile Ser Gly Lys Glu Ala Ala Glu Leu Phe Tyr Asn Asn Asp Leu
50 55 60

Ile Glu Arg Gln Gly Thr Leu Pro Lys Arg Val Val Asn Thr Leu Phe
65 70 75 80

Gly Lys Gly Ala Ile His Thr Thr Gly Gly Lys Lys His Ile Asp Arg
85 90 95

Lys Ala Leu Phe Met Ser Leu Met Thr Glu Glu Asn Leu Glu Tyr Leu
100 105 110

```
Arg Glu Leu Thr Arg Ser Thr Trp Phe Met Asn Thr Glu Arg Met Glu
        115                 120                 125

Arg Met Asp Glu Val Asn Val Tyr Lys Glu Ser Ile Ile Leu Leu Thr
    130                 135                 140

Lys Val Gly Phe Arg Trp Ala Gly Val Ile Ala Ser Pro Glu Glu Ile
145                 150                 155                 160

Glu Ser Cys Ala Gln Asp Met Asp Thr Met Ile Asp Ser Phe Lys Asn
                165                 170                 175

Ile Gly Thr Ala Phe Lys Gly Tyr Arg Glu Ala Lys Lys Ala Arg Asp
            180                 185                 190

Arg Val Glu Thr Phe Leu Glu Asn Gln Ile Ile Ala Val Arg Glu Gly
        195                 200                 205

Lys Leu Thr Pro Pro Gln Gly Thr Ala Leu His Glu Phe Ser His Trp
    210                 215                 220

Glu Asp Phe Glu Gly Asn Leu Met Asp Ser Arg Leu Cys Ala Ile Asp
225                 230                 235                 240

Leu Met Asn Val Val Arg Pro Leu Val Ala Ile Asn Arg Phe Ile Ser
                245                 250                 255

Phe Gly Val Lys Ala Leu His Asp Tyr Pro Gly Glu Ala Glu Lys Val
            260                 265                 270

Phe Asn Asn Glu Asn Asp Tyr Ala Tyr Lys Phe Val Gln Glu Val Arg
        275                 280                 285

Arg Phe Tyr Pro Phe Val Pro Tyr Leu Pro Gly Lys Ala Ala Val Asp
    290                 295                 300

Ile Glu Phe Asp Gly Tyr Thr Ile Glu Lys Asp Thr Phe Leu Val Leu
305                 310                 315                 320

Asp Ile Tyr Gly Thr Leu His Arg Asp Gly Leu Trp Asp Asn Pro Glu
                325                 330                 335

Arg Phe Tyr Pro Asn Arg Phe Ser Asp Trp Asp Gly Ser Pro Phe Asp
            340                 345                 350

Leu Ile Pro Gln Gly Gly Gly Asp Tyr Tyr Thr Asn His Arg Cys Ala
```

355 360 365

Gly Glu Trp Met Thr Ile Ile Ile Met Glu Glu Ser Met Lys Tyr Phe
370 375 380

Ala Arg Asn Ile Ser Tyr Asp Met Lys Lys Asp Gln Asp Leu Ser Val
385 390 395 400

Asn Leu Asn Lys Leu Pro Gly Arg Val Val Ser Gly Thr Ile Ile Glu
405 410 415

Asn Val Asn Ala Leu Val Asn Arg Asn Val Glu Ser Val
420 425

<210> 73
<211> 429
<212> PRT
<213> Staphylococcus sciuri

<400> 73

Met Gly Lys Gln Ile Pro Lys Asp Arg Gly Leu Asp Ser Thr Phe Lys
1 5 10 15

Val Leu Lys Glu Gly Tyr Lys Tyr Val Pro Asn Arg Leu Glu Lys Phe
20 25 30

Asp Thr Asn Ile Phe Glu Leu Arg Ala Leu Gly Gly Arg Arg Thr Val
35 40 45

Val Ile Ser Gly Lys Glu Ala Ala Glu Leu Phe Tyr Asn Asn Asp Leu
50 55 60

Ile Glu Arg Gln Gly Thr Leu Pro Lys Arg Val Val Asn Thr Leu Phe
65 70 75 80

Gly Lys Gly Ala Ile His Thr Thr Gly Gly Lys Lys His Ile Asp Arg
85 90 95

Lys Ala Leu Phe Met Ser Leu Met Thr Glu Glu Asn Leu Glu Tyr Leu
100 105 110

Arg Glu Leu Thr Arg Ser Thr Trp Phe Met Asn Thr Glu Arg Met Glu
115 120 125

Arg Met Asp Glu Val Asn Val Tyr Lys Glu Ser Ile Ile Leu Leu Thr
130 135 140

Lys Val Gly Phe Arg Trp Ala Gly Val Ile Ala Ser Pro Glu Glu Ile

145 150 155 160

Glu Ser Cys Ala Arg Asp Met Asp Thr Met Ile Asp Ser Phe Lys Asn
165 170 175

Ile Gly Thr Ala Phe Lys Gly Tyr Arg Glu Ala Lys Lys Ala Arg Asp
180 185 190

Arg Val Glu Thr Phe Leu Glu Asn Gln Ile Ile Ala Val Arg Glu Gly
195 200 205

Lys Leu Thr Pro Pro Gln Gly Thr Ala Leu His Glu Phe Ser His Trp
210 215 220

Glu Asp Phe Glu Gly Asn Leu Met Asp Ser Arg Leu Cys Ala Ile Asp
225 230 235 240

Leu Met Asn Val Val Arg Pro Leu Val Ala Ile Asn Arg Phe Ile Ser
245 250 255

Phe Gly Val Lys Ala Leu His Asp Tyr Pro Gly Glu Ala Glu Lys Val
260 265 270

Phe Asn Asn Glu Asn Asp Tyr Ala Tyr Lys Phe Val Gln Glu Val Arg
275 280 285

Arg Phe Tyr Pro Phe Val Pro Tyr Leu Pro Gly Lys Ala Ala Val Asp
290 295 300

Ile Glu Phe Asp Gly Tyr Thr Ile Glu Lys Asp Thr Phe Leu Val Leu
305 310 315 320

Asp Ile Tyr Gly Thr Leu His Arg Asp Gly Leu Trp Glu Asn Pro Glu
325 330 335

Arg Phe Tyr Pro Asn Arg Phe Ser Asp Trp Asp Gly Ser Pro Phe Asp
340 345 350

Leu Ile Pro Gln Gly Gly Gly Asp Tyr Tyr Thr Asn His Arg Cys Ala
355 360 365

Gly Glu Trp Met Thr Ile Ile Ile Met Glu Glu Ser Met Lys Tyr Phe
370 375 380

Ala Arg Asn Ile Ser Tyr Asp Met Lys Lys Asp Gln Asp Leu Ser Val
385 390 395 400

Asn Leu Asn Lys Leu Pro Gly Arg Val Val Ser Gly Thr Ile Ile Glu
405 410 415

Asn Val Asn Ala Leu Val Asn Arg Asn Val Glu Ser Val
420 425

<210> 74
<211> 429
<212> PRT
<213> Staphylococcus sp.

<400> 74

Met Gly Lys Gln Ile Pro Lys Asp Arg Gly Leu Asp Ser Thr Phe Lys
1 5 10 15

Val Leu Lys Glu Gly Tyr Lys Tyr Val Pro Asn Arg Leu Glu Lys Phe
20 25 30

Asp Thr Asn Ile Phe Glu Leu Arg Ala Leu Gly Gly Arg Arg Thr Val
35 40 45

Val Ile Ser Gly Lys Glu Ala Ala Glu Leu Phe Tyr Asn Asn Asp Leu
50 55 60

Ile Glu Arg Gln Gly Thr Leu Pro Lys Arg Val Val Asn Thr Leu Phe
65 70 75 80

Gly Lys Gly Ala Ile His Thr Thr Gly Gly Lys Lys His Ile Asp Arg
85 90 95

Lys Ala Leu Phe Met Ser Leu Met Thr Glu Glu Asn Leu Glu Tyr Leu
100 105 110

Arg Glu Leu Thr Arg Ser Thr Trp Phe Met Asn Thr Glu Arg Met Glu
115 120 125

Arg Met Asp Glu Val Asn Val Tyr Lys Glu Ser Ile Ile Leu Leu Thr
130 135 140

Lys Val Gly Phe Arg Trp Ala Gly Val Ile Ala Ser Pro Glu Glu Ile
145 150 155 160

Glu Ser Cys Ala Gln Asp Met Asp Thr Met Ile Asp Ser Phe Lys Asn
165 170 175

Ile Gly Thr Ala Phe Lys Gly Tyr Arg Glu Ala Lys Lys Ala Arg Asp
180 185 190

```
Arg Val Glu Thr Phe Leu Glu Asn Gln Ile Ile Ala Val Arg Glu Gly
        195                 200                 205

Lys Leu Thr Pro Pro Gln Gly Thr Ala Leu His Glu Phe Ser His Trp
    210                 215                 220

Glu Asp Phe Glu Gly Asn Leu Met Asp Ser Arg Leu Cys Ala Ile Asp
225                 230                 235                 240

Leu Met Asn Val Val Arg Pro Leu Val Ala Ile Asn Arg Phe Ile Ser
                245                 250                 255

Phe Gly Val Lys Ala Leu His Asp Tyr Pro Gly Glu Ala Glu Lys Val
            260                 265                 270

Phe Asn Asn Glu Asn Asp Tyr Ala Tyr Lys Phe Val Gln Glu Val Arg
        275                 280                 285

Arg Phe Tyr Pro Phe Val Pro Tyr Leu Pro Gly Lys Ala Ala Val Asp
    290                 295                 300

Ile Glu Phe Asp Gly Tyr Thr Ile Glu Lys Asp Thr Phe Leu Val Leu
305                 310                 315                 320

Asp Ile Tyr Gly Thr Leu His Arg Asp Gly Leu Trp Glu Asn Pro Glu
                325                 330                 335

Arg Phe Tyr Pro Asn Arg Phe Ser Asp Trp Asp Gly Ser Pro Phe Asp
            340                 345                 350

Leu Ile Pro Gln Gly Gly Gly Asp Tyr Tyr Thr Asn His Arg Cys Ala
        355                 360                 365

Gly Glu Trp Met Thr Ile Ile Ile Met Glu Glu Ser Met Lys Tyr Phe
    370                 375                 380

Ala Arg Asn Ile Ser Tyr Asp Met Lys Lys Asp Gln Asp Leu Ser Val
385                 390                 395                 400

Asn Leu Asn Lys Leu Pro Gly Arg Val Val Ser Gly Thr Ile Ile Glu
                405                 410                 415

Asn Val Asn Ala Leu Val Asn Arg Asn Val Glu Ser Val
            420                 425
```

<210> 75
<211> 429
<212> PRT

<213> Staphylococcus vitulinus

<400> 75

Met Gly Lys Gln Ile Pro Lys Asp Arg Gly Leu Asp Ser Thr Leu Lys
1 5 10 15

Val Leu Lys Glu Gly Tyr Lys Tyr Val Pro Asn Arg Leu Glu Lys Phe
20 25 30

Asp Thr Asn Ile Phe Glu Leu Arg Ala Leu Gly Gly Arg Arg Thr Val
35 40 45

Val Ile Ser Gly Lys Glu Ala Ala Glu Leu Phe Tyr Asn Asn Glu Leu
50 55 60

Thr Glu Arg Gln Gly Thr Leu Pro Lys Arg Val Val Asn Thr Leu Phe
65 70 75 80

Gly Lys Gly Ala Ile His Thr Thr Gly Gly Lys Lys His Ile Asp Arg
85 90 95

Lys Ala Leu Phe Met Ser Leu Met Thr Glu Glu Asn Leu Asp Tyr Leu
100 105 110

Arg Glu Leu Thr Arg Ser Thr Trp Phe Met Asn Thr Glu Arg Met Glu
115 120 125

Arg Met Asp Glu Val Asn Val Tyr Lys Glu Ser Ile Ile Leu Leu Thr
130 135 140

Lys Val Gly Phe Arg Trp Ala Gly Ile Ile Ala Ser Pro Glu Glu Ile
145 150 155 160

Glu Ser Cys Ala His Asp Met Asp Thr Met Ile Asp Ser Phe Lys Asn
165 170 175

Ile Gly Thr Val Phe Lys Gly Tyr Arg Glu Ala Lys Gln Ala Arg Ala
180 185 190

Arg Val Glu Thr Phe Leu Glu Asn Gln Ile Ile Ala Val Arg Glu Gly
195 200 205

Lys Leu Thr Pro Pro Gln Gly Thr Ala Leu His Glu Phe Ser His Trp
210 215 220

Glu Asp Phe Glu Gly Asn Leu Met Asp Ser Arg Leu Cys Ala Ile Asp
225 230 235 240

Leu Met Asn Val Val Arg Pro Leu Val Ala Ile Asn Arg Phe Ile Ser
                245                 250                 255

Phe Gly Val Lys Ala Leu His Asp Tyr Pro Gly Glu Ala Glu Lys Val
            260                 265                 270

Phe Asn Asn Glu Asp Asp Tyr Ala Tyr Lys Phe Val Gln Glu Ile Arg
        275                 280                 285

Arg Phe Tyr Pro Phe Val Pro Phe Leu Pro Gly Lys Ala Ala Val Asp
    290                 295                 300

Ile Glu Phe Glu Gly Tyr Thr Ile Glu Lys Asp Thr Phe Leu Val Leu
305                 310                 315                 320

Asp Ile Tyr Gly Thr Leu His Arg Glu Gly Leu Trp Glu Asn Pro Glu
                325                 330                 335

Arg Phe Tyr Pro Asn Arg Phe Ser Asp Trp Asp Gly Ser Pro Phe Asp
            340                 345                 350

Leu Ile Pro Gln Gly Gly Gly Asp Tyr His Thr Asn His Arg Cys Ala
        355                 360                 365

Gly Glu Trp Met Thr Leu Ile Ile Met Glu Glu Ser Met Lys Tyr Phe
    370                 375                 380

Ala Arg Asn Ile Ser Tyr Asp Val Lys Lys Asp Gln Asp Leu Ser Val
385                 390                 395                 400

Asn Leu Asn Lys Leu Pro Gly Arg Val Val Ser Gly Thr Ile Ile Glu
                405                 410                 415

Asn Val Asn Ala Leu Val Asn Arg Asn Val Glu Ser Val
            420                 425

<210> 76
<211> 429
<212> PRT
<213> Staphylococcus vitulinus

<400> 76

Met Gly Lys Gln Ile Pro Lys Asp Arg Gly Leu Asp Ser Thr Leu Lys
1               5                   10                  15

Val Leu Lys Glu Gly Tyr Lys Tyr Val Pro Asn Arg Leu Glu Lys Phe
            20                  25                  30

```
Asp Thr Asn Ile Phe Glu Leu Arg Ala Leu Gly Gly Arg Arg Thr Val
        35                  40                  45

Val Ile Ser Gly Lys Glu Ala Ala Glu Leu Phe Tyr Asn Asn Glu Leu
    50                  55                  60

Thr Glu Arg Gln Gly Thr Leu Pro Lys Arg Val Val Asn Thr Leu Phe
65                  70                  75                  80

Gly Lys Gly Ala Ile His Thr Thr Gly Gly Lys Lys His Ile Asp Arg
                85                  90                  95

Lys Ala Leu Phe Met Ser Leu Met Thr Glu Glu Asn Leu Asp Tyr Leu
            100                 105                 110

Arg Glu Leu Thr Arg Ser Thr Trp Phe Met Asn Thr Glu Arg Met Glu
        115                 120                 125

Arg Met Asp Glu Val Asn Val Tyr Lys Glu Ser Ile Ile Leu Leu Thr
    130                 135                 140

Lys Val Gly Phe Arg Trp Ala Gly Ile Ile Ala Ser Pro Glu Glu Ile
145                 150                 155                 160

Glu Ser Cys Ala Gln Asp Met Asp Thr Met Ile Asp Ser Phe Lys Asn
                165                 170                 175

Ile Gly Thr Val Phe Lys Gly Tyr Arg Glu Ala Lys Gln Ala Arg Ala
            180                 185                 190

Arg Val Glu Thr Phe Leu Glu Asn Gln Ile Ile Ala Val Arg Glu Gly
        195                 200                 205

Lys Leu Thr Pro Pro Gln Gly Thr Ala Leu His Glu Phe Ser His Trp
    210                 215                 220

Glu Asp Phe Glu Gly Asn Leu Met Asp Ser Arg Leu Cys Ala Ile Asp
225                 230                 235                 240

Leu Met Asn Val Val Arg Pro Leu Val Ala Ile Asn Arg Phe Ile Ser
                245                 250                 255

Phe Gly Val Lys Ala Leu His Asp Tyr Pro Gly Glu Ala Glu Lys Val
            260                 265                 270

Phe Asn Asn Glu Asp Asp Tyr Ala Tyr Lys Phe Val Gln Glu Ile Arg
        275                 280                 285
```

Arg Phe Tyr Pro Phe Val Pro Phe Leu Pro Gly Lys Ala Ala Val Asp
    290                 295                 300

Ile Glu Phe Glu Gly Tyr Thr Ile Glu Lys Asp Thr Phe Leu Val Leu
305                 310                 315                 320

Asp Ile Tyr Gly Thr Leu His Arg Glu Gly Leu Trp Glu Asn Pro Glu
                325                 330                 335

Arg Phe Tyr Pro Asn Arg Phe Ser Asp Trp Asp Gly Ser Pro Phe Asp
            340                 345                 350

Leu Ile Pro Gln Gly Gly Gly Asp Tyr His Thr Asn His Arg Cys Ala
        355                 360                 365

Gly Glu Trp Met Thr Leu Ile Ile Met Glu Glu Ser Met Lys Tyr Phe
    370                 375                 380

Ala Arg Asn Ile Ser Tyr Asp Val Lys Lys Asp Gln Asp Leu Ser Val
385                 390                 395                 400

Asn Leu Asn Lys Leu Pro Gly Arg Val Val Ser Gly Thr Ile Ile Glu
                405                 410                 415

Asn Val Asn Ala Leu Val Asn Arg Asn Val Glu Ser Val
            420                 425

<210> 77
<211> 429
<212> PRT
<213> Staphylococcus sp.

<400> 77

Met Gly Lys Gln Ile Pro Lys Asp Arg Gly Leu Asp Ser Thr Leu Lys
1               5                   10                  15

Val Leu Lys Glu Gly Tyr Ile Tyr Val Pro Asn Arg Leu Glu Lys Phe
            20                  25                  30

Asp Thr Asn Ile Phe Glu Leu Arg Ala Leu Gly Gly Arg Arg Thr Val
        35                  40                  45

Val Ile Ser Gly Lys Glu Ala Ala Glu Leu Phe Tyr Asn Asn Glu Leu
    50                  55                  60

Thr Glu Arg Gln Gly Thr Leu Pro Lys Arg Val Val Asn Thr Leu Phe
65                  70                  75                  80

```
Gly Lys Gly Ala Ile His Thr Thr Gly Gly Lys Lys His Ile Asp Arg
                85                  90                  95

Lys Ala Leu Phe Met Ser Leu Met Thr Glu Glu Asn Leu Asp Tyr Leu
            100                 105                 110

Arg Glu Leu Thr Arg Ser Thr Trp Phe Met Asn Thr Glu Arg Met Glu
        115                 120                 125

Arg Met Asp Glu Val Asn Val Tyr Lys Glu Ser Ile Ile Leu Leu Thr
    130                 135                 140

Lys Val Gly Phe Arg Trp Ala Gly Ile Ile Ala Ser Pro Glu Glu Ile
145                 150                 155                 160

Glu Ser Cys Ala His Asp Met Asp Thr Met Ile Asp Ser Phe Lys Asn
                165                 170                 175

Ile Gly Thr Val Phe Lys Gly Tyr Arg Glu Ala Lys Gln Ala Arg Ala
            180                 185                 190

Arg Val Glu Thr Phe Leu Glu Asn Gln Ile Ile Ala Val Arg Glu Gly
        195                 200                 205

Lys Leu Thr Pro Pro Gln Gly Thr Ala Leu His Glu Phe Ser His Trp
    210                 215                 220

Glu Asp Phe Glu Gly Asn Leu Met Asp Ser Arg Leu Cys Ala Ile Asp
225                 230                 235                 240

Leu Met Asn Val Val Arg Pro Leu Val Ala Ile Asn Arg Phe Ile Ser
                245                 250                 255

Phe Gly Val Lys Ala Leu His Asp Tyr Pro Gly Glu Ala Glu Lys Val
            260                 265                 270

Phe Asn Asn Glu Asp Asp Tyr Ala Tyr Lys Phe Val Gln Glu Ile Arg
        275                 280                 285

Arg Phe Tyr Pro Phe Val Pro Phe Leu Pro Gly Lys Ala Ala Val Asp
    290                 295                 300

Ile Glu Phe Glu Gly Tyr Thr Ile Glu Lys Asp Thr Phe Leu Val Leu
305                 310                 315                 320

Asp Ile Tyr Gly Thr Leu His Arg Glu Gly Leu Trp Glu Asn Pro Glu
```

```
                325                 330                 335

Arg Phe Tyr Pro Asn Arg Phe Ser Asp Trp Asp Gly Ser Pro Phe Asp
            340                 345                 350

Leu Ile Pro Gln Gly Gly Gly Asp Tyr His Thr Asn His Arg Cys Ala
        355                 360                 365

Gly Glu Trp Met Thr Leu Ile Ile Met Glu Glu Ser Met Lys Tyr Phe
    370                 375                 380

Ala Arg Asn Ile Ser Tyr Asp Val Lys Lys Asp Gln Asp Leu Ser Val
385                 390                 395                 400

Asn Leu Asn Lys Leu Pro Gly Arg Val Val Ser Gly Thr Ile Ile Glu
                405                 410                 415

Asn Val Asn Ala Leu Val Asn Arg Asn Val Glu Ser Val
            420                 425


<210>  78
<211>  429
<212>  PRT
<213>  Staphylococcus sciuri

<400>  78

Met Gly Lys Gln Ile Pro Lys Asp Arg Gly Leu Asp Ser Thr Phe Lys
1               5                   10                  15

Val Leu Lys Glu Gly Tyr Lys Tyr Val Pro Asn Arg Leu Glu Lys Phe
            20                  25                  30

Asp Thr Asn Ile Phe Glu Leu Arg Ala Leu Gly Gly Arg Arg Thr Val
        35                  40                  45

Val Ile Ser Gly Lys Glu Ala Thr Glu Leu Phe Tyr Asn Asn Asp Leu
    50                  55                  60

Ile Glu Arg Gln Gly Thr Leu Pro Lys Arg Val Val Asn Thr Leu Phe
65                  70                  75                  80

Gly Lys Gly Ala Ile His Thr Thr Gly Gly Lys Lys His Ile Asp Arg
                85                  90                  95

Lys Ala Leu Phe Met Ser Leu Met Thr Glu Glu Asn Leu Glu Tyr Leu
            100                 105                 110

Arg Glu Leu Thr Arg Ser Thr Trp Phe Met Asn Thr Glu Arg Met Glu
```

```
        115                 120                 125

Arg Met Asp Glu Val Asn Val Tyr Lys Glu Ser Ile Ile Leu Leu Thr
    130                 135                 140

Lys Val Gly Phe Arg Trp Ala Gly Val Ile Ala Ser Pro Glu Glu Ile
145                 150                 155                 160

Glu Ser Cys Ala Gln Asp Met Asp Thr Met Ile Asp Ser Phe Lys Asn
                165                 170                 175

Ile Gly Thr Ala Phe Lys Gly Tyr Arg Glu Ala Lys Lys Ala Arg Asp
            180                 185                 190

Arg Val Glu Thr Phe Leu Glu Asn Gln Ile Ile Ala Val Arg Glu Gly
        195                 200                 205

Lys Leu Thr Pro Pro Gln Gly Thr Ala Leu His Glu Phe Ser His Trp
    210                 215                 220

Glu Asp Phe Glu Gly Asn Leu Met Asp Ser Arg Leu Cys Ala Ile Asp
225                 230                 235                 240

Leu Met Asn Val Val Arg Pro Leu Val Ala Ile Asn Arg Phe Ile Ser
                245                 250                 255

Phe Gly Val Lys Ala Leu His Asp Tyr Pro Gly Glu Ala Glu Lys Val
            260                 265                 270

Phe Asn Asn Glu Asn Asp Tyr Ala Tyr Lys Phe Val Gln Glu Val Arg
        275                 280                 285

Arg Phe Tyr Pro Phe Val Pro Tyr Leu Pro Gly Lys Ala Ala Val Asp
    290                 295                 300

Ile Glu Phe Asp Gly Tyr Thr Ile Glu Lys Asp Thr Phe Leu Val Leu
305                 310                 315                 320

Asp Ile Tyr Gly Thr Leu His Arg Asp Gly Leu Trp Glu Asn Pro Glu
                325                 330                 335

Arg Phe Tyr Pro Asn Arg Phe Ser Asp Trp Asp Gly Ser Pro Phe Asp
            340                 345                 350

Leu Ile Pro Gln Gly Gly Gly Asp Tyr Tyr Thr Asn His Arg Cys Ala
        355                 360                 365
```

Gly Glu Trp Met Thr Ile Ile Ile Met Glu Glu Ser Met Lys Tyr Phe
    370                 375                 380

Ala Arg Asn Ile Ser Tyr Asp Met Lys Lys Asp Gln Asp Leu Ser Val
385                 390                 395                 400

Asn Leu Asn Lys Leu Pro Gly Arg Val Val Ser Gly Thr Ile Ile Glu
                405                 410                 415

Asn Val Asn Ala Leu Val Asn Arg Asn Val Glu Ser Val
            420                 425

<210> 79
<211> 429
<212> PRT
<213> Staphylococcus stepanovicii

<400> 79

Met Gly Lys Gln Ile Pro Lys Glu Arg Gly Leu Asp Ser Thr Phe Lys
1               5                   10                  15

Val Leu Lys Glu Gly Tyr Lys Tyr Val Pro Asn Arg Leu Glu Lys His
            20                  25                  30

Asn Ser Asn Ile Phe Glu Leu Arg Ala Leu Gly Gly Arg Arg Thr Val
        35                  40                  45

Val Phe Ser Gly Lys Glu Ala Ala Glu Val Phe Tyr Asn Asn Asp Leu
    50                  55                  60

Ile Glu Arg Gln Gly Thr Leu Pro Lys Arg Val Val Asn Thr Leu Phe
65                  70                  75                  80

Gly Lys Gly Ala Ile His Thr Thr Gly Gly Lys Lys His Ile Asp Arg
                85                  90                  95

Lys Ala Leu Phe Met Ser Leu Met Thr Glu Glu Asn Leu Asp Tyr Leu
            100                 105                 110

Arg Glu Leu Thr Arg Ser Phe Trp Phe Thr Asn Ile Glu Arg Met Glu
        115                 120                 125

Arg Met Asp Glu Val Asn Val Tyr Lys Glu Ser Ile Ile Leu Leu Thr
    130                 135                 140

Lys Val Gly Phe Arg Trp Ala Gly Val Ile Ala Ser Pro Glu Glu Ile
145                 150                 155                 160

Glu Arg Cys Ala Glu Asp Met Asp Thr Met Ile Asp Ser Phe Lys Asn
165 170 175

Ile Gly Thr Ala Phe Lys Gly Tyr Lys Glu Ala Lys Gln Ala Arg Asp
180 185 190

Arg Val Glu Thr Phe Leu Glu Asn Gln Ile Ile Ala Val Arg Glu Gly
195 200 205

Lys Leu Thr Pro Pro Gln Gly Thr Ala Leu Tyr Glu Phe Ser His Trp
210 215 220

Glu Asp Phe Glu Gly Asn Pro Met Asp Ser Arg Leu Cys Ala Ile Asp
225 230 235 240

Leu Met Asn Val Val Arg Pro Leu Val Ala Ile Asn Arg Phe Val Ser
245 250 255

Phe Gly Val Lys Ala Leu His Asp Tyr Pro Gly Glu Ala Glu Lys Val
260 265 270

Phe Asn Asn Glu Asn Asp Tyr Ala Tyr Lys Phe Val Gln Glu Val Arg
275 280 285

Arg Phe Tyr Pro Phe Val Pro Phe Leu Pro Gly Arg Ala Ala Val Asp
290 295 300

Ile Glu Phe Glu Asp Tyr Thr Ile Glu Lys Gly Thr Phe Leu Val Leu
305 310 315 320

Asp Ile Tyr Gly Thr Leu His Lys Glu Gly Leu Trp Glu Asn Pro Glu
325 330 335

Arg Phe Tyr Pro Asn Arg Phe Ser Asp Trp Asp Gly Ser Pro Phe Asp
340 345 350

Leu Ile Pro Gln Gly Gly Gly Asp Tyr Asn Thr Asn His Arg Cys Ala
355 360 365

Gly Glu Trp Met Thr Ile Ile Ile Met Glu Glu Ser Met Lys Phe Phe
370 375 380

Ala Lys Asp Ile Ser Tyr Asp Val Lys Lys Asp Gln Asp Gln Ser Val
385 390 395 400

Asn Leu Asn Lys Leu Pro Gly Arg Val Ala Ser Gly Met Ile Ile Glu
405 410 415

Asn Val Asn Ala Leu Val Asn Arg Asn Val Glu Ser Val
420 425

<210> 80
<211> 429
<212> PRT
<213> Staphylococcus sp.

<400> 80

Met Gly Lys Gln Ile Pro Lys Asp Arg Gly Leu Asp Ser Thr Phe Lys
1 5 10 15

Val Leu Lys Glu Gly Tyr Asn Tyr Val Pro Ser Arg Leu Glu Lys Phe
20 25 30

Asp Ser Asn Ile Phe Glu Leu Arg Ala Leu Gly Gly Arg Arg Thr Val
35 40 45

Val Ile Ser Gly Lys Glu Ala Ala Glu Leu Phe Tyr Asn Asn Glu Leu
50 55 60

Ile Glu Arg Gln Gly Thr Leu Pro Lys Arg Val Val Asn Thr Leu Phe
65 70 75 80

Gly Lys Gly Ala Ile His Thr Thr Gly Gly Lys Lys His Ile Asp Arg
85 90 95

Lys Ala Leu Phe Met Ser Leu Met Thr Glu Glu Asn Leu Asp Tyr Leu
100 105 110

Arg Glu Leu Thr Arg Ser Thr Trp Phe Met Asn Thr Glu Arg Met Glu
115 120 125

Arg Met Asp Glu Val Asn Val Tyr Lys Glu Ser Ile Val Leu Leu Thr
130 135 140

Lys Val Gly Phe Arg Trp Ala Gly Ile Ile Ala Ser Pro Glu Glu Ile
145 150 155 160

Glu Ser Cys Ala Lys Asp Met Asp Thr Met Ile Asp Ser Phe Lys Asn
165 170 175

Ile Gly Thr Ala Phe Lys Gly Tyr Lys Glu Ala Lys Gln Ala Arg Ala
180 185 190

Arg Val Glu Thr Phe Leu Glu Asn Gln Ile Ile Ala Val Arg Glu Gly
195 200 205

Lys Leu Thr Pro Pro Gln Gly Thr Ala Leu His Glu Phe Ser His Trp
210 215 220

Glu Asp Phe Glu Gly Asn Leu Met Asp Ser Arg Leu Cys Ala Ile Asp
225 230 235 240

Leu Met Asn Val Val Arg Pro Leu Val Ala Ile Asn Arg Phe Val Ser
245 250 255

Phe Gly Val Lys Ala Leu His Asp Tyr Pro Gly Glu Ala Glu Lys Val
260 265 270

Phe Asn Asn Glu Asp Asp Tyr Ala Tyr Lys Phe Val Gln Glu Val Arg
275 280 285

Arg Phe Tyr Pro Phe Val Pro Phe Leu Pro Gly Lys Ala Ala Val Asp
290 295 300

Ile Glu Phe Asp Gly Tyr Thr Ile Glu Lys Asp Thr Phe Leu Val Met
305 310 315 320

Asp Ile Tyr Gly Thr Leu His Lys Glu Gly Leu Trp Glu Asn Pro Glu
325 330 335

Arg Phe Tyr Pro Asn Arg Phe Ser Asp Trp Asp Gly Ser Pro Phe Asp
340 345 350

Leu Ile Pro Gln Gly Gly Gly Asp Tyr Asn Thr Asn His Arg Cys Ala
355 360 365

Gly Glu Trp Met Thr Ile Ile Ile Met Glu Glu Ser Met Lys Tyr Phe
370 375 380

Ala Arg Asn Ile Ser Tyr Asp Met Lys Gln Asp Gln Asp Leu Ser Val
385 390 395 400

Lys Leu Asn Lys Leu Pro Gly Arg Val Val Ser Gly Thr Ile Ile Glu
405 410 415

Asn Val Asn Ala Leu Val Asn Arg Asn Val Glu Ser Val
420 425

<210> 81
<211> 429
<212> PRT
<213> Staphylococcus fleurettii

<400> 81

Met Gly Lys Gln Ile Pro Lys Asp Arg Gly Leu Asp Ser Thr Phe Lys
1               5                   10                  15

Val Leu Lys Glu Gly Tyr Asn Tyr Val Pro Ser Arg Leu Glu Lys Phe
            20                  25                  30

Asp Ser Asn Ile Phe Glu Leu Arg Ala Leu Gly Gly Arg Arg Thr Val
        35                  40                  45

Val Ile Ser Gly Lys Glu Ala Ala Glu Leu Phe Tyr Asn Asn Glu Leu
    50                  55                  60

Ile Glu Arg Gln Gly Thr Leu Pro Lys Arg Val Val Asn Thr Leu Phe
65                  70                  75                  80

Gly Lys Gly Ala Ile His Thr Thr Gly Gly Lys Lys His Ile Asp Arg
                85                  90                  95

Lys Ala Leu Phe Met Ser Leu Met Thr Glu Glu Asn Leu Asp Tyr Leu
            100                 105                 110

Arg Glu Leu Thr Arg Ser Thr Trp Phe Met Asn Thr Glu Arg Met Glu
        115                 120                 125

Arg Met Asp Glu Val Asn Val Tyr Lys Glu Ser Ile Val Leu Leu Thr
    130                 135                 140

Lys Val Gly Phe Arg Trp Ala Gly Ile Ile Ala Ser Pro Glu Glu Ile
145                 150                 155                 160

Glu Ser Cys Ala Lys Asp Met Asp Thr Met Ile Asp Ser Phe Lys Asn
                165                 170                 175

Ile Gly Thr Ala Phe Lys Gly Tyr Lys Glu Ala Lys Gln Ala Arg Ala
            180                 185                 190

Arg Val Glu Thr Phe Leu Glu Asn Gln Ile Ile Ala Val Arg Glu Gly
        195                 200                 205

Lys Leu Thr Pro Pro Gln Gly Thr Ala Leu His Glu Phe Ser His Trp
    210                 215                 220

Glu Asp Phe Glu Gly Asn Leu Met Asp Ser Arg Leu Cys Ala Ile Asp
225                 230                 235                 240

Leu Met Asn Val Val Arg Pro Leu Val Ala Ile Asn Arg Phe Val Ser
                245                 250                 255

Phe Gly Val Lys Ala Leu His Asp Tyr Pro Gly Glu Ala Glu Lys Val
260 265 270

Phe Asn Asn Glu Asp Asp Tyr Ala Tyr Lys Phe Val Gln Glu Val Arg
275 280 285

Arg Phe Tyr Pro Phe Val Pro Phe Leu Pro Gly Lys Ala Ala Val Asp
290 295 300

Ile Glu Phe Asp Gly Tyr Thr Ile Glu Lys Asp Thr Phe Leu Ile Met
305 310 315 320

Asp Ile Tyr Gly Thr Leu His Lys Glu Gly Leu Trp Glu Asn Pro Glu
325 330 335

Arg Phe Tyr Pro Asn Arg Phe Ser Asp Trp Asp Gly Ser Pro Phe Asp
340 345 350

Leu Ile Pro Gln Gly Gly Gly Asp Tyr Asn Thr Asn His Arg Cys Ala
355 360 365

Gly Glu Trp Met Thr Ile Ile Ile Met Glu Glu Ser Met Lys Tyr Phe
370 375 380

Ala Arg Asn Ile Ser Tyr Asp Met Lys Gln Asp Gln Asp Leu Ser Val
385 390 395 400

Lys Leu Asn Lys Leu Pro Gly Arg Val Val Ser Gly Thr Ile Ile Glu
405 410 415

Asn Val Asn Ala Leu Val Asn Arg Asn Val Glu Ser Val
420 425

<210> 82
<211> 425
<212> PRT
<213> Abyssicoccus albus

<400> 82

Met Gly Lys Ser Ile Pro Arg Asp Arg Gly Met Asp Asn Thr Val Lys
1 5 10 15

Ala Leu Lys Lys Gly Tyr Thr Tyr Ile Pro Glu Leu Arg Glu Arg Leu
20 25 30

Asn Ser Asn Asp Ile Val Ala Thr Arg Ala Leu Gly Gly Lys Lys Thr
35 40 45

Val Ile Ile Ser Gly Lys Asp Ala Ala Glu Leu Phe Tyr Asp Asn Asp
    50                  55                  60

Lys Ile Glu Arg Gln Gly Thr Leu Pro Pro Arg Val Val Lys Thr Leu
65                  70                  75                  80

Phe Gly Lys Gly Ala Ile His Thr Thr Thr Gly Lys Thr His Ile Asp
                85                  90                  95

Arg Lys Ala Leu Phe Met Ser Leu Met Thr Glu Glu Asn Leu Ser Tyr
            100                 105                 110

Leu Arg Glu Leu Thr Arg Leu Glu Trp Tyr Tyr Asn Thr Thr Arg Met
        115                 120                 125

Ser His Met Asp Glu Val Asn Val Tyr Lys Glu Ala Ile Tyr Val Leu
    130                 135                 140

Thr Lys Val Gly Ile Lys Trp Ala Gly Leu His Ala Ser Glu Asp Glu
145                 150                 155                 160

Ile Asn Gln Tyr Ala Glu Asp Met Asp Thr Met Ile Asp Ser Phe Lys
                165                 170                 175

Ala Ile Gly Thr Met Phe Lys Gly Tyr Lys Glu Ala Arg Lys Ala Arg
            180                 185                 190

Asp Arg Val Glu Thr Trp Leu Glu Ala Gln Ile Gln Gly Val Arg Asp
        195                 200                 205

Gly Glu Ile Thr Pro Pro Glu Gly Thr Ala Leu Tyr Glu Phe Ala His
    210                 215                 220

Trp Lys Asp Tyr Glu Gly Asn Pro Met Asp Ala Arg Leu Ala Ala Ile
225                 230                 235                 240

Asp Leu Met Asn Val Ile Arg Pro Leu Val Ala Ile Asn Arg Phe Val
                245                 250                 255

Ala Phe Gly Thr Leu Ala Met Tyr Glu Phe Pro Lys Glu Arg Thr Lys
            260                 265                 270

Val Ala Gln Asn Glu Glu Asp Tyr Val Tyr Lys Phe Ile Gln Glu Val
        275                 280                 285

Arg Arg Phe Tyr Pro Phe Val Pro Tyr Leu Pro Gly Lys Ala Gly Lys

290 295 300

Asp Ile Glu Phe Arg Gly Tyr Glu Ile Lys Lys Asp Thr Phe Met Val
305 310 315 320

Leu Asp Val Tyr Gly Thr Leu His Arg Glu Asp Leu Trp Glu Asn Ala
325 330 335

Asp Gln Phe Ile Pro Glu Arg Phe Glu Asp Trp Asp Gly Ser Pro Phe
340 345 350

Asp Leu Ile Pro Gln Gly Gly Gly Asp Tyr Tyr Thr Asn His Arg Cys
355 360 365

Ala Gly Glu Trp Met Thr Val Ile Val Met Glu Glu Ser Met Lys Tyr
370 375 380

Phe Ala Gln Ala Ile Lys Tyr Glu Val Pro Ala Gln Asp Leu Thr Val
385 390 395 400

Asp Leu Asn Lys Leu Pro Gly Arg Val Lys Ser Gly Phe Ile Ile Lys
405 410 415

Asn Val Glu Arg Asp Ile Asn Arg Phe
420 425

<210> 83
<211> 425
<212> PRT
<213> Auricoccus indicus

<400> 83

Met Gly Lys Ser Ile Pro Arg Asp Arg Gly Met Asp Asn Thr Val Lys
1 5 10 15

Ala Leu Lys Lys Gly Tyr Thr Tyr Ile Pro Glu Leu Arg Glu Arg Leu
20 25 30

Asn Ser Asn Asp Ile Val Ala Thr Arg Ala Leu Gly Gly Lys Lys Thr
35 40 45

Val Ile Ile Ser Gly Lys Asp Ala Ala Glu Leu Phe Tyr Asp Asn Asp
50 55 60

Lys Ile Glu Arg Gln Gly Thr Leu Pro Pro Arg Val Val Lys Thr Leu
65 70 75 80

Phe Gly Lys Gly Ala Ile His Thr Thr Ser Gly Lys Thr His Ile Asp

85 90 95

Arg Lys Ala Leu Phe Met Ser Leu Met Thr Glu Glu Asn Leu Ser Tyr
100 105 110

Leu Arg Glu Leu Thr Arg Leu Glu Trp Tyr Tyr Asn Thr Thr Arg Met
115 120 125

Ser His Met Asp Glu Val Asn Val Tyr Lys Glu Ala Ile Tyr Val Leu
130 135 140

Thr Lys Val Gly Ile Lys Trp Ala Gly Leu His Ala Ser Glu Asp Glu
145 150 155 160

Ile Asn Gln Tyr Ala Glu Asp Met Asp Thr Met Ile Asp Ser Phe Lys
165 170 175

Ala Ile Gly Thr Met Phe Lys Gly Tyr Lys Glu Ala Arg Lys Ala Arg
180 185 190

Asp Arg Val Glu Thr Trp Leu Glu Ala Gln Ile Gln Gly Val Arg Asp
195 200 205

Gly Glu Ile Thr Pro Pro Glu Gly Thr Ala Leu Tyr Glu Phe Ala His
210 215 220

Trp Lys Asp Tyr Glu Gly Asn Pro Met Asp Ala Arg Leu Ala Ala Ile
225 230 235 240

Asp Leu Met Asn Val Ile Arg Pro Leu Val Ala Ile Asn Arg Phe Val
245 250 255

Ala Phe Gly Thr Leu Ala Met Tyr Glu Phe Pro Lys Glu Arg Thr Lys
260 265 270

Val Ala Gln Asn Glu Glu Asp Tyr Val Tyr Lys Phe Ile Gln Glu Val
275 280 285

Arg Arg Phe Tyr Pro Phe Val Pro Tyr Leu Pro Gly Lys Ala Gly Lys
290 295 300

Asp Ile Glu Phe Arg Gly Tyr Glu Ile Lys Lys Asp Thr Phe Met Val
305 310 315 320

Leu Asp Val Tyr Gly Thr Leu His Arg Glu Asp Leu Trp Glu Asn Ala
325 330 335

Asp Gln Phe Ile Pro Glu Arg Phe Glu Asp Trp Asp Gly Ser Pro Phe
340 345 350

Asp Leu Ile Pro Gln Gly Gly Gly Asp Tyr Tyr Thr Asn His Arg Cys
355 360 365

Ala Gly Glu Trp Met Thr Val Ile Val Met Glu Glu Ser Met Lys Tyr
370 375 380

Phe Ala Gln Ala Ile Lys Tyr Glu Val Pro Ala Gln Asp Leu Thr Val
385 390 395 400

Asp Leu Asn Lys Leu Pro Gly Arg Val Lys Ser Gly Phe Ile Ile Lys
405 410 415

Asn Val Glu Arg Asp Ile Asn Arg Phe
420 425

<210> 84
<211> 423
<212> PRT
<213> Aliicoccus persicus

<400> 84

Met Ser Thr Ile Lys Lys Asp Lys Gly Leu Asp Asn Thr Leu Lys Ala
1 5 10 15

Ile Lys Gln Gly Tyr Lys Tyr Thr Glu Asn Gln Arg Lys Arg Leu Asp
20 25 30

Ser Asn Asn Ile Phe Glu Thr Arg Gly Leu Gly Gly Lys Arg Ile Ile
35 40 45

Phe Ile Ser Gly Lys Glu Ala Ala Glu Thr Phe Tyr Asp Asn Asp Lys
50 55 60

Ile Glu Arg Glu Gly Met Leu Pro Lys Arg Val Val Ser Thr Leu Phe
65 70 75 80

Gly Lys Gly Ala Ile His Thr Thr Thr Gly Lys Val His Ile Asp Arg
85 90 95

Lys Ala Leu Phe Met Ser Leu Met Thr Glu Gly Asn Leu Gln Tyr Leu
100 105 110

Arg Glu Leu Thr Arg Asn Gln Trp Arg Ser Asn Thr Ile Asn Met Glu
115 120 125

```
Arg Gln Asp Thr Val Asn Val Tyr Arg Glu Ala Ile Val Gln Leu Thr
    130                 135                 140

His Ile Gly Thr Arg Trp Ala Gly Val Gln Ala Pro Ala Glu Glu Ile
145                 150                 155                 160

Asp Arg Ile Ala Thr Asp Met Asp Leu Met Ile Asp Ser Phe Lys Gly
                165                 170                 175

Leu Gly Leu Ser Phe Lys Gly Phe Arg Glu Ser Lys Ala Ala Arg Arg
            180                 185                 190

Arg Val Glu Asp Trp Leu Glu Asp Gln Ile His Lys Thr Arg Lys Gly
        195                 200                 205

Val Ile His Pro Pro Glu Gly Thr Ala Leu Tyr Glu Phe Ala His Trp
    210                 215                 220

Glu Asp Phe Glu Gly Asn Pro Met Asp Ser Arg Leu Cys Ala Ile Asp
225                 230                 235                 240

Leu Met Asn Thr Phe Arg Pro Leu Ile Ala Ile Asn Arg Phe Val Thr
                245                 250                 255

Phe Gly Val Leu Ala Leu His Gln Tyr Pro Glu Thr Arg Glu Lys Ile
            260                 265                 270

Lys Asn Asp Pro Asp Tyr Ala Tyr Met Phe Ser Gln Glu Val Arg Arg
        275                 280                 285

Tyr Tyr Pro Phe Val Pro Phe Leu Pro Ala Lys Ala Lys Val Asp Phe
    290                 295                 300

Glu Phe Glu Gly His Lys Ile Asp Lys Asp Val Met Ile Leu Leu Asp
305                 310                 315                 320

Val Tyr Gly Thr Met His Arg Asp Asp Val Phe Asp Asn Pro Asn Glu
                325                 330                 335

Phe Tyr Pro Glu Arg Phe Lys Asp Trp Asp Gly Ser Pro Phe Asp Leu
            340                 345                 350

Ile Pro Gln Gly Gly Gly Asp Tyr His Thr Asn His Arg Cys Ala Gly
        355                 360                 365

Glu Trp Met Thr Ile Ile Ile Met Glu Glu Thr Met Lys Tyr Phe Ala
    370                 375                 380
```

Glu Arg Ile Thr Tyr Asp Val Pro Glu Gln Asp Leu Thr Val Asp Leu
385                 390                 395                 400

Glu Lys Leu Pro Gly Ser Val Ala Ser Gly Met Asn Ile Thr Asn Val
                405                 410                 415

Arg Glu Asn Val Asn Arg Lys
            420

<210>  85
<211>  423
<212>  PRT
<213>  Staphylococcus lutrae

<400>  85

Met Asn Lys Lys Leu Pro Lys Asp Thr Gly Leu Asp Asn Thr Leu Lys
1               5                   10                  15

Ile Ile Asn Glu Ala Tyr Thr Tyr Val Pro Lys Arg Leu Glu Lys Phe
            20                  25                  30

Gly Thr Lys Ala Phe Glu Thr Arg Ala Leu Gly Met Lys Pro Ile Thr
        35                  40                  45

Val Met Ser Gly Lys Ala Ala Ala Glu Leu Phe Tyr Asp Asn Asp Lys
    50                  55                  60

Val Ser Arg Thr Gly Thr Leu Pro Lys Arg Val Val His Thr Leu Phe
65                  70                  75                  80

Gly Lys Gly Ala Ile His Thr Thr Glu Gly Lys Val His Val Asn Arg
                85                  90                  95

Lys Ala Leu Phe Met Ser Leu Met Thr Glu Lys Asn Leu Lys Tyr Leu
            100                 105                 110

Arg Glu Leu Thr Arg His Tyr Trp Phe Met His Thr Glu Arg Met Gln
        115                 120                 125

Asn Met Asp Glu Val Ser Val Tyr Lys Glu Ala Gly Ile Ile Leu Thr
    130                 135                 140

Lys Val Gly Phe Arg Trp Ala Gly Leu Lys Gln Thr Asp Glu Glu Ala
145                 150                 155                 160

Val Lys Asn Ala Glu Asp Met Asn Lys Met Ile Asp Ser Phe Ser Arg
                165                 170                 175

Leu Gly Gln Ser Val Lys Gly Tyr Arg Gln Ala Lys Lys Ala Arg Ala
180 185 190

Arg Val Glu Gln Phe Leu Arg Asp Gln Ile Lys Ala Val Arg Glu Asn
195 200 205

Lys Gln Tyr Ala Glu Pro Gly Thr Ala Leu Tyr Glu Phe Ala His Trp
210 215 220

Lys Asp Leu Lys Gly Gln Pro Met Asp Leu His Leu Cys Ala Val Asp
225 230 235 240

Leu Met Asn Ile Val Arg Pro Leu Val Ala Val Asn Arg Phe Ile Ser
245 250 255

Tyr Ala Val Lys Ala Leu Ile Glu Phe Asp Gln Glu Arg Gln Lys Leu
260 265 270

Gln Val Thr His Asp Pro Asn Tyr Ala Tyr Lys Phe Ala Gln Glu Val
275 280 285

Arg Arg Ile Phe Pro Phe Val Pro Phe Leu Pro Gly Lys Leu Lys Lys
290 295 300

Thr Ile Asp Phe Asp Gly Phe Lys Ile Lys Lys Gly Thr Met Val Ala
305 310 315 320

Leu Asp Ile Phe Gly Thr Thr His Asp Pro Glu Leu Phe Lys Asn Pro
325 330 335

Tyr Gln Phe Asn Pro Asn Arg Phe Glu Lys Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Ile Ile Val Met Glu Glu Thr Val Gln Tyr
370 375 380

Phe Ala Asn Lys Ile Asp Phe Glu Val Pro Ala Gln Asp Leu Ser Val
385 390 395 400

Lys Leu Asp Arg Phe Pro Gly Lys Val Thr Ser Gly Thr Leu Ile Lys
405 410 415

Asn Val Arg Pro Arg Arg Ser
420

<210> 86
<211> 425
<212> PRT
<213> Staphylococcus aureus

<400> 86

```
Met Ala Lys Gln Leu Pro Lys Asp Pro Gly Leu Asp Asn Thr Phe Lys
1               5                   10                  15

Val Leu Lys Glu Ala Tyr Thr Tyr Val Pro Cys Arg Leu Glu Lys Phe
            20                  25                  30

Asn Ser Lys Ala Phe Gln Thr Thr Gly Met Gly Met Lys Pro Ile Ala
        35                  40                  45

Val Ile Ser Gly Lys Glu Ala Ala Glu Leu Phe Tyr Asn Asn Asp Val
    50                  55                  60

Met Gln Arg Glu Lys Thr Leu Pro Lys Arg Val Val Asn Thr Leu Phe
65                  70                  75                  80

Gly Lys Gly Ala Ile His Thr Thr Lys Gly Lys Val His Val Asp Arg
                85                  90                  95

Lys Ala Leu Phe Met Ser Leu Met Thr Glu Glu Asn Leu Lys Tyr Leu
            100                 105                 110

Arg Glu Leu Thr Arg Asn His Trp Phe Met His Thr Glu His Met Gln
        115                 120                 125

Asn Gln Lys Glu Val Asn Ile Tyr Lys Glu Ser Ile Tyr Val Leu Thr
    130                 135                 140

Lys Ile Gly Phe Arg Trp Ala Gly Ile His Gln Thr Thr Glu Glu Ala
145                 150                 155                 160

Glu Gln Asn Ala Lys Asp Met Asp Ile Met Ile Asp Ser Phe Lys Gly
                165                 170                 175

Leu Gly Gln Thr Ile Gly Gly Gly Tyr Arg Lys Ala Lys Lys Ala Arg
            180                 185                 190

Ala Arg Val Glu Gln Phe Leu Glu Lys Gln Ile Ile Ala Val Arg Lys
        195                 200                 205

Gly Lys Ile Ser Ala Glu Pro Gly Thr Ala Leu Tyr Glu Phe Ala His
    210                 215                 220
```

Trp Glu Asp Tyr Lys Gly Asn Pro Met Asp Ala Arg Leu Cys Ala Ile
225 230 235 240

Asp Leu Met Asn Val Val Arg Pro Leu Ala Ala Val Asn Arg Phe Val
245 250 255

Ser Tyr Ala Val Lys Ala Met Ile Glu Tyr Asp Gln Glu Arg Ile Lys
260 265 270

Leu Gln Val Ser Asp Asp Pro Asn Tyr Ala Tyr Lys Phe Ala Gln Glu
275 280 285

Val Arg Arg Ile Phe Pro Phe Val Pro Phe Leu Pro Gly Lys Leu Lys
290 295 300

Lys Asn Ile Glu Phe Asp Gly Tyr Arg Ile Lys Lys Gly Thr Phe Thr
305 310 315 320

Leu Leu Asp Val Phe Gly Thr Thr His Asp Pro Lys Leu Phe Glu Asn
325 330 335

Pro Tyr Gln Phe Asn Pro Asp Arg Phe Glu Asn Trp Asp Gly Ser Pro
340 345 350

Phe Asp Leu Ile Pro Gln Gly Gly Gly Asp Phe Tyr Thr Asn His Arg
355 360 365

Cys Ala Gly Glu Trp Met Thr Ile Ile Val Met Glu Glu Thr Ile Lys
370 375 380

Tyr Phe Ala Asn Lys Ile Asp Phe Asp Ala Pro Ser Gln Asp Leu Ser
385 390 395 400

Val Lys Leu Asp Gln Phe Pro Gly Lys Val Thr Ser Gly Thr Ile Ile
405 410 415

Gln Asn Val Arg Pro Arg Val Gln Arg
420 425

<210> 87
<211> 425
<212> PRT
<213> Staphylococcus agnetis

<400> 87

Met Ala Lys Gln Leu Pro Lys Asp Pro Gly Leu Asp Asn Thr Phe Lys
1 5 10 15

```
Val Leu Lys Glu Ala Tyr Thr Tyr Val Pro Cys Arg Leu Glu Lys Phe
            20                  25                  30

Asn Ser Lys Ala Phe Gln Thr Thr Gly Met Gly Met Lys Pro Ile Ala
        35                  40                  45

Val Ile Ser Gly Lys Asp Ala Ala Glu Leu Phe Tyr Asn Asn Asp Val
    50                  55                  60

Met Gln Arg Glu Lys Thr Leu Pro Lys Arg Val Val Asn Thr Leu Phe
65                  70                  75                  80

Gly Lys Gly Ala Ile His Thr Thr Lys Gly Lys Val His Val Asp Arg
                85                  90                  95

Lys Ala Leu Phe Met Ser Leu Met Thr Glu Glu Asn Leu Lys Tyr Leu
            100                 105                 110

Arg Glu Leu Thr Arg Asn His Trp Phe Met His Thr Glu His Met Gln
        115                 120                 125

Asn Gln Lys Glu Val Asn Ile Tyr Lys Glu Ser Ile Tyr Val Leu Thr
    130                 135                 140

Lys Ile Gly Phe Arg Trp Ala Gly Ile His Gln Thr Ala Glu Glu Ala
145                 150                 155                 160

Glu Gln Asn Ala Lys Asp Met Asp Ile Met Ile Asp Ser Phe Lys Gly
                165                 170                 175

Leu Gly Gln Thr Ile Gly Gly Gly Tyr Arg Lys Ala Lys Lys Ala Arg
            180                 185                 190

Ala Arg Val Glu Gln Phe Leu Glu Lys Gln Ile Ile Ala Val Arg Lys
        195                 200                 205

Gly Lys Ile Asn Ala Glu Pro Gly Thr Ala Leu Tyr Glu Phe Ala His
    210                 215                 220

Trp Glu Asp Tyr Lys Gly Asn Pro Met Asp Ala Arg Leu Cys Ala Ile
225                 230                 235                 240

Asp Leu Met Asn Val Val Arg Pro Leu Ala Ala Val Asn Arg Phe Val
                245                 250                 255

Ser Tyr Ala Val Lys Ala Met Ile Glu Tyr Asp Gln Glu Arg Ile Lys
```

260 265 270

Leu Gln Val Ser Asp Asp Pro Asn Tyr Ala Tyr Lys Phe Ala Gln Glu
275 280 285

Val Arg Arg Ile Phe Pro Phe Val Pro Phe Leu Pro Gly Lys Leu Lys
290 295 300

Lys Asn Ile Glu Phe Asp Gly Tyr Arg Ile Lys Lys Gly Thr Phe Thr
305 310 315 320

Leu Leu Asp Val Phe Gly Thr Thr His Asp Pro Glu Leu Phe Glu Asn
325 330 335

Pro Tyr Gln Phe Asn Pro Asp Arg Phe Glu Asn Trp Asp Gly Ser Pro
340 345 350

Phe Asp Leu Ile Pro Gln Gly Gly Gly Asp Phe Tyr Thr Asn His Arg
355 360 365

Cys Ala Gly Glu Trp Met Thr Ile Ile Val Met Glu Glu Thr Ile Lys
370 375 380

Tyr Phe Ala Asn Lys Ile Asp Phe Glu Ala Pro Ser Gln Asp Leu Ser
385 390 395 400

Val Lys Leu Asp Gln Phe Pro Gly Lys Val Thr Ser Gly Thr Ile Ile
405 410 415

Gln Asn Val Arg Pro Arg Val Gln Arg
420 425

<210> 88
<211> 425
<212> PRT
<213> Staphylococcus agnetis

<400> 88

Met Ala Lys Gln Leu Pro Lys Asp Pro Gly Leu Asp Asn Thr Phe Lys
1 5 10 15

Val Leu Lys Glu Ala Tyr Thr Tyr Val Pro Cys Arg Leu Glu Lys Phe
20 25 30

Asn Ser Lys Ala Phe Gln Thr Thr Gly Met Gly Met Lys Pro Ile Ala
35 40 45

Val Ile Ser Gly Lys Glu Ala Ala Glu Leu Phe Tyr Asn Asn Asp Val

50 55 60

Met Gln Arg Glu Lys Thr Leu Pro Lys Arg Val Val Asn Thr Leu Phe
65 70 75 80

Gly Lys Gly Ala Ile His Thr Thr Lys Gly Lys Val His Val Asp Arg
85 90 95

Lys Ala Leu Phe Met Ser Leu Met Thr Glu Glu Asn Leu Lys Tyr Leu
100 105 110

Arg Glu Leu Thr Arg Asn His Trp Phe Met His Thr Glu His Met Gln
115 120 125

Asn Gln Lys Glu Val Asn Ile Tyr Lys Glu Ser Ile Tyr Val Leu Thr
130 135 140

Lys Ile Gly Phe Arg Trp Ala Gly Ile His Gln Thr Ala Glu Glu Ala
145 150 155 160

Glu Gln Asn Ala Lys Asp Met Asp Ile Met Ile Asp Ser Phe Lys Gly
165 170 175

Leu Gly Gln Thr Ile Gly Gly Gly Tyr Arg Lys Ala Lys Lys Ala Arg
180 185 190

Ala Arg Val Glu Gln Phe Leu Glu Lys Gln Ile Ile Ala Val Arg Lys
195 200 205

Gly Lys Ile Asn Ala Glu Pro Gly Thr Ala Leu Tyr Glu Phe Ala His
210 215 220

Trp Glu Asp Tyr Lys Gly Asn Pro Met Asp Ala Arg Leu Cys Ala Ile
225 230 235 240

Asp Leu Met Asn Val Val Arg Pro Leu Ala Ala Val Asn Arg Phe Val
245 250 255

Ser Tyr Ala Val Lys Ala Met Ile Glu Tyr Asp Gln Glu Arg Ile Lys
260 265 270

Leu Gln Val Ser Asp Asp Pro Asn Tyr Ala Tyr Lys Phe Ala Gln Glu
275 280 285

Val Arg Arg Ile Phe Pro Phe Val Pro Phe Leu Pro Gly Lys Leu Lys
290 295 300

Lys Asn Ile Glu Phe Asp Gly Tyr Arg Ile Lys Lys Gly Thr Phe Thr
305 310 315 320

Leu Leu Asp Val Phe Gly Thr Thr His Asp Pro Glu Leu Phe Glu Asn
325 330 335

Pro Tyr Gln Phe Asn Pro Asp Arg Phe Glu Asn Trp Asp Gly Ser Pro
340 345 350

Phe Asp Leu Ile Pro Gln Gly Gly Gly Asp Phe Tyr Thr Asn His Arg
355 360 365

Cys Ala Gly Glu Trp Met Thr Ile Ile Val Met Glu Glu Thr Ile Lys
370 375 380

Tyr Phe Ala Asn Lys Ile Asp Phe Glu Ala Pro Ser Gln Asp Leu Ser
385 390 395 400

Val Lys Leu Asp Gln Phe Pro Gly Lys Val Thr Ser Gly Thr Ile Ile
405 410 415

Gln Asn Val Arg Pro Arg Val Gln Arg
420 425

<210> 89
<211> 425
<212> PRT
<213> Staphylococcus agnetis

<400> 89

Met Ala Lys Gln Leu Pro Lys Asp Pro Gly Leu Asp Asn Thr Phe Lys
1 5 10 15

Val Leu Lys Glu Ala Tyr Thr Tyr Val Pro Cys Arg Leu Glu Lys Phe
20 25 30

Asn Ser Lys Ala Phe Gln Thr Thr Gly Met Gly Met Lys Pro Ile Ala
35 40 45

Val Ile Ser Gly Lys Glu Ala Ala Glu Leu Phe Tyr Asn Asn Asp Val
50 55 60

Met Gln Arg Glu Lys Thr Leu Pro Lys Arg Val Val Asn Thr Leu Phe
65 70 75 80

Gly Lys Gly Ala Ile His Thr Thr Lys Gly Lys Val His Val Asp Arg
85 90 95

Lys Ala Leu Phe Met Ser Leu Met Thr Glu Glu Asn Leu Lys Tyr Leu
100 105 110

Arg Glu Leu Thr Arg Asn His Trp Phe Met His Thr Glu His Met Gln
115 120 125

Asn Gln Arg Glu Val Asn Ile Tyr Lys Glu Ser Ile Tyr Val Leu Thr
130 135 140

Lys Ile Gly Phe Arg Trp Ala Gly Ile His Gln Thr Ala Glu Glu Ala
145 150 155 160

Glu Gln Asn Ala Lys Asp Met Asp Ile Met Ile Asp Ser Phe Lys Gly
165 170 175

Leu Gly Gln Thr Ile Gly Gly Gly Tyr Arg Lys Ala Lys Lys Ala Arg
180 185 190

Ala Arg Val Glu Gln Phe Leu Glu Lys Gln Ile Ile Ala Val Arg Lys
195 200 205

Gly Lys Ile Asn Ala Glu Pro Gly Thr Ala Leu Tyr Glu Phe Ala His
210 215 220

Trp Glu Asp Tyr Lys Gly Asn Pro Met Asp Ala Arg Leu Cys Ala Ile
225 230 235 240

Asp Leu Met Asn Val Val Arg Pro Leu Ala Ala Val Asn Arg Phe Val
245 250 255

Ser Tyr Ala Val Lys Ala Met Ile Glu Tyr Asp Gln Glu Arg Ile Lys
260 265 270

Leu Gln Val Ser Asp Asp Pro Asn Tyr Ala Tyr Lys Phe Ala Gln Glu
275 280 285

Val Arg Arg Ile Phe Pro Phe Val Pro Phe Leu Pro Gly Lys Leu Lys
290 295 300

Lys Asn Ile Glu Phe Asp Gly Tyr Arg Ile Lys Lys Gly Thr Phe Thr
305 310 315 320

Leu Leu Asp Val Phe Gly Thr Thr His Asp Pro Glu Leu Phe Glu Asn
325 330 335

Pro Tyr Gln Phe Asn Pro Asp Arg Phe Glu Asn Trp Asp Gly Ser Pro
340 345 350

Phe Asp Leu Ile Pro Gln Gly Gly Gly Asp Phe Tyr Thr Asn His Arg
355 360 365

Cys Ala Gly Glu Trp Met Thr Ile Ile Val Met Glu Glu Thr Ile Lys
370 375 380

Tyr Phe Ala Asn Lys Ile Asp Phe Glu Ala Pro Ser Gln Asp Leu Ser
385 390 395 400

Val Lys Leu Asp Gln Phe Pro Gly Lys Val Thr Ser Gly Thr Ile Ile
405 410 415

Gln Asn Val Arg Pro Arg Val Gln Arg
420 425

<210> 90
<211> 425
<212> PRT
<213> Staphylococcus agnetis

<400> 90

Met Ala Lys Gln Leu Pro Lys Asp Pro Gly Leu Asp Asn Thr Phe Lys
1 5 10 15

Val Leu Lys Glu Ala Tyr Thr Tyr Val Pro Cys Arg Leu Glu Lys Phe
20 25 30

Asn Ser Lys Ala Phe Gln Thr Thr Gly Met Gly Met Lys Pro Ile Ala
35 40 45

Val Ile Ser Gly Lys Glu Ala Ala Glu Leu Phe Tyr Asn Asn Asp Val
50 55 60

Met Gln Arg Glu Lys Thr Leu Pro Lys Arg Val Val Asn Thr Leu Phe
65 70 75 80

Gly Lys Gly Ala Ile His Thr Thr Lys Gly Lys Val His Val Asp Arg
85 90 95

Lys Ala Leu Phe Met Ser Leu Met Thr Glu Glu Asn Leu Lys Tyr Leu
100 105 110

Arg Glu Leu Thr Arg Asn His Trp Phe Met His Thr Glu His Met Gln
115 120 125

Asn Gln Lys Glu Val Asn Ile Tyr Lys Glu Ser Ile Tyr Val Leu Thr
130 135 140

Lys Ile Gly Phe Arg Trp Ala Gly Ile His Gln Thr Thr Glu Glu Ala
145 150 155 160

Glu Gln Asn Ala Lys Asp Met Asp Ile Met Ile Asp Ser Phe Lys Gly
165 170 175

Leu Gly Gln Thr Ile Gly Gly Gly Tyr Arg Lys Ala Lys Lys Ala Arg
180 185 190

Ala Arg Val Glu Gln Phe Leu Glu Lys Gln Ile Ile Ala Val Arg Lys
195 200 205

Gly Lys Ile Asn Ala Glu Pro Gly Thr Ala Leu Tyr Glu Phe Ala His
210 215 220

Trp Glu Asp Tyr Lys Gly Asn Pro Met Asp Ala Arg Leu Cys Ala Ile
225 230 235 240

Asp Leu Met Asn Val Val Arg Pro Leu Ala Ala Val Asn Arg Phe Val
245 250 255

Ser Tyr Ala Val Lys Ala Met Ile Glu Tyr Asp Gln Glu Arg Ile Lys
260 265 270

Leu Gln Val Ser Asp Asp Pro Asn Tyr Ala Tyr Lys Phe Ala Gln Glu
275 280 285

Val Arg Arg Ile Phe Pro Phe Val Pro Phe Leu Pro Gly Lys Leu Lys
290 295 300

Lys Asn Ile Glu Phe Asp Gly Tyr Arg Ile Lys Lys Gly Thr Phe Thr
305 310 315 320

Leu Leu Asp Val Phe Gly Thr Thr His Asp Pro Glu Leu Phe Glu Asn
325 330 335

Pro Tyr Gln Phe Asn Pro Asp Arg Phe Glu Asn Trp Asp Gly Ser Pro
340 345 350

Phe Asp Leu Ile Pro Gln Gly Gly Gly Asp Phe Tyr Thr Asn His Arg
355 360 365

Cys Ala Gly Glu Trp Met Thr Ile Ile Val Met Glu Glu Thr Ile Lys
370 375 380

Tyr Phe Ala Asn Lys Ile Asp Phe Glu Ala Pro Ser Gln Asp Leu Ser
385 390 395 400

Val Lys Leu Asp Gln Phe Pro Gly Lys Val Thr Ser Gly Thr Ile Ile
405 410 415

Gln Asn Val Arg Pro Arg Val Gln Arg
420 425

<210> 91
<211> 425
<212> PRT
<213> Staphylococcus agnetis

<400> 91

Met Ala Lys Gln Leu Pro Lys Asp Pro Gly Leu Asp Asn Thr Phe Lys
1 5 10 15

Val Leu Lys Glu Ala Tyr Thr Tyr Val Pro Cys Arg Leu Glu Lys Phe
20 25 30

Asn Ser Lys Ala Phe Gln Thr Thr Gly Met Gly Met Lys Pro Ile Ala
35 40 45

Val Ile Ser Gly Lys Glu Ala Ala Glu Leu Phe Tyr Asn Asn Asp Val
50 55 60

Met Gln Arg Glu Lys Thr Leu Pro Lys Arg Val Val Asn Thr Leu Phe
65 70 75 80

Gly Lys Gly Ala Ile His Thr Thr Lys Gly Lys Val His Val Asp Arg
85 90 95

Lys Ala Leu Phe Met Ser Leu Met Thr Glu Glu Asn Leu Lys Tyr Leu
100 105 110

Arg Glu Leu Thr Arg Asn His Trp Phe Met His Thr Glu His Met Gln
115 120 125

Asn Gln Lys Glu Val Asn Ile Tyr Lys Glu Ser Val Tyr Val Leu Thr
130 135 140

Lys Ile Gly Phe Arg Trp Ala Gly Ile His Gln Thr Ala Glu Glu Ala
145 150 155 160

Glu Gln Asn Ala Lys Asp Met Asp Ile Met Ile Asp Ser Phe Lys Gly
165 170 175

Leu Gly Gln Thr Ile Gly Gly Gly Tyr Arg Lys Ala Lys Lys Ala Arg
180 185 190

Ala Arg Val Glu Gln Phe Leu Glu Asn Gln Ile Ile Ala Val Arg Lys
195 200 205

Gly Lys Ile Asn Ala Glu Pro Gly Thr Ala Leu Tyr Glu Phe Ala His
210 215 220

Trp Glu Asp Tyr Lys Gly Asn Pro Met Asp Ala Arg Leu Cys Ala Ile
225 230 235 240

Asp Leu Met Asn Val Val Arg Pro Leu Ala Ala Val Asn Arg Phe Val
245 250 255

Ser Tyr Ala Val Lys Ala Met Ile Glu Tyr Asp Gln Glu Arg Ile Lys
260 265 270

Leu Gln Val Ser Asp Asp Pro Asn Tyr Ala Tyr Lys Phe Ala Gln Glu
275 280 285

Val Arg Arg Ile Phe Pro Phe Val Pro Phe Leu Pro Gly Lys Leu Lys
290 295 300

Lys Asn Ile Glu Phe Asp Gly Tyr Arg Ile Lys Lys Gly Thr Phe Thr
305 310 315 320

Leu Leu Asp Val Phe Gly Thr Thr His Asp Pro Glu Leu Phe Glu Asn
325 330 335

Pro Tyr Gln Phe Asn Pro Asp Arg Phe Glu Asn Trp Asp Gly Ser Pro
340 345 350

Phe Asp Leu Ile Pro Gln Gly Gly Gly Asp Phe Tyr Thr Asn His Arg
355 360 365

Cys Ala Gly Glu Trp Met Thr Ile Ile Val Met Glu Glu Thr Ile Lys
370 375 380

Tyr Phe Ala Asn Lys Ile Asp Phe Glu Ala Pro Ser Gln Asp Leu Ser
385 390 395 400

Val Lys Leu Asp Gln Phe Pro Gly Lys Val Thr Ser Gly Thr Ile Ile
405 410 415

Gln Asn Val Arg Pro Arg Val Gln Arg
420 425

<210> 92

<211> 423
<212> PRT
<213> Salinicoccus sediminis

<400> 92

```
Met Gly Thr Ile Lys Arg Asp Lys Gly Leu Asp Asn Thr Leu Lys Val
1               5                   10                  15

Met Lys Gln Gly Tyr Leu Tyr Ile Thr Asn Gln Arg Lys Arg Leu Gly
            20                  25                  30

Ser Glu Asn Ile Phe Glu Thr Arg Ala Leu Gly Gly Lys Lys Ile Ile
        35                  40                  45

Val Ile Ser Gly Lys Asp Ala Ala Glu Leu Phe Tyr Asp Asn Asp Lys
    50                  55                  60

Ile Glu Arg Ser Gly Thr Leu Pro Lys Arg Val Val Asn Thr Leu Phe
65                  70                  75                  80

Gly Lys Gly Ala Ile His Thr Thr Thr Gly Lys Lys His Ile Asp Arg
                85                  90                  95

Lys Ala Leu Phe Met Ser Leu Met Thr Glu Gly Asn Leu Lys His Leu
            100                 105                 110

Arg Glu Leu Thr Arg Asn His Trp Tyr Met Asn Thr Gln Lys Met Glu
        115                 120                 125

Gln Met Asp Gln Ile Asn Ile Tyr Arg Glu Ser Ile Val Gln Leu Thr
    130                 135                 140

Lys Ile Gly Thr Lys Trp Ala Gly Val Gln Ala Pro Glu Glu Lys Ile
145                 150                 155                 160

Glu Glu Ile Ala Thr Asp Met Asp Val Met Ile Asp Ser Phe Lys Gly
                165                 170                 175

Leu Gly Gly Lys Phe Lys Gly Tyr Lys Ala Ser Val Asp Ala Arg Lys
            180                 185                 190

Arg Val Glu Asp Trp Leu Glu Asp Gln Ile Ile Gln Thr Arg Lys Gly
        195                 200                 205

Lys Ile Phe Pro Pro Glu Gly Thr Ala Leu Tyr Glu Phe Ala His Trp
    210                 215                 220

Glu Asp Tyr Lys Gly Asn Pro Met Asp Ser Arg Leu Cys Ala Ile Asp
```

225 230 235 240

Leu Met Asn Thr Phe Arg Pro Leu Ile Ala Ile Asn Arg Phe Val Ser
245 250 255

Phe Gly Leu Leu Ala Met His Glu His Pro Ile Thr Arg Glu Lys Ile
260 265 270

Lys Ser Asp Asp Asp Tyr Ala Tyr Met Phe Ser Gln Glu Val Arg Arg
275 280 285

Phe Tyr Pro Phe Val Pro Phe Leu Pro Ala Lys Ala Lys Thr Asp Ile
290 295 300

Gln Phe Glu Gly His Pro Ile Glu Lys Asp Thr Met Ile Ala Leu Asp
305 310 315 320

Ile Tyr Gly Thr Met His Arg Glu Asp Val Phe Glu Asn Pro Asp Asp
325 330 335

Phe Tyr Pro Glu Arg Phe Lys Asp Trp Asp Gly Ser Pro Phe Asp Leu
340 345 350

Ile Pro Gln Gly Gly Gly Asp Tyr Tyr Thr Asn His Arg Cys Ala Gly
355 360 365

Glu Trp Met Thr Ile Ile Ile Met Glu Glu Thr Met Lys Tyr Phe Ala
370 375 380

Glu Arg Val Thr Tyr Asp Val Pro Glu Gln Asp Leu Thr Val Asp Leu
385 390 395 400

Asn Ser Ile Pro Gly Tyr Val Lys Ser Gly Phe Ile Ile Glu Asn Leu
405 410 415

Arg Glu Val Val Asp Arg Lys
420

<210> 93
<211> 423
<212> PRT
<213> Salinicoccus carnicancri

<400> 93

Met Gly Thr Ile Ser Arg Asp Lys Gly Leu Asp Asn Thr Leu Lys Val
1 5 10 15

Met Lys Gln Gly Tyr Leu Tyr Met Thr Asn Gln Arg Lys Arg Leu Gly

20 25 30

Ser Glu Asn Ile Phe Glu Thr Arg Ala Leu Gly Gly Lys Lys Ile Ile
35 40 45

Val Ile Ser Gly Lys Asp Ala Ala Glu Leu Phe Tyr Asp Asn Asp Lys
50 55 60

Ile Glu Arg Ser Gly Thr Leu Pro Lys Arg Val Val Asn Thr Leu Phe
65 70 75 80

Gly Lys Gly Ala Ile His Thr Thr Thr Gly Lys Lys His Ile Asp Arg
85 90 95

Lys Ala Leu Phe Met Ser Leu Met Thr Glu Gly Asn Leu Lys His Leu
100 105 110

Arg Glu Leu Thr Arg Asn His Trp Tyr Met Asn Thr Gln Lys Met Glu
115 120 125

Gln Met Glu Gln Ile Asn Ile Tyr Arg Glu Ser Ile Val Gln Leu Thr
130 135 140

Lys Ile Gly Thr Lys Trp Ala Gly Val Gln Ala Pro Glu Glu Lys Ile
145 150 155 160

Glu Glu Ile Ala Thr Asp Met Asp Val Met Ile Asp Ser Phe Lys Gly
165 170 175

Leu Gly Gly Lys Phe Lys Gly Tyr Lys Ala Ser Val Asp Ala Arg Lys
180 185 190

Arg Val Glu Asp Trp Leu Glu Asp Gln Ile Ile Gln Thr Arg Lys Gly
195 200 205

Lys Ile Phe Pro Pro Glu Gly Thr Ala Leu Tyr Glu Phe Ala His Trp
210 215 220

Glu Asp Tyr Lys Gly Asn Pro Met Asp Ser Arg Leu Cys Ala Ile Asp
225 230 235 240

Leu Met Asn Thr Phe Arg Pro Leu Ile Ala Ile Asn Arg Phe Val Ser
245 250 255

Phe Gly Leu Leu Ala Met His Glu His Pro Ile Thr Arg Glu Lys Ile
260 265 270

Lys Ser Asp Asp Asp Tyr Ala Tyr Met Phe Ser Gln Glu Val Arg Arg
275 280 285

Phe Tyr Pro Phe Val Pro Phe Leu Pro Ala Lys Ala Lys Thr Asp Ile
290 295 300

Gln Phe Glu Gly His Pro Ile Glu Lys Asp Thr Met Ile Val Leu Asp
305 310 315 320

Ile Tyr Gly Thr Met His Arg Glu Asp Val Phe Glu Asn Pro Asp Asp
325 330 335

Phe Tyr Pro Glu Arg Phe Arg Asp Trp Asp Gly Ser Pro Phe Asp Leu
340 345 350

Ile Pro Gln Gly Gly Gly Asp Tyr Tyr Thr Asn His Arg Cys Ala Gly
355 360 365

Glu Trp Met Thr Ile Ile Ile Met Glu Glu Thr Met Lys Tyr Phe Ala
370 375 380

Gly Arg Val Thr Tyr Asp Val Pro Glu Gln Asp Leu Thr Val Asp Leu
385 390 395 400

Asn Ser Ile Pro Gly Tyr Val Lys Ser Gly Phe Ile Ile Glu Asn Leu
405 410 415

Arg Glu Val Val Asp Arg Lys
420

<210> 94
<211> 425
<212> PRT
<213> Staphylococcus agnetis

<400> 94

Met Ala Lys Gln Leu Pro Lys Asp Pro Gly Leu Asp Asn Thr Phe Lys
1 5 10 15

Val Leu Lys Glu Ala Tyr Thr Tyr Val Pro Cys Arg Leu Glu Lys Phe
20 25 30

Asn Ser Lys Ala Phe Gln Thr Thr Gly Met Gly Met Lys Pro Ile Ala
35 40 45

Val Ile Ser Gly Lys Glu Ala Ala Glu Leu Phe Tyr Asn Asn Asp Val
50 55 60

Met Gln Arg Glu Lys Thr Leu Pro Lys Arg Val Val Asn Thr Leu Phe
65 70 75 80

Gly Lys Gly Ala Ile His Thr Thr Lys Gly Lys Val His Val Asp Arg
85 90 95

Lys Ala Leu Phe Met Ser Leu Met Thr Glu Glu Asn Leu Lys Tyr Leu
100 105 110

Arg Glu Leu Thr Arg Asn His Trp Phe Met His Thr Glu His Met Gln
115 120 125

Asn Gln Lys Glu Val Asn Ile Tyr Lys Glu Ser Ile Tyr Val Leu Thr
130 135 140

Lys Ile Gly Phe Arg Trp Ala Gly Ile His Gln Thr Thr Glu Glu Ala
145 150 155 160

Glu Gln Asn Ala Lys Asp Met Asp Ile Met Ile Asp Ser Phe Lys Gly
165 170 175

Leu Gly Gln Thr Ile Gly Gly Gly Tyr Arg Lys Ala Lys Lys Ala Arg
180 185 190

Ala Arg Val Glu Gln Phe Leu Glu Arg Gln Ile Ile Ala Val Arg Lys
195 200 205

Gly Lys Ile Asn Ala Glu Pro Ser Thr Ala Leu Tyr Glu Phe Ala His
210 215 220

Trp Glu Asp Tyr Lys Gly Asn Pro Met Asp Ala Arg Leu Cys Ala Ile
225 230 235 240

Asp Leu Met Asn Val Val Arg Pro Leu Ala Ala Val Asn Arg Phe Val
245 250 255

Ser Tyr Ala Val Lys Ala Met Ile Glu Tyr Asp Gln Glu Arg Ile Lys
260 265 270

Leu Gln Val Ser Asp Asp Pro Asn Tyr Ala Tyr Lys Phe Ala Gln Glu
275 280 285

Val Arg Arg Ile Phe Pro Phe Val Pro Phe Leu Pro Gly Lys Leu Lys
290 295 300

Lys Asn Ile Glu Phe Asp Gly Tyr Arg Ile Lys Lys Gly Thr Phe Thr
305 310 315 320

Leu Leu Asp Val Phe Gly Thr Thr His Asp Pro Glu Leu Phe Glu Asn
                325                 330                 335

Pro Tyr Gln Phe Asn Pro Asp Arg Phe Glu Asn Trp Asp Gly Ser Pro
            340                 345                 350

Phe Asp Leu Ile Pro Gln Gly Gly Gly Asp Phe Tyr Thr Asn His Arg
        355                 360                 365

Cys Ala Gly Glu Trp Met Thr Ile Ile Val Met Glu Glu Thr Ile Lys
    370                 375                 380

Tyr Phe Ala Asn Lys Ile Asp Phe Glu Ala Pro Ser Gln Asp Leu Ser
385                 390                 395                 400

Val Lys Leu Asp Gln Phe Pro Gly Lys Val Thr Ser Gly Thr Ile Ile
                405                 410                 415

Lys Asn Val Arg Pro Arg Val Gln Arg
            420                 425

<210> 95
<211> 425
<212> PRT
<213> Staphylococcus agnetis

<400> 95

Met Ala Lys Gln Leu Pro Lys Asp Pro Gly Leu Asp Asn Thr Phe Lys
1               5                   10                  15

Val Leu Lys Glu Ala Tyr Thr Tyr Val Pro Cys Arg Leu Glu Lys Phe
            20                  25                  30

Asn Ser Lys Ala Phe Gln Thr Thr Gly Met Gly Met Lys Pro Ile Ala
        35                  40                  45

Val Ile Asn Gly Lys Glu Ala Ala Glu Leu Phe Tyr Asn Asn Asp Val
    50                  55                  60

Met Gln Arg Glu Lys Thr Leu Pro Lys Arg Val Val Asn Thr Leu Phe
65                  70                  75                  80

Gly Lys Gly Ala Ile His Thr Thr Lys Gly Lys Val His Val Asp Arg
                85                  90                  95

Lys Ala Leu Phe Met Ser Leu Met Thr Glu Glu Asn Leu Lys Tyr Leu
            100                 105                 110

Arg Glu Leu Thr Arg Asn His Trp Phe Met His Thr Glu His Met Gln
115 120 125

Asn Gln Lys Glu Val Asn Ile Tyr Lys Glu Ser Ile Tyr Val Leu Thr
130 135 140

Lys Ile Gly Phe Arg Trp Ala Gly Ile His Gln Thr Ala Glu Glu Ala
145 150 155 160

Glu Gln Asn Ala Gln Asp Met Asp Ile Met Ile Asp Ser Phe Lys Gly
165 170 175

Leu Gly Gln Thr Ile Gly Gly Gly Tyr Arg Lys Ala Lys Lys Ala Arg
180 185 190

Ala Arg Val Glu Gln Phe Leu Glu Lys Gln Ile Ile Ala Val Arg Lys
195 200 205

Gly Lys Ile Asn Ala Glu Pro Gly Thr Ala Leu Tyr Glu Phe Ala His
210 215 220

Trp Glu Asp Tyr Lys Gly Asn Pro Met Asp Ala Arg Leu Cys Ala Ile
225 230 235 240

Asp Leu Met Asn Val Val Arg Pro Leu Ala Ala Val Asn Arg Phe Val
245 250 255

Ser Tyr Ala Val Lys Ala Met Ile Glu Tyr Asp Gln Glu Arg Ile Lys
260 265 270

Leu Gln Val Ser Asp Asp Pro Asn Tyr Ala Tyr Lys Phe Ala Gln Glu
275 280 285

Val Arg Arg Ile Phe Pro Phe Val Pro Phe Leu Pro Gly Lys Leu Lys
290 295 300

Lys Asn Ile Glu Phe Asp Gly Tyr Arg Ile Lys Lys Gly Thr Phe Thr
305 310 315 320

Leu Leu Asp Val Phe Gly Thr Thr His Asp Pro Glu Leu Phe Glu Asn
325 330 335

Pro Tyr Gln Phe Asn Pro Asp Arg Phe Glu Asn Trp Asp Gly Ser Pro
340 345 350

Phe Asp Leu Ile Pro Gln Gly Gly Gly Asp Phe Tyr Thr Asn His Arg
355 360 365

Cys Ala Gly Glu Trp Met Thr Ile Ile Val Met Glu Glu Thr Ile Lys
370 375 380

Tyr Phe Ala Asn Lys Ile Asp Phe Glu Ala Pro Ser Gln Asp Leu Ser
385 390 395 400

Val Lys Leu Asp Gln Phe Pro Gly Lys Val Thr Ser Gly Thr Ile Ile
405 410 415

Gln Asn Val Arg Pro Arg Val Gln Arg
420 425

<210> 96
<211> 425
<212> PRT
<213> Staphylococcus agnetis

<400> 96

Met Ala Lys Gln Leu Pro Lys Asp Pro Gly Leu Asp Asn Thr Phe Lys
1 5 10 15

Val Leu Lys Glu Ala Tyr Thr Tyr Val Pro Cys Arg Leu Glu Lys Phe
20 25 30

Asn Ser Lys Ala Phe Gln Thr Thr Gly Met Gly Met Lys Pro Ile Ala
35 40 45

Val Ile Asn Gly Lys Glu Ala Ala Glu Leu Phe Tyr Asn Asn Asp Val
50 55 60

Met Gln Arg Glu Lys Thr Leu Pro Lys Arg Val Val Asn Thr Leu Phe
65 70 75 80

Gly Lys Gly Ala Ile His Thr Thr Lys Gly Lys Val His Val Asp Arg
85 90 95

Lys Ala Leu Phe Met Ser Leu Met Thr Glu Glu Asn Leu Lys Tyr Leu
100 105 110

Arg Glu Leu Thr Arg Asn His Trp Phe Met His Thr Glu His Met Gln
115 120 125

Asn Gln Lys Glu Val Asn Ile Tyr Lys Glu Ser Ile Tyr Val Leu Thr
130 135 140

Lys Ile Gly Phe Arg Trp Ala Gly Ile His Gln Thr Ala Glu Glu Ala
145 150 155 160

Glu Gln Asn Ala Gln Asp Met Asp Ile Met Ile Asp Ser Phe Lys Gly
165 170 175

Leu Gly Gln Thr Ile Gly Gly Gly Tyr Arg Lys Ala Lys Lys Ala Arg
180 185 190

Ala Arg Val Glu Gln Phe Leu Glu Lys Gln Ile Ile Ala Val Arg Lys
195 200 205

Gly Lys Ile Asn Ala Glu Pro Gly Thr Ala Leu Tyr Glu Phe Ala His
210 215 220

Trp Glu Asp Tyr Lys Gly Asn Pro Met Asp Ala Arg Leu Cys Ala Ile
225 230 235 240

Asp Leu Met Asn Val Val Arg Pro Leu Ala Ala Val Asn Arg Phe Val
245 250 255

Ser Tyr Ala Val Lys Ala Met Ile Glu Tyr Asp Gln Glu Arg Ile Lys
260 265 270

Leu Gln Val Ser Asp Asp Pro Asn Tyr Ala Tyr Lys Phe Ala Gln Glu
275 280 285

Val Arg Arg Ile Phe Pro Phe Val Pro Phe Leu Pro Gly Lys Leu Lys
290 295 300

Lys Asn Ile Glu Phe Asp Gly Tyr Arg Ile Lys Lys Gly Thr Phe Thr
305 310 315 320

Leu Leu Asp Val Phe Gly Thr Thr His Asp Pro Glu Leu Phe Glu Asn
325 330 335

Pro Tyr Gln Phe Asn Pro Asp Arg Phe Glu Asn Trp Asp Gly Ser Pro
340 345 350

Phe Asp Leu Ile Pro Gln Gly Gly Gly Asp Phe Tyr Thr Asn His Arg
355 360 365

Cys Ala Gly Glu Trp Met Thr Ile Ile Val Met Glu Glu Thr Ile Lys
370 375 380

Tyr Phe Ala Asn Lys Ile Asp Phe Glu Ala Pro Ser Gln Asp Leu Phe
385 390 395 400

Val Lys Leu Asp Gln Phe Pro Gly Lys Val Thr Ser Gly Thr Ile Ile

405 410 415

Gln Asn Val Arg Pro Arg Val Gln Arg
420 425

<210> 97
<211> 425
<212> PRT
<213> Staphylococcus agnetis

<400> 97

Met Ala Lys Gln Leu Pro Lys Asp Pro Gly Leu Asp Asn Thr Phe Lys
1 5 10 15

Val Leu Lys Glu Ala Tyr Thr Tyr Val Pro Cys Arg Leu Glu Lys Phe
20 25 30

Asn Ser Lys Ala Phe Gln Thr Thr Gly Met Gly Met Lys Pro Ile Ala
35 40 45

Val Ile Ser Gly Lys Glu Ala Ala Glu Leu Phe Tyr Asn Asn Asp Val
50 55 60

Met Gln Arg Glu Lys Thr Leu Pro Lys Arg Val Val Asn Thr Leu Phe
65 70 75 80

Gly Lys Gly Ala Ile His Thr Thr Lys Gly Lys Val His Val Asp Arg
85 90 95

Lys Ala Leu Phe Met Ser Leu Met Thr Glu Glu Asn Leu Lys Tyr Leu
100 105 110

Arg Glu Leu Thr Arg Asn His Trp Phe Met His Thr Glu His Met Gln
115 120 125

Asn Gln Arg Glu Val Asn Ile Tyr Lys Glu Ser Ile Tyr Val Leu Thr
130 135 140

Lys Ile Gly Phe Arg Trp Ala Gly Ile His Gln Thr Ala Glu Glu Ala
145 150 155 160

Glu Gln Asn Ala Lys Asp Met Asp Ile Met Ile Ala Ser Phe Lys Gly
165 170 175

Leu Gly Gln Thr Ile Gly Gly Gly Tyr Arg Lys Ala Lys Lys Ala Arg
180 185 190

Ala Arg Val Glu Gln Phe Leu Glu Lys Gln Ile Ile Ala Val Arg Lys

195 200 205

Gly Lys Ile Asn Ala Glu Pro Gly Thr Ala Leu Tyr Glu Phe Ala His
210 215 220

Trp Glu Asp Tyr Lys Gly Asn Pro Met Asp Ala Arg Leu Cys Ala Ile
225 230 235 240

Asp Leu Met Asn Val Val Arg Pro Leu Ala Ala Val Asn Arg Phe Val
245 250 255

Ser Tyr Ala Val Lys Ala Met Ile Glu Tyr Asp Gln Glu Arg Ile Lys
260 265 270

Leu Gln Val Ser Asp Asp Pro Asn Tyr Ala Tyr Lys Phe Ala Gln Glu
275 280 285

Val Arg Arg Ile Phe Pro Phe Val Pro Phe Leu Pro Gly Lys Leu Lys
290 295 300

Lys Asn Ile Glu Phe Asp Gly Tyr Arg Ile Lys Lys Gly Thr Phe Thr
305 310 315 320

Leu Leu Asp Val Phe Gly Thr Thr His Asp Pro Glu Leu Phe Glu Asn
325 330 335

Pro Tyr Gln Phe Asn Pro Asp Arg Phe Glu Asn Trp Asp Gly Ser Pro
340 345 350

Phe Asp Leu Ile Pro Gln Gly Gly Gly Asp Phe Tyr Thr Asn His Arg
355 360 365

Cys Ala Gly Glu Trp Met Thr Ile Ile Val Met Glu Glu Thr Ile Lys
370 375 380

Tyr Phe Ala Asn Lys Ile Asp Phe Glu Ala Pro Ser Gln Asp Leu Ser
385 390 395 400

Val Lys Leu Asp Gln Phe Pro Gly Lys Val Thr Ser Gly Thr Ile Ile
405 410 415

Gln Asn Val Arg Pro Arg Val Gln Arg
420 425

<210> 98
<211> 424
<212> PRT
<213> Staphylococcus sp.

<400> 98

Met Ala Lys Lys Leu Pro Lys Asp Ser Gly Leu Asp Asn Thr Phe Lys
1               5                   10                  15

Val Leu Lys Glu Ala Tyr Thr Tyr Val Pro Cys Arg Leu Glu Lys Phe
            20                  25                  30

Asn Thr Lys Ala Phe Gln Thr Lys Ala Met Gly Met Lys Pro Ile Val
        35                  40                  45

Val Ile Ser Gly Lys Glu Ala Ala Glu Leu Phe Tyr Asn Asn Asp Val
    50                  55                  60

Met Gln Arg Glu Lys Thr Leu Pro Lys Arg Val Val Asn Thr Leu Phe
65                  70                  75                  80

Gly Lys Gly Ala Ile His Thr Thr Lys Gly Lys Val His Val Asp Arg
                85                  90                  95

Lys Ala Leu Phe Met Ser Leu Met Thr Glu Glu Asn Leu Lys Tyr Leu
            100                 105                 110

Arg Glu Leu Ala Arg Asn His Trp Phe Val His Thr Glu His Met Gln
        115                 120                 125

Thr Gln Asp Glu Val Asn Ile Tyr Lys Glu Ser Ile Tyr Val Leu Thr
    130                 135                 140

Lys Ile Gly Phe Arg Trp Ala Gly Ile Arg Gln Thr Ala Glu Glu Ala
145                 150                 155                 160

Glu Arg Asn Ala Lys Asp Met Asp Val Met Ile Asp Ser Phe Lys Gly
                165                 170                 175

Leu Gly Gln Thr Leu Gly Ser Gly Tyr Arg Lys Ala Lys Lys Ala Arg
            180                 185                 190

Ala Arg Val Glu Gln Phe Leu Glu Asp Gln Ile Val Ala Val Arg Asn
        195                 200                 205

Gly Glu Ile Lys Ala Glu Pro Gly Thr Ala Leu Tyr Glu Phe Ala His
    210                 215                 220

Trp Glu Asp Tyr Lys Gly Asn Gln Met Asp Ala Arg Leu Cys Ala Val
225                 230                 235                 240

Asp Leu Met Asn Val Val Arg Pro Leu Ala Ala Val Asn Arg Phe Val
245 250 255

Ser Tyr Ala Val Lys Ala Met Ile Glu Tyr Asp Gln Glu Arg Leu Lys
260 265 270

Leu Gln Val Thr Asp Asp Pro Asn Tyr Ala Tyr Lys Phe Ala Gln Glu
275 280 285

Val Arg Arg Ile Phe Pro Phe Val Pro Phe Leu Pro Gly Lys Phe Lys
290 295 300

Lys Thr Val Glu Phe Asp Gly Tyr Arg Ile Lys Lys Gly Thr Phe Thr
305 310 315 320

Leu Leu Asp Val Phe Gly Thr Thr His Asp Pro Asp Leu Phe Glu Asn
325 330 335

Pro Tyr Gln Phe Asn Pro Asp Arg Phe Asp Asn Trp Asp Gly Ser Pro
340 345 350

Phe Asp Leu Ile Pro Gln Gly Gly Gly Asp Phe Tyr Thr Asn His Arg
355 360 365

Cys Ala Gly Glu Trp Met Thr Ile Ile Val Met Glu Glu Thr Ile Lys
370 375 380

Tyr Phe Ala Asn Lys Ile Asp Phe Asp Ala Pro Ala Gln Asp Leu Ser
385 390 395 400

Val Lys Leu Asp Gln Phe Pro Gly Lys Val Thr Ser Gly Thr Leu Ile
405 410 415

Lys Asn Val Arg Pro Arg Leu Gln
420

<210> 99
<211> 424
<212> PRT
<213> Staphylococcus hyicus

<400> 99

Met Ala Lys Lys Leu Pro Lys Asp Ser Gly Leu Asp Asn Thr Phe Lys
1 5 10 15

Val Leu Lys Glu Ala Tyr Thr Tyr Val Pro Cys Arg Leu Glu Lys Phe
20 25 30

Asn Thr Lys Ala Phe Gln Thr Lys Ala Met Gly Met Lys Pro Ile Val
35 40 45

Val Ile Ser Gly Lys Glu Ala Ala Glu Leu Phe Tyr Asn Asn Asp Val
50 55 60

Met Gln Arg Glu Lys Thr Leu Pro Lys Arg Val Val Asn Thr Leu Phe
65 70 75 80

Gly Lys Gly Ala Ile His Thr Thr Lys Gly Lys Val His Val Asp Arg
85 90 95

Lys Ala Leu Phe Met Ser Leu Met Thr Glu Glu Asn Leu Lys Tyr Leu
100 105 110

Arg Glu Leu Ala Arg Asn His Trp Phe Val His Thr Glu His Met Gln
115 120 125

Thr Gln Asp Glu Val Asn Ile Tyr Lys Glu Ser Ile Tyr Val Leu Thr
130 135 140

Lys Ile Gly Phe Arg Trp Ala Gly Ile Arg Gln Thr Ala Glu Glu Ala
145 150 155 160

Glu Arg Asn Ala Lys Asp Met Asp Val Met Ile Asp Ser Phe Lys Gly
165 170 175

Leu Gly Gln Thr Leu Gly Ser Gly Tyr Arg Lys Ala Lys Lys Ala Arg
180 185 190

Ala Arg Val Glu Gln Phe Leu Glu Asp Gln Ile Val Ala Val Arg Asn
195 200 205

Gly Glu Ile Lys Ala Glu Pro Gly Thr Ala Leu Tyr Glu Phe Ala His
210 215 220

Trp Glu Asp Tyr Lys Gly Asn Gln Met Asp Ala Arg Leu Cys Ala Val
225 230 235 240

Asp Leu Met Asn Val Val Arg Pro Leu Ala Ala Val Asn Arg Phe Val
245 250 255

Ser Tyr Ala Val Lys Ala Met Ile Glu Tyr Asp Gln Glu Cys Leu Lys
260 265 270

Leu Gln Val Thr Asp Asp Pro Asn Tyr Ala Tyr Lys Phe Ala Gln Glu
275 280 285

Val Arg Arg Ile Phe Pro Phe Val Pro Phe Leu Pro Gly Lys Phe Lys
    290                 295                 300

Lys Thr Val Glu Phe Asp Gly Tyr Arg Ile Lys Lys Gly Thr Phe Thr
305                 310                 315                 320

Leu Leu Asp Val Phe Gly Thr Thr His Asp Pro Asp Leu Phe Glu Asn
                325                 330                 335

Pro Tyr Gln Phe Asn Pro Asp Arg Phe Asp Asn Trp Asp Gly Ser Pro
            340                 345                 350

Phe Asp Leu Ile Pro Gln Gly Gly Gly Asp Phe Tyr Thr Asn His Arg
        355                 360                 365

Cys Ala Gly Glu Trp Met Thr Ile Ile Val Met Glu Glu Thr Ile Lys
    370                 375                 380

Tyr Phe Ala Asn Lys Ile Asp Phe Asp Ala Pro Ala Gln Asp Leu Ser
385                 390                 395                 400

Val Lys Leu Asp Gln Phe Pro Gly Lys Val Thr Ser Gly Thr Leu Ile
                405                 410                 415

Lys Asn Val Arg Pro Arg Leu Gln
            420

<210> 100
<211> 424
<212> PRT
<213> Salinicoccus qingdaonensis

<400> 100

Met Ala Thr Ile Lys Arg Asp Lys Gly Leu Asp Asn Ser Val Lys Val
1               5                   10                  15

Met Lys Gln Gly Tyr Leu Tyr Thr Thr Asn Gln Arg Glu Arg Leu Gly
            20                  25                  30

Val Thr Asp Gly Val Phe Glu Thr Arg Ala Leu Gly Gly Lys Arg Ile
        35                  40                  45

Ile Val Leu Ser Gly Lys Asp Gly Ala Glu Leu Phe Tyr Asp Asn Asp
    50                  55                  60

Lys Ile Glu Arg Ser Gly Thr Leu Pro Lys Arg Val Val Asn Thr Leu
65                  70                  75                  80

Phe Gly Lys Gly Ala Ile His Thr Thr Thr Gly Lys Val His Ile Asp
85 90 95

Arg Lys Ala Leu Phe Met Ser Leu Met Thr Glu Gly Asn Leu Lys Tyr
100 105 110

Leu Arg Glu Leu Thr Arg Asn Leu Trp Phe Ala Asn Thr Gln Arg Met
115 120 125

Glu Ser Met Asp Gln Val Asn Val Tyr Arg Glu Ser Ile Ile Val Leu
130 135 140

Thr Lys Val Gly Thr Arg Trp Ala Gly Val Gln Ala Pro Glu Lys Glu
145 150 155 160

Ile Glu Asn Ile Ala Thr Asp Met Asp Ile Met Ile Asp Ser Phe Lys
165 170 175

Ala Ile Gly Ser Ala Phe Lys Gly Tyr Arg Glu Ala Lys Ala Ala Arg
180 185 190

Arg Arg Val Glu Asp Trp Leu Glu Asp Gln Ile Ile Gln Thr Arg Lys
195 200 205

Gly Lys Ile His Pro Pro Lys Gly Thr Ala Leu Tyr Glu Phe Ala His
210 215 220

Trp Lys Asp Tyr Lys Gly Glu Pro Met Asp Ser Arg Leu Cys Gly Ile
225 230 235 240

Asp Leu Met Asn Thr Phe Arg Pro Leu Ile Ala Ile Asn Arg Phe Val
245 250 255

Ala Phe Gly Ala Leu Ala Met Tyr Glu Asn Pro Val Ala Arg Glu Lys
260 265 270

Ile Lys Gln Asp Asp Asp Tyr Ala Tyr Met Phe Ala Gln Glu Val Arg
275 280 285

Arg Phe Tyr Pro Phe Val Pro Tyr Leu Pro Ala Lys Ala Lys Val Asp
290 295 300

Phe Gln Tyr Lys Gly Tyr Glu Ile Glu Lys Asp Thr Met Leu Ala Leu
305 310 315 320

Asp Ile Tyr Gly Thr Met His Asp Pro Asn Val Trp Glu Asp Pro Asn
325 330 335

Glu Phe Tyr Pro Glu Arg Phe Lys Asp Trp Asp Gly Ser Pro Phe Asp
340 345 350

Leu Ile Pro Gln Gly Gly Gly Asp Tyr His Thr Asn His Arg Cys Ala
355 360 365

Gly Glu Trp Met Thr Val Ile Ile Met Glu Glu Thr Met Lys Tyr Phe
370 375 380

Ala Ser Arg Ile Asn Tyr Asp Val Pro Glu Gln Asp Leu Thr Val Asp
385 390 395 400

Leu Asn Ser Leu Pro Gly Tyr Ile Lys Ser Gly Phe Val Ile Glu Asn
405 410 415

Val Gln Glu Asn Val Asp Arg Thr
420

<210> 101
<211> 424
<212> PRT
<213> Salinicoccus alkaliphilus

<400> 101

Met Ala Thr Ile Lys Lys Asp Lys Gly Ile Asp Asn Thr Ala Lys Ile
1 5 10 15

Ala Lys Gln Gly Tyr Leu Tyr Thr Thr Asn Gln Arg Glu Arg Leu Gly
20 25 30

Val Lys Asp Gly Val Phe Glu Thr Arg Gly Leu Gly Gly Lys Arg Ile
35 40 45

Ile Ile Leu Ser Gly Lys Asp Gly Ala Glu Leu Phe Tyr Asp Asn Asp
50 55 60

Lys Val Glu Arg Ser Gly Thr Leu Pro Lys Arg Val Val Asn Thr Leu
65 70 75 80

Phe Gly Lys Gly Ala Ile His Thr Thr Thr Gly Lys Val His Val Asp
85 90 95

Arg Lys Ala Leu Phe Met Ser Leu Met Thr Glu Gly Asn Leu Gln Tyr
100 105 110

Leu Arg Glu Leu Thr Arg Asn His Trp Leu Met Asn Thr Gln Arg Met
115 120 125

Glu Asn Met Asp Gln Val Asn Val Tyr Arg Glu Ser Ile Ile Leu Leu
130 135 140

Thr Lys Val Gly Thr Arg Trp Ala Gly Val Gln Ala Pro Glu Lys Glu
145 150 155 160

Ile Glu Asn Ile Ala Thr Asp Met Asp Ile Met Ile Asp Ser Phe Lys
165 170 175

Ala Ile Gly Ser Ala Phe Lys Gly Tyr Arg Ala Ser Lys Ala Ala Arg
180 185 190

Arg Arg Val Glu Asp Trp Leu Glu Asp Gln Ile Ile Gln Thr Arg Lys
195 200 205

Gly Lys Ile His Pro Pro Lys Gly Thr Ala Leu Tyr Glu Phe Ala His
210 215 220

Trp Lys Asp Tyr Lys Gly Glu Pro Met Asp Ser Arg Leu Cys Gly Ile
225 230 235 240

Asp Leu Met Asn Thr Phe Arg Pro Leu Ile Ala Ile Asn Arg Phe Ile
245 250 255

Ala Phe Gly Ala Leu Ala Met His Glu Asn Pro Val Ala Arg Glu Lys
260 265 270

Ile Lys Gln Asp Asp Asp Tyr Ala Tyr Met Phe Ala Gln Glu Val Arg
275 280 285

Arg Phe Tyr Pro Phe Val Pro Tyr Leu Pro Gly Lys Val Lys Glu Asp
290 295 300

Phe Gln Tyr Lys Gly Tyr Asp Phe Glu Lys Asp Thr Met Leu Ala Ile
305 310 315 320

Asp Ile Tyr Gly Thr Met His Asp Pro Asp Val Trp Glu Asn Pro Asn
325 330 335

Glu Phe Tyr Pro Glu Arg Phe Lys Asp Trp Asp Gly Ser Pro Phe Asp
340 345 350

Leu Ile Pro Gln Gly Gly Gly Asp Tyr Tyr Thr Asn His Arg Cys Ala
355 360 365

Gly Glu Trp Met Thr Ile Ile Ile Met Gln Glu Thr Met Lys Tyr Phe

370 375 380

Ala Ser Arg Ile Thr Tyr Asp Val Pro Glu Gln Asp Leu Thr Val Asp
385 390 395 400

Leu Asn Ser Leu Pro Gly Tyr Val Lys Ser Gly Phe Ile Ile Glu Asn
405 410 415

Val Arg Glu Val Val Asp Arg Asp
420

<210> 102
<211> 425
<212> PRT
<213> Staphylococcus chromogenes

<400> 102

Met Ala Lys Lys Leu Pro Lys Asp Ser Gly Leu Asp Asn Thr Phe Lys
1 5 10 15

Ile Leu Asn Glu Ala Tyr Thr Tyr Val Pro Cys Arg Leu Glu Lys Phe
20 25 30

Gly Thr Lys Ala Phe Glu Thr Ala Ala Ile Gly Met Lys Pro Met Thr
35 40 45

Val Ile Ser Gly Lys Glu Ala Ala Glu Leu Phe Tyr Asp Asn Glu Leu
50 55 60

Met Gln Arg Glu Lys Thr Leu Pro Lys Arg Val Val Asn Thr Leu Phe
65 70 75 80

Gly Lys Gly Ala Ile His Thr Thr Lys Gly Lys Val His Val Asp Arg
85 90 95

Lys Ala Leu Phe Met Ser Leu Met Thr Glu Glu Asn Leu Lys Tyr Leu
100 105 110

Arg Asp Leu Thr Arg Asn Tyr Trp Phe Met His Thr Glu Glu Met Gln
115 120 125

Arg Gln Asp Glu Val Asn Ile Tyr Lys Thr Ser Ile Tyr Ile Leu Thr
130 135 140

Lys Ile Gly Phe Arg Trp Ala Gly Leu Gln Gln Thr Ser Glu Glu Ala
145 150 155 160

Lys Arg Asn Ala Arg Asp Met Asp Ile Met Ile Asp Ser Phe Gln Gly

165 170 175

Leu Gly Gln Thr Phe Gly Gly Gly Tyr Arg Lys Ala Lys Lys Ala Arg
180 185 190

Ala Arg Val Glu Lys Phe Leu Glu Asn Gln Ile Val Ala Val Arg Glu
195 200 205

Gly Lys Met Lys Ala Gln Pro Gly Thr Ala Leu Tyr Glu Phe Ala His
210 215 220

Trp Lys Asp Tyr Lys Gly His Gln Met Asp Pro His Leu Cys Ala Val
225 230 235 240

Glu Leu Met Asn Val Val Arg Pro Leu Ala Ala Ile Asn Arg Phe Val
245 250 255

Ser Tyr Val Val Lys Ala Met Leu Glu Phe Asp Gln Glu Arg Ile Lys
260 265 270

Leu Gln Val Ala Asp Asp Pro Asn Tyr Ala Tyr Lys Phe Ala Gln Glu
275 280 285

Val Arg Arg Ile Phe Pro Phe Val Pro Phe Leu Pro Gly Lys Leu Lys
290 295 300

Lys Asp Val Glu Phe Asp Gly Phe Lys Ile Lys Lys Gly Thr Phe Thr
305 310 315 320

Leu Leu Asp Ile Phe Gly Thr Thr His Asp Pro Glu Leu Phe Glu Asn
325 330 335

Pro Tyr Gln Phe Asn Pro Asp Arg Phe Asp Asn Trp Asp Gly Ser Pro
340 345 350

Phe Asp Leu Ile Pro Gln Gly Gly Gly Asp Phe Tyr Thr Asn His Arg
355 360 365

Cys Ala Gly Glu Trp Met Thr Ile Ile Ile Met Glu Glu Thr Ile Lys
370 375 380

Tyr Phe Ala Thr Lys Ile Asp Phe Asp Ala Pro Val Gln Asp Leu Ser
385 390 395 400

Val Lys Leu Asp Gln Phe Pro Gly Lys Val Thr Ser Gly Pro Val Ile
405 410 415

His Arg Val Arg Pro Arg Val Gln Arg
420 425

<210> 103
<211> 425
<212> PRT
<213> Staphylococcus chromogenes

<400> 103

Met Ala Lys Lys Leu Pro Lys Asp Ser Gly Leu Asp Asn Thr Phe Lys
1 5 10 15

Ile Leu Asn Glu Ala Tyr Thr Tyr Val Pro Cys Arg Leu Glu Lys Phe
20 25 30

Gly Thr Lys Ala Phe Glu Thr Ala Ala Ile Gly Met Lys Pro Met Thr
35 40 45

Val Ile Ser Gly Lys Glu Ala Ala Glu Leu Phe Tyr Asp Asn Glu Leu
50 55 60

Met Gln Arg Glu Lys Thr Leu Pro Lys Arg Val Val Asn Thr Leu Phe
65 70 75 80

Gly Lys Gly Ala Ile His Thr Thr Lys Gly Lys Val His Val Asp Arg
85 90 95

Lys Ala Leu Phe Met Ser Leu Met Thr Glu Glu Asn Leu Lys Tyr Leu
100 105 110

Arg Asp Leu Thr Arg Asn Tyr Trp Phe Met His Thr Glu Glu Met Gln
115 120 125

Arg Gln Asp Glu Val Asn Ile Tyr Lys Thr Ser Ile Tyr Ile Leu Thr
130 135 140

Lys Ile Gly Phe Arg Trp Ala Gly Leu Gln Gln Thr Ser Glu Glu Ala
145 150 155 160

Lys Arg Asn Ala Arg Asp Met Asp Ile Met Ile Asp Ser Phe Gln Gly
165 170 175

Leu Gly Gln Thr Phe Gly Gly Gly Tyr Arg Lys Ala Lys Lys Ala Arg
180 185 190

Ala Arg Val Glu Lys Phe Leu Glu Asn Gln Ile Val Ala Val Arg Glu
195 200 205

Gly Lys Met Lys Ala Gln Pro Gly Thr Ala Leu Tyr Glu Phe Ala His
    210                 215                 220

Trp Lys Asp Tyr Lys Gly His Gln Met Asp Pro His Leu Cys Ala Val
225                 230                 235                 240

Glu Leu Met Asn Val Val Arg Pro Leu Ala Ala Ile Asn Arg Phe Val
                245                 250                 255

Ser Tyr Ala Val Lys Ala Met Leu Glu Phe Asp Gln Glu Arg Ile Lys
            260                 265                 270

Leu Gln Val Ala Asp Asp Pro Asn Tyr Ala Tyr Lys Phe Ala Gln Glu
        275                 280                 285

Val Arg Arg Ile Phe Pro Phe Val Pro Phe Leu Pro Gly Lys Leu Lys
    290                 295                 300

Lys Asp Val Glu Phe Asp Gly Phe Lys Ile Lys Lys Gly Thr Phe Thr
305                 310                 315                 320

Leu Leu Asp Ile Phe Gly Thr Thr His Asp Ser Glu Leu Phe Glu Asn
                325                 330                 335

Pro Tyr Gln Phe Asn Pro Asp Arg Phe Glu Asn Trp Asp Gly Ser Pro
            340                 345                 350

Phe Asp Leu Ile Pro Gln Gly Gly Gly Asp Phe Tyr Thr Asn His Arg
        355                 360                 365

Cys Ala Gly Glu Trp Met Thr Ile Ile Ile Met Glu Glu Thr Ile Lys
    370                 375                 380

Tyr Phe Ala Thr Lys Ile Asp Phe Asp Ala Pro Val Gln Asp Leu Ser
385                 390                 395                 400

Val Lys Leu Asp Gln Phe Pro Gly Lys Val Thr Ser Gly Pro Val Ile
                405                 410                 415

His Arg Val Arg Pro Arg Val Gln Arg
            420                 425

<210> 104
<211> 425
<212> PRT
<213> Staphylococcus chromogenes

<400> 104

```
Met Ala Lys Lys Leu Pro Lys Asp Ser Gly Leu Asp Asn Thr Phe Lys
1               5                   10                  15

Ile Leu Asn Glu Ala Tyr Thr Tyr Val Pro Cys Arg Leu Glu Lys Phe
            20                  25                  30

Gly Thr Lys Ala Phe Glu Thr Ala Ala Ile Gly Met Lys Pro Met Thr
        35                  40                  45

Val Ile Ser Gly Lys Glu Ala Ala Glu Leu Phe Tyr Asp Asn Glu Leu
    50                  55                  60

Met Gln Arg Glu Lys Thr Leu Pro Lys Arg Val Val Asn Thr Leu Phe
65                  70                  75                  80

Gly Lys Gly Ala Ile His Thr Thr Lys Gly Lys Val His Val Asp Arg
                85                  90                  95

Lys Ala Leu Phe Met Ser Leu Met Thr Glu Glu Asn Leu Lys Tyr Leu
            100                 105                 110

Arg Asp Leu Thr Arg Asn Tyr Trp Phe Met His Thr Glu Glu Met Gln
        115                 120                 125

Arg Gln Asp Glu Val Asn Ile Tyr Lys Thr Ser Ile Tyr Ile Leu Thr
    130                 135                 140

Lys Ile Gly Phe Arg Trp Ala Gly Leu Gln Gln Thr Ser Glu Glu Ala
145                 150                 155                 160

Lys Arg Asn Ala Arg Asp Met Asp Ile Met Ile Asp Ser Phe Gln Gly
                165                 170                 175

Leu Gly Gln Thr Phe Gly Gly Gly Tyr Arg Lys Ala Lys Lys Ala Arg
            180                 185                 190

Ala Arg Val Glu Lys Phe Leu Glu Asn Gln Ile Val Ala Val Arg Glu
        195                 200                 205

Gly Lys Met Lys Ala Gln Pro Gly Thr Ala Leu Tyr Glu Phe Ala His
    210                 215                 220

Trp Lys Asp Tyr Lys Gly His Gln Met Asp Pro His Leu Cys Ala Val
225                 230                 235                 240

Glu Leu Met Asn Val Val Arg Pro Leu Ala Ala Ile Asn Arg Phe Val
                245                 250                 255
```

Ser Tyr Ala Val Lys Ala Met Leu Glu Phe Asp Gln Glu Arg Ile Lys
260 265 270

Leu Gln Val Ala Asp Asp Pro Asn Tyr Ala Tyr Lys Phe Ala Gln Glu
275 280 285

Val Arg Arg Ile Phe Pro Phe Val Pro Phe Leu Pro Gly Lys Leu Lys
290 295 300

Lys Asp Val Lys Phe Asp Gly Phe Lys Ile Lys Lys Gly Thr Phe Thr
305 310 315 320

Leu Leu Asp Ile Phe Gly Thr Thr His Asp Pro Glu Leu Phe Glu Asn
325 330 335

Pro Tyr Gln Phe Asn Pro Asp Arg Phe Glu Asn Trp Asp Gly Ser Pro
340 345 350

Phe Asp Leu Ile Pro Gln Gly Gly Gly Asp Phe Tyr Thr Asn His Arg
355 360 365

Cys Ala Gly Glu Trp Met Thr Ile Ile Ile Met Glu Glu Thr Ile Lys
370 375 380

Tyr Phe Ala Thr Lys Ile Asp Phe Asp Ala Pro Val Gln Asp Leu Ser
385 390 395 400

Val Lys Leu Asp Gln Phe Pro Gly Lys Val Thr Ser Gly Pro Val Ile
405 410 415

His Arg Val Arg Pro Arg Val Gln Arg
420 425

<210> 105
<211> 425
<212> PRT
<213> Staphylococcus chromogenes

<400> 105

Met Ala Lys Lys Leu Pro Lys Asp Ser Gly Leu Asp Asn Thr Phe Lys
1 5 10 15

Ile Leu Asn Glu Ala Tyr Thr Tyr Val Pro Cys Arg Leu Glu Lys Phe
20 25 30

Gly Thr Lys Ala Phe Glu Thr Ala Ala Ile Gly Met Lys Pro Met Thr
35 40 45

Val Ile Ser Gly Lys Glu Ala Ala Glu Leu Phe Tyr Asp Asn Glu Leu
    50                  55                  60

Met Gln Arg Glu Lys Thr Leu Pro Lys Arg Val Val Asn Thr Leu Phe
65                  70                  75                  80

Gly Lys Gly Ala Ile His Thr Thr Lys Gly Lys Val His Val Asp Arg
                85                  90                  95

Lys Ala Leu Phe Met Ser Leu Met Thr Glu Glu Asn Leu Lys Tyr Leu
            100                 105                 110

Arg Asp Leu Thr Arg Asn Tyr Trp Phe Met His Thr Glu Glu Met Gln
        115                 120                 125

Arg Gln Asp Glu Val Asn Ile Tyr Lys Thr Ser Ile Tyr Ile Leu Thr
    130                 135                 140

Lys Ile Gly Phe Arg Trp Ala Gly Leu Gln Gln Thr Ser Glu Glu Ala
145                 150                 155                 160

Lys Arg Asn Ala Arg Asp Met Asp Ile Met Ile Asp Ser Phe Gln Gly
                165                 170                 175

Leu Gly Gln Thr Phe Gly Gly Gly Tyr Arg Lys Ala Lys Lys Ala Arg
            180                 185                 190

Ala Arg Val Glu Lys Phe Leu Glu Asn Gln Ile Val Ala Val Arg Glu
        195                 200                 205

Gly Lys Met Lys Ala Gln Pro Gly Thr Ala Leu Tyr Glu Phe Ala His
    210                 215                 220

Trp Lys Asp Tyr Lys Gly His Gln Met Asp Pro His Leu Cys Ala Val
225                 230                 235                 240

Glu Leu Met Asn Val Val Arg Pro Leu Ala Ala Ile Asn Arg Phe Val
                245                 250                 255

Ser Tyr Val Val Lys Ala Met Leu Glu Phe Asp Gln Glu Arg Ile Lys
            260                 265                 270

Leu Gln Val Ala Asp Asp Pro Asn Tyr Ala Tyr Lys Phe Ala Gln Glu
        275                 280                 285

Val Arg Arg Ile Phe Pro Phe Val Pro Phe Leu Pro Gly Lys Leu Lys
    290                 295                 300

Lys Asp Val Glu Phe Asp Gly Phe Lys Ile Lys Lys Gly Thr Phe Thr
305 310 315 320

Leu Leu Asp Ile Phe Gly Thr Thr His Asp Pro Glu Leu Phe Glu Asn
325 330 335

Pro Tyr Gln Phe Asn Pro Asp Arg Phe Glu Asn Trp Asp Gly Ser Pro
340 345 350

Phe Asp Leu Ile Pro Gln Gly Gly Gly Asp Phe Tyr Thr Asn His Arg
355 360 365

Cys Ala Gly Glu Trp Met Thr Ile Ile Ile Met Glu Glu Thr Ile Lys
370 375 380

Tyr Phe Ala Thr Lys Ile Asp Phe Asp Ala Pro Val Gln Asp Leu Ser
385 390 395 400

Val Lys Leu Asp Gln Phe Pro Gly Lys Val Thr Ser Gly Pro Val Ile
405 410 415

His Arg Val Arg Pro Arg Val Gln Arg
420 425

<210> 106
<211> 425
<212> PRT
<213> Staphylococcus chromogenes

<400> 106

Met Ala Lys Lys Leu Pro Lys Asp Ser Gly Leu Asp Asn Thr Phe Lys
1 5 10 15

Ile Leu Asn Glu Ala Tyr Thr Tyr Val Pro Cys Arg Leu Glu Lys Phe
20 25 30

Gly Thr Lys Ala Phe Glu Thr Ala Ala Ile Gly Met Lys Pro Met Thr
35 40 45

Val Ile Ser Gly Lys Glu Ala Ala Glu Leu Phe Tyr Asp Asn Glu Leu
50 55 60

Met Gln Arg Glu Lys Thr Leu Pro Lys Arg Val Val Asn Thr Leu Phe
65 70 75 80

Gly Lys Gly Ala Ile His Thr Thr Lys Gly Lys Val His Val Asp Arg
85 90 95

Lys Ala Leu Phe Met Ser Leu Met Thr Glu Glu Asn Leu Lys Tyr Leu
100 105 110

Arg Asp Leu Thr Arg Asn Tyr Trp Phe Met His Thr Glu Asp Met Gln
115 120 125

Arg Gln Asp Glu Val Asn Ile Tyr Lys Thr Ser Ile Tyr Ile Leu Thr
130 135 140

Lys Ile Gly Phe Arg Trp Ala Gly Leu Gln Gln Thr Ser Glu Glu Ala
145 150 155 160

Lys Arg Asn Ala Arg Asp Met Asp Ile Met Ile Asp Ser Phe Gln Gly
165 170 175

Leu Gly Gln Thr Phe Gly Gly Gly Tyr Arg Lys Ala Lys Lys Ala Arg
180 185 190

Ala Arg Val Glu Lys Phe Leu Glu Asn Gln Ile Val Ala Val Arg Glu
195 200 205

Gly Lys Met Lys Ala Gln Pro Gly Thr Ala Leu Tyr Glu Phe Ala His
210 215 220

Trp Lys Asp Tyr Lys Gly His Gln Met Asp Pro His Leu Cys Ala Val
225 230 235 240

Glu Leu Met Asn Val Val Arg Pro Leu Ala Ala Ile Asn Arg Phe Val
245 250 255

Ser Tyr Ala Val Lys Ala Met Leu Glu Phe Asp Gln Glu Arg Ile Lys
260 265 270

Leu Gln Val Ala Asp Asp Pro Asn Tyr Ala Tyr Lys Phe Ala Gln Glu
275 280 285

Val Arg Arg Ile Phe Pro Phe Val Pro Phe Leu Pro Gly Lys Leu Lys
290 295 300

Lys Asp Val Glu Phe Asp Gly Phe Lys Ile Lys Lys Gly Thr Phe Thr
305 310 315 320

Leu Leu Asp Ile Phe Gly Thr Thr His Asp Pro Glu Leu Phe Glu Asn
325 330 335

Pro Tyr Gln Phe Asn Pro Asp Arg Phe Glu Asn Trp Asp Gly Ser Pro

340 345 350

Phe Asp Leu Ile Pro Gln Gly Gly Gly Asp Phe Tyr Thr Asn His Arg
355 360 365

Cys Ala Gly Glu Trp Met Thr Ile Ile Ile Met Glu Glu Thr Ile Lys
370 375 380

Tyr Phe Ala Thr Lys Ile Asp Phe Asp Ala Pro Val Gln Asp Leu Ser
385 390 395 400

Val Lys Leu Asp Gln Phe Pro Gly Lys Val Thr Ser Gly Pro Val Ile
405 410 415

His Arg Val Arg Pro Arg Val Gln Arg
420 425

<210> 107
<211> 425
<212> PRT
<213> Staphylococcus chromogenes

<400> 107

Met Ala Lys Lys Leu Pro Lys Asp Ser Gly Leu Asp Asn Thr Phe Lys
1 5 10 15

Ile Leu Asn Glu Ala Tyr Thr Tyr Val Pro Cys Arg Leu Glu Lys Phe
20 25 30

Gly Thr Lys Ala Phe Glu Thr Ala Ala Ile Gly Met Lys Pro Met Thr
35 40 45

Val Ile Ser Gly Lys Glu Ala Ala Glu Leu Phe Tyr Asp Asn Glu Leu
50 55 60

Met Gln Arg Glu Lys Thr Leu Pro Lys Arg Val Val Asn Thr Leu Phe
65 70 75 80

Gly Lys Gly Ala Ile His Thr Thr Lys Gly Lys Val His Val Asp Arg
85 90 95

Lys Ala Leu Phe Met Ser Leu Met Thr Glu Glu Asn Leu Lys Tyr Leu
100 105 110

Arg Asp Leu Thr Arg Asn Tyr Trp Phe Met His Thr Glu Glu Met Gln
115 120 125

Arg Gln Asp Glu Val Asn Ile Tyr Lys Thr Ser Ile Tyr Ile Leu Thr

130 135 140

Lys Ile Gly Phe Arg Trp Ala Gly Leu Gln Gln Thr Ser Glu Glu Ala
145 150 155 160

Lys Arg Asn Ala Arg Asp Met Asp Ile Met Ile Asp Ser Phe Gln Gly
165 170 175

Leu Gly Gln Thr Phe Gly Gly Gly Tyr Arg Lys Ala Lys Lys Ala Arg
180 185 190

Ala Arg Val Glu Lys Phe Leu Glu Asn Gln Ile Val Ala Val Arg Glu
195 200 205

Gly Lys Met Lys Ala Gln Pro Gly Thr Ala Leu Tyr Glu Phe Ala His
210 215 220

Trp Lys Asp Tyr Lys Gly His Gln Met Asp Pro His Leu Cys Ala Val
225 230 235 240

Glu Leu Met Asn Val Val Arg Pro Leu Ala Ala Ile Asn Arg Phe Val
245 250 255

Ser Tyr Ala Val Lys Ala Met Leu Glu Phe Asp Gln Glu Arg Ile Lys
260 265 270

Leu Gln Val Ala Asp Asp Pro Asn Tyr Ala Tyr Lys Phe Ala Gln Glu
275 280 285

Val Arg Arg Ile Phe Pro Phe Val Pro Phe Leu Pro Gly Lys Leu Lys
290 295 300

Lys Asp Val Glu Phe Asp Gly Phe Lys Ile Lys Lys Gly Thr Phe Thr
305 310 315 320

Leu Leu Asp Ile Phe Gly Thr Thr His Asp Pro Glu Leu Phe Glu Asn
325 330 335

Pro Tyr Gln Phe Asn Pro Asp Arg Phe Glu Asn Trp Asp Gly Ser Pro
340 345 350

Phe Asp Leu Ile Pro Gln Gly Gly Gly Asp Phe Tyr Thr Asn His Arg
355 360 365

Cys Ala Gly Glu Trp Met Thr Ile Ile Ile Met Glu Glu Thr Ile Lys
370 375 380

Tyr Phe Ala Thr Lys Ile Asp Phe Asp Ala Pro Val Gln Asp Leu Ser
385 390 395 400

Val Lys Leu Asp Gln Phe Pro Gly Lys Val Thr Ser Gly Pro Val Ile
405 410 415

His Arg Val Arg Pro Arg Val Gln Arg
420 425

<210> 108
<211> 425
<212> PRT
<213> Staphylococcus chromogenes

<400> 108

Met Ala Lys Lys Leu Pro Lys Asp Ser Gly Leu Asp Asn Thr Phe Lys
1 5 10 15

Ile Leu Asn Glu Ala Tyr Thr Tyr Val Pro Cys Arg Leu Glu Lys Phe
20 25 30

Gly Thr Lys Ala Phe Glu Thr Ala Ala Ile Gly Met Lys Pro Met Thr
35 40 45

Val Ile Ser Gly Lys Glu Ala Ala Glu Leu Phe Tyr Asp Asn Glu Leu
50 55 60

Met Gln Arg Glu Lys Thr Leu Pro Lys Arg Val Val Asn Thr Leu Phe
65 70 75 80

Gly Lys Gly Ala Ile His Thr Thr Lys Gly Lys Val His Val Asp Arg
85 90 95

Lys Ala Leu Phe Met Ser Leu Met Thr Glu Glu Asn Leu Lys Tyr Leu
100 105 110

Arg Asp Leu Thr Arg Asn Tyr Trp Phe Met His Thr Glu Asp Met Gln
115 120 125

Arg Gln Asp Glu Val Asn Ile Tyr Lys Thr Ser Ile Tyr Ile Leu Thr
130 135 140

Lys Ile Gly Phe Arg Trp Ala Gly Leu Gln Gln Thr Ser Glu Glu Ala
145 150 155 160

Lys Arg Asn Ala Arg Asp Met Asp Ile Met Ile Asp Ser Phe Gln Gly
165 170 175

Leu Gly Gln Thr Phe Gly Gly Gly Tyr Arg Lys Ala Lys Lys Ala Arg
180 185 190

Ala Arg Val Glu Lys Phe Leu Glu Asn Gln Ile Val Ala Val Arg Glu
195 200 205

Gly Lys Met Lys Ala Gln Pro Gly Thr Ala Leu Tyr Glu Phe Val His
210 215 220

Trp Lys Asp Tyr Lys Gly His Gln Met Asp Pro His Leu Cys Ala Val
225 230 235 240

Glu Leu Met Asn Val Val Arg Pro Leu Ala Ala Ile Asn Arg Phe Val
245 250 255

Ser Tyr Ala Val Lys Ala Met Leu Glu Phe Asp Gln Glu Arg Ile Lys
260 265 270

Leu Gln Val Ala Asp Asp Pro Asn Tyr Ala Tyr Lys Phe Ala Gln Glu
275 280 285

Val Arg Arg Ile Phe Pro Phe Val Pro Phe Leu Pro Gly Lys Leu Lys
290 295 300

Lys Asp Val Glu Phe Asp Gly Phe Lys Ile Lys Lys Gly Thr Phe Thr
305 310 315 320

Leu Leu Asp Ile Phe Gly Thr Thr His Asp Pro Glu Leu Phe Glu Asn
325 330 335

Pro Tyr Gln Phe Asn Pro Asp Arg Phe Glu Asn Trp Asp Gly Ser Pro
340 345 350

Phe Asp Leu Ile Pro Gln Gly Gly Gly Asp Phe Tyr Thr Asn His Arg
355 360 365

Cys Ala Gly Glu Trp Met Thr Ile Ile Ile Met Glu Glu Thr Ile Lys
370 375 380

Tyr Phe Ala Thr Lys Ile Asp Phe Asp Ala Pro Val Gln Asp Leu Ser
385 390 395 400

Val Lys Leu Asp Gln Phe Pro Gly Lys Val Thr Ser Gly Pro Val Ile
405 410 415

His Arg Val Arg Pro Arg Val Gln Arg
420 425

<210> 109
<211> 425
<212> PRT
<213> Staphylococcus chromogenes

<400> 109

```
Met Ala Lys Lys Leu Pro Lys Asp Ser Gly Leu Asp Asn Thr Phe Lys
1               5                   10                  15

Ile Leu Asn Glu Ala Tyr Thr Tyr Val Pro Cys Arg Leu Glu Lys Phe
            20                  25                  30

Gly Thr Lys Ala Phe Glu Thr Ala Ala Ile Gly Met Lys Pro Met Thr
        35                  40                  45

Val Ile Ser Gly Lys Glu Ala Ala Glu Leu Phe Tyr Asp Asn Glu Leu
    50                  55                  60

Met Gln Arg Glu Lys Thr Leu Pro Lys Arg Val Val Asn Thr Leu Phe
65                  70                  75                  80

Gly Lys Gly Ala Ile His Thr Thr Lys Gly Lys Val His Val Asp Arg
                85                  90                  95

Lys Ala Leu Phe Met Ser Leu Met Thr Glu Glu Asn Leu Lys Tyr Leu
            100                 105                 110

Arg Asp Leu Thr Arg Asn Tyr Trp Phe Met His Thr Glu Glu Met Gln
        115                 120                 125

Arg Gln Glu Glu Val Asn Ile Tyr Lys Thr Ser Ile Tyr Ile Leu Thr
    130                 135                 140

Lys Ile Gly Phe Arg Trp Ala Gly Leu Gln Gln Thr Ser Glu Glu Ala
145                 150                 155                 160

Lys Arg Asn Ala Arg Asp Met Asp Ile Met Ile Asp Ser Phe Gln Gly
                165                 170                 175

Leu Gly Gln Thr Phe Gly Gly Gly Tyr Arg Lys Ala Lys Lys Ala Arg
            180                 185                 190

Ala Arg Val Glu Lys Phe Leu Glu Asn Gln Ile Val Ala Val Arg Glu
        195                 200                 205

Gly Lys Met Lys Ala Gln Pro Gly Thr Ala Leu Tyr Glu Phe Ala His
    210                 215                 220
```

Trp Lys Asp Tyr Lys Gly His Gln Met Asp Pro His Leu Cys Ala Val
225 230 235 240

Glu Leu Met Asn Val Val Arg Pro Leu Ala Ala Ile Asn Arg Phe Val
245 250 255

Ser Tyr Ala Val Lys Ala Met Leu Glu Phe Asp Gln Glu Arg Ile Lys
260 265 270

Leu Gln Val Ala Asp Asp Leu Asn Tyr Ala Tyr Lys Phe Ala Gln Glu
275 280 285

Val Arg Arg Ile Phe Pro Phe Val Pro Phe Leu Pro Gly Lys Leu Lys
290 295 300

Lys Asp Val Glu Phe Asp Gly Phe Lys Ile Lys Lys Gly Thr Phe Thr
305 310 315 320

Leu Leu Asp Ile Phe Gly Thr Thr His Asp Pro Glu Leu Phe Glu Asn
325 330 335

Pro Tyr Gln Phe Asn Pro Asp Arg Phe Glu Asn Trp Asp Gly Ser Pro
340 345 350

Phe Asp Leu Ile Pro Gln Gly Gly Gly Asp Phe Tyr Thr Asn His Arg
355 360 365

Cys Ala Gly Glu Trp Met Thr Ile Ile Ile Met Glu Glu Thr Ile Lys
370 375 380

Tyr Phe Ala Thr Lys Ile Asp Phe Asp Ala Pro Val Gln Asp Leu Ser
385 390 395 400

Val Lys Leu Asp Gln Phe Pro Gly Lys Val Thr Ser Gly Pro Val Ile
405 410 415

His Arg Val Arg Pro Arg Val Gln Arg
420 425

<210> 110
<211> 425
<212> PRT
<213> Staphylococcus chromogenes

<400> 110

Met Ala Lys Lys Leu Pro Lys Asp Ser Gly Leu Asp Asn Thr Phe Lys
1 5 10 15

Ile Leu Asn Glu Ala Tyr Thr Tyr Val Pro Cys Arg Leu Glu Lys Phe
20 25 30

Gly Thr Lys Ala Phe Glu Thr Ala Ala Leu Gly Met Lys Pro Met Thr
35 40 45

Val Ile Ser Gly Lys Glu Ala Ala Glu Leu Phe Tyr Asn Asn Glu Leu
50 55 60

Met Gln Arg Glu Lys Thr Leu Pro Lys Arg Val Val Asn Thr Leu Phe
65 70 75 80

Gly Lys Gly Ala Ile His Thr Thr Lys Gly Lys Val His Val Asp Arg
85 90 95

Lys Ala Leu Phe Met Ser Leu Met Thr Glu Glu Asn Leu Lys Tyr Leu
100 105 110

Arg Asp Leu Thr Arg Asn Tyr Trp Phe Met His Thr Glu Glu Met Gln
115 120 125

Arg Gln Asp Glu Val Asn Ile Tyr Lys Thr Ser Ile Tyr Ile Leu Thr
130 135 140

Lys Ile Gly Phe Arg Trp Ala Gly Leu Gln Gln Thr Ser Glu Glu Ala
145 150 155 160

Lys Arg Asn Ala Arg Asp Met Asp Ile Met Ile Asp Ser Phe Gln Gly
165 170 175

Leu Gly Gln Thr Phe Gly Gly Gly Tyr Arg Lys Ala Lys Lys Ala Arg
180 185 190

Ala Arg Val Glu Lys Phe Leu Glu Asn Gln Ile Val Ala Val Arg Glu
195 200 205

Gly Lys Met Lys Ala Gln Pro Gly Thr Ala Leu Tyr Glu Phe Ala His
210 215 220

Trp Lys Asp Tyr Lys Gly His Gln Met Asp Pro His Leu Cys Ala Val
225 230 235 240

Glu Leu Met Asn Val Val Arg Pro Leu Ala Ala Val Asn Arg Phe Val
245 250 255

Ser Tyr Ala Val Lys Ala Met Leu Glu Phe Asp Gln Glu Arg Ile Lys
260 265 270

Leu Gln Val Ala Asp Asp Pro Asn Tyr Ala Tyr Lys Phe Ala Gln Glu
        275                 280                 285

Val Arg Arg Ile Phe Pro Phe Val Pro Phe Leu Pro Gly Lys Leu Lys
    290                 295                 300

Lys Asp Val Glu Phe Asp Gly Phe Lys Ile Lys Lys Gly Thr Phe Thr
305                 310                 315                 320

Leu Leu Asp Ile Phe Gly Thr Thr His Asp Pro Glu Leu Phe Glu Asn
                325                 330                 335

Pro Tyr Gln Phe Asn Pro Asp Arg Phe Glu Asn Trp Asp Gly Ser Pro
            340                 345                 350

Phe Asp Leu Ile Pro Gln Gly Gly Gly Asp Phe Tyr Thr Asn His Arg
        355                 360                 365

Cys Ala Gly Glu Trp Met Thr Ile Ile Ile Met Glu Glu Thr Ile Lys
    370                 375                 380

Tyr Phe Ala Thr Lys Ile Asp Phe Asp Ala Pro Val Gln Asp Leu Ser
385                 390                 395                 400

Val Lys Leu Asp Gln Phe Pro Gly Lys Val Thr Ser Gly Pro Val Ile
                405                 410                 415

His Arg Val Arg Pro Arg Val Gln Arg
            420                 425

<210> 111
<211> 425
<212> PRT
<213> Staphylococcus chromogenes

<400> 111

Met Ala Lys Lys Leu Pro Lys Asp Ser Gly Leu Asp Asn Thr Phe Lys
1               5                   10                  15

Ile Leu Asn Glu Ala Tyr Thr Tyr Val Pro Cys Arg Leu Glu Lys Phe
            20                  25                  30

Gly Thr Lys Ala Phe Glu Thr Ala Ala Ile Gly Met Lys Pro Met Thr
        35                  40                  45

Val Ile Ser Gly Lys Glu Ala Ala Glu Leu Phe Tyr Asp Asn Glu Leu
    50                  55                  60

Met Gln Arg Glu Lys Thr Leu Pro Lys Arg Val Val Asn Thr Leu Phe
65                  70                  75                  80

Gly Lys Gly Ala Ile His Thr Thr Lys Gly Lys Val His Val Asp Arg
                85                  90                  95

Lys Ala Leu Phe Met Ser Leu Met Thr Glu Glu Asn Leu Lys Tyr Leu
            100                 105                 110

Arg Asp Leu Thr Arg Asn Cys Trp Phe Met His Thr Glu Glu Met Gln
        115                 120                 125

Arg Gln Asp Glu Val Asn Ile Tyr Lys Thr Ser Ile Tyr Ile Leu Thr
    130                 135                 140

Lys Ile Gly Phe Arg Trp Ala Gly Leu Gln Gln Thr Ser Glu Glu Ala
145                 150                 155                 160

Lys Arg Asn Ala Arg Asp Met Asp Ile Met Ile Asp Ser Phe Gln Gly
                165                 170                 175

Leu Gly Gln Thr Phe Gly Gly Gly Tyr Arg Lys Ala Lys Lys Ala Arg
            180                 185                 190

Ala Arg Val Glu Lys Phe Leu Glu Asn Gln Ile Val Ala Val Arg Glu
        195                 200                 205

Gly Lys Met Lys Ala Gln Pro Gly Thr Ala Leu Tyr Glu Phe Ala His
    210                 215                 220

Trp Lys Asp Tyr Lys Gly His Gln Met Asp Pro His Leu Cys Ala Val
225                 230                 235                 240

Glu Leu Met Asn Val Val Arg Pro Leu Ala Ala Ile Asn Arg Phe Val
                245                 250                 255

Ser Tyr Ala Val Lys Ala Met Leu Glu Phe Asp Gln Glu Arg Ile Lys
            260                 265                 270

Leu Gln Val Ala Asp Asp Pro Asn Tyr Ala Tyr Lys Phe Ala Gln Glu
        275                 280                 285

Val Arg Arg Ile Phe Pro Phe Val Pro Phe Leu Pro Gly Lys Leu Lys
    290                 295                 300

Lys Asp Val Glu Phe Asp Gly Phe Lys Ile Lys Lys Gly Thr Phe Thr

305 310 315 320

Leu Leu Asp Ile Phe Gly Thr Thr His Asp Pro Glu Leu Phe Glu Asn
325 330 335

Pro Tyr Gln Phe Asn Pro Asp Arg Phe Glu Asn Trp Asp Gly Ser Pro
340 345 350

Phe Asp Leu Ile Pro Gln Gly Gly Gly Asp Phe Tyr Thr Asn His Arg
355 360 365

Cys Ala Gly Glu Trp Met Thr Ile Ile Ile Met Glu Glu Thr Ile Lys
370 375 380

Tyr Phe Ala Thr Lys Ile Asp Phe Asp Ala Pro Val Gln Asp Leu Ser
385 390 395 400

Val Lys Leu Asp Gln Phe Pro Gly Lys Val Thr Ser Gly Pro Val Ile
405 410 415

His Arg Val Arg Pro Arg Val Gln Arg
420 425

<210> 112
<211> 425
<212> PRT
<213> Staphylococcus chromogenes

<400> 112

Met Ala Lys Lys Leu Pro Lys Asp Ser Gly Leu Asp Asn Thr Phe Lys
1 5 10 15

Ile Leu Asn Glu Ala Tyr Thr Tyr Val Pro Cys Arg Leu Glu Lys Phe
20 25 30

Gly Thr Lys Ala Phe Glu Thr Ala Ala Ile Gly Met Lys Pro Met Thr
35 40 45

Val Ile Ser Gly Lys Glu Ala Ala Glu Leu Phe Tyr Asp Asn Glu Leu
50 55 60

Met Gln Arg Glu Lys Thr Leu Pro Lys Arg Val Val Asn Thr Leu Phe
65 70 75 80

Gly Lys Gly Ala Ile His Thr Thr Lys Gly Lys Val His Val Asp Arg
85 90 95

Lys Ala Leu Phe Met Ser Leu Met Thr Glu Glu Asn Leu Lys Tyr Leu

100 105 110

Arg Asp Leu Thr Arg Asn Tyr Trp Phe Met His Thr Glu Glu Met Gln
115 120 125

Arg Gln Asp Glu Val Asn Ile Tyr Lys Thr Ser Ile Tyr Ile Leu Thr
130 135 140

Lys Ile Gly Phe Arg Trp Ala Gly Leu Gln Gln Thr Ser Glu Glu Ala
145 150 155 160

Lys Arg Asn Ala Arg Asp Met Asp Ile Met Ile Asp Ser Phe Gln Gly
165 170 175

Leu Gly Gln Thr Phe Gly Gly Gly Tyr Arg Lys Ala Lys Lys Ala Arg
180 185 190

Ala Arg Val Glu Lys Phe Leu Glu Asn Gln Ile Val Ala Val Arg Glu
195 200 205

Gly Lys Met Lys Ala Gln Pro Gly Thr Ala Leu Tyr Glu Phe Ala His
210 215 220

Trp Lys Asp Tyr Lys Gly His Gln Val Asp Pro His Leu Cys Ala Val
225 230 235 240

Glu Leu Met Asn Val Val Arg Pro Leu Ala Ala Ile Asn Arg Phe Val
245 250 255

Ser Tyr Ala Val Lys Ala Met Leu Glu Phe Asp Gln Glu Arg Ile Lys
260 265 270

Leu Gln Val Ala Asp Asp Pro Asn Tyr Ala Tyr Lys Phe Ala Gln Glu
275 280 285

Val Arg Arg Ile Phe Pro Phe Val Pro Phe Leu Pro Gly Lys Leu Lys
290 295 300

Lys Asp Val Glu Phe Asp Gly Phe Lys Ile Lys Lys Gly Thr Phe Thr
305 310 315 320

Leu Leu Asp Ile Phe Gly Thr Thr His Asp Pro Glu Leu Phe Glu Asn
325 330 335

Pro Tyr Gln Phe Asn Pro Asp Arg Phe Glu Asn Trp Asp Gly Ser Pro
340 345 350

Phe Asp Leu Ile Pro Gln Gly Gly Gly Asp Phe Tyr Thr Asn His Arg
355 360 365

Cys Ala Gly Glu Trp Met Thr Ile Ile Ile Met Glu Glu Thr Ile Lys
370 375 380

Tyr Phe Ala Thr Lys Ile Asp Phe Asp Ala Pro Val Gln Asp Leu Ser
385 390 395 400

Val Lys Leu Asp Gln Phe Pro Gly Lys Val Thr Ser Gly Pro Val Ile
405 410 415

His Arg Val Arg Pro Arg Val Gln Arg
420 425

<210> 113
<211> 425
<212> PRT
<213> Staphylococcus chromogenes

<400> 113

Met Ala Lys Lys Leu Pro Lys Asp Ser Gly Leu Asp Asn Thr Phe Lys
1 5 10 15

Ile Leu Asn Glu Ala Tyr Thr Tyr Val Pro Cys Arg Leu Glu Lys Phe
20 25 30

Gly Thr Lys Ala Phe Glu Thr Ala Ala Ile Gly Met Lys Pro Met Thr
35 40 45

Val Ile Ser Gly Lys Glu Ala Ala Glu Leu Phe Tyr Asp Asn Glu Leu
50 55 60

Met Gln Arg Glu Lys Thr Leu Pro Lys Arg Val Val Asn Thr Leu Phe
65 70 75 80

Gly Lys Gly Ala Ile His Thr Thr Lys Gly Lys Val His Val Asp Arg
85 90 95

Lys Ala Leu Phe Met Ser Leu Ile Thr Glu Glu Asn Leu Lys Tyr Leu
100 105 110

Arg Asp Leu Thr Arg Asn Tyr Trp Phe Met His Thr Glu Asp Met Gln
115 120 125

Arg Gln Asp Glu Val Asn Ile Tyr Lys Thr Ser Ile Tyr Ile Leu Thr
130 135 140

```
Lys Ile Gly Phe Arg Trp Ala Gly Leu Gln Gln Thr Ser Glu Glu Ala
145                 150                 155                 160

Lys Arg Asn Ala Arg Asp Met Asp Ile Met Ile Asp Ser Phe Gln Gly
                165                 170                 175

Leu Gly Gln Thr Phe Gly Gly Gly Tyr Arg Lys Ala Lys Lys Ala Arg
            180                 185                 190

Ala Arg Val Glu Lys Phe Leu Glu Asn Gln Ile Val Ala Val Arg Glu
        195                 200                 205

Gly Lys Met Lys Ala Gln Pro Gly Thr Ala Leu Tyr Glu Phe Val His
    210                 215                 220

Trp Lys Asp Tyr Lys Gly His Gln Met Asp Pro His Leu Cys Ala Val
225                 230                 235                 240

Glu Leu Met Asn Val Val Arg Pro Leu Ala Ala Ile Asn Arg Phe Val
                245                 250                 255

Ser Tyr Ala Val Lys Ala Met Leu Glu Phe Asp Gln Glu Arg Ile Lys
            260                 265                 270

Leu Gln Val Ala Asp Asp Pro Asn Tyr Ala Tyr Lys Phe Ala Gln Glu
        275                 280                 285

Val Arg Arg Ile Phe Pro Phe Val Pro Phe Leu Pro Gly Lys Leu Lys
    290                 295                 300

Lys Asp Val Glu Phe Asp Gly Phe Lys Ile Lys Lys Gly Thr Phe Thr
305                 310                 315                 320

Leu Leu Asp Ile Phe Gly Thr Thr His Asp Pro Glu Leu Phe Glu Asn
                325                 330                 335

Pro Tyr Gln Phe Asn Pro Asp Arg Phe Glu Asn Trp Asp Gly Ser Pro
            340                 345                 350

Phe Asp Leu Ile Pro Gln Gly Gly Gly Asp Phe Tyr Thr Asn His Arg
        355                 360                 365

Cys Ala Gly Glu Trp Met Thr Ile Ile Ile Met Glu Glu Thr Ile Lys
    370                 375                 380

Tyr Phe Ala Thr Lys Ile Asp Phe Asp Ala Pro Val Gln Asp Leu Ser
385                 390                 395                 400
```

Val Lys Leu Asp Gln Phe Pro Gly Lys Val Thr Ser Gly Pro Val Ile
405 410 415

His Arg Val Arg Pro Arg Val Gln Arg
420 425

<210> 114
<211> 422
<212> PRT
<213> Nosocomiicoccus sp.

<400> 114

Met Ala Lys Ile Lys Arg Asp Lys Gly Leu Asp Asn Thr Leu Lys Val
1 5 10 15

Phe Lys Gln Gly Tyr Leu Tyr Thr Thr Asn Gln Arg Glu Arg Leu Gly
20 25 30

Ala Glu Val Phe Glu Thr Arg Ala Leu Gly Gly Lys Ser Tyr Val Val
35 40 45

Leu Ser Gly Lys Glu Gly Ala Glu Leu Phe Tyr Asp Asn Asp Lys Ile
50 55 60

Glu Arg Glu Gly Gly Leu Pro Lys Arg Val Val Asn Thr Leu Phe Gly
65 70 75 80

Lys Gly Ala Ile His Thr Thr Thr Gly Lys Gln His Ile Asp Arg Lys
85 90 95

Ala Leu Phe Met Ser Leu Met Thr Glu Gly Asn Leu Glu Tyr Val Arg
100 105 110

Lys Leu Thr Arg Asn Tyr Trp Lys Ala Asn Thr Ala Arg Met Glu Ala
115 120 125

Met Gly Asp Val Asn Val Tyr His Glu Ser Ile Val Leu Leu Thr Arg
130 135 140

Ile Gly Met Arg Trp Ala Gly Val Thr Ala Pro Glu Glu Glu Ile Glu
145 150 155 160

Arg Ile Ala Glu Asp Met Asp Ile Met Ile Asp Ser Phe Lys Gly Leu
165 170 175

Gly Asn Ala Phe Lys Gly Tyr Lys Ser Ser Lys Asp Ala Arg Lys Arg
180 185 190

Val Glu Asp Trp Leu Glu Asp Gln Ile Ile Lys Thr Arg Asn Gly Glu
195 200 205

Ile Asn Pro Pro Lys Gly Ser Ser Leu Tyr Glu Leu Ala His Trp Arg
210 215 220

Asp Tyr Glu Gly Asn Gln Met Asp Ser Arg Leu Ala Gly Ile Asp Leu
225 230 235 240

Met Asn Thr Phe Arg Pro Leu Ile Ala Ile Asn Arg Phe Val Ser Tyr
245 250 255

Gly Val Leu Ala Leu Tyr Glu His Pro Glu Ala Ile Arg Lys Met Ser
260 265 270

Thr Val Glu Asp Tyr Pro Tyr Met Phe Ala Gln Glu Val Arg Arg Phe
275 280 285

Tyr Pro Phe Val Pro Phe Leu Pro Gly Lys Thr Lys Val Glu Val Glu
290 295 300

His Lys Gly Val Thr Ile Pro Lys Asp Gln Arg Leu Val Ile Asp Val
305 310 315 320

Tyr Gly Thr Leu His Ser Glu Glu Leu Trp Asp Gln Pro Asn Lys Phe
325 330 335

Ile Pro Glu Arg Phe Arg Asp Trp Asp Gly Ser Pro Phe Asp Met Ile
340 345 350

Pro Gln Gly Gly Gly Asp Tyr Trp Thr Asn His Arg Cys Ala Gly Glu
355 360 365

Trp Ile Thr Ile Ile Ile Met Glu Glu Thr Met Lys Tyr Phe Ala Asn
370 375 380

Glu Ile Thr Trp Asp Val Pro Glu Gln Asp Leu Thr Ile Asp Leu Asn
385 390 395 400

Ser Ile Pro Gly Tyr Ile Asn Ser Gly Met Val Ile Asn Asn Val Arg
405 410 415

Glu Lys Val Asp Arg Phe
420

<210> 115
<211> 422

<212> PRT
<213> Nosocomiicoccus sp.

<400> 115

Met Ala Lys Ile Lys Arg Asp Lys Gly Leu Asp Asn Thr Leu Lys Val
1               5                   10                  15

Phe Lys Gln Gly Tyr Leu Tyr Thr Thr Asn Gln Arg Glu Arg Leu Gly
            20                  25                  30

Ala Glu Val Phe Glu Thr Arg Ala Leu Gly Gly Lys Ser Tyr Val Val
        35                  40                  45

Leu Ser Gly Lys Glu Gly Ala Glu Leu Phe Tyr Asp Asn Asp Lys Ile
    50                  55                  60

Glu Arg Glu Gly Gly Leu Pro Lys Arg Val Val Asn Thr Leu Phe Gly
65                  70                  75                  80

Lys Gly Ala Ile His Thr Thr Thr Gly Lys Gln His Ile Asp Arg Lys
                85                  90                  95

Ala Leu Phe Met Ser Leu Met Thr Glu Gly Asn Leu Glu Tyr Val Arg
            100                 105                 110

Lys Leu Thr Arg Asn Tyr Trp Lys Ala Asn Thr Ala Arg Met Glu Ala
        115                 120                 125

Met Gly Asp Val Asn Val Tyr His Glu Ser Ile Val Leu Leu Thr Arg
    130                 135                 140

Ile Gly Met Arg Trp Ala Gly Val Thr Ala Pro Glu Glu Glu Ile Glu
145                 150                 155                 160

Arg Ile Ala Glu Asp Met Asp Ile Met Ile Asp Ser Phe Lys Gly Leu
                165                 170                 175

Gly Asn Ala Phe Lys Gly Tyr Lys Ser Ser Lys Asp Ala Arg Lys Arg
            180                 185                 190

Val Glu Asp Trp Leu Glu Asp Gln Ile Ile Lys Thr Arg Asn Gly Glu
        195                 200                 205

Ile Asn Pro Pro Lys Gly Ser Ser Leu Tyr Glu Leu Ala His Trp Arg
    210                 215                 220

Asp Tyr Glu Gly Asn Gln Met Asp Ser Arg Leu Ala Gly Ile Asp Leu
225                 230                 235                 240

Met Asn Thr Phe Arg Pro Leu Ile Ala Ile Asn Arg Phe Val Ser Tyr
245 250 255

Gly Val Leu Ala Leu Tyr Glu His Pro Glu Ala Ile Arg Lys Met Ser
260 265 270

Thr Val Glu Asp Tyr Pro Tyr Met Phe Ala Gln Glu Val Arg Arg Phe
275 280 285

Tyr Pro Phe Val Pro Phe Leu Pro Gly Lys Thr Lys Val Glu Val Glu
290 295 300

His Lys Gly Val Thr Ile Pro Lys Asp Gln Arg Leu Val Ile Asp Val
305 310 315 320

Tyr Gly Thr Leu His Ser Glu Glu Leu Trp Asp Gln Pro Asn Lys Phe
325 330 335

Ile Pro Glu Arg Phe Lys Asp Trp Asp Gly Ser Pro Phe Asp Met Ile
340 345 350

Pro Gln Gly Gly Gly Asp Tyr Trp Thr Asn His Arg Cys Ala Gly Glu
355 360 365

Trp Ile Thr Ile Ile Ile Met Glu Glu Thr Met Lys Tyr Phe Ala Asn
370 375 380

Glu Ile Thr Trp Asp Val Pro Glu Gln Asp Leu Thr Ile Asp Leu Asn
385 390 395 400

Ser Ile Pro Gly Tyr Ile Asn Ser Gly Met Val Ile Asn Asn Val Arg
405 410 415

Glu Lys Val Asp Arg Phe
420

<210> 116
<211> 423
<212> PRT
<213> Nosocomiicoccus massiliensis

<400> 116

Met Met Ala Lys Ile Lys Arg Asp Lys Gly Leu Asp Asn Thr Leu Arg
1 5 10 15

Val Phe Lys Gln Gly Tyr Leu Tyr Thr Thr Asn Gln Arg Glu Arg Leu
20 25 30

```
Gly Ala Glu Val Phe Glu Thr Arg Ala Leu Gly Gly Lys Ser Tyr Val
        35                  40                  45

Val Leu Ser Gly Lys Glu Gly Ala Glu Leu Phe Tyr Asp Asn Asp Lys
    50                  55                  60

Ile Glu Arg Glu Gly Gly Leu Pro Arg Arg Val Val Asn Thr Leu Phe
65                  70                  75                  80

Gly Lys Gly Ala Ile His Thr Thr Thr Gly Lys Gln His Ile Asp Arg
                85                  90                  95

Lys Ala Leu Phe Met Ser Leu Met Thr Glu Gly Asn Leu Glu Tyr Val
            100                 105                 110

Arg Lys Leu Thr Arg Asn Tyr Trp Lys Ala Asn Thr Ala Arg Met Glu
        115                 120                 125

Ala Met Gly Asp Val Asn Val Tyr His Glu Ser Ile Val Leu Leu Thr
    130                 135                 140

Arg Ile Gly Met Arg Trp Ala Gly Val Thr Ala Pro Glu Glu Glu Ile
145                 150                 155                 160

Glu Arg Ile Ala Glu Asp Met Asp Ile Met Ile Asp Ser Phe Lys Gly
                165                 170                 175

Leu Gly Asn Ala Phe Lys Gly Tyr Lys Ser Ser Lys Asp Ala Arg Lys
            180                 185                 190

Arg Val Glu Asp Trp Leu Glu Asp Gln Ile Ile Lys Thr Arg Asn Gly
        195                 200                 205

Glu Ile Asn Pro Pro Lys Gly Ser Ser Leu Tyr Glu Phe Ala His Trp
    210                 215                 220

Arg Asp Tyr Glu Gly Asn His Met Asp Ser Arg Leu Ala Gly Ile Asp
225                 230                 235                 240

Leu Met Asn Thr Phe Arg Pro Leu Ile Ala Ile Asn Arg Phe Val Ser
                245                 250                 255

Tyr Gly Val Leu Ala Leu Tyr Glu Asn Pro Glu Ala Ile Arg Lys Met
            260                 265                 270

Ser Thr Val Glu Asp Tyr Pro Tyr Met Phe Ala Gln Glu Val Arg Arg
```

275 280 285

Phe Tyr Pro Phe Val Pro Phe Leu Pro Gly Lys Thr Lys Val Glu Val
290 295 300

Glu His Lys Gly Val Thr Ile Pro Lys Asp Gln Arg Leu Val Ile Asp
305 310 315 320

Val Tyr Gly Thr Leu His Ser Glu Glu Leu Trp Asp Gln Pro Asn Lys
325 330 335

Phe Ile Pro Glu Arg Phe Lys Asp Trp Asp Gly Ser Pro Phe Asp Met
340 345 350

Ile Pro Gln Gly Gly Gly Asp Tyr Trp Thr Asn His Arg Cys Ala Gly
355 360 365

Glu Trp Ile Thr Ile Ile Ile Met Glu Glu Thr Met Lys Tyr Phe Ala
370 375 380

Asn Glu Ile Thr Trp Asp Val Pro Glu Gln Asp Leu Thr Ile Asp Leu
385 390 395 400

Asn Ser Ile Pro Gly Tyr Ile Asn Ser Gly Met Val Ile Asn Asn Val
405 410 415

Arg Glu Lys Val Asp Arg Phe
420

<210> 117
<211> 422
<212> PRT
<213> Nosocomiicoccus massiliensis

<400> 117

Met Ala Lys Ile Lys Arg Asp Lys Gly Leu Asp Asn Thr Leu Lys Val
1 5 10 15

Phe Lys Gln Gly Tyr Leu Tyr Thr Thr Asn Gln Arg Glu Arg Leu Gly
20 25 30

Ala Glu Val Phe Glu Thr Arg Ala Leu Gly Gly Lys Ser Tyr Val Val
35 40 45

Leu Ser Gly Lys Glu Gly Ala Glu Leu Phe Tyr Asp Asn Asp Lys Ile
50 55 60

Glu Arg Glu Gly Gly Leu Pro Lys Arg Val Val Asn Thr Leu Phe Gly

65 70 75 80

Lys Gly Ala Ile His Thr Thr Thr Gly Lys Gln His Ile Asp Arg Lys
85 90 95

Ala Leu Phe Met Ser Leu Met Thr Glu Gly Asn Leu Glu Tyr Val Arg
100 105 110

Lys Leu Thr Arg Asn Tyr Trp Lys Ala Asn Thr Ala Arg Met Glu Ala
115 120 125

Met Gly Asp Val Asn Val Tyr His Glu Ser Ile Val Leu Leu Thr Arg
130 135 140

Ile Gly Met Arg Trp Ala Gly Val Thr Ala Pro Glu Glu Glu Ile Glu
145 150 155 160

Arg Ile Ala Glu Asp Met Asp Ile Met Ile Asp Ser Phe Lys Gly Leu
165 170 175

Gly Asn Ala Phe Lys Gly Tyr Lys Ser Ser Lys Asp Ala Arg Lys Arg
180 185 190

Val Glu Asp Trp Leu Glu Asp Gln Ile Ile Lys Thr Arg Asn Gly Glu
195 200 205

Ile Asn Pro Pro Lys Gly Ser Ser Leu Tyr Glu Leu Ala His Trp Arg
210 215 220

Asp Tyr Glu Gly Asn Gln Met Asp Ser Arg Leu Ala Gly Ile Asp Leu
225 230 235 240

Met Asn Thr Phe Arg Pro Leu Ile Ala Ile Asn Arg Phe Val Ser Tyr
245 250 255

Gly Val Leu Ala Leu Tyr Glu His Pro Glu Ala Ile Arg Lys Met Ser
260 265 270

Thr Val Glu Asp Tyr Pro Tyr Met Phe Ala Gln Glu Val Arg Arg Phe
275 280 285

Tyr Pro Phe Val Pro Phe Leu Pro Gly Lys Thr Lys Val Glu Val Glu
290 295 300

His Lys Gly Val Thr Ile Pro Lys Asp Gln Arg Leu Val Ile Asp Val
305 310 315 320

Tyr Gly Thr Leu His Ser Glu Glu Leu Trp Asn Gln Pro Asn Lys Phe
325 330 335

Ile Pro Glu Arg Phe Lys Asp Trp Asp Gly Ser Pro Phe Asp Met Ile
340 345 350

Pro Gln Gly Gly Gly Asp Tyr Trp Thr Asn His Arg Cys Ala Gly Glu
355 360 365

Trp Ile Thr Ile Ile Ile Met Glu Glu Thr Met Lys Tyr Phe Ala Asn
370 375 380

Glu Ile Thr Trp Asp Val Pro Glu Gln Asp Leu Thr Ile Asp Leu Asn
385 390 395 400

Ser Ile Pro Gly Tyr Ile Asn Ser Gly Met Val Ile Asn Asn Val Arg
405 410 415

Glu Lys Val Asp Arg Phe
420

<210> 118
<211> 422
<212> PRT
<213> Nosocomiicoccus massiliensis

<400> 118

Met Ala Lys Ile Lys Arg Asp Lys Gly Leu Asp Asn Thr Leu Arg Val
1 5 10 15

Phe Lys Gln Gly Tyr Leu Tyr Thr Thr Asn Gln Arg Glu Arg Leu Gly
20 25 30

Ala Glu Val Phe Glu Thr Arg Ala Leu Gly Gly Lys Ser Tyr Val Val
35 40 45

Leu Ser Gly Lys Glu Gly Ala Glu Leu Phe Tyr Asp Asn Asp Lys Ile
50 55 60

Glu Arg Glu Gly Gly Leu Pro Arg Arg Val Val Asn Thr Leu Phe Gly
65 70 75 80

Lys Gly Ala Ile His Thr Thr Thr Gly Lys Gln His Ile Asp Arg Lys
85 90 95

Ala Leu Phe Met Ser Leu Met Thr Glu Gly Asn Leu Glu Tyr Val Arg
100 105 110

```
Lys Leu Thr Arg Asn Tyr Trp Lys Ala Asn Thr Ala Arg Met Glu Ala
        115                 120                 125

Met Gly Asp Val Asn Val Tyr His Glu Ser Ile Val Leu Leu Thr Arg
    130                 135                 140

Ile Gly Met Arg Trp Ala Gly Val Thr Ala Pro Glu Glu Glu Ile Glu
145                 150                 155                 160

Arg Ile Ala Glu Asp Met Asp Ile Met Ile Asp Ser Phe Lys Gly Leu
                165                 170                 175

Gly Asn Ala Phe Lys Gly Tyr Lys Ser Ser Lys Asp Ala Arg Lys Arg
            180                 185                 190

Val Glu Asp Trp Leu Glu Asp Gln Ile Ile Lys Thr Arg Asn Gly Glu
        195                 200                 205

Ile Asn Pro Pro Lys Gly Ser Ser Leu Tyr Glu Phe Ala His Trp Arg
    210                 215                 220

Asp Tyr Glu Gly Asn His Met Asp Ser Arg Leu Ala Gly Ile Asp Leu
225                 230                 235                 240

Met Asn Thr Phe Arg Pro Leu Ile Ala Ile Asn Arg Phe Val Ser Tyr
                245                 250                 255

Gly Val Leu Ala Leu Tyr Glu Asn Pro Glu Ala Ile Arg Lys Met Ser
            260                 265                 270

Thr Val Glu Asp Tyr Pro Tyr Met Phe Ala Gln Glu Val Arg Arg Phe
        275                 280                 285

Tyr Pro Phe Val Pro Phe Leu Pro Gly Lys Thr Lys Val Glu Val Glu
    290                 295                 300

His Lys Gly Val Thr Ile Pro Lys Asp Gln Arg Leu Val Ile Asp Val
305                 310                 315                 320

Tyr Gly Thr Leu His Ser Glu Glu Leu Trp Asp Gln Pro Asn Lys Phe
                325                 330                 335

Ile Pro Glu Arg Phe Lys Asp Trp Asp Gly Ser Pro Phe Asp Met Ile
            340                 345                 350

Pro Gln Gly Gly Gly Asp Tyr Trp Thr Asn His Arg Cys Ala Gly Glu
        355                 360                 365
```

Trp Ile Thr Ile Ile Ile Met Glu Glu Thr Met Lys Tyr Phe Ala Asn
    370                 375                 380

Glu Ile Thr Trp Asp Val Pro Glu Gln Asp Leu Thr Ile Asp Leu Asn
385                 390                 395                 400

Ser Ile Pro Gly Tyr Ile Asn Ser Gly Met Val Ile Asn Asn Val Arg
                405                 410                 415

Glu Lys Val Asp Arg Phe
            420

<210>  119
<211>  422
<212>  PRT
<213>  Nosocomiicoccus sp.

<400>  119

Met Ala Lys Ile Lys Arg Asp Lys Gly Leu Asp Asn Thr Leu Lys Val
1               5                   10                  15

Phe Lys Gln Gly Tyr Leu Tyr Thr Thr Asn Gln Arg Glu Arg Leu Gly
            20                  25                  30

Ala Glu Val Phe Glu Thr Arg Ala Leu Gly Gly Lys Ser Tyr Val Val
        35                  40                  45

Leu Ser Gly Lys Glu Gly Ala Glu Leu Phe Tyr Asp Asn Asp Lys Ile
    50                  55                  60

Glu Arg Glu Gly Gly Leu Pro Lys Arg Val Val Asn Thr Leu Phe Gly
65                  70                  75                  80

Lys Gly Ala Ile His Thr Thr Thr Gly Lys Gln His Ile Asp Arg Lys
                85                  90                  95

Ala Leu Phe Met Ser Leu Met Thr Glu Gly Asn Leu Glu Tyr Val Arg
            100                 105                 110

Lys Leu Thr Arg Asn Tyr Trp Lys Ala Asn Thr Ala Arg Met Glu Ala
        115                 120                 125

Met Gly Asp Val Asn Val Tyr Gln Glu Ser Ile Val Leu Leu Thr Arg
    130                 135                 140

Ile Gly Met Arg Trp Ala Gly Val Thr Ala Pro Glu Glu Glu Ile Glu
145                 150                 155                 160

Arg Ile Ala Glu Asp Met Asp Ile Met Ile Asp Ser Phe Lys Gly Leu
165 170 175

Gly Asn Ala Phe Lys Gly Tyr Lys Ser Ser Lys Asp Ala Arg Lys Arg
180 185 190

Val Glu Asp Trp Leu Glu Asp Gln Ile Ile Lys Thr Arg Asn Gly Glu
195 200 205

Ile Asn Pro Pro Lys Gly Ser Ser Leu Tyr Glu Leu Ala His Trp Arg
210 215 220

Asp Tyr Glu Gly Asn Gln Met Asp Ser Arg Leu Ala Gly Ile Asp Leu
225 230 235 240

Met Asn Thr Phe Arg Pro Leu Ile Ala Ile Asn Arg Phe Val Ser Tyr
245 250 255

Gly Val Leu Ala Leu Tyr Glu His Arg Glu Ala Ile Arg Lys Met Ser
260 265 270

Thr Val Glu Asp Tyr Pro Tyr Met Phe Ala Gln Glu Val Arg Arg Phe
275 280 285

Tyr Pro Phe Val Pro Phe Leu Pro Gly Lys Thr Lys Val Glu Val Glu
290 295 300

His Lys Gly Val Thr Ile Pro Lys Asp Gln Arg Leu Val Ile Asp Val
305 310 315 320

Tyr Gly Thr Leu His Ser Glu Glu Leu Trp Asp Gln Pro Asn Lys Phe
325 330 335

Ile Pro Glu Arg Phe Arg Asp Trp Asp Gly Ser Pro Phe Asp Met Ile
340 345 350

Pro Gln Gly Gly Gly Asp Tyr Trp Thr Asn His Arg Cys Ala Gly Glu
355 360 365

Trp Ile Thr Ile Ile Ile Met Glu Glu Thr Met Lys Tyr Phe Ala Asn
370 375 380

Glu Ile Thr Trp Asp Val Pro Glu Gln Asp Leu Thr Ile Asp Leu Asn
385 390 395 400

Ser Ile Pro Gly Tyr Ile Asn Ser Gly Met Val Ile Asn Asn Val Arg
405 410 415

Glu Lys Val Asp Arg Phe
420

<210> 120
<211> 347
<212> PRT
<213> Salinicoccus sediminis

<400> 120

Met Gly Thr Ile Lys Arg Asp Lys Gly Leu Asp Asn Thr Leu Lys Val
1 5 10 15

Met Lys Gln Gly Tyr Leu Tyr Ile Thr Asn Gln Arg Lys Arg Leu Gly
20 25 30

Ser Glu Asn Ile Phe Glu Thr Arg Ala Leu Gly Gly Lys Lys Ile Ile
35 40 45

Val Ile Ser Gly Lys Asp Ala Ala Glu Leu Phe Tyr Asp Asn Asp Lys
50 55 60

Ile Glu Arg Ser Gly Thr Leu Pro Lys Arg Val Val Asn Thr Leu Phe
65 70 75 80

Gly Lys Gly Ala Ile His Thr Thr Thr Gly Lys Lys His Ile Asp Arg
85 90 95

Lys Ala Leu Phe Met Ser Leu Met Thr Glu Gly Asn Leu Lys His Leu
100 105 110

Arg Glu Leu Thr Arg Asn His Trp Tyr Met Asn Thr Gln Lys Met Glu
115 120 125

Gln Met Asp Gln Ile Asn Ile Tyr Arg Glu Ser Ile Val Gln Leu Thr
130 135 140

Lys Ile Gly Thr Lys Trp Ala Gly Val Gln Ala Pro Glu Glu Lys Ile
145 150 155 160

Glu Glu Ile Ala Thr Asp Met Asp Val Met Ile Asp Ser Phe Lys Gly
165 170 175

Leu Gly Gly Lys Phe Lys Gly Tyr Lys Ala Ser Val Asp Ala Arg Lys
180 185 190

Arg Val Glu Asp Trp Leu Glu Asp Gln Ile Ile Gln Thr Arg Lys Gly
195 200 205

Lys Ile Phe Pro Pro Glu Gly Thr Ala Leu Tyr Glu Phe Ala His Trp
    210                 215                 220

Glu Asp Tyr Lys Gly Asn Pro Met Asp Ser Arg Leu Cys Ala Ile Asp
225                 230                 235                 240

Leu Met Asn Thr Phe Arg Pro Leu Ile Ala Ile Asn Arg Phe Val Ser
                245                 250                 255

Phe Gly Leu Leu Ala Met His Glu His Pro Ile Thr Arg Glu Lys Ile
            260                 265                 270

Lys Ser Asp Asp Asp Tyr Ala Tyr Met Phe Ser Gln Glu Val Arg Arg
        275                 280                 285

Phe Tyr Pro Phe Val Pro Phe Leu Pro Ala Lys Ala Lys Thr Asp Ile
    290                 295                 300

Gln Phe Glu Gly His Pro Ile Glu Lys Asp Thr Met Ile Ala Leu Asp
305                 310                 315                 320

Ile Tyr Gly Thr Met His Arg Glu Asp Val Phe Glu Asn Pro Asp Asp
                325                 330                 335

Phe Tyr Pro Glu Arg Phe Lys Asp Trp Asp Gly
            340                 345

<210> 121
<211> 414
<212> PRT
<213> Pontibacillus halophilus

<400> 121

Met Asn Val Asn Val Ala Lys Asp Arg Leu Asp Gln Thr Ile Ser Leu
1               5                   10                  15

Val Arg Glu Gly Tyr Ala Phe Ile Pro Asn Arg Leu His Ser Gln His
            20                  25                  30

Leu Asp Val Tyr Glu Thr Lys Leu Leu Gly Gln Arg Ile Ala Leu Ile
        35                  40                  45

Gly Gly Glu Glu Gly Ala Asn Leu Phe Tyr Asp Ser Ser Arg Met Lys
    50                  55                  60

Arg Asn Gly Ala Leu Pro Lys Pro Val Leu Lys Thr Leu Phe Gly Glu
65                  70                  75                  80

Gly Gly Val His Thr Lys Asp Gly Ala Ala His Ala His Arg Lys Gln
85 90 95

Leu Phe Met Ser Leu Met Thr Pro Asp Ala Leu Arg Arg Leu Ser Asn
100 105 110

Leu Thr Thr Ala Tyr Trp Glu Glu Tyr Ala Met Lys Trp Glu Lys Gln
115 120 125

Arg Lys Val Val Leu Tyr Asp Glu Ser Arg Glu Leu Leu Cys Gln Val
130 135 140

Ala Cys Asp Trp Ala Gly Ile Pro Leu Gly Lys Lys Glu Val Glu Gln
145 150 155 160

Arg Thr Glu Asp Leu Ser Lys Met Ile Asp Gly Phe Ser Ala Leu Gly
165 170 175

Pro Lys His Ile Glu Ser Arg Arg Ala Arg Asn Arg Ser Glu Lys Trp
180 185 190

Ile Arg Ser Ile Ile Glu Asp Val Arg Ala Asn Lys Leu Glu Thr Arg
195 200 205

Glu Gly Ser Ala Ile His Glu Met Ala Phe His Leu Asp Glu Asn Gly
210 215 220

Asp Arg Leu Asp Thr Glu Ile Ala Ala Val Glu Leu Leu Asn Val Ile
225 230 235 240

Arg Pro Leu Val Ala Ile Ser Lys Phe Leu Ala Phe Gly Ala Lys Ala
245 250 255

Tyr Ala Asp Tyr Pro Glu Thr Arg Glu Lys Thr Lys Glu Ser Asp Glu
260 265 270

Tyr Ile Leu Arg Phe Ala Gln Glu Val Arg Arg Tyr Tyr Pro Phe Ala
275 280 285

Pro Phe Leu Gly Ala Lys Val Lys His Asp Phe Thr Trp Gln Gly Tyr
290 295 300

Thr Phe Arg Glu Asn Gln Leu Val Met Ile Asp Leu Phe Gly Thr Asn
305 310 315 320

His Asp Pro Arg Leu Trp Glu Asn Pro Asp Gln Phe Asp Pro Asp Arg

325 330 335

Phe Lys Asp Trp Lys Gly Gly Leu Phe Asp Leu Ile Pro Gln Gly Gly
340 345 350

Gly Asp Tyr Tyr Asp Gly His Arg Cys Pro Gly Glu Trp Pro Thr Ile
355 360 365

Glu Val Leu Lys Ala Ser Phe Gly Tyr Leu Ser Arg Lys Ile Asp Tyr
370 375 380

Lys Val Pro Lys Gln Asp His Ser Tyr Ser Leu Ser Lys Met Pro Ala
385 390 395 400

Leu Pro Lys Ser Gly Met Ile Leu Lys Lys Val Arg Arg Lys
405 410

<210> 122
<211> 420
<212> PRT
<213> Macrococcus sp.

<400> 122

Met Ser Lys Leu Lys Glu Ser Gly Phe Asp His Thr Leu Thr Met Leu
1 5 10 15

Lys Glu Gly Tyr Thr Tyr Ile Pro Asn Arg Thr Glu Lys Tyr Gln Ser
20 25 30

Pro Ile Phe Phe Asn Arg Val Leu Gly Gly Lys Pro Cys Ala Ile Ile
35 40 45

Ser Gly Lys Glu Ala Ala Glu Leu Phe Tyr Asn Asn Asp Leu Ile Glu
50 55 60

Arg Lys Gly Thr Met Pro Lys Arg Ile Val Lys Thr Leu Phe Gly Lys
65 70 75 80

Gly Ala Ile His Thr Thr Thr Gly Lys Val His Ile Asp Arg Lys Ala
85 90 95

Leu Phe Met Ser Leu Met Thr Asp Glu Asn Leu Ser Leu Leu Arg Gln
100 105 110

Ile Thr Lys Asp Ala Trp Glu Asn Glu Val Asp Ser Leu Ile Lys Val
115 120 125

Asn Glu Val Asn Val Tyr Lys Lys Ser Ser Glu Val Leu Thr Lys Val

130 135 140

Gly Leu Lys Trp Ala Gly Ile Glu Asp Thr Pro Lys Asn Ile Asp Lys
145 150 155 160

Ile Ala Lys Glu Met Asp Leu Met Ile Asp Ser Phe Gly Gln Leu Gly
165 170 175

Gly Ala Tyr Lys Gly Tyr Arg Ala Ala Gln Lys Ala Arg Ala Asn Val
180 185 190

Glu Ala Tyr Leu Gln Asn Gln Ile Lys Lys Val Arg Lys Gly Lys Leu
195 200 205

Lys Pro Arg Glu Asn Ser Ala Leu Tyr Ala Phe Ser His Trp Lys Asp
210 215 220

Met Asn Asn Lys Pro Met Asp Leu Ala Leu Cys Ala Val Asp Leu Met
225 230 235 240

Asn Val Ile Arg Pro Leu Val Ala Ile Asn Arg Phe Val Thr Tyr Gly
245 250 255

Val Leu Ala Ile His Glu Phe Asn Gly Glu Lys Asp Lys Leu Lys Phe
260 265 270

Gly Thr Asp Asp Tyr Val Tyr Gln Phe Val Gln Glu Val Arg Arg Phe
275 280 285

Tyr Pro Phe Val Pro Tyr Leu Pro Gly Lys Ala Lys Lys Lys Leu Thr
290 295 300

Phe Asp Asn Gln Lys Ile Lys Lys Asp Gln Met Ile Leu Leu Asp Val
305 310 315 320

Tyr Gly Thr Leu His Asn Glu Lys Leu Trp Glu Tyr Pro Asn Arg Phe
325 330 335

Asp Pro Thr Arg Phe Glu Asn Trp Asp Gly Ser Pro Phe Asp Leu Ile
340 345 350

Pro Gln Gly Gly Gly Asp Tyr His Thr Asn His Arg Cys Ala Gly Glu
355 360 365

Trp Met Thr Ile Ile Ile Met Glu Glu Thr Met Lys Phe Phe Thr Arg
370 375 380

Thr Ile Ser Phe Glu Val Pro Pro Gln Asp Phe Thr Val Asp Leu Thr
385 390 395 400

Lys Leu Pro Gly Lys Val Ala Ser Gly Met Asn Ile Arg Gln Val Gln
405 410 415

Ala Leu Asn Asn
420

<210> 123
<211> 426
<212> PRT
<213> Staphylococcus lentus

<400> 123

Met Gly Lys Asn Ile Pro Arg Glu Arg Gly Leu Asp Asn Thr Ile Thr
1 5 10 15

Val Leu Lys Glu Ala Tyr Glu Tyr Val Pro Asn Arg Leu Glu Lys His
20 25 30

Asn Thr Asn Ile Phe Glu Leu Arg Ala Leu Gly Gly Arg Arg Thr Val
35 40 45

Val Ile Ser Gly Lys Glu Ala Ala Glu Leu Phe Tyr Asn Asn Asp Leu
50 55 60

Ile Glu Arg Gln Gly Thr Leu Pro Lys Arg Ile Val Asn Thr Leu Phe
65 70 75 80

Gly Lys Gly Ala Ile His Thr Thr Gly Gly Lys Lys His Ile Asp Arg
85 90 95

Lys Ala Leu Phe Met Ser Leu Met Thr Glu Glu Asn Leu Glu Tyr Leu
100 105 110

Arg Glu Leu Thr Arg Asn His Trp Phe Met Asn Thr Glu Arg Met Glu
115 120 125

Arg Met Asp Glu Val Asn Val Tyr Lys Glu Ser Ile Leu Met Leu Thr
130 135 140

Lys Ile Gly Phe Arg Trp Ala Gly Ile Ile Ala Ser Pro Glu Glu Ile
145 150 155 160

Glu Gln Cys Ala Ala Asp Met Asp Thr Met Ile Asp Ser Phe Lys Asn
165 170 175

```
Ile Gly Thr Val Phe Lys Gly Tyr Arg Glu Ala Lys Gln Ala Arg Asp
            180                 185                 190

Arg Val Glu Thr Phe Leu Glu Asn Gln Ile Ile Ala Val Arg Glu Gly
        195                 200                 205

Lys Ile Asn Pro Pro Gln Gly Thr Ala Leu Tyr Glu Phe Ser His Trp
    210                 215                 220

Glu Asp Phe Glu Gly Asn Leu Met Asp Ser Arg Leu Cys Ser Ile Asp
225                 230                 235                 240

Leu Met Asn Val Val Arg Pro Leu Val Ala Ile Asn Arg Phe Val Ser
                245                 250                 255

Phe Gly Val Lys Ala Phe His Asp Tyr Pro Gly Glu Ala Glu Arg Val
            260                 265                 270

Phe Asn Asn Glu Asp Asp Tyr Ala Tyr Lys Phe Ile Gln Glu Val Arg
        275                 280                 285

Arg Phe Tyr Pro Phe Val Pro Tyr Leu Pro Gly Lys Ala Ala Val Asp
    290                 295                 300

Ile Asp Phe Glu Gly Tyr Thr Ile Glu Lys Asp Thr Phe Leu Val Leu
305                 310                 315                 320

Asp Ile Tyr Gly Thr Leu His Arg Asp Gly Leu Trp Glu Asn Pro Glu
                325                 330                 335

Arg Phe Tyr Pro Asn Arg Phe Asn Asp Trp Asp Gly Ser Pro Phe Asp
            340                 345                 350

Leu Ile Pro Gln Gly Gly Gly Asp Tyr Tyr Thr Asn His Arg Cys Ala
        355                 360                 365

Gly Glu Trp Met Thr Ile Ile Ile Met Glu Glu Thr Met Lys Tyr Phe
    370                 375                 380

Ala Gln Asn Ile Ser Tyr Asp Val Lys Lys Asp Gln Asp Thr Ser Val
385                 390                 395                 400

Asn Leu Asn Lys Leu Pro Gly Arg Val Val Asp Gly Met Ile Ile Thr
                405                 410                 415

Asn Val Asn Ala Leu Val Asn Arg Asn Val
            420                 425
```

<210> 124
<211> 426
<212> PRT
<213> Staphylococcus lentus

<400> 124

Met Gly Lys Asn Ile Pro Arg Glu Arg Gly Leu Asp Asn Thr Ile Thr
1               5                   10                  15

Val Leu Lys Glu Ala Tyr Glu Tyr Val Pro Asn Arg Leu Glu Lys His
            20                  25                  30

Asn Thr Asn Ile Phe Glu Leu Arg Ala Leu Gly Gly Arg Arg Thr Val
        35                  40                  45

Val Ile Ser Gly Lys Glu Ala Ala Glu Leu Phe Tyr Asn Asn Asp Leu
    50                  55                  60

Ile Glu Arg Gln Gly Thr Leu Pro Lys Arg Ile Val Asn Thr Leu Phe
65                  70                  75                  80

Gly Lys Gly Ala Ile His Thr Thr Gly Gly Lys Lys His Ile Asp Arg
                85                  90                  95

Lys Ala Leu Phe Met Ser Leu Met Thr Glu Glu Asn Leu Glu Tyr Leu
            100                 105                 110

Arg Glu Leu Thr Arg Asn His Trp Phe Met Asn Thr Glu Arg Met Glu
        115                 120                 125

Arg Met Asp Glu Val Asn Val Tyr Lys Glu Ser Ile Leu Met Leu Thr
    130                 135                 140

Lys Ile Gly Phe Arg Trp Ala Gly Ile Ile Ala Ser Pro Glu Glu Ile
145                 150                 155                 160

Glu Gln Cys Ala Ala Asp Met Asp Thr Met Ile Asp Ser Phe Lys Asn
                165                 170                 175

Ile Gly Thr Val Phe Lys Gly Tyr Arg Glu Ala Lys Gln Ala Arg Asp
            180                 185                 190

Arg Val Glu Thr Phe Leu Glu Asn Gln Ile Ile Ala Val Arg Glu Gly
        195                 200                 205

Lys Ile Asn Pro Pro Gln Gly Thr Ala Leu Tyr Glu Phe Ser His Trp
    210                 215                 220

Glu Asp Phe Glu Gly Asn Leu Met Asp Ser Arg Leu Cys Ser Ile Asp
225 230 235 240

Leu Met Asn Val Val Arg Pro Leu Val Ala Ile Asn Arg Phe Val Ser
245 250 255

Phe Gly Val Lys Ala Phe His Asp Tyr Pro Gly Glu Ala Glu Arg Val
260 265 270

Phe Asn Asn Glu Asp Asp Tyr Ala Tyr Lys Phe Ile Gln Glu Val Arg
275 280 285

Arg Phe Tyr Pro Phe Val Pro Tyr Leu Pro Gly Lys Ala Ala Val Asp
290 295 300

Ile Asp Phe Glu Gly Tyr Thr Ile Glu Lys Asp Thr Phe Leu Val Leu
305 310 315 320

Asp Ile Tyr Gly Thr Leu His Arg Glu Gly Leu Trp Glu Asn Pro Glu
325 330 335

Arg Phe Tyr Pro Asn Arg Phe Asn Asp Trp Asp Gly Ser Pro Phe Asp
340 345 350

Leu Ile Pro Gln Gly Gly Gly Asp Tyr Tyr Thr Asn His Arg Cys Ala
355 360 365

Gly Glu Trp Met Thr Ile Ile Ile Met Glu Glu Thr Met Lys Tyr Phe
370 375 380

Ala Gln Asn Ile Ser Tyr Asp Val Lys Lys Asp Gln Asp Thr Ser Val
385 390 395 400

Asn Leu Asn Lys Leu Pro Gly Arg Val Val Asp Gly Met Ile Ile Thr
405 410 415

Asn Val Asn Ala Leu Val Asn Arg Asn Val
420 425

<210> 125
<211> 426
<212> PRT
<213> Staphylococcus lentus

<400> 125

Met Gly Lys Asn Ile Pro Arg Glu Arg Gly Leu Asp Asn Thr Ile Thr
1 5 10 15

```
Val Leu Lys Glu Ala Tyr Glu Tyr Val Pro Asn Arg Leu Glu Lys His
            20                  25                  30

Asp Thr Asn Ile Phe Glu Leu Arg Ala Leu Gly Gly Arg Arg Thr Val
        35                  40                  45

Val Ile Ser Gly Lys Glu Ala Ala Glu Leu Phe Tyr Asn Asn Asp Leu
    50                  55                  60

Ile Glu Arg Gln Gly Thr Leu Pro Lys Arg Ile Val Asn Thr Leu Phe
65                  70                  75                  80

Gly Lys Gly Ala Ile His Thr Thr Gly Gly Lys Lys His Ile Asp Arg
                85                  90                  95

Lys Ala Leu Phe Met Ser Leu Met Thr Glu Glu Asn Leu Glu Tyr Leu
            100                 105                 110

Arg Glu Leu Thr Arg Asn His Trp Phe Met Asn Thr Glu Arg Met Glu
        115                 120                 125

Arg Met Asp Glu Val Asn Val Tyr Lys Glu Ser Ile Leu Met Leu Thr
    130                 135                 140

Lys Ile Gly Phe Arg Trp Ala Gly Ile Ile Ala Ser Pro Glu Glu Ile
145                 150                 155                 160

Glu Gln Cys Ala Ala Asp Met Asp Thr Met Ile Asp Ser Phe Lys Asn
                165                 170                 175

Ile Gly Thr Val Phe Lys Gly Tyr Arg Glu Ala Lys Gln Ala Arg Asp
            180                 185                 190

Arg Val Glu Thr Phe Leu Glu Asn Gln Ile Ile Ala Val Arg Glu Gly
        195                 200                 205

Lys Ile Asn Pro Pro Gln Gly Thr Ala Leu Tyr Glu Phe Ser His Trp
    210                 215                 220

Glu Asp Phe Glu Gly Asn Leu Met Asp Ser Arg Leu Cys Ser Ile Asp
225                 230                 235                 240

Leu Met Asn Val Val Arg Pro Leu Val Ala Ile Asn Arg Phe Val Ser
                245                 250                 255

Phe Gly Val Lys Ala Phe His Asp Tyr Pro Gly Glu Ala Glu Arg Val
            260                 265                 270
```

Phe Asn Asn Glu Asp Asp Tyr Ala Tyr Lys Phe Ile Gln Glu Val Arg
275 280 285

Arg Phe Tyr Pro Phe Val Pro Tyr Leu Pro Gly Lys Ala Ala Val Asp
290 295 300

Ile Asp Phe Glu Gly Tyr Thr Ile Glu Lys Asp Thr Phe Leu Val Leu
305 310 315 320

Asp Ile Tyr Gly Thr Leu His Arg Glu Gly Leu Trp Glu Asn Pro Glu
325 330 335

Arg Phe Tyr Pro Asn Arg Phe Asn Asp Trp Asp Gly Ser Pro Phe Asp
340 345 350

Leu Ile Pro Gln Gly Gly Gly Asp Tyr Tyr Thr Asn His Arg Cys Ala
355 360 365

Gly Glu Trp Met Thr Ile Ile Ile Met Glu Glu Thr Met Lys Tyr Phe
370 375 380

Ala Gln Asn Ile Ser Tyr Asp Val Lys Lys Asp Gln Asp Thr Ser Val
385 390 395 400

Asn Leu Asn Lys Leu Pro Gly Arg Val Val Asp Gly Met Ile Ile Thr
405 410 415

Asn Val Asn Ala Leu Val Asn Arg Asn Val
420 425

<210> 126
<211> 422
<212> PRT
<213> Staphylococcus pseudintermedius

<400> 126

Met Ala Lys Lys Leu Pro Lys Asp Thr Gly Leu Asp Asn Thr Leu Lys
1 5 10 15

Met Ile Asn Glu Ala Tyr Thr Tyr Val Pro Lys Arg Leu Glu Lys Phe
20 25 30

Gly Thr Lys Ala Phe Glu Thr Arg Ala Leu Gly Met Lys Pro Ile Val
35 40 45

Val Ile Ser Gly Lys Ala Ala Ala Glu Leu Phe Tyr Asp Asn Asp Lys
50 55 60

Ile Ser Arg Lys Gly Thr Leu Pro Lys Arg Ile Val His Thr Leu Phe
65 70 75 80

Gly Lys Gly Ala Ile His Thr Thr Glu Gly Lys Val His Val Asp Arg
85 90 95

Lys Ala Leu Phe Met Ser Leu Met Thr Glu Glu Asn Leu Lys Tyr Leu
100 105 110

Arg Glu Leu Thr Arg Asn Tyr Trp Phe Met His Thr Glu Arg Met Gln
115 120 125

Asn Lys Asp Glu Val Asn Val Tyr Gln Glu Ala Gly Leu Ile Leu Thr
130 135 140

Lys Val Gly Phe Arg Trp Ala Gly Leu Lys Gln Thr Asp Glu Gln Ala
145 150 155 160

Ala Gln Asn Ala Glu Asp Met Asn Thr Met Ile Asp Ser Phe Ser Gly
165 170 175

Leu Gly Gln Ser Leu Lys Gly Tyr Arg Glu Ala Lys Lys Ala Arg Ala
180 185 190

Arg Val Glu Gln Phe Leu Gln Glu Gln Ile Glu Ala Val Arg Val Gly
195 200 205

Gln Gln Tyr Ala Glu Pro Gly Thr Ala Leu Tyr Glu Phe Ala His Trp
210 215 220

Lys Asp Leu Asn Asp Gln Pro Met Asp Pro His Leu Cys Ala Val Asp
225 230 235 240

Leu Met Asn Ile Val Arg Pro Leu Val Ala Val Asn Arg Phe Val Ser
245 250 255

Tyr Gly Val Lys Ala Leu Ile Glu Phe Asp Gln Glu Arg Lys Lys Leu
260 265 270

Gln Val Thr Asn Asp Pro Asn Tyr Ala Tyr Lys Phe Ala Gln Glu Val
275 280 285

Arg Arg Ile Phe Pro Phe Val Pro Phe Leu Pro Gly Arg Leu Lys Lys
290 295 300

Thr Val Glu Phe Asp Gly Phe Lys Leu Lys Lys Gly Thr Leu Thr Val

305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Val Val Pro Ala Gln Asp Leu Ser Val
385 390 395 400

Lys Leu Ser Gln Phe Pro Gly Lys Val Thr Ser Gly Thr Met Ile Lys
405 410 415

Asn Val Tyr Pro Arg Ile
420

<210> 127
<211> 422
<212> PRT
<213> Staphylococcus pseudintermedius

<400> 127

Met Ala Lys Lys Leu Pro Lys Asp Thr Gly Leu Asp Asn Thr Leu Lys
1 5 10 15

Met Ile Asn Glu Ala Tyr Thr Tyr Val Pro Lys Arg Leu Glu Lys Phe
20 25 30

Gly Thr Lys Ala Phe Glu Thr Arg Ala Leu Gly Met Lys Pro Ile Val
35 40 45

Val Ile Ser Gly Lys Ala Ala Ala Glu Leu Phe Tyr Asp Asn Asp Lys
50 55 60

Ile Ser Arg Lys Gly Thr Leu Pro Lys Arg Ile Val His Thr Leu Phe
65 70 75 80

Gly Lys Gly Ala Ile His Thr Thr Glu Gly Lys Val His Val Asp Arg
85 90 95

Lys Ala Leu Phe Met Ser Leu Met Thr Glu Glu Asn Leu Lys Tyr Leu

```
            100                 105                 110

Arg Glu Leu Thr Arg Asn Tyr Trp Phe Met His Thr Glu Arg Met Gln
        115                 120                 125

Asn Lys Asp Glu Val Asn Val Tyr Gln Glu Ala Gly Leu Ile Leu Thr
    130                 135                 140

Lys Val Gly Phe Arg Trp Ala Gly Leu Lys Gln Thr Asp Glu Gln Ala
145                 150                 155                 160

Ala Gln Asn Ala Glu Asp Met Asn Thr Met Ile Asp Ser Phe Ser Gly
                165                 170                 175

Leu Gly Gln Ser Leu Lys Gly Tyr Arg Glu Ala Lys Lys Ala Arg Ala
            180                 185                 190

Arg Val Glu Gln Phe Leu Gln Glu Gln Ile Glu Ala Val Arg Val Gly
        195                 200                 205

Gln Gln Tyr Ala Glu Pro Gly Thr Ala Leu Tyr Glu Phe Ala His Trp
    210                 215                 220

Lys Asp Leu Asn Asp Gln Pro Met Asp Pro His Leu Cys Ala Val Asp
225                 230                 235                 240

Leu Met Asn Ile Val Arg Pro Leu Val Ala Val Asn Arg Phe Val Ser
                245                 250                 255

Tyr Gly Val Lys Ala Leu Ile Glu Phe Asp Gln Glu Arg Lys Lys Leu
            260                 265                 270

Gln Val Thr Asn Asp Pro Asn Tyr Ala Tyr Lys Phe Ala Gln Glu Val
        275                 280                 285

Arg Arg Ile Phe Pro Phe Val Pro Phe Leu Pro Gly Arg Leu Lys Lys
    290                 295                 300

Thr Val Glu Phe Asp Gly Phe Lys Leu Lys Lys Gly Thr Leu Thr Val
305                 310                 315                 320

Leu Asp Ile Phe Gly Thr Thr His Asp Pro Glu Leu Phe Glu Asn Pro
                325                 330                 335

Tyr Gln Phe Asn Pro Asp Arg Phe Asp Asn Trp Asp Gly Ser Pro Phe
            340                 345                 350
```

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Val Ala Pro Pro Gln Asp Leu Ser Val
385 390 395 400

Lys Leu Ser Gln Phe Pro Gly Lys Val Thr Ser Gly Thr Met Ile Lys
405 410 415

Asn Val Tyr Pro Arg Ile
420

<210> 128
<211> 422
<212> PRT
<213> Staphylococcus pseudintermedius

<400> 128

Met Ala Lys Lys Leu Pro Lys Asp Thr Gly Leu Asp Asn Thr Leu Lys
1 5 10 15

Met Ile Asn Glu Ala Tyr Thr Tyr Val Pro Lys Arg Leu Glu Lys Phe
20 25 30

Gly Thr Lys Ala Phe Glu Thr Arg Ala Leu Gly Met Lys Pro Ile Val
35 40 45

Val Ile Ser Gly Lys Ala Ala Ala Glu Leu Phe Tyr Asp Asn Asp Lys
50 55 60

Ile Ser Arg Lys Gly Thr Leu Pro Lys Arg Ile Val His Thr Leu Phe
65 70 75 80

Gly Lys Gly Ala Ile His Thr Thr Glu Gly Lys Val His Val Asp Arg
85 90 95

Lys Ala Leu Phe Met Ser Leu Met Thr Glu Lys Asn Leu Lys Tyr Leu
100 105 110

Arg Glu Leu Thr Arg Asn Tyr Trp Phe Met His Thr Glu Arg Met Gln
115 120 125

Asn Lys Asp Glu Val Asn Val Tyr Gln Glu Ala Gly Leu Ile Leu Thr
130 135 140

Lys Val Gly Phe Arg Trp Ala Gly Leu Lys Gln Thr Asp Glu Gln Val
145 150 155 160

Ala Gln Asn Ala Glu Asp Met Asn Thr Met Ile Asp Ser Phe Ser Gly
165 170 175

Leu Gly Gln Ser Leu Lys Gly Tyr Arg Glu Ala Lys Lys Ala Arg Ala
180 185 190

Arg Val Glu Gln Phe Leu Gln Glu Gln Ile Glu Ala Val Arg Val Gly
195 200 205

Gln Gln Tyr Ala Glu Pro Gly Thr Ala Leu Tyr Glu Phe Ala His Trp
210 215 220

Lys Asp Leu Asn Asp Gln Pro Met Asp Pro His Leu Cys Ala Val Asp
225 230 235 240

Leu Met Asn Ile Val Arg Pro Leu Val Ala Val Asn Arg Phe Val Ser
245 250 255

Tyr Gly Val Lys Ala Leu Ile Glu Phe Asp Gln Glu Arg Lys Lys Leu
260 265 270

Gln Val Thr Asn Asp Pro Asn Tyr Ala Tyr Lys Phe Ala Gln Glu Val
275 280 285

Arg Arg Ile Phe Pro Phe Val Pro Phe Leu Pro Gly Arg Leu Lys Lys
290 295 300

Thr Val Glu Phe Asp Gly Phe Lys Leu Lys Lys Gly Thr Leu Thr Val
305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Val Val Pro Ala Gln Asp Leu Ser Val
385 390 395 400

Lys Leu Ser Gln Phe Pro Gly Lys Val Thr Ser Gly Thr Met Ile Lys
405 410 415

Asn Val Tyr Pro Arg Ile
420

<210> 129
<211> 426
<212> PRT
<213> Macrococcus brunensis

<400> 129

Met Ala Lys Arg Met Pro Lys Val Lys Gly Leu Asp Gln Thr Leu Thr
1 5 10 15

Val Leu Lys Glu Gly Tyr Glu Phe Ile Pro Asn Arg Thr Lys Glu Leu
20 25 30

Asn Thr Asn Val Phe Glu Met Arg Val Leu Gly Gly Lys Lys Ala Val
35 40 45

Val Ile Ser Gly Lys Glu Ala Ala Glu Leu Phe Tyr Asp Asn Asp Lys
50 55 60

Thr Glu Arg Ser Gly Thr Leu Pro Lys Arg Leu Val Lys Thr Leu Phe
65 70 75 80

Gly Lys Gly Ala Ile His Thr Thr Thr Gly Asp Lys His Lys Asn Arg
85 90 95

Lys Thr Leu Phe Met Ser Leu Met Thr Asp Asn Asn Leu Glu Tyr Leu
100 105 110

Arg Lys Leu Thr Arg Asn Tyr Trp Phe Glu Asp Thr Ala Arg Leu Gln
115 120 125

Ala Met Asp Gln Thr Asn Val Tyr Leu Glu Ser Thr Leu Val Leu Leu
130 135 140

Lys Val Gly Phe Arg Phe Ala Asp Val Pro Phe Asp Thr Pro Glu Arg
145 150 155 160

Met Glu Gln Tyr Ala Lys Asp Met Asn Ala Met Val Asp Ser Phe Gly
165 170 175

Ser Ile Gly Thr Ala Tyr Ser Gly Tyr Lys Arg Ala Leu Asn Ala Arg
180 185 190

Asp Arg Val Glu Asp Tyr Leu Glu Lys Gln Ile Ile Ala Thr Arg Lys
195 200 205

Gly Lys Leu Phe Pro Glu Glu Gly Ser Gly Leu Tyr Glu Phe Ser His
210 215 220

Trp Lys Asp Met Asn Gly Lys Gln Met Asp Ser Arg Leu Ala Ala Val
225 230 235 240

Asp Leu Met Asn Thr Ile Arg Pro Leu Val Ala Ile Asn Arg Phe Ile
245 250 255

Ser Phe Gly Val Leu Ala Met His Glu Tyr Pro Gly Glu Arg Ala Arg
260 265 270

Val Ala Gln Asn Asp Asn Ser Tyr Ala Tyr Lys Phe Ala Gln Glu Val
275 280 285

Arg Arg Tyr Tyr Pro Phe Val Pro Phe Leu Pro Gly Lys Ala Lys Lys
290 295 300

Asn Ile Thr Phe Asp Gly Tyr Lys Ile Glu Lys Asp Thr Met Leu Ile
305 310 315 320

Leu Asp Ile Phe Gly Thr Leu His Ser Glu Ser Leu Trp Gln Asn Ala
325 330 335

Glu Gln Phe Tyr Pro Glu Arg Phe Glu Asn Trp Asp Gly Ser Pro Phe
340 345 350

Asp Leu Ile Pro Gln Gly Gly Gly Asp Tyr Trp Thr Asn His Arg Cys
355 360 365

Ala Gly Glu Trp Met Thr Val Ile Ile Met Glu Glu Ser Met Lys Tyr
370 375 380

Phe Ala Gln Glu Ile Gln Tyr Asp Val Pro Ala Gln Asp Phe Thr Val
385 390 395 400

Asp Arg Thr Lys Leu Pro Gly Gln Ile Asn Ser Gly Met Ile Ile Glu
405 410 415

Asn Ile Arg Asn Asn Phe Asp Arg Arg Arg
420 425

<210> 130
<211> 422

<212> PRT
<213> Staphylococcus pseudintermedius

<400> 130

```
Met Ala Lys Lys Leu Pro Lys Asp Thr Gly Leu Asp Asn Thr Leu Lys
1               5                   10                  15

Met Ile Asn Glu Ala Tyr Thr Tyr Val Pro Lys Arg Leu Glu Lys Phe
            20                  25                  30

Gly Thr Lys Ala Phe Glu Thr Arg Ala Leu Gly Met Lys Pro Ile Val
        35                  40                  45

Val Ile Ser Gly Lys Ala Ala Ala Glu Leu Phe Tyr Asp Asn Asp Lys
    50                  55                  60

Ile Ser Arg Lys Gly Thr Leu Pro Lys Arg Ile Val His Thr Leu Phe
65                  70                  75                  80

Gly Lys Gly Ala Ile His Thr Thr Glu Gly Lys Val His Val Asp Arg
                85                  90                  95

Lys Ala Leu Phe Met Ser Leu Met Thr Glu Lys Asn Leu Lys Tyr Leu
            100                 105                 110

Arg Glu Leu Thr Arg Asn Tyr Trp Phe Met His Thr Glu Arg Met Gln
        115                 120                 125

Asn Lys Asp Glu Val Asn Val Tyr Gln Glu Ala Gly Leu Ile Leu Thr
    130                 135                 140

Lys Val Gly Phe Arg Trp Ala Gly Leu Lys Gln Thr Asp Glu Gln Ala
145                 150                 155                 160

Val Gln Asn Ala Glu Asp Met Asn Thr Met Ile Asp Ser Phe Ser Gly
                165                 170                 175

Leu Gly Gln Ser Leu Lys Gly Tyr Arg Glu Ala Lys Lys Ala Arg Ala
            180                 185                 190

Arg Val Glu Gln Phe Leu Gln Glu Gln Ile Glu Ala Val Arg Val Gly
        195                 200                 205

Gln Gln Tyr Ala Glu Pro Gly Thr Ala Leu Tyr Glu Phe Ala His Trp
    210                 215                 220

Lys Asp Leu Asn Asp Gln Pro Met Asp Pro His Leu Cys Ala Val Asp
225                 230                 235                 240
```

Leu Met Asn Ile Val Arg Pro Leu Val Ala Val Asn Arg Phe Val Ser
245 250 255

Tyr Gly Val Lys Ala Leu Thr Glu Phe Asp Gln Glu Arg Lys Lys Leu
260 265 270

Gln Val Thr Asn Asp Pro Asn Tyr Ala Tyr Lys Phe Ala Gln Glu Val
275 280 285

Arg Arg Ile Phe Pro Phe Val Pro Phe Leu Pro Gly Arg Leu Lys Lys
290 295 300

Thr Val Glu Phe Asp Gly Phe Lys Leu Lys Lys Gly Thr Leu Thr Val
305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Val Val Pro Ala Gln Asp Leu Ser Val
385 390 395 400

Lys Leu Ser Gln Phe Pro Gly Lys Val Thr Ser Gly Thr Met Ile Lys
405 410 415

Asn Val Tyr Pro Arg Ile
420

<210> 131
<211> 422
<212> PRT
<213> Staphylococcus pseudintermedius

<400> 131

Met Ala Lys Lys Leu Pro Lys Asp Thr Gly Leu Asp Asn Thr Leu Lys
1 5 10 15

Met Ile Tyr Glu Ala Tyr Thr Tyr Val Pro Lys Arg Leu Glu Lys Phe
20 25 30

Gly Thr Lys Ala Phe Glu Thr Arg Ala Leu Gly Met Lys Pro Ile Val
35 40 45

Val Ile Ser Gly Lys Ala Ala Ala Glu Leu Phe Tyr Asp Asn Asp Lys
50 55 60

Ile Ser Arg Lys Gly Thr Leu Pro Lys Arg Ile Val His Thr Leu Phe
65 70 75 80

Gly Lys Gly Ala Ile His Thr Thr Glu Gly Lys Val His Val Asp Arg
85 90 95

Lys Ala Leu Phe Met Ser Leu Met Thr Glu Lys Asn Leu Lys Tyr Leu
100 105 110

Arg Glu Leu Thr Arg Asn Tyr Trp Phe Met His Thr Glu Arg Met Gln
115 120 125

Asn Lys Asp Glu Val Asn Val Tyr Gln Glu Ala Gly Leu Ile Leu Thr
130 135 140

Lys Val Gly Phe Arg Trp Ala Gly Leu Lys Gln Thr Asp Glu Gln Ala
145 150 155 160

Val Gln Asn Ala Glu Asp Met Asn Thr Met Ile Asp Ser Phe Ser Gly
165 170 175

Leu Gly Gln Ser Leu Lys Gly Tyr Arg Glu Ala Lys Lys Ala Arg Ala
180 185 190

Arg Val Glu Gln Phe Leu Gln Glu Gln Ile Glu Ala Val Arg Val Gly
195 200 205

Gln Gln Tyr Ala Glu Pro Gly Thr Ala Leu Tyr Glu Phe Ala His Trp
210 215 220

Lys Asp Leu Asn Asp Gln Pro Met Asp Pro His Leu Cys Ala Val Asp
225 230 235 240

Leu Met Asn Ile Val Arg Pro Leu Val Ala Val Asn Arg Phe Val Ser
245 250 255

Tyr Gly Val Lys Ala Leu Ile Glu Phe Asp Gln Glu Arg Lys Lys Leu
260 265 270

Gln Val Thr Asn Asp Pro Asn Tyr Ala Tyr Lys Phe Ala Gln Glu Val

275 280 285

Arg Arg Ile Phe Pro Phe Val Pro Phe Leu Pro Gly Arg Leu Lys Lys
290 295 300

Thr Val Glu Phe Asp Gly Phe Lys Leu Lys Lys Gly Thr Leu Thr Val
305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Val Val Pro Ala Gln Asp Leu Ser Val
385 390 395 400

Lys Leu Ser Gln Phe Pro Gly Lys Val Thr Ser Gly Thr Met Ile Lys
405 410 415

Asn Val Tyr Pro Arg Ile
420

<210> 132
<211> 422
<212> PRT
<213> Staphylococcus pseudintermedius

<400> 132

Met Ala Lys Lys Leu Pro Lys Asp Thr Gly Leu Asp Asn Thr Leu Lys
1 5 10 15

Met Ile Asn Glu Ala Tyr Thr Tyr Val Pro Lys Arg Leu Glu Lys Phe
20 25 30

Gly Thr Lys Ala Phe Glu Thr Arg Ala Leu Gly Met Lys Pro Ile Val
35 40 45

Val Ile Ser Gly Lys Ala Ala Ala Glu Leu Phe Tyr Asp Asn Asp Lys
50 55 60

Ile Ser Arg Lys Gly Thr Leu Pro Lys Arg Ile Val His Thr Leu Phe

65 70 75 80

Gly Lys Gly Ala Ile His Thr Thr Glu Gly Lys Val His Val Asp Arg
85 90 95

Lys Ala Leu Phe Met Ser Leu Met Thr Glu Lys Asn Leu Lys Tyr Leu
100 105 110

Arg Glu Leu Thr Arg Asn Tyr Trp Phe Met His Thr Glu Arg Met Gln
115 120 125

Asn Lys Asp Glu Val Asn Val Tyr Gln Glu Ala Gly Leu Ile Leu Thr
130 135 140

Lys Val Gly Phe Arg Trp Ala Gly Leu Arg Gln Thr Asp Glu Gln Ala
145 150 155 160

Val Gln Asn Ala Glu Asp Met Asn Thr Met Ile Asp Ser Phe Ser Gly
165 170 175

Leu Gly Gln Ser Leu Lys Gly Tyr Arg Glu Ala Lys Lys Ala Arg Ala
180 185 190

Arg Val Glu Gln Phe Leu Gln Glu Gln Ile Glu Ala Val Arg Val Gly
195 200 205

Gln Gln Tyr Ala Glu Pro Gly Thr Ala Leu Tyr Glu Phe Ala His Trp
210 215 220

Lys Asp Leu Asn Asp Gln Pro Met Asp Pro His Leu Cys Ala Val Asp
225 230 235 240

Leu Met Asn Ile Val Arg Pro Leu Val Ala Val Asn Arg Phe Val Ser
245 250 255

Tyr Gly Val Lys Ala Leu Ile Glu Phe Asp Gln Glu Arg Lys Lys Leu
260 265 270

Gln Val Thr Asn Asp Pro Asn Tyr Ala Tyr Lys Phe Ala Gln Glu Val
275 280 285

Arg Arg Ile Phe Pro Phe Val Pro Phe Leu Pro Gly Arg Leu Lys Lys
290 295 300

Thr Val Glu Phe Asp Gly Phe Lys Leu Lys Lys Gly Thr Leu Thr Val
305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Val Val Pro Ala Gln Asp Leu Ser Val
385 390 395 400

Lys Leu Ser Gln Phe Pro Gly Lys Val Thr Ser Gly Thr Met Ile Lys
405 410 415

Asn Val Tyr Pro Arg Ile
420

<210> 133
<211> 422
<212> PRT
<213> Staphylococcus sp.

<400> 133

Met Ala Lys Lys Leu Pro Lys Asp Thr Gly Leu Asp Asn Thr Leu Lys
1 5 10 15

Met Ile Asn Glu Ala Tyr Thr Tyr Val Pro Lys Arg Leu Glu Lys Phe
20 25 30

Gly Thr Lys Ala Phe Glu Thr Arg Ala Leu Gly Met Lys Pro Ile Val
35 40 45

Val Ile Ser Gly Lys Ala Ala Ala Glu Leu Phe Tyr Asp Asn Asp Lys
50 55 60

Ile Ser Arg Lys Gly Thr Leu Pro Lys Arg Ile Val His Thr Leu Phe
65 70 75 80

Gly Lys Gly Ala Ile His Thr Thr Glu Gly Lys Val His Val Asp Arg
85 90 95

Lys Ala Leu Phe Met Ser Leu Met Thr Glu Lys Asn Leu Lys Tyr Leu
100 105 110

Arg Glu Leu Thr Arg Asn Tyr Trp Phe Met His Thr Glu Arg Met Gln
115 120 125

Asn Lys Asp Glu Val Asn Val Tyr Gln Glu Ala Gly Leu Ile Leu Thr
130 135 140

Lys Val Gly Phe Arg Trp Ala Gly Leu Lys Gln Thr Asp Glu Gln Ala
145 150 155 160

Val Gln Asn Ala Glu Asp Met Asn Thr Met Ile Asp Ser Phe Ser Gly
165 170 175

Leu Gly Gln Ser Leu Lys Gly Tyr Arg Glu Ala Lys Lys Ala Arg Ala
180 185 190

Arg Val Glu Gln Phe Leu Gln Glu Gln Ile Glu Ala Val Arg Val Gly
195 200 205

Gln Gln Tyr Ala Glu Pro Gly Thr Ala Leu Tyr Glu Phe Ala His Trp
210 215 220

Lys Asp Leu Asn Asp Gln Pro Met Asp Pro His Leu Cys Ala Val Asp
225 230 235 240

Leu Met Asn Ile Val Arg Pro Leu Val Ala Val Asn Arg Phe Val Ser
245 250 255

Tyr Gly Val Lys Ala Leu Ile Glu Phe Asp Gln Glu Arg Lys Lys Leu
260 265 270

Gln Val Thr Asn Asp Pro Asn Tyr Ala Tyr Lys Phe Ala Gln Glu Val
275 280 285

Arg Arg Ile Phe Pro Phe Val Pro Phe Leu Pro Gly Arg Leu Lys Lys
290 295 300

Thr Val Glu Phe Asp Gly Phe Lys Leu Lys Lys Gly Thr Leu Thr Val
305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
    370                 375                 380

Phe Ala Asn Lys Ile Asp Phe Val Val Pro Ala Gln Asp Leu Ser Val
385                 390                 395                 400

Lys Leu Ser Gln Phe Pro Gly Lys Val Thr Ser Gly Thr Met Ile Lys
                405                 410                 415

Asn Val Tyr Pro Arg Ile
            420

<210>  134
<211>  422
<212>  PRT
<213>  Staphylococcus pseudintermedius

<400>  134

Met Ala Lys Lys Leu Pro Lys Asp Thr Gly Leu Asp Asn Thr Leu Lys
1               5                   10                  15

Met Ile Asn Glu Ala Tyr Thr Tyr Val Pro Lys Arg Leu Glu Lys Phe
            20                  25                  30

Gly Thr Lys Ala Phe Glu Thr Arg Ala Leu Gly Met Lys Pro Ile Val
        35                  40                  45

Val Ile Ser Gly Lys Ala Ala Ala Glu Leu Phe Tyr Asp Asn Asp Lys
    50                  55                  60

Ile Ser Arg Lys Gly Thr Leu Pro Lys Arg Ile Val His Thr Leu Phe
65                  70                  75                  80

Gly Lys Gly Ala Ile His Thr Thr Glu Gly Lys Val His Val Asp Arg
                85                  90                  95

Lys Ala Leu Phe Met Ser Leu Met Thr Glu Lys Asn Leu Lys Tyr Leu
            100                 105                 110

Arg Glu Leu Thr Arg Asn Tyr Trp Phe Met His Thr Glu Arg Met Gln
        115                 120                 125

Asn Lys Asp Glu Val Asn Val Tyr Gln Glu Ala Gly Leu Ile Leu Thr
    130                 135                 140

Lys Val Gly Phe Arg Trp Ala Gly Leu Lys Gln Thr Asp Glu Gln Ala
145                 150                 155                 160

Ala Gln Asn Ala Glu Asp Met Asn Thr Met Ile Asp Ser Phe Ser Gly
165 170 175

Leu Gly Gln Ser Leu Lys Gly Tyr Arg Glu Ala Lys Lys Ala Arg Ala
180 185 190

Arg Val Glu Gln Phe Leu Gln Glu Gln Ile Glu Ala Val Arg Val Gly
195 200 205

Gln Gln Tyr Ala Glu Pro Gly Thr Ala Leu Tyr Glu Phe Ala His Trp
210 215 220

Lys Asp Leu Asn Asp Gln Pro Met Asp Pro His Leu Cys Ala Val Asp
225 230 235 240

Leu Met Asn Ile Val Arg Pro Leu Val Ala Val Asn Arg Phe Val Ser
245 250 255

Tyr Gly Val Lys Ala Leu Ile Glu Phe Asp Gln Glu Arg Lys Lys Leu
260 265 270

Gln Val Thr Asn Asp Pro Asn Tyr Ala Tyr Lys Phe Ala Gln Glu Val
275 280 285

Arg Arg Ile Phe Pro Phe Val Pro Phe Leu Pro Gly Arg Leu Lys Lys
290 295 300

Thr Val Glu Phe Asp Gly Phe Lys Leu Lys Lys Gly Thr Leu Thr Val
305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Val Val Pro Ala Gln Asp Leu Ser Val
385 390 395 400

Lys Leu Ser Gln Phe Pro Gly Lys Val Thr Ser Gly Thr Met Ile Lys
405 410 415

Asn Val Tyr Pro Arg Ile
420

<210> 135
<211> 422
<212> PRT
<213> Staphylococcus pseudintermedius

<400> 135

Met Ala Lys Lys Leu Pro Lys Asp Thr Gly Leu Asp Asn Thr Leu Lys
1 5 10 15

Met Ile Asn Glu Ala Tyr Thr Tyr Val Pro Lys Arg Leu Glu Lys Phe
20 25 30

Gly Thr Lys Ala Phe Glu Thr Arg Ala Leu Gly Met Lys Pro Ile Val
35 40 45

Val Ile Ser Gly Lys Ala Ala Ala Glu Leu Phe Tyr Asp Asn Asp Lys
50 55 60

Ile Ser Arg Lys Gly Thr Leu Pro Lys Arg Ile Val His Thr Leu Phe
65 70 75 80

Gly Lys Gly Ala Ile His Thr Thr Glu Gly Lys Val His Val Asp Arg
85 90 95

Lys Ala Leu Phe Met Ser Leu Met Thr Glu Lys Asn Leu Lys Tyr Leu
100 105 110

Arg Glu Leu Thr Arg Asn Tyr Trp Phe Met His Thr Glu Arg Met Gln
115 120 125

Asn Lys Asp Glu Val Asn Val Tyr Gln Glu Ala Gly Leu Ile Leu Thr
130 135 140

Lys Val Gly Phe Arg Trp Ala Gly Leu Lys Gln Thr Asp Glu Gln Ala
145 150 155 160

Ala Gln Asn Ala Glu Asp Met Asn Thr Met Ile Asp Ser Phe Ser Gly
165 170 175

Leu Gly Gln Ser Leu Lys Gly Tyr Arg Glu Ala Lys Lys Ala Arg Ala
180 185 190

Arg Val Glu Gln Phe Leu Gln Glu Gln Ile Glu Ala Val Arg Val Gly
195 200 205

```
Gln Gln Tyr Ala Glu Pro Gly Thr Ala Leu Tyr Glu Phe Ala His Trp
    210                 215                 220

Lys Asp Leu Asn Asp Gln Pro Met Asp Pro His Leu Cys Ala Val Asp
225                 230                 235                 240

Leu Met Asn Ile Val Arg Pro Leu Val Ala Val Asn Arg Phe Val Ser
                245                 250                 255

Tyr Gly Val Lys Ala Leu Ile Glu Phe Asp Gln Glu Arg Lys Lys Leu
            260                 265                 270

Gln Val Thr Asn Asp Pro Asn Tyr Ala Tyr Lys Phe Ala Gln Glu Val
        275                 280                 285

Arg Arg Ile Phe Pro Phe Val Pro Phe Leu Pro Gly Arg Leu Lys Lys
    290                 295                 300

Thr Val Glu Phe Asp Gly Phe Lys Leu Lys Lys Gly Thr Leu Thr Val
305                 310                 315                 320

Leu Asp Ile Phe Gly Thr Thr His Asp Pro Glu Leu Phe Glu Asn Pro
                325                 330                 335

Tyr Gln Phe Asn Pro Asp Arg Phe Asp Asn Trp Asp Gly Ser Pro Phe
            340                 345                 350

Asp Leu Ile Pro Gln Gly Gly Gly Asp Phe Tyr Thr Asn His Arg Cys
        355                 360                 365

Ala Gly Glu Trp Met Thr Val Ile Val Met Glu Glu Thr Ile Gln Tyr
    370                 375                 380

Phe Ala Asn Lys Ile Asp Phe Val Ala Pro Pro Gln Asp Leu Ser Val
385                 390                 395                 400

Lys Leu Ser Gln Phe Pro Gly Lys Val Thr Ser Gly Thr Met Ile Lys
                405                 410                 415

Asn Val Tyr Pro Arg Ile
            420
```

<210> 136
<211> 422
<212> PRT
<213> Staphylococcus pseudintermedius

<400> 136

Met Ala Lys Lys Leu Pro Lys Asp Thr Gly Leu Asp Asn Thr Leu Lys
1               5                   10                  15

Met Ile Asn Glu Ala Tyr Thr Tyr Val Pro Lys Arg Leu Glu Lys Phe
            20                  25                  30

Gly Thr Lys Ala Phe Glu Thr Arg Ala Leu Gly Met Lys Pro Ile Val
        35                  40                  45

Val Ile Ser Gly Lys Ala Ala Ala Glu Leu Phe Tyr Asp Asn Asp Lys
    50                  55                  60

Ile Ser Arg Lys Gly Thr Leu Pro Lys Arg Ile Val His Thr Leu Phe
65                  70                  75                  80

Gly Lys Gly Ala Ile His Thr Thr Glu Gly Lys Val His Val Asp Arg
                85                  90                  95

Lys Ala Leu Phe Met Ser Leu Met Thr Glu Lys Asn Leu Lys Tyr Leu
            100                 105                 110

Arg Glu Leu Thr Arg Asn Tyr Trp Phe Met His Thr Glu Arg Met Gln
        115                 120                 125

Asn Lys Asp Glu Val Asn Val Tyr Gln Glu Ala Gly Leu Ile Leu Thr
    130                 135                 140

Lys Val Gly Phe Arg Trp Ala Gly Leu Lys Gln Thr Asp Glu Gln Ala
145                 150                 155                 160

Ala Gln Asn Ala Glu Asp Met Asn Thr Met Ile Asp Ser Phe Ser Gly
                165                 170                 175

Leu Gly Gln Ser Leu Lys Gly Tyr Arg Glu Ala Lys Lys Ala Arg Ala
            180                 185                 190

Arg Val Glu Gln Phe Leu Gln Glu Gln Ile Glu Ala Val Arg Val Gly
        195                 200                 205

Gln Gln Tyr Ala Glu Pro Gly Thr Ala Leu Tyr Glu Phe Ala His Trp
    210                 215                 220

Lys Asp Leu Asn Asp Gln Pro Met Asp Pro His Leu Cys Ala Val Asp
225                 230                 235                 240

Leu Met Asn Ile Val Arg Pro Leu Val Ala Val Asn Arg Phe Val Ser

245 250 255

Tyr Gly Val Lys Ala Leu Ile Glu Phe Asp Gln Glu Arg Lys Lys Leu
260 265 270

Gln Val Thr Asn Asp Pro Asn Tyr Ala Tyr Lys Phe Ser Gln Glu Val
275 280 285

Arg Arg Ile Phe Pro Phe Val Pro Phe Leu Pro Gly Arg Leu Lys Lys
290 295 300

Thr Val Glu Phe Asp Gly Phe Lys Leu Lys Lys Gly Thr Leu Thr Val
305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Val Val Pro Ala Gln Asp Leu Ser Val
385 390 395 400

Lys Leu Ser Gln Phe Pro Gly Lys Val Thr Ser Gly Thr Met Ile Lys
405 410 415

Asn Val Tyr Pro Arg Ile
420

<210> 137
<211> 422
<212> PRT
<213> Staphylococcus pseudintermedius

<400> 137

Met Ala Lys Lys Leu Pro Lys Asp Thr Gly Leu Asp Asn Thr Leu Lys
1 5 10 15

Met Ile Asn Glu Ala Tyr Thr Tyr Val Pro Lys Arg Leu Glu Lys Phe
20 25 30

Gly Thr Lys Ala Phe Glu Thr Arg Ala Leu Gly Met Lys Pro Ile Val

35 40 45

Val Ile Ser Gly Lys Ala Ala Ala Glu Leu Phe Tyr Asp Asn Asp Lys
50 55 60

Ile Ser Arg Lys Gly Thr Leu Pro Lys Arg Ile Val His Thr Leu Phe
65 70 75 80

Gly Lys Gly Ala Ile His Thr Thr Glu Gly Lys Val His Val Asp Arg
85 90 95

Lys Ala Leu Phe Met Ser Leu Met Thr Glu Lys Asn Leu Lys Tyr Leu
100 105 110

Arg Glu Leu Thr Arg Asn Tyr Trp Phe Met His Thr Glu Arg Met Gln
115 120 125

Asn Lys Asp Glu Val Asn Val Tyr Gln Glu Ala Gly Leu Ile Leu Thr
130 135 140

Lys Val Gly Phe Arg Trp Ala Gly Leu Lys Gln Thr Asp Glu Gln Ala
145 150 155 160

Val Gln Asn Ala Glu Asp Met Asn Thr Met Ile Asp Ser Phe Ser Gly
165 170 175

Leu Gly Gln Ser Leu Lys Gly Tyr Arg Glu Ala Lys Lys Ala Arg Ala
180 185 190

Arg Val Glu Gln Phe Leu Gln Glu Gln Ile Glu Ala Val Arg Val Gly
195 200 205

Gln Gln Tyr Ala Glu Pro Gly Thr Ala Leu Tyr Glu Phe Ala His Trp
210 215 220

Lys Asp Leu Asn Asp Gln Pro Met Asp Pro His Leu Cys Ala Val Asp
225 230 235 240

Leu Met Asn Ile Val Arg Pro Leu Val Ala Val Asn Arg Phe Val Ser
245 250 255

Tyr Gly Val Lys Ala Leu Ile Glu Phe Asp Gln Glu Arg Lys Lys Leu
260 265 270

Gln Val Thr Asn Asp Pro Asn Tyr Ala Tyr Lys Phe Ser Gln Glu Val
275 280 285

Arg Arg Ile Phe Pro Phe Val Pro Phe Leu Pro Gly Arg Leu Lys Lys
    290                 295                 300

Thr Val Glu Phe Asp Gly Phe Lys Leu Lys Lys Gly Thr Leu Thr Val
305                 310                 315                 320

Leu Asp Ile Phe Gly Thr Thr His Asp Pro Glu Leu Phe Glu Asn Pro
                325                 330                 335

Tyr Gln Phe Asn Pro Asp Arg Phe Asp Asn Trp Asp Gly Ser Pro Phe
            340                 345                 350

Asp Leu Ile Pro Gln Gly Gly Gly Asp Phe Tyr Thr Asn His Arg Cys
        355                 360                 365

Ala Gly Glu Trp Met Thr Val Ile Val Met Glu Glu Thr Ile Gln Tyr
    370                 375                 380

Phe Ala Asn Lys Ile Asp Phe Val Val Pro Ala Gln Asp Leu Ser Val
385                 390                 395                 400

Lys Leu Ser Gln Phe Pro Gly Lys Val Thr Ser Gly Thr Met Ile Lys
                405                 410                 415

Asn Val Tyr Pro Arg Ile
            420

<210> 138
<211> 422
<212> PRT
<213> Staphylococcus pseudintermedius

<400> 138

Met Ala Lys Lys Leu Pro Lys Asp Thr Gly Leu Asp Asn Thr Leu Lys
1               5                   10                  15

Met Ile Asn Glu Ala Tyr Thr Tyr Val Pro Lys Arg Leu Glu Lys Phe
            20                  25                  30

Gly Thr Lys Ala Phe Glu Thr Arg Ala Leu Gly Met Lys Pro Ile Val
        35                  40                  45

Val Ile Ser Gly Lys Ala Ala Ala Glu Leu Phe Tyr Asp Asn Asp Lys
    50                  55                  60

Ile Ser Arg Lys Gly Thr Leu Pro Lys Arg Ile Val His Thr Leu Phe
65                  70                  75                  80

```
Gly Lys Gly Ala Ile His Thr Thr Glu Gly Lys Val His Val Asp Arg
                85                  90                  95

Lys Ala Leu Phe Met Ser Leu Met Thr Glu Lys Asn Leu Lys Tyr Leu
            100                 105                 110

Arg Glu Leu Thr Arg Asn Tyr Trp Phe Met His Thr Glu Arg Met Gln
        115                 120                 125

Asn Lys Asp Glu Val Asn Val Tyr Gln Glu Ala Gly Leu Ile Leu Thr
    130                 135                 140

Lys Val Gly Phe Arg Trp Ala Gly Leu Arg Gln Thr Asp Glu Gln Ala
145                 150                 155                 160

Val Gln Asn Ala Glu Asp Met Asn Thr Met Ile Asp Ser Phe Ser Gly
                165                 170                 175

Leu Gly Gln Ser Leu Lys Gly Tyr Arg Glu Ala Lys Lys Ala Arg Ala
            180                 185                 190

Arg Val Glu Gln Phe Leu Gln Glu Gln Ile Glu Ala Val Arg Val Gly
        195                 200                 205

Gln Gln Tyr Ala Glu Pro Gly Thr Ala Leu Tyr Glu Phe Ala His Trp
    210                 215                 220

Lys Asp Leu Asn Asp Gln Pro Met Asp Pro His Leu Cys Ala Val Asp
225                 230                 235                 240

Leu Met Asn Ile Val Arg Pro Leu Val Ala Val Asn Arg Phe Val Ser
                245                 250                 255

Tyr Gly Val Lys Ala Leu Ile Glu Phe Asp Gln Glu Arg Lys Lys Leu
            260                 265                 270

Gln Val Thr Asn Asp Pro Asn Tyr Ala Tyr Lys Phe Ala Gln Glu Val
        275                 280                 285

Arg Arg Ile Phe Pro Phe Val Pro Phe Leu Pro Gly Arg Leu Lys Lys
    290                 295                 300

Thr Val Glu Phe Asp Gly Phe Lys Leu Lys Lys Gly Thr Leu Thr Val
305                 310                 315                 320

Leu Asp Ile Leu Gly Thr Thr His Asp Pro Glu Leu Phe Glu Asn Pro
                325                 330                 335
```

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Val Ala Pro Pro Gln Asp Leu Ser Val
385 390 395 400

Lys Leu Ser Gln Phe Pro Gly Lys Val Thr Ser Gly Thr Met Ile Lys
405 410 415

Asn Val Tyr Pro Arg Ile
420

<210> 139
<211> 422
<212> PRT
<213> Staphylococcus pseudintermedius

<400> 139

Met Ala Lys Lys Leu Pro Lys Asp Thr Gly Leu Asp Asn Thr Leu Lys
1 5 10 15

Met Ile Asn Glu Ala Tyr Thr Tyr Val Pro Lys Arg Leu Glu Lys Phe
20 25 30

Gly Thr Lys Ala Phe Glu Thr Arg Ala Leu Gly Met Lys Pro Ile Val
35 40 45

Val Ile Ser Gly Lys Ala Ala Ala Glu Leu Phe Tyr Asp Asn Asp Lys
50 55 60

Ile Ser Arg Lys Gly Thr Leu Pro Lys Arg Ile Val His Thr Leu Phe
65 70 75 80

Gly Lys Gly Ala Ile His Thr Thr Glu Gly Lys Val His Val Asp Arg
85 90 95

Lys Ala Leu Phe Met Ser Leu Met Thr Glu Lys Asn Leu Lys Tyr Leu
100 105 110

Arg Glu Leu Thr Arg Asn Tyr Trp Phe Met His Thr Glu Arg Met Gln
115 120 125

Asn Lys Asp Glu Val Asn Val Tyr Gln Glu Ala Gly Leu Ile Leu Thr
    130                 135                 140

Lys Val Gly Phe Arg Trp Ala Gly Leu Arg Gln Thr Asp Glu Gln Ala
145                 150                 155                 160

Val Gln Asn Ala Glu Asp Met Asn Thr Met Ile Asp Ser Phe Ser Gly
                165                 170                 175

Leu Gly Gln Ser Leu Lys Gly Tyr Arg Glu Ala Lys Lys Ala Arg Ala
            180                 185                 190

Arg Val Glu Gln Phe Leu Gln Glu Gln Ile Glu Ala Val Arg Val Gly
        195                 200                 205

Gln Gln Tyr Ala Glu Pro Gly Thr Ala Leu Tyr Glu Phe Ala His Trp
    210                 215                 220

Lys Asp Leu Asn Asp Gln Pro Met Asp Pro His Leu Cys Ala Val Asp
225                 230                 235                 240

Leu Met Asn Ile Val Arg Pro Leu Val Ala Val Asn Arg Phe Val Ser
                245                 250                 255

Tyr Gly Val Lys Ala Leu Ile Glu Phe Asp Gln Glu Arg Lys Lys Leu
            260                 265                 270

Gln Val Thr Asn Asp Pro Asn Tyr Ala Tyr Lys Phe Ala Gln Glu Val
        275                 280                 285

Arg Arg Ile Phe Pro Phe Val Pro Phe Leu Pro Gly Arg Leu Lys Lys
    290                 295                 300

Thr Val Glu Phe Asp Gly Phe Lys Leu Lys Lys Gly Thr Leu Thr Val
305                 310                 315                 320

Leu Asp Ile Phe Gly Thr Thr His Asp Pro Glu Leu Phe Glu Asn Pro
                325                 330                 335

Tyr Gln Phe Asn Pro Asp Arg Phe Asp Asn Trp Asp Gly Ser Pro Phe
            340                 345                 350

Asp Leu Ile Pro Gln Gly Gly Gly Asp Phe Tyr Thr Asn His Arg Cys
        355                 360                 365

Ala Gly Glu Trp Met Thr Val Ile Val Met Glu Glu Thr Ile Gln Tyr
    370                 375                 380

Phe Ala Asn Lys Ile Asp Phe Val Ala Pro Pro Gln Asp Leu Ser Val
385                 390                 395                 400

Lys Leu Ser Gln Phe Pro Gly Lys Val Thr Ser Gly Thr Met Ile Lys
                405                 410                 415

Asn Val Tyr Pro Arg Ile
            420

<210> 140
<211> 422
<212> PRT
<213> Staphylococcus pseudintermedius

<400> 140

Met Ala Lys Lys Leu Pro Lys Asp Thr Gly Leu Asp Asn Thr Leu Lys
1               5                   10                  15

Ile Ile Asn Glu Ala Tyr Thr Tyr Val Pro Lys Arg Leu Glu Lys Phe
            20                  25                  30

Gly Thr Lys Ala Phe Glu Thr Arg Ala Leu Gly Met Lys Pro Ile Val
        35                  40                  45

Val Ile Ser Gly Lys Ala Ala Ala Glu Leu Phe Tyr Asp Asn Asp Lys
    50                  55                  60

Ile Ser Arg Lys Gly Thr Leu Pro Lys Arg Ile Val His Thr Leu Phe
65                  70                  75                  80

Gly Lys Gly Ala Ile His Thr Thr Glu Gly Lys Val His Val Asp Arg
                85                  90                  95

Lys Ala Leu Phe Met Ser Leu Met Thr Glu Lys Asn Leu Lys Tyr Leu
            100                 105                 110

Arg Glu Leu Thr Arg Asn Tyr Trp Phe Met His Thr Glu Arg Met Gln
        115                 120                 125

Asn Lys Asp Glu Val Asn Val Tyr Gln Glu Ala Gly Leu Ile Leu Thr
    130                 135                 140

Lys Val Gly Phe Arg Trp Ala Gly Leu Lys Gln Thr Asp Glu Gln Ala
145                 150                 155                 160

Ala Gln Asn Ala Glu Asp Met Asn Thr Met Ile Asp Ser Phe Ser Gly
                165                 170                 175

Leu Gly Gln Ser Leu Lys Gly Tyr Arg Glu Ala Lys Lys Ala Arg Ala
180 185 190

Arg Val Glu Gln Phe Leu Gln Glu Gln Ile Glu Ala Val Arg Val Gly
195 200 205

Gln Gln Tyr Ala Glu Pro Gly Thr Ala Leu Tyr Glu Phe Ala His Trp
210 215 220

Lys Asp Leu Asn Asp Gln Pro Met Asp Pro His Leu Cys Ala Val Asp
225 230 235 240

Leu Met Asn Ile Val Arg Pro Leu Val Ala Val Asn Arg Phe Val Ser
245 250 255

Tyr Gly Val Lys Ala Leu Ile Glu Phe Asp Gln Glu Arg Lys Lys Leu
260 265 270

Gln Val Thr Asn Asp Pro Asn Tyr Ala Tyr Lys Phe Ala Gln Glu Val
275 280 285

Arg Arg Ile Phe Pro Phe Val Pro Phe Leu Pro Gly Arg Leu Lys Lys
290 295 300

Thr Val Glu Phe Asp Gly Phe Lys Leu Lys Lys Gly Thr Leu Thr Val
305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Val Ala Pro Ala Gln Asp Leu Ser Val
385 390 395 400

Lys Leu Ser Gln Phe Pro Gly Lys Val Thr Ser Gly Thr Met Ile Lys
405 410 415

Asn Val Tyr Pro Arg Ile

420

<210> 141
<211> 422
<212> PRT
<213> Staphylococcus pseudintermedius

<400> 141

```
Met Ala Lys Lys Leu Pro Lys Asp Thr Gly Leu Asp Asn Thr Leu Lys
1               5                   10                  15

Met Ile Asn Glu Ala Tyr Thr Tyr Val Pro Lys Arg Leu Glu Lys Phe
            20                  25                  30

Gly Thr Lys Ala Phe Glu Thr Arg Ala Leu Gly Met Lys Thr Ile Val
        35                  40                  45

Val Ile Ser Gly Lys Ala Ala Ala Glu Leu Phe Tyr Asp Asn Asp Lys
    50                  55                  60

Ile Ser Arg Lys Gly Thr Leu Pro Lys Arg Ile Val His Thr Leu Phe
65                  70                  75                  80

Gly Lys Gly Ala Ile His Thr Thr Glu Gly Lys Val His Val Asp Arg
                85                  90                  95

Lys Ala Leu Phe Met Ser Leu Met Thr Glu Lys Asn Leu Lys Tyr Leu
            100                 105                 110

Arg Glu Leu Thr Arg Asn Tyr Trp Phe Met His Thr Glu Arg Met Gln
        115                 120                 125

Asn Lys Asp Glu Val Asn Val Tyr Gln Glu Ala Gly Leu Ile Leu Thr
    130                 135                 140

Lys Val Gly Phe Arg Trp Ala Gly Leu Lys Gln Thr Asp Glu Gln Ala
145                 150                 155                 160

Ala Gln Asn Ala Glu Asp Met Asn Thr Met Ile Asp Ser Phe Ser Gly
                165                 170                 175

Leu Gly Gln Ser Leu Lys Gly Tyr Arg Glu Ala Lys Lys Ala Arg Ala
            180                 185                 190

Arg Val Glu Gln Phe Leu Gln Glu Gln Ile Glu Ala Val Arg Val Gly
        195                 200                 205

Gln Gln Tyr Ala Glu Pro Gly Thr Ala Leu Tyr Glu Phe Ala His Trp
```

210 215 220

Lys Asp Leu Asn Asp Gln Pro Met Asp Pro His Leu Cys Ala Val Asp
225 230 235 240

Leu Met Asn Ile Val Arg Pro Leu Val Ala Val Asn Arg Phe Val Ser
245 250 255

Tyr Gly Val Lys Ala Leu Ile Glu Phe Asp Gln Glu Arg Lys Lys Leu
260 265 270

Gln Val Thr Asn Asp Pro Asn Tyr Ala Tyr Lys Phe Ala Gln Glu Val
275 280 285

Arg Arg Ile Phe Pro Phe Val Pro Phe Leu Pro Gly Arg Leu Lys Lys
290 295 300

Thr Val Glu Phe Asp Gly Phe Lys Leu Lys Lys Gly Thr Leu Thr Val
305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Val Ala Pro Ala Gln Asp Leu Ser Val
385 390 395 400

Lys Leu Ser Gln Phe Pro Gly Lys Val Thr Ser Gly Thr Met Ile Lys
405 410 415

Asn Val Tyr Pro Arg Ile
420

<210> 142
<211> 422
<212> PRT
<213> Staphylococcus pseudintermedius

<400> 142

Met Ala Lys Lys Leu Pro Lys Asp Thr Gly Leu Asp Asn Thr Leu Lys

```
1               5                   10                  15

Met Ile Asn Glu Ala Tyr Thr Tyr Val Pro Lys Arg Leu Glu Lys Phe
            20                  25                  30

Gly Thr Lys Ala Phe Glu Thr Arg Ala Leu Gly Met Lys Pro Ile Val
        35                  40                  45

Val Ile Ser Gly Lys Ala Ala Ala Glu Leu Phe Tyr Asp Asn Asp Lys
    50                  55                  60

Ile Ser Arg Lys Gly Thr Leu Pro Lys Arg Ile Val His Thr Leu Phe
65                  70                  75                  80

Gly Lys Gly Ala Ile His Thr Thr Glu Gly Lys Val His Val Asp Arg
                85                  90                  95

Lys Ala Leu Phe Met Ser Leu Met Thr Glu Lys Asn Leu Lys Tyr Leu
            100                 105                 110

Arg Glu Leu Thr Arg Asn Tyr Trp Phe Met His Thr Glu Arg Met Gln
        115                 120                 125

Asn Lys Asp Glu Val Asn Val Tyr Gln Glu Thr Gly Leu Ile Leu Thr
    130                 135                 140

Lys Val Gly Phe Arg Trp Ala Gly Leu Lys Gln Thr Asp Glu Gln Ala
145                 150                 155                 160

Ala Gln Asn Ala Glu Asp Met Asn Thr Met Ile Asp Ser Phe Ser Gly
                165                 170                 175

Leu Gly Gln Ser Leu Lys Gly Tyr Arg Glu Ala Lys Lys Ala Arg Ala
            180                 185                 190

Arg Val Glu Gln Phe Leu Gln Glu Gln Ile Glu Ala Val Arg Val Gly
        195                 200                 205

Gln Gln Tyr Ala Glu Pro Gly Thr Ala Leu Tyr Glu Phe Ala His Trp
    210                 215                 220

Lys Asp Leu Asn Asp Gln Pro Met Asp Pro His Leu Cys Ala Val Asp
225                 230                 235                 240

Leu Met Asn Ile Val Arg Pro Leu Val Ala Val Asn Arg Phe Val Ser
                245                 250                 255
```

Tyr Gly Val Lys Ala Leu Ile Glu Phe Asp Gln Glu Arg Lys Lys Leu
260 265 270

Gln Val Thr Asn Asp Pro Asn Tyr Ala Tyr Lys Phe Ala Gln Glu Val
275 280 285

Arg Arg Ile Phe Pro Phe Val Pro Phe Leu Pro Gly Arg Leu Lys Lys
290 295 300

Thr Val Glu Phe Asp Gly Phe Lys Leu Lys Lys Gly Thr Leu Thr Val
305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Val Val Pro Ala Gln Asp Leu Ser Val
385 390 395 400

Lys Leu Ser Gln Phe Pro Gly Lys Val Thr Ser Gly Thr Met Ile Lys
405 410 415

Asn Val Tyr Pro Arg Ile
420

<210> 143
<211> 422
<212> PRT
<213> Staphylococcus pseudintermedius

<400> 143

Met Ala Lys Lys Leu Pro Lys Asp Thr Gly Leu Asp Asn Thr Leu Lys
1 5 10 15

Met Ile Asn Glu Ala Tyr Thr Tyr Val Pro Lys Arg Leu Glu Lys Phe
20 25 30

Gly Thr Lys Ala Phe Glu Thr Arg Ala Leu Gly Met Lys Pro Ile Val
35 40 45

Val Ile Ser Gly Lys Ala Ala Ala Glu Leu Phe Tyr Asp Asn Asp Lys
    50                  55                  60

Ile Ser Arg Lys Gly Thr Leu Pro Lys Arg Ile Val His Thr Leu Phe
65                  70                  75                  80

Gly Lys Gly Ala Ile His Thr Thr Glu Gly Lys Val His Val Asp Arg
                85                  90                  95

Lys Ala Leu Phe Met Ser Leu Met Thr Glu Lys Asn Leu Lys Tyr Leu
            100                 105                 110

Arg Glu Leu Thr Arg Asn Tyr Trp Phe Met His Thr Glu Arg Met Gln
        115                 120                 125

Asn Lys Asp Glu Val Asn Val Tyr Gln Glu Ala Gly Leu Ile Leu Thr
    130                 135                 140

Lys Val Gly Phe Arg Trp Ala Gly Leu Lys Gln Thr Asp Glu Gln Ala
145                 150                 155                 160

Ala Gln Asn Ala Glu Asp Met Asn Thr Met Ile Asp Ser Phe Ser Gly
                165                 170                 175

Leu Gly Gln Ser Leu Lys Gly Tyr Arg Glu Ala Lys Lys Ala Arg Ala
            180                 185                 190

Arg Val Glu Gln Phe Leu Gln Glu Gln Ile Glu Ala Val Arg Val Gly
        195                 200                 205

Gln Gln Tyr Ala Glu Pro Gly Thr Ala Leu Tyr Glu Phe Ala His Trp
    210                 215                 220

Lys Asp Leu Asn Asp Gln Pro Met Asp Pro His Leu Cys Ala Val Asp
225                 230                 235                 240

Leu Met Asn Ile Val Arg Pro Leu Val Ala Val Asn Arg Phe Val Ser
                245                 250                 255

Tyr Gly Val Lys Ala Leu Ile Glu Phe Asp Gln Glu Arg Lys Lys Leu
            260                 265                 270

Gln Val Thr Asn Asp Pro Asn Tyr Ala Tyr Lys Phe Ala Gln Glu Val
        275                 280                 285

Arg Arg Ile Phe Pro Phe Val Pro Phe Leu Pro Gly Arg Leu Lys Lys
    290                 295                 300

Thr Val Glu Phe Asp Gly Phe Lys Leu Lys Lys Gly Thr Leu Thr Val
305                 310                 315                 320

Leu Asp Ile Phe Gly Thr Thr His Asp Pro Glu Leu Phe Glu Asn Pro
                325                 330                 335

Tyr Gln Leu Asn Pro Asp Arg Phe Asp Asn Trp Asp Gly Ser Pro Phe
            340                 345                 350

Asp Leu Ile Pro Gln Gly Gly Gly Asp Phe Tyr Thr Asn His Arg Cys
        355                 360                 365

Ala Gly Glu Trp Met Thr Val Ile Val Met Glu Glu Thr Ile Gln Tyr
    370                 375                 380

Phe Ala Asn Lys Ile Asp Phe Val Val Pro Ala Gln Asp Leu Ser Val
385                 390                 395                 400

Lys Leu Ser Gln Phe Pro Gly Lys Val Thr Ser Gly Thr Met Ile Lys
                405                 410                 415

Asn Val Tyr Pro Arg Ile
            420

<210>  144
<211>  422
<212>  PRT
<213>  Staphylococcus pseudintermedius

<400>  144

Met Ala Asn Lys Leu Pro Lys Asp Thr Gly Leu Asp Asn Thr Leu Lys
1               5                   10                  15

Met Ile Asn Glu Ala Tyr Thr Tyr Val Pro Lys Arg Leu Glu Lys Phe
            20                  25                  30

Gly Thr Lys Ala Phe Glu Thr Arg Ala Leu Gly Met Lys Pro Ile Val
        35                  40                  45

Val Ile Ser Gly Lys Ala Ala Ala Glu Leu Phe Tyr Asp Asn Asp Lys
    50                  55                  60

Ile Ser Arg Lys Gly Thr Leu Pro Lys Arg Ile Val His Thr Leu Phe
65                  70                  75                  80

Gly Lys Gly Ala Ile His Thr Thr Glu Gly Lys Val His Val Asp Arg
                85                  90                  95

```
Lys Ala Leu Phe Met Ser Leu Met Thr Glu Lys Asn Leu Lys Tyr Leu
            100                 105                 110

Arg Glu Leu Thr Arg Asn Tyr Trp Phe Met His Thr Glu Arg Met Gln
        115                 120                 125

Asn Lys Asp Glu Val Asn Val Tyr Gln Glu Ala Gly Leu Ile Leu Thr
    130                 135                 140

Lys Val Gly Phe Arg Trp Ala Gly Leu Arg Gln Thr Asp Glu Gln Ala
145                 150                 155                 160

Val Gln Asn Ala Glu Asp Met Asn Thr Met Ile Asp Ser Phe Ser Gly
                165                 170                 175

Leu Gly Gln Ser Leu Lys Gly Tyr Arg Glu Ala Lys Lys Ala Arg Ala
            180                 185                 190

Arg Val Glu Gln Phe Leu Gln Glu Gln Ile Glu Ala Val Arg Val Gly
        195                 200                 205

Gln Gln Tyr Ala Glu Pro Gly Thr Ala Leu Tyr Glu Phe Ala His Trp
    210                 215                 220

Lys Asp Leu Asn Asp Gln Pro Met Asp Pro His Leu Cys Ala Val Asp
225                 230                 235                 240

Leu Met Asn Ile Val Arg Pro Leu Val Ala Val Asn Arg Phe Val Ser
                245                 250                 255

Tyr Gly Val Lys Ala Leu Ile Glu Phe Asp Gln Glu Arg Lys Lys Leu
            260                 265                 270

Gln Val Thr Asn Asp Pro Asn Tyr Ala Tyr Lys Phe Ala Gln Glu Val
        275                 280                 285

Arg Arg Ile Phe Pro Phe Val Pro Phe Leu Pro Gly Arg Leu Lys Lys
    290                 295                 300

Thr Val Glu Phe Asp Gly Phe Lys Leu Lys Lys Gly Thr Leu Thr Val
305                 310                 315                 320

Leu Asp Ile Phe Gly Thr Thr His Asp Pro Glu Leu Phe Glu Asn Pro
                325                 330                 335

Tyr Gln Phe Asn Pro Asp Arg Phe Asp Asn Trp Asp Gly Ser Pro Phe
            340                 345                 350
```

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Val Ala Pro Pro Gln Asp Leu Ser Val
385 390 395 400

Lys Leu Ser Gln Phe Pro Gly Lys Val Thr Ser Gly Thr Met Ile Lys
405 410 415

Asn Val Tyr Pro Arg Ile
420

<210> 145
<211> 422
<212> PRT
<213> Staphylococcus pseudintermedius

<400> 145

Met Ala Lys Lys Leu Pro Lys Asp Thr Gly Leu Asp Asn Thr Leu Lys
1 5 10 15

Met Ile Asn Glu Ala Tyr Thr Tyr Val Pro Lys Arg Leu Glu Lys Phe
20 25 30

Gly Thr Lys Ala Phe Glu Thr Arg Ala Leu Gly Met Lys Pro Ile Val
35 40 45

Val Ile Ser Gly Lys Ala Ala Ala Glu Leu Phe Tyr Asp Asn Asp Lys
50 55 60

Ile Ser Arg Lys Gly Thr Leu Pro Lys Arg Ile Val His Thr Leu Phe
65 70 75 80

Gly Lys Gly Ala Ile His Thr Thr Glu Gly Lys Val His Val Asp Arg
85 90 95

Lys Ala Leu Phe Met Ser Leu Met Thr Glu Lys Asn Leu Lys Tyr Leu
100 105 110

Cys Glu Leu Thr Arg Asn Tyr Trp Phe Met His Thr Glu Arg Met Gln
115 120 125

Asn Lys Asp Glu Val Asn Val Tyr Gln Glu Ala Gly Leu Ile Leu Thr
130 135 140

Lys Val Gly Phe Arg Trp Ala Gly Leu Arg Gln Thr Asp Glu Gln Ala
145 150 155 160

Val Gln Asn Ala Glu Asp Met Asn Thr Met Ile Asp Ser Phe Ser Gly
165 170 175

Leu Gly Gln Ser Leu Lys Gly Tyr Arg Glu Ala Lys Lys Ala Arg Ala
180 185 190

Arg Val Glu Gln Phe Leu Gln Glu Gln Ile Glu Ala Val Arg Val Gly
195 200 205

Gln Gln Tyr Ala Glu Pro Gly Thr Ala Leu Tyr Glu Phe Ala His Trp
210 215 220

Lys Asp Leu Asn Asp Gln Pro Met Asp Pro His Leu Cys Ala Val Asp
225 230 235 240

Leu Met Asn Ile Val Arg Pro Leu Val Ala Val Asn Arg Phe Val Ser
245 250 255

Tyr Gly Val Lys Ala Leu Ile Glu Phe Asp Gln Glu Arg Lys Lys Leu
260 265 270

Gln Val Thr Asn Asp Pro Asn Tyr Ala Tyr Lys Phe Ala Gln Glu Val
275 280 285

Arg Arg Ile Phe Pro Phe Val Pro Phe Leu Pro Gly Arg Leu Lys Lys
290 295 300

Thr Val Glu Phe Asp Gly Phe Lys Leu Lys Lys Gly Thr Leu Thr Val
305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

Asp Leu Ile Pro Gln Gly Gly Gly Asp Phe Tyr Thr Asn His Arg Cys
355 360 365

Ala Gly Glu Trp Met Thr Val Ile Val Met Glu Glu Thr Ile Gln Tyr
370 375 380

Phe Ala Asn Lys Ile Asp Phe Val Val Pro Ala Gln Asp Leu Ser Val

385 390 395 400

Lys Leu Ser Gln Phe Pro Gly Lys Val Thr Ser Gly Thr Met Ile Lys
405 410 415

Asn Val Tyr Pro Arg Ile
420

<210> 146
<211> 371
<212> PRT
<213> Staphylococcus pseudintermedius

<400> 146

Gly Lys Ala Ala Ala Glu Leu Phe Tyr Asp Asn Asp Lys Ile Ser Arg
1 5 10 15

Lys Gly Thr Leu Pro Lys Arg Ile Val His Thr Leu Phe Gly Lys Gly
20 25 30

Ala Ile His Thr Thr Glu Gly Lys Val His Val Asp Arg Lys Ala Leu
35 40 45

Phe Met Ser Leu Met Thr Glu Lys Asn Leu Lys Tyr Leu Arg Glu Leu
50 55 60

Thr Arg Asn Tyr Trp Phe Met His Thr Glu Arg Met Gln Asn Lys Asp
65 70 75 80

Glu Val Asn Val Tyr Gln Glu Ala Gly Leu Ile Leu Thr Lys Val Gly
85 90 95

Phe Arg Trp Ala Gly Leu Arg Gln Thr Asp Glu Gln Ala Val Gln Asn
100 105 110

Ala Glu Asp Met Asn Thr Met Ile Asp Ser Phe Ser Gly Leu Gly Gln
115 120 125

Ser Leu Lys Gly Tyr Arg Glu Ala Lys Lys Ala Arg Ala Arg Val Glu
130 135 140

Gln Phe Leu Gln Glu Gln Ile Glu Ala Val Arg Val Gly Gln Gln Tyr
145 150 155 160

Ala Glu Pro Gly Thr Ala Leu Tyr Glu Phe Ala His Trp Lys Asp Leu
165 170 175

Asn Asp Gln Pro Met Asp Pro His Leu Cys Ala Val Asp Leu Met Asn

180 185 190

Ile Val Arg Pro Leu Val Ala Val Asn Arg Phe Val Ser Tyr Gly Val
195 200 205

Lys Ala Leu Ile Glu Phe Asp Gln Glu Arg Lys Lys Leu Gln Val Thr
210 215 220

Asn Asp Pro Asn Tyr Ala Tyr Lys Phe Ala Gln Glu Val Arg Arg Ile
225 230 235 240

Phe Pro Phe Val Pro Phe Leu Pro Gly Arg Leu Lys Lys Thr Val Glu
245 250 255

Phe Asp Gly Phe Lys Leu Lys Lys Gly Thr Leu Thr Val Leu Asp Ile
260 265 270

Leu Gly Thr Thr His Asp Pro Glu Leu Phe Glu Asn Pro Tyr Gln Phe
275 280 285

Asn Pro Asp Arg Phe Asp Asn Trp Asp Gly Ser Pro Phe Asp Leu Ile
290 295 300

Pro Gln Gly Gly Gly Asp Phe Tyr Thr Asn His Arg Cys Ala Gly Glu
305 310 315 320

Trp Met Thr Val Ile Val Met Glu Glu Thr Ile Gln Tyr Phe Ala Asn
325 330 335

Lys Ile Asp Phe Val Ala Pro Pro Gln Asp Leu Ser Val Lys Leu Ser
340 345 350

Gln Phe Pro Gly Lys Val Thr Ser Gly Thr Met Ile Lys Asn Val Tyr
355 360 365

Pro Arg Ile
370

<210> 147
<211> 352
<212> PRT
<213> Staphylococcus pseudintermedius

<400> 147

Met Ala Lys Lys Leu Pro Lys Asp Thr Gly Leu Asp Asn Thr Leu Lys
1 5 10 15

Met Ile Asn Glu Ala Tyr Thr Tyr Val Pro Lys Arg Leu Glu Lys Phe

20 25 30

Gly Thr Lys Ala Phe Glu Thr Arg Ala Leu Gly Met Lys Pro Ile Val
35 40 45

Val Ile Ser Gly Lys Ala Ala Ala Glu Leu Phe Tyr Asp Asn Asp Lys
50 55 60

Ile Ser Arg Lys Gly Thr Leu Pro Lys Arg Ile Val His Thr Leu Phe
65 70 75 80

Gly Lys Gly Ala Ile His Thr Thr Glu Gly Lys Val His Val Asp Arg
85 90 95

Lys Ala Leu Phe Met Ser Leu Met Thr Glu Lys Asn Leu Lys Tyr Leu
100 105 110

Arg Glu Leu Thr Arg Asn Tyr Trp Phe Met His Thr Glu Arg Met Gln
115 120 125

Asn Lys Asp Glu Val Asn Val Tyr Gln Glu Ala Gly Leu Ile Leu Thr
130 135 140

Lys Val Gly Phe Arg Trp Ala Gly Leu Lys Gln Thr Asp Glu Gln Ala
145 150 155 160

Ala Gln Asn Ala Glu Asp Met Asn Thr Met Ile Asp Ser Phe Ser Gly
165 170 175

Leu Gly Gln Ser Leu Lys Gly Tyr Arg Glu Ala Lys Lys Ala Arg Ala
180 185 190

Arg Val Glu Gln Phe Leu Gln Glu Gln Ile Glu Ala Val Arg Val Gly
195 200 205

Gln Gln Tyr Ala Glu Pro Gly Thr Ala Leu Tyr Glu Phe Ala His Trp
210 215 220

Lys Asp Leu Asn Asp Gln Pro Met Asp Pro His Leu Cys Ala Val Asp
225 230 235 240

Leu Met Asn Ile Val Arg Pro Leu Val Ala Val Asn Arg Phe Val Ser
245 250 255

Tyr Gly Val Lys Ala Leu Ile Glu Phe Asp Gln Glu Arg Lys Lys Leu
260 265 270

Gln Val Thr Asn Asp Pro Asn Tyr Ala Tyr Lys Phe Ala Gln Glu Val
275 280 285

Arg Arg Ile Phe Pro Phe Val Pro Phe Leu Pro Gly Arg Leu Lys Lys
290 295 300

Thr Val Glu Phe Asp Gly Phe Lys Leu Lys Lys Gly Thr Leu Thr Val
305 310 315 320

Leu Asp Ile Phe Gly Thr Thr His Asp Pro Glu Leu Phe Glu Asn Pro
325 330 335

Tyr Gln Phe Asn Pro Asp Arg Phe Asp Asn Trp Asp Gly Ser Pro Phe
340 345 350

<210> 148
<211> 423
<212> PRT
<213> Jeotgalicoccus saudimassiliensis

<400> 148

Met Met Ala Ser Leu Lys Arg Asp Lys Gly Leu Asp Asn Thr Leu Lys
1 5 10 15

Leu Met Lys Gln Gly Tyr Leu Tyr Thr Thr Asn Gln Arg Asn Arg Leu
20 25 30

Asp Thr Thr Val Phe Glu Thr Lys Val Leu Gly Gly Lys Pro Phe Ala
35 40 45

Val Val Thr Gly Lys Glu Gly Ala Glu Met Phe Tyr Asn Asn Asp Val
50 55 60

Val Gln Arg Glu Gly Met Leu Pro Lys Arg Ile Val Asn Thr Leu Phe
65 70 75 80

Gly Lys Gly Ala Ile His Thr Ile Asp Gly Lys Lys His Ile Asp Arg
85 90 95

Lys Ala Leu Phe Met Ser Leu Met Thr Glu Gly Asn Leu Asp Tyr Val
100 105 110

Arg Glu Leu Thr Arg Thr Leu Trp Arg Ala Asn Thr Gln Arg Met Glu
115 120 125

Ser Met Asp Gln Val Asn Ile Tyr Arg Glu Ser Ile Val Leu Leu Thr
130 135 140

Lys Val Gly Thr Arg Trp Ala Gly Val Gln Ala Pro Pro Glu Asp Ile
145 150 155 160

Glu Arg Ile Ala Thr Asp Met Asp Ile Met Ile Asp Ser Phe Lys Gly
165 170 175

Leu Gly Gly Ala Phe Lys Gly Tyr Lys Glu Ser Lys Ala Ala Arg Arg
180 185 190

Arg Val Glu Asp Trp Leu Glu Glu Gln Ile Leu Glu Thr Arg Lys Gly
195 200 205

Asn Ile His Pro Pro Glu Gly Thr Ala Leu Tyr Glu Phe Ala His Trp
210 215 220

Glu Asp Tyr Leu Gly Asn Pro Met Asp Ser Arg Asn Cys Ala Ile Asp
225 230 235 240

Leu Met Asn Thr Phe Arg Pro Leu Ile Ala Ile Asn Arg Phe Val Ser
245 250 255

Phe Gly Leu Leu Ala Met His Asp Asn Pro Ile Thr Arg Glu Lys Ile
260 265 270

Lys Ser Glu Pro Asp Tyr Ala Tyr Lys Phe Ala Gln Glu Val Arg Arg
275 280 285

Phe Tyr Pro Phe Val Pro Phe Leu Pro Gly Lys Ala Lys Val Asp Ile
290 295 300

Asp Phe Gln Gly Val Thr Ile Pro Ala Gly His Gly Leu Ala Leu Asp
305 310 315 320

Val Tyr Gly Thr Met His Asp Glu Thr Leu Trp Glu Asp Pro Asn Glu
325 330 335

Phe Arg Pro Glu Arg Phe Glu Asp Trp Asp Gly Ser Pro Phe Asp Leu
340 345 350

Ile Pro Gln Gly Gly Gly Asp Tyr Trp Thr Asn His Arg Cys Ala Gly
355 360 365

Glu Trp Ile Thr Val Ile Ile Met Glu Glu Thr Met Lys Tyr Phe Ala
370 375 380

Glu Lys Val Thr Tyr Asp Val Pro Glu Gln Asp Leu Thr Val Asp Leu
385 390 395 400

Asn Ser Ile Pro Gly Tyr Val Lys Ser Gly Phe Ile Ile Ser Asn Val
405 410 415

Gln Glu Val Val Asp Arg Lys
420

<210> 149
<211> 423
<212> PRT
<213> Jeotgalicoccus sp.

<400> 149

Met Met Ala Ser Leu Lys Arg Asp Lys Gly Leu Asp Asn Thr Leu Lys
1 5 10 15

Leu Met Lys Gln Gly Tyr Leu Tyr Thr Thr Asn Gln Arg Asn Arg Leu
20 25 30

Asn Thr Thr Val Phe Glu Thr Lys Val Leu Gly Gly Lys Pro Phe Ala
35 40 45

Val Val Thr Gly Lys Glu Gly Ala Glu Met Phe Tyr Asn Asn Asp Ile
50 55 60

Val Gln Arg Glu Gly Met Leu Pro Lys Arg Ile Val Asn Thr Leu Phe
65 70 75 80

Gly Lys Gly Ala Ile His Thr Ile Asp Gly Lys Lys His Ile Asp Arg
85 90 95

Lys Ala Leu Phe Met Ser Leu Met Thr Glu Gly Asn Leu Asn Tyr Val
100 105 110

Arg Glu Leu Thr Arg Thr Leu Trp Gln Ala Asn Thr Gln Arg Met Glu
115 120 125

Ser Met Asp Gln Val Asn Ile Tyr Arg Glu Ser Ile Val Leu Leu Thr
130 135 140

Lys Val Gly Thr Arg Trp Ala Gly Val Gln Ala Pro Pro Glu Asp Ile
145 150 155 160

Glu Arg Ile Ala Thr Asp Met Asp Ile Met Ile Asp Ser Phe Lys Gly
165 170 175

Leu Gly Gly Val Phe Lys Gly Tyr Lys Glu Ser Lys Ala Ala Arg Arg
180 185 190

Arg Val Glu Asp Trp Leu Glu Glu Gln Ile Leu Glu Thr Arg Lys Gly
        195                 200                 205

Asn Ile His Pro Pro Glu Gly Thr Ala Leu Tyr Glu Phe Ala His Trp
    210                 215                 220

Glu Asp Tyr Leu Gly Asn Pro Met Asp Ser Arg Asn Cys Ala Ile Asp
225                 230                 235                 240

Leu Met Asn Thr Phe Arg Pro Leu Ile Ala Ile Asn Arg Phe Val Ser
                245                 250                 255

Phe Gly Leu Leu Ala Met His Asp Asn Pro Ile Thr Arg Glu Lys Ile
            260                 265                 270

Lys Ser Glu Pro Asp Tyr Ala Tyr Lys Phe Ala Gln Glu Val Arg Arg
        275                 280                 285

Phe Tyr Pro Phe Val Pro Phe Leu Pro Gly Lys Ala Lys Val Asp Ile
    290                 295                 300

Asp Phe Gln Gly Val Thr Ile Pro Ala Gly His Gly Leu Ala Ile Asp
305                 310                 315                 320

Val Tyr Gly Thr Met His Asp Glu Thr Leu Trp Glu Asn Pro Asn Glu
                325                 330                 335

Phe Arg Pro Glu Arg Phe Glu Asp Trp Asp Gly Ser Pro Phe Asp Leu
            340                 345                 350

Ile Pro Gln Gly Gly Gly Asp Tyr Trp Thr Asn His Arg Cys Ala Gly
        355                 360                 365

Glu Trp Ile Thr Val Ile Ile Met Glu Glu Thr Met Lys Tyr Phe Ala
    370                 375                 380

Glu Lys Val Thr Tyr Asp Val Pro Glu Gln Asp Leu Thr Val Asp Leu
385                 390                 395                 400

Asn Ser Ile Pro Gly Tyr Val Lys Ser Gly Phe Ile Ile Ser Asn Val
                405                 410                 415

Gln Glu Val Val Asp Arg Lys
            420

<210> 150
<211> 422

<212> PRT
<213> Jeotgalicoccus saudimassiliensis

<400> 150

Met Ala Ser Leu Lys Arg Asp Lys Gly Leu Asp Asn Thr Leu Lys Leu
1               5                   10                  15

Met Lys Gln Gly Tyr Leu Tyr Thr Thr Asn Gln Arg Asn Arg Leu Asp
            20                  25                  30

Thr Thr Val Phe Glu Thr Lys Val Leu Gly Gly Lys Pro Phe Ala Val
        35                  40                  45

Val Thr Gly Lys Glu Gly Ala Glu Met Phe Tyr Asn Asn Asp Val Val
    50                  55                  60

Gln Arg Glu Gly Met Leu Pro Lys Arg Ile Val Asn Thr Leu Phe Gly
65                  70                  75                  80

Lys Gly Ala Ile His Thr Ile Asp Gly Lys Lys His Ile Asp Arg Lys
                85                  90                  95

Ala Leu Phe Met Ser Leu Met Thr Glu Gly Asn Leu Asp Tyr Val Arg
            100                 105                 110

Glu Leu Thr Arg Thr Leu Trp Arg Ala Asn Thr Gln Arg Met Glu Ser
        115                 120                 125

Met Asp Gln Val Asn Ile Tyr Arg Glu Ser Ile Val Leu Leu Thr Lys
    130                 135                 140

Val Gly Thr Arg Trp Ala Gly Val Gln Ala Pro Pro Glu Asp Ile Glu
145                 150                 155                 160

Arg Ile Ala Thr Asp Met Asp Ile Met Ile Asp Ser Phe Lys Gly Leu
                165                 170                 175

Gly Gly Ala Phe Lys Gly Tyr Lys Glu Ser Lys Ala Ala Arg Arg Arg
            180                 185                 190

Val Glu Asp Trp Leu Glu Glu Gln Ile Leu Glu Thr Arg Lys Gly Asn
        195                 200                 205

Ile His Pro Pro Glu Gly Thr Ala Leu Tyr Glu Phe Ala His Trp Glu
    210                 215                 220

Asp Tyr Leu Gly Asn Pro Met Asp Ser Arg Asn Cys Ala Ile Asp Leu
225                 230                 235                 240

Met Asn Thr Phe Arg Pro Leu Ile Ala Ile Asn Arg Phe Val Ser Phe
245 250 255

Gly Leu Leu Ala Met His Asp Asn Pro Ile Thr Arg Glu Lys Ile Lys
260 265 270

Ser Glu Pro Asp Tyr Ala Tyr Lys Phe Ala Gln Glu Val Arg Arg Phe
275 280 285

Tyr Pro Phe Val Pro Phe Leu Pro Gly Lys Ala Lys Val Asp Ile Asp
290 295 300

Phe Gln Gly Val Thr Ile Pro Ala Gly His Gly Leu Ala Leu Asp Val
305 310 315 320

Tyr Gly Thr Met His Asp Glu Thr Leu Trp Glu Asp Pro Asn Glu Phe
325 330 335

Arg Pro Glu Arg Phe Glu Asp Trp Asp Gly Ser Pro Phe Asp Leu Ile
340 345 350

Pro Gln Gly Gly Gly Asp Tyr Trp Thr Asn His Arg Cys Ala Gly Glu
355 360 365

Trp Ile Thr Val Ile Ile Met Glu Glu Thr Met Lys Tyr Phe Ala Glu
370 375 380

Lys Val Thr Tyr Asp Val Pro Glu Gln Asp Leu Thr Val Asp Leu Asn
385 390 395 400

Ser Ile Pro Gly Tyr Val Lys Ser Gly Phe Ile Ile Ser Asn Val Gln
405 410 415

Glu Val Val Asp Arg Lys
420

<210> 151
<211> 441
<212> PRT
<213> Macrococcus caseolyticus

<400> 151

Met Arg Val Glu Phe Thr Ile Asn Tyr Ile Asn Val Glu Gly Ile Ser
1 5 10 15

Met Ser Lys Arg Val Pro Lys Asp Arg Gly Ile Asp Asn Ser Leu Lys
20 25 30

```
Ile Met Lys Glu Gly Tyr Glu Tyr Val Pro Ala Arg Met Lys Lys Phe
        35                  40                  45

Asn Thr Asn Ile Phe Glu Thr Arg Val Leu Gly Gly Lys Thr Ala Val
    50                  55                  60

Val Ile Ser Gly Lys Asp Ala Ala Glu Leu Phe Tyr Asp Asn Asp Lys
65                  70                  75                  80

Thr Glu Arg Lys Gly Thr Leu Pro Lys Arg Val Val Lys Thr Leu Phe
                85                  90                  95

Gly Lys Gly Ala Ile His Thr Thr Thr Gly Lys Lys His Ile Asp Arg
            100                 105                 110

Lys Ala Leu Phe Met Ser Leu Met Thr Asp Glu Asn Leu Ala Tyr Leu
        115                 120                 125

Arg Lys Leu Thr Arg Ile Tyr Trp Phe Gln Asn Ile Glu His Met Gln
    130                 135                 140

Tyr Lys Gln Lys Val Asn Val Tyr Glu Glu Ala Thr Glu Leu Leu Thr
145                 150                 155                 160

Lys Val Gly Leu Arg Trp Ala Gly Ile Val Asp His Pro Glu Asn Ile
                165                 170                 175

Gln Lys Met Ala Asp Asp Met Asn Lys Met Ile Asp Ser Phe Ser Ala
            180                 185                 190

Ile Gly Ser Leu Tyr Gly Gly Tyr Arg Glu Ala Lys Lys Ala Arg Ala
        195                 200                 205

Arg Val Glu Gln Phe Leu Glu Asp Gln Ile Thr Ala Val Arg Lys Gly
    210                 215                 220

Lys Ile His Pro Glu Lys Gly Thr Ala Leu Tyr Glu Phe Ser His Trp
225                 230                 235                 240

Glu Asp Met Asn Gly Lys Pro Met Asp Ala Arg Leu Cys Ala Val Asp
                245                 250                 255

Leu Met Asn Val Ile Arg Pro Leu Val Ala Ile Asn Lys Phe Val Ser
            260                 265                 270

Phe Gly Val Leu Ala Leu His Glu Phe Pro Gly Glu Arg Val Arg Val
```

        275                 280                 285

Ala Leu Asn Glu Gly Asp Tyr Ala Tyr Lys Phe Val Gln Glu Val Arg
    290                 295                 300

Arg Tyr Tyr Pro Phe Val Pro Phe Leu Pro Gly Lys Ala Lys Glu Asn
305                 310                 315                 320

Ile Thr Phe Asp Gly Tyr Lys Ile Gln Lys Asp Thr Met Met Leu Leu
                325                 330                 335

Asp Ile Tyr Gly Thr Leu His Arg Asp Asp Leu Phe Ser Glu Pro Glu
            340                 345                 350

Arg Phe Asn Pro Tyr Arg Phe Asp Asn Trp Asp Gly Ser Pro Phe Asp
        355                 360                 365

Leu Ile Pro Gln Gly Gly Gly Asp Tyr Tyr Thr Asn His Arg Cys Ala
    370                 375                 380

Gly Glu Trp Met Thr Ile Ile Ile Met Glu Glu Thr Met Lys Phe Phe
385                 390                 395                 400

Ala Asn Glu Ile Ser Tyr Asp Val Pro Pro Gln Asp Phe Thr Val Asp
                405                 410                 415

Thr Thr Lys Phe Pro Gly Lys Val Ala Ser Gly Met Asp Ile Glu Asn
            420                 425                 430

Ile Arg Val Asn Ile Asp Arg Thr Lys
        435                 440

<210>  152
<211>  1266
<212>  DNA
<213>  Artificial Sequence

<220>
<223>  Codon optimized artificial DNA sequence

<400>  152
atggcgaccc tgaagcgtga caaaggcctg gataacaccc tgaaggtgct gaaacagggt        60

tacctgtata ccaccaacca acgtaaccgt ctgaacacca gcgttttcca gaccaaagcg       120

ctgggtggca agccgtttgt ggttgtgacc ggcaaagagg gtgcggaaat gttctacaac       180

aacgacgttg tgcaacgtga gggcatgctg ccgaaacgta tcgtgaacac cctgttcggc       240

aagggtgcga ttcacaccgt tgacggcaag aaacacgtgg atcgtaaggc gctgtttatg       300

agcctgatga ccgagggtaa cctgaactac gttcgtgaac tgacccgtac cctgtggcac       360

gcgaacaccc agcgtatgga gagcatggac gaagtgaaca tctatcgtga aagcattgtt 420

ctgctgacca aagtgggcac ccgttgggcg ggtgttcaag ctccgccgga ggatatcgaa 480

cgtattgcga ccgacatgga tatcatgatt gacagcttcc gtgcgctggg tggcgcgttt 540

aagggttata aagcgagcaa agaggcgcgt cgtcgtgtgg aagattggct ggaggaacag 600

atcattgaga cccgtaaggg caacatccac ccgccggaag gtaccgcgct gtacgagttc 660

gcgcactggg aagactatct gggcaacccg atggatagcc gtacctgcgc gattgacctg 720

atgaacacct ttcgtccgct gatcgcgatt aaccgtttcg tgagctttgg tctgcacgcg 780

atgaacgaga acccgatcac ccgtgagaag attaaaagcg aaccggacta cgcgtataaa 840

ttcgcgcagg aagtgcgtcg ttactatccg ttcgttccgt ttctgccggg caaggcgaaa 900

gttgacatcg attttcaagg tgttaccatc ccggcgggcg tgggtctggc gctggatgtt 960

tacggtacca cccacgacga gagcctgtgg gacgatccga acgaattccg tccggagcgt 1020

tttgaaacct gggacggcag cccgttcgat ctgatcccgc agggtggcgg tgattattgg 1080

accaaccacc gttgcgcggg cgagtggatc accgtgatca ttatggagga aaccatgaaa 1140

tacttcgcgg aaaagattac ctatgacgtg ccggagcaag acctggaagt tgatctgaac 1200

agcatcccgg gctacgttaa aagcggtttt gttattaaga acgtgcgtga ggttgtggat 1260

cgtacc 1266

<210> 153
<211> 436
<212> PRT
<213> Jeotgalicoccus sp.

<400> 153

Met His His His His His His Glu Asn Leu Tyr Phe Gln Gly Met Ala
1 5 10 15

Thr Leu Lys Arg Asp Lys Gly Leu Asp Asn Thr Leu Lys Val Leu Lys
20 25 30

Gln Gly Tyr Leu Tyr Thr Thr Asn Gln Arg Asn Arg Leu Asn Thr Ser
35 40 45

Val Phe Gln Thr Lys Ala Leu Gly Gly Lys Pro Phe Val Val Val Thr
50 55 60

Gly Lys Glu Gly Ala Glu Met Phe Tyr Asn Asn Asp Val Val Gln Arg
65 70 75 80

Glu Gly Met Leu Pro Lys Arg Ile Val Asn Thr Leu Phe Gly Lys Gly
85 90 95

Ala Ile His Thr Val Asp Gly Lys Lys His Val Asp Arg Lys Ala Leu
100 105 110

Phe Met Ser Leu Met Thr Glu Gly Asn Leu Asn Tyr Val Arg Glu Leu
115 120 125

Thr Arg Thr Leu Trp His Ala Asn Thr Gln Arg Met Glu Ser Met Asp
130 135 140

Glu Val Asn Ile Tyr Arg Glu Ser Ile Val Leu Leu Thr Lys Val Gly
145 150 155 160

Thr Arg Trp Ala Gly Val Gln Ala Pro Pro Glu Asp Ile Glu Arg Ile
165 170 175

Ala Thr Asp Met Asp Ile Met Ile Asp Ser Phe Arg Ala Leu Gly Gly
180 185 190

Ala Phe Lys Gly Tyr Lys Ala Ser Lys Glu Ala Arg Arg Arg Val Glu
195 200 205

Asp Trp Leu Glu Glu Gln Ile Ile Glu Thr Arg Lys Gly Asn Ile His
210 215 220

Pro Pro Glu Gly Thr Ala Leu Tyr Glu Phe Ala His Trp Glu Asp Tyr
225 230 235 240

Leu Gly Asn Pro Met Asp Ser Arg Thr Cys Ala Ile Asp Leu Met Asn
245 250 255

Thr Phe Arg Pro Leu Ile Ala Ile Asn Arg Phe Val Ser Phe Gly Leu
260 265 270

His Ala Met Asn Glu Asn Pro Ile Thr Arg Glu Lys Ile Lys Ser Glu
275 280 285

Pro Asp Tyr Ala Tyr Lys Phe Ala Gln Glu Val Arg Arg Tyr Tyr Pro
290 295 300

Phe Val Pro Phe Leu Pro Gly Lys Ala Lys Val Asp Ile Asp Phe Gln
305 310 315 320

Gly Val Thr Ile Pro Ala Gly Val Gly Leu Ala Leu Asp Val Tyr Gly
325 330 335

Thr Thr His Asp Glu Ser Leu Trp Asp Asp Pro Asn Glu Phe Arg Pro

340 345 350

Glu Arg Phe Glu Thr Trp Asp Gly Ser Pro Phe Asp Leu Ile Pro Gln
355 360 365

Gly Gly Gly Asp Tyr Trp Thr Asn His Arg Cys Ala Gly Glu Trp Ile
370 375 380

Thr Val Ile Ile Met Glu Glu Thr Met Lys Tyr Phe Ala Glu Lys Ile
385 390 395 400

Thr Tyr Asp Val Pro Glu Gln Asp Leu Glu Val Asp Leu Asn Ser Ile
405 410 415

Pro Gly Tyr Val Lys Ser Gly Phe Val Ile Lys Asn Val Arg Glu Val
420 425 430

Val Asp Arg Thr
435

<210> 154
<211> 1308
<212> DNA
<213> Artificial Sequence

<220>
<223> Codon optimized artificial DNA sequence

<400> 154
catatgcacc accaccacca ccactcaaaa cgcgttccca aagatagagg gattgataac 60

agcctgaaga tcatgaaaga gggttacgaa tatgttccgg cgcgtatgaa gaagttcaac 120

accaacatct tcgagacccg tgtgctgggt ggcaagaccg cggtggttat cagcggcaaa 180

gatgcggcgg agctgttcta cgacaacgat aagaccgaac gtaaaggcac cctgccgaag 240

cgtgtggtta aaccctgtt cggcaagggt gcgattcaca ccaccaccgg taagaaacac 300

atcgatcgta aagcgctgtt tatgagcctg atgaccgacg agaacctggc gtacctgcgt 360

aagctgaccc gtatttattg gtttcagaac atcgagcaca tgcagtacaa gcaaaaagtg 420

aacgtttatg aggaagcgac cgaactgctg accaaagttg gcctgcgttg ggcgggtatt 480

gtggatcacc cggaaaacat ccaaaagatg gcggacgata tgaacaaaat gattgacagc 540

ttcagcgcga tcggcagcct gtacggtggc tatcgtgagg cgaagaaagc gcgtgcgcgt 600

gttgagcagt ttctggaaga ccaaattacc gcggtgcgta agggcaaaat ccacccggaa 660

aagggcaccg cgctgtacga gttcagccac tgggaagaca tgaacggtaa accgatggat 720

gcgcgtctgt gcgcggtgga cctgatgaac gttattcgtc cgctggttgc gatcaacaag 780

ttcgtgagct ttggcgttct ggcgctgcac gagttcccgg gtgaacgtgt gcgtgttgcg 840

ctgaacgagg gcgattacgc gtataaattt gtgcaggaag ttcgtcgtta ctatccgttc 900

gtgccgtttc tgccgggcaa ggcgaaagag aacattacct tcgacggtta caagatccaa 960

aaagacacca tgatgctgct ggatatttat ggcaccctgc accgtgacga cctgttcagc 1020

gagccggaac gtttcaaccc gtaccgtttt gacaactggg atggcagccc gtttgatctg 1080

atcccgcagg gtggcggtga ctactatacc aaccaccgtt gcgcgggcga gtggatgacc 1140

atcattatca tggaggaaac catgaagttc tttgcgaacg aaatcagcta tgacgttccg 1200

ccgcaagact tcaccgtgga taccaccaag tttccgggca aagttgcgag cggtatggat 1260

attgaaaaca tccgtgtgaa catcgaccgt accaaataat gaaagctt 1308

<210> 155
<211> 431
<212> PRT
<213> Micrococcus lylae

<400> 155

Met His His His His His His Ser Lys Arg Val Pro Lys Asp Arg Gly
1 5 10 15

Ile Asp Asn Ser Leu Lys Ile Met Lys Glu Gly Tyr Glu Tyr Val Pro
20 25 30

Ala Arg Met Lys Lys Phe Asn Thr Asn Ile Phe Glu Thr Arg Val Leu
35 40 45

Gly Gly Lys Thr Ala Val Val Ile Ser Gly Lys Asp Ala Ala Glu Leu
50 55 60

Phe Tyr Asp Asn Asp Lys Thr Glu Arg Lys Gly Thr Leu Pro Lys Arg
65 70 75 80

Val Val Lys Thr Leu Phe Gly Lys Gly Ala Ile His Thr Thr Thr Gly
85 90 95

Lys Lys His Ile Asp Arg Lys Ala Leu Phe Met Ser Leu Met Thr Asp
100 105 110

Glu Asn Leu Ala Tyr Leu Arg Lys Leu Thr Arg Ile Tyr Trp Phe Gln
115 120 125

Asn Ile Glu His Met Gln Tyr Lys Gln Lys Val Asn Val Tyr Glu Glu
130 135 140

Ala Thr Glu Leu Leu Thr Lys Val Gly Leu Arg Trp Ala Gly Ile Val
145 150 155 160

Asp His Pro Glu Asn Ile Gln Lys Met Ala Asp Asp Met Asn Lys Met
165 170 175

Ile Asp Ser Phe Ser Ala Ile Gly Ser Leu Tyr Gly Gly Tyr Arg Glu
180 185 190

Ala Lys Lys Ala Arg Ala Arg Val Glu Gln Phe Leu Glu Asp Gln Ile
195 200 205

Thr Ala Val Arg Lys Gly Lys Ile His Pro Glu Lys Gly Thr Ala Leu
210 215 220

Tyr Glu Phe Ser His Trp Glu Asp Met Asn Gly Lys Pro Met Asp Ala
225 230 235 240

Arg Leu Cys Ala Val Asp Leu Met Asn Val Ile Arg Pro Leu Val Ala
245 250 255

Ile Asn Lys Phe Val Ser Phe Gly Val Leu Ala Leu His Glu Phe Pro
260 265 270

Gly Glu Arg Val Arg Val Ala Leu Asn Glu Gly Asp Tyr Ala Tyr Lys
275 280 285

Phe Val Gln Glu Val Arg Arg Tyr Tyr Pro Phe Val Pro Phe Leu Pro
290 295 300

Gly Lys Ala Lys Glu Asn Ile Thr Phe Asp Gly Tyr Lys Ile Gln Lys
305 310 315 320

Asp Thr Met Met Leu Leu Asp Ile Tyr Gly Thr Leu His Arg Asp Asp
325 330 335

Leu Phe Ser Glu Pro Glu Arg Phe Asn Pro Tyr Arg Phe Asp Asn Trp
340 345 350

Asp Gly Ser Pro Phe Asp Leu Ile Pro Gln Gly Gly Gly Asp Tyr Tyr
355 360 365

Thr Asn His Arg Cys Ala Gly Glu Trp Met Thr Ile Ile Ile Met Glu
370 375 380

Glu Thr Met Lys Phe Phe Ala Asn Glu Ile Ser Tyr Asp Val Pro Pro
385 390 395 400

Gln Asp Phe Thr Val Asp Thr Thr Lys Phe Pro Gly Lys Val Ala Ser

405 410 415

Gly Met Asp Ile Glu Asn Ile Arg Val Asn Ile Asp Arg Thr Lys
420 425 430

<210> 156
<211> 453
<212> PRT
<213> Staphylococcus massiliensis

<400> 156

Met Phe Val Asp Ser Ile Leu Val Leu Arg Leu Asn Leu Leu Lys Thr
1 5 10 15

Gly Ile Gln Leu Glu Met Lys Asn Gly Gly Ile Lys Val Ala Lys Lys
20 25 30

Leu Pro Lys Val Lys Gly Leu Asp Asn Thr Val Asp Ile Ile Lys Gly
35 40 45

Gly Tyr Thr Tyr Val Pro Gly Lys Leu Glu Glu Phe Asp Ser Lys Ala
50 55 60

Phe Glu Val Arg Ala Leu Gly Gly Lys Lys Ile Ala Val Met Ser Gly
65 70 75 80

Lys Glu Ala Ala Glu Ile Phe Tyr Asp Asn Glu Lys Met Glu Arg Gln
85 90 95

Gly Thr Leu Pro Lys Arg Ile Val Asn Thr Leu Phe Gly Lys Gly Ala
100 105 110

Ile His Thr Thr Ala Gly Lys Lys His Val Asp Arg Lys Ala Leu Phe
115 120 125

Met Ser Leu Met Thr Asp Glu Asn Leu Asn Tyr Leu Arg Glu Leu Thr
130 135 140

Arg Asn Tyr Trp Phe Met Asn Thr Glu Arg Met Gln Ser Met Asp Lys
145 150 155 160

Val Asn Val Tyr Asn Glu Ser Ile Tyr Met Leu Thr Lys Ile Gly Phe
165 170 175

Arg Trp Ala Gly Ile Ile Gln Thr Pro Glu Glu Ala Glu Gln Asn Ala
180 185 190

Lys Asp Met Asp Thr Met Ile Asn Ser Phe Val Ser Leu Gly Ser Ala

```
        195                 200                 205

Tyr Lys Gly Tyr Lys Lys Ala Lys Lys Ala Arg Lys Arg Val Glu Asp
    210                 215                 220

Phe Leu Glu Lys Gln Ile Ile Asp Val Arg Lys Gly Lys Leu His Pro
225                 230                 235                 240

Glu Glu Gly Thr Ala Leu Tyr Glu Phe Ala His Trp Glu Asp Leu Asn
                245                 250                 255

Asp Asn Pro Met Asp Ser His Leu Cys Ala Val Asp Leu Met Asn Val
            260                 265                 270

Val Arg Pro Leu Ala Ala Ile Asn Arg Phe Ile Ser Tyr Gly Val Lys
        275                 280                 285

Val Leu Ile Glu Phe Asp Gln Glu Lys Glu Lys Leu Arg Leu Glu Asn
    290                 295                 300

Asn Glu Asp Tyr Ala Tyr Lys Phe Ala Gln Glu Val Arg Arg Ile Phe
305                 310                 315                 320

Pro Phe Val Pro Tyr Leu Pro Gly Arg Ala Ala Val Asp Leu Glu Tyr
                325                 330                 335

Asp Gly Tyr Lys Ile Pro Ala Gly Met Met Thr Ala Leu Asp Val Tyr
            340                 345                 350

Gly Thr Thr His Asp Glu Asp Leu Trp Glu Asn Pro Asp Gln Phe Asn
        355                 360                 365

Pro Asn Arg Phe Asp Asn Trp Asp Gly Ser Pro Phe Asp Leu Ile Pro
    370                 375                 380

Gln Gly Gly Gly Asp Phe Tyr Thr Asn His Arg Cys Ala Gly Glu Trp
385                 390                 395                 400

Ile Thr Val Ile Ile Met Glu Glu Thr Met Lys Tyr Phe Ala Asn Lys
                405                 410                 415

Ile Glu Phe Asp Val Pro Ser Gln Asp Leu Ser Val Lys Leu Asp Lys
            420                 425                 430

Leu Pro Gly Asn Val Thr Ser Gly Thr Ile Ile Ser Asn Val Arg Pro
        435                 440                 445
```

Arg Val Ala Arg Lys
    450

<210> 157
<211> 425
<212> PRT
<213> Staphylococcus massiliensis

<400> 157

Met Ala Lys Lys Leu Pro Lys Val Lys Gly Leu Asp Asn Thr Val Asp
1               5                   10                  15

Ile Ile Lys Gly Gly Tyr Thr Tyr Val Pro Gly Lys Leu Glu Glu Phe
            20                  25                  30

Asp Ser Lys Ala Phe Glu Val Arg Ala Leu Gly Gly Lys Lys Ile Ala
        35                  40                  45

Val Met Ser Gly Lys Glu Ala Ala Glu Ile Phe Tyr Asp Asn Glu Lys
    50                  55                  60

Met Glu Arg Gln Gly Thr Leu Pro Lys Arg Ile Val Asn Thr Leu Phe
65                  70                  75                  80

Gly Lys Gly Ala Ile His Thr Thr Ala Gly Lys Lys His Val Asp Arg
                85                  90                  95

Lys Ala Leu Phe Met Ser Leu Met Thr Asp Glu Asn Leu Asn Tyr Leu
            100                 105                 110

Arg Glu Leu Thr Arg Asn Tyr Trp Phe Met Asn Thr Glu Arg Met Gln
        115                 120                 125

Ser Met Asp Lys Val Asn Val Tyr Asn Glu Ser Ile Tyr Met Leu Thr
    130                 135                 140

Lys Ile Gly Phe Arg Trp Ala Gly Ile Ile Gln Thr Pro Glu Glu Ala
145                 150                 155                 160

Glu Gln Asn Ala Lys Asp Met Asp Thr Met Ile Asn Ser Phe Val Ser
                165                 170                 175

Leu Gly Ser Ala Tyr Lys Gly Tyr Lys Lys Ala Lys Lys Ala Arg Lys
            180                 185                 190

Arg Val Glu Asp Phe Leu Glu Lys Gln Ile Ile Asp Val Arg Lys Gly
        195                 200                 205

Lys Leu His Pro Glu Glu Gly Thr Ala Leu Tyr Glu Phe Ala His Trp
    210                 215                 220

Glu Asp Leu Asn Asp Asn Pro Met Asp Ser His Leu Cys Ala Val Asp
225                 230                 235                 240

Leu Met Asn Val Val Arg Pro Leu Ala Ala Ile Asn Arg Phe Ile Ser
                245                 250                 255

Tyr Gly Val Lys Val Leu Ile Glu Phe Asp Gln Glu Lys Glu Lys Leu
            260                 265                 270

Arg Leu Glu Asn Asn Glu Asp Tyr Ala Tyr Lys Phe Ala Gln Glu Val
        275                 280                 285

Arg Arg Ile Phe Pro Phe Val Pro Tyr Leu Pro Gly Arg Ala Ala Val
    290                 295                 300

Asp Leu Glu Tyr Asp Gly Tyr Lys Ile Pro Ala Gly Met Met Thr Ala
305                 310                 315                 320

Leu Asp Val Tyr Gly Thr Thr His Asp Glu Asp Leu Trp Glu Asn Pro
                325                 330                 335

Asp Gln Phe Asn Pro Asn Arg Phe Asp Asn Trp Asp Gly Ser Pro Phe
            340                 345                 350

Asp Leu Ile Pro Gln Gly Gly Gly Asp Phe Tyr Thr Asn His Arg Cys
        355                 360                 365

Ala Gly Glu Trp Ile Thr Val Ile Ile Met Glu Glu Thr Met Lys Tyr
    370                 375                 380

Phe Ala Asn Lys Ile Glu Phe Asp Val Pro Ser Gln Asp Leu Ser Val
385                 390                 395                 400

Lys Leu Asp Lys Leu Pro Gly Asn Val Thr Ser Gly Thr Ile Ile Ser
                405                 410                 415

Asn Val Arg Pro Arg Val Ala Arg Lys
            420                 425

<210> 158
<211> 422
<212> PRT
<213> Jeotgalicoccus halophilus

<400> 158

Met Ala Ser Leu Lys Arg Asp Lys Gly Leu Asp Asn Thr Leu Lys Val
1               5                   10                  15

Met Lys Gln Gly Tyr Leu Tyr Thr Thr Asn Gln Arg Asn Arg Leu Asn
            20                  25                  30

Thr Thr Val Phe Glu Thr Lys Ala Leu Gly Gly Lys Pro Phe Ala Val
        35                  40                  45

Val Thr Gly Lys Glu Gly Ala Glu Met Phe Tyr Asn Asn Asp Ile Val
    50                  55                  60

Gln Arg Glu Gly Met Leu Pro Lys Arg Ile Val Asn Thr Leu Phe Gly
65                  70                  75                  80

Lys Gly Ala Ile His Thr Ile Asp Gly Lys Lys His Val Asp Arg Lys
                85                  90                  95

Ala Leu Phe Met Ser Leu Met Thr Glu Gly Asn Leu Asn Tyr Val Arg
            100                 105                 110

Glu Leu Thr Arg Thr Leu Trp Gln Ala Asn Thr Gln Arg Met Glu Ser
        115                 120                 125

Met Asp Glu Val Asn Ile Tyr Arg Glu Ser Ile Val Leu Leu Thr Lys
    130                 135                 140

Val Gly Thr Arg Trp Ala Gly Val Gln Ala Pro Pro Glu Gln Ile Glu
145                 150                 155                 160

Arg Ile Ala Thr Asp Met Asp Ile Met Ile Asp Ser Phe Lys Gly Leu
                165                 170                 175

Gly Gly Val Phe Lys Gly Tyr Lys Glu Ser Lys Ala Ala Arg Arg Arg
            180                 185                 190

Val Glu Asp Trp Leu Glu Asp Gln Ile Leu Glu Thr Arg Lys Gly Asn
        195                 200                 205

Ile His Pro Pro Glu Gly Thr Ala Leu Tyr Glu Phe Ala His Trp Glu
    210                 215                 220

Asp Tyr Leu Gly Asn Pro Met Asp Ser Arg Asn Cys Ala Ile Asp Leu
225                 230                 235                 240

Met Asn Thr Phe Arg Pro Leu Ile Ala Ile Asn Arg Phe Val Ser Phe
                245                 250                 255

Gly Leu Leu Ala Met His Asp Asn Pro Val Ser Arg Glu Lys Ile Lys
260 265 270

Ser Glu Pro Asp Tyr Ala Tyr Lys Phe Ala Gln Glu Val Arg Arg Tyr
275 280 285

Tyr Pro Phe Val Pro Phe Leu Pro Gly Lys Ala Lys Thr Asp Ile Asp
290 295 300

Phe Gln Gly Val Thr Ile Pro Ala Gly His Gly Leu Ala Ile Asp Val
305 310 315 320

Tyr Gly Thr Leu His Asp Glu Ser Leu Trp Glu Asp Pro Asn Glu Phe
325 330 335

Arg Pro Glu Arg Phe Glu Gly Trp Asp Gly Ser Pro Phe Asp Leu Ile
340 345 350

Pro Gln Gly Gly Gly Asp Tyr Trp Thr Asn His Arg Cys Ala Gly Glu
355 360 365

Trp Ile Thr Val Ile Ile Met Glu Glu Thr Met Lys Tyr Phe Ala Glu
370 375 380

Lys Val Thr Tyr Asp Val Pro Glu Gln Asp Leu Thr Val Asp Leu Asn
385 390 395 400

Ser Ile Pro Gly Tyr Val Lys Ser Gly Phe Val Ile Lys Asn Val Gln
405 410 415

Glu Val Val Asp Arg Arg
420

<210> 159
<211> 1332
<212> DNA
<213> Artificial Sequence

<220>
<223> Codon optimized artificial DNA sequence

<400> 159
catatgcatc accaccacca ccacgagaac ctgtacttcc agggcatggc gaagcgtatg 60

ccgaaggtga aaggtctgga ccaaaccctg accgttctga aagagggtta tgaattcatt 120

ccgaaccgta cccgtaaact gaacaccaac gtttttgaga ttcgtatcct gggtggcaag 180

aaagcggtgg ttatcagcgg caaagaggcg gcggaactgt tttacgacaa cgataaaacc 240

gaacgtgcgg gtaccctgcc gaagcgtctg gtgaaaaccc tgttcggcaa gggtgcgatt 300

cacaccacca ccggtgataa gcacaaaaac cgtaaaagcc tgtttatgag cctgatgacc 360

gacaacaacc tggagtacct gcgtaaactg acccgtaact attggttcga agataccgcg 420

cgtctgcagg cgatggacca aaccaacgtg taccaggaag cgaccctggt tctgctgaag 480

attggcttcc gttttgcgga cgttccgttc gataccccgg agcgtatgga acaatatgcg 540

aaagacatga acgcgatggt ggatagcttt ggcagcatcg gtaccgcgta caccggttat 600

cgtcgtgcgc tgaacgcgcg tgaccgtgtt gaggattacc tggaaaagca gatcattgcg 660

acccgtaagg gcaaactgtt cccggaggaa ggcagcggtc tgtatgagtt tagccactgg 720

aaggacatga acggtaaaca catggatagc cgtctggcgg cggtggacct gatgaacacc 780

atccgtccgc tggttgcgat taaccgtttc atcagctttg gcgtgctggc gatgcacgag 840

tacccgggtg aacgtgcgcg tgtggcgcag aacgacaaca gctacgcgta taaattcgcg 900

caagaagtgc gtcgttacta tccgttcgtt ccgtttctgc cgggcaaggc gaaacaaaac 960

attaccttcg agggttacaa gatcgacaaa gataccatgc tgattctgga tatctttggt 1020

accctgcaca gcgaaagcct gtgggagaac gcggaacagt tctatccgga gcgttttgaa 1080

aactgggacg gcagcccgtt tgatctgatt ccgcaaggtg gcggtgatta ctataccaac 1140

caccgttgcg cgggcgagtg gatgaccatc attatcatgg aggaaaccat gaagtacttc 1200

gcgcgtgaaa tccagtatga cgtgccgccg caagacttta ccgttgatcg taccaaactg 1260

ccgggccaga ttaacagcgg tatgattatc gagaacatcc gtaacaactt cgatcgtcgt 1320

cgttaactcg ag 1332

<210> 160
<211> 1320
<212> DNA
<213> Artificial Sequence

<220>
<223> Codon optimized artificial DNA sequence

<400> 160
catatgcatc accaccacca ccacgagaac ctgtacttcc agggcatggc gaagaaactg 60

ccgaaagata ccggtctgga caacaccctg aagatcatta acgaagcgta cacctatgtg 120

ccgaagcgtc tggagaaatt cggcaccaag gcgtttgaaa cccgtgcgct gggcatgaaa 180

ccggtggttg tgatcagcgg caaggcggcg gcggagctgt tttatgacaa cgataaaatc 240

agccgtaagg gcaccctgcc gaaacgtatt gttcacaccc tgttcggcaa gggtgcgatc 300

cacaccaccg agggtaaagt tcacgtggat cgtaaggcgc tgtttatgag cctgatgacc 360

gagaagaacc tgaaatacct gcgtgaactg acccgtaact attggttcat gcacaccgag 420

cgtatgcaga acaaagatga ggttaacgtg taccaagaag cgggcctgat tctgaccaaa 480

gtgggctttc gttgggcggg tctgaagcag accgacgagc aggcggcgca aaacgcggaa 540

gatatgaaca ccatgatcga cagcttcagc ggcctgggtc agagcctgaa aggttatcgt 600

gaagcgaaga aagcgcgtgc gcgtgtggag caatttctgc aggaccaaat cgaagcggtg 660

cgtgcgggtc agcaatatgc ggagccgggt accgcgctgt atgaattcgc gcactggaag 720

gacctgaacg atcaaccgat ggatccgcac ctgtgcgcgg ttgacctgat gaacattgtg 780

cgtccgctgg ttgcggtgaa ccgtttcgtt agctacggtg tgaaagcgct gatcgagttt 840

gatcaggagc gtaagaaact gcaagttacc cacgacccga actacgcgta taagttcgcg 900

caggaagtgc gtcgtatttt cccgtttgtg ccgtttctgc cgggccgtct gaaacaaacc 960

gtggaattcg atggctttaa gctgaagaaa ggtaccctga ccgttctgga tatcttcggt 1020

accacccacg acccggagct gtttgaaaac ccgtatcagt tcaacccgga ccgttttgat 1080

aactgggacg gcagcccgtt cgatctgatt ccgcaaggtg gcggtgactt ttacaccaac 1140

caccgttgcg cgggcgagtg gatgaccgtt atcgtgatgg aggaaaccat tcagtatttc 1200

gcgaacaaaa tcgactttga agcgccggcg caggatctga gcgttaaact ggaccaattc 1260

ccgggcaagg tgaccagcgg taccatcatt aagagcgttt acccgcgtat ttaactcgag 1320

<210> 161
<211> 1317
<212> DNA
<213> Artificial Sequence

<220>
<223> Codon optimized artificial DNA sequence

<400> 161
catatgcatc accaccacca ccacgagaac ctgtacttcc agggcatgaa caagattccg 60

aaggacaaag gtctggataa caccctgaag atcgtgaaag aagcgtacac ctatgttccg 120

cgtcgtctgg aggcgttcga caccaacgtg tttgaaaccc gtgcgctggg catgaagaaa 180

gttctggtgg ttagcggtaa gaaagcggcg gagctgtttt atgataacga aaagatcagc 240

cgtaaaggcg cgctgccgaa acgtattgtg aacaccctgt ttggtcaggg tgcgatccac 300

accaccgagg gcaagcaaca cgttgaccgt aaagcgctgt ttatgagcct gatgaccacc 360

aagaacctga aatacctgcg tgaactgacc cgtaactatt ggttcgcgca cacccagcgt 420

atggaggaca tggatgaagt gaacgtttac gaggaagcga ccctgctgct gaccaagatt 480

ggctttcgtt gggcgggtct gaaacagacc cacgaggaag cggtgcaaaa cgcgaaggac 540

atggatctgc tgatcgatag ctttagcgcg ctgggtcaaa gcccggcggg ttataagaaa 600

gcgaagaaag cgcgtgagcg tgtggaaaag tttctggagc accagattaa agcggttcgt 660

gagggcgaac aatacgcggc gccgaacacc gcgctgtatg aattcgcgca ctggaaggac 720

ctgaacgata aaccgatgga cctgcacctg tgcgcggtgg atctgatgaa cgtggtgcgt 780

ccgctggttg cggttaaccg tttcgtgagc tacgcggtta aagcgctgat cgagtttgac 840

caggaacgtc tgaagctgca ggttaccaaa gatcaaaact acgcgtataa gtttgcgcag 900

gaagtgcgtc gtattttccc gtttgttccg ttcctgccgg gccgtctgaa acaaaacatc 960

gaatttgacg gttacagcct gaagaaaaac accctgatca ttctggacgt gttcggtacc 1020

acccacgatc cgaagctgtt tgagaacccg tatcagttca acccgaaccg ttttgaagac 1080

tgggatggca gcccgttcga cctgatcccg caaggtggcg gtgattttta caccaaccac 1140

cgttgcgcgg gcgagtggat gaccgtgatc gttatggagg aaaccatcaa ctacttcgcg 1200

aacaagatcg acttcagcgt gccgacccag gacctgagcg ttaagctgga tcaattcccg 1260

ggcaaagtta ccagcggtac cgtgatccgt aacgttcgtc cgcgtcgtta actcgag 1317

<210> 162
<211> 1326
<212> DNA
<213> Artificial Sequence

<220>
<223> Codon optimized artificial DNA sequence

<400> 162
catatgcatc accaccacca ccacgagaac ctgtactttc agggcatgag caagcaactg 60

aacatcccga aagaggaagg cctggaccac agcctggcgc tgatccagga aggttaccaa 120

tatattacca gccgtcgtac caagtttaaa agcgacctgt tcgaaacccg tctgctgggc 180

cagaaagtgg tttgcattgg tggccaagag ggtgcggaac tgttttatga caacgagaag 240

ttcgaacgta agggtgcggt tccgaagcgt atccagaaaa gcctgtttgg cgaaaacgcg 300

attcacaccc tggatggcga ggcgcacgaa caccgtaagc tgctgttcat gagcctgatg 360

accgaggaac gtctggagct gctggcggaa ctgacccgtg aggaatggcg tctggcggcg 420

gcggagtggg cgaaaatgga acaggtggtt ctgtttgacg aggttcagca aattctgtgc 480

cgtgttgcgt gccgttgggc gggcgttccg atcgatgcga ttgaagtgaa gccgctggcg 540

cgtgacttta ttgcgatggt tgatgcgttt ggtgcggtgg gtccgcgtca ccaaaagggc 600

cgtaaagcgc gtaagaaaat ggaggactac ttcaagggtc tgatcattga aatccgtcac 660

cgtcgtatcg agccgattga aggtaccgcg ctgcacaaaa ttgcgtggca ccgtgaccac 720

aaggagaaac acctggatga acacaccgcg gcggtggagc tgctgaacgt tctgcgtccg 780

atcgtggcga ttacctacct ggttaccttt ggcgcgctgg cgctgttcga gcacaacgtg 840

tggaaggaaa aactgcagag cggtgatgag gaaagcgttc agatgttcgc gcaagaagtg 900

cgtcgtttct atccgtttgc gccgttcctg ggtgcgaagg ttaaaaacgg ctttgagtgg 960

aagggtcaca ccttcgaaaa aggccaactg gtgctgctgg acgtttacgg taccaaccac 1020

gataaccgtc tgtgggagga accgtatgtg tttaacccgg agcgtttccg taactggaac 1080

ggtggcatgt ttgacctgat cccgcagggt ggcggtgatg agtacaccgg ccaccgttgc 1140

ccgggtgaaa tggcgaccat ccaagtgatg aaaaccagct tcagctttct gaccaacgag 1200

atgatttata acgtgccggt tggtcaggac ctgagctaca gcctgagccg tatgccgacc 1260

tatccggaaa gcggcttcat cattcagggt gttagcatca agagccaaac caccgtgtaa 1320

ctcgag 1326

<210> 163
<211> 1326
<212> DNA
<213> Artificial Sequence

<220>
<223> Codon optimized artificial DNA sequence

<400> 163
catatgcatc accaccacca ccacgagaac ctgtactttc agggcatgag caagcaactg 60

aacatcccga aagaggaagg cctggaccac agcctggcgc tgatccagga aggttaccaa 120

tatattacca gccgtcgtac caagtttaaa agcgacctgt tcgaaacccg tctgctgggc 180

cagaaagtgg tttgcattgg tggccaagag ggtgcggaac tgttttatga caacgagaag 240

ttcgaacgta agggtgcggt tccgaagcgt atccagaaaa gcctgtttgg cgaaaacgcg 300

attcacaccc tggatggcga ggcgcacgaa caccgtaagc tgctgttcat gagcctgatg 360

accgaggaac gtctggagct gctggcggaa ctgacccgtg aggaatggcg tctggcggcg 420

gcggagtggg cgaaaatgga acaggtggtt ctgtttgacg aggttcagca aattctgtgc 480

cgtgttgcgt gccgttgggc gggcgttccg atcgatgcga ttgaagtgaa gccgctggcg 540

cgtgacttta ttgcgatggt tgatgcgttt ggtgcggtgg gtccgcgtca ccaaaagggc 600

cgtaaagcgc gtaagaaaat ggaggactac ttcaagggtc tgatcattga aatccgtcac 660

cgtcgtatcg agccgattga aggtaccgcg ctgcacaaaa ttgcgtggca ccgtgaccac 720

aaggagaaac acctggatga acacaccgcg gcggtggagc tgctgaacgt tctgcgtccg 780

atcgtggcga ttacctacct ggttaccttt ggcgcgctgg cgctgttcga gcacaacgtg 840

tggaaggaaa aactgcagag cggtgatgag gaaagcgttc agatgttcgc gcaagaagtg 900

cgtcgtttct atccgtttgc gccgttcctg ggtgcgaagg ttaaaaacgg ctttgagtgg 960

aagggtcaca ccttcgaaaa aggccaactg gtgctgctgg acgtttacgg taccaaccac 1020

gataaccgtc tgtgggagga accgtatgtg tttaacccgg agcgtttccg taactggaac 1080

ggtggcatgt ttgacctgat cccgcagggt ggcggtgatg agtacaccgg ccaccgttgc 1140

ccgggtgaaa tggcgaccat ccaagtgatg aaaaccagct tcagctttct gaccaacgag 1200

atgatttata acgtgccggt tggtcaggac ctgagctaca gcctgagccg tatgccgacc 1260

tatccggaaa gcggcttcat cattcagggt gttagcatca agagccaaac caccgtgtaa 1320

ctcgag 1326

<210> 164
<211> 1329
<212> DNA
<213> Artificial Sequence

<220>
<223> Codon optimized artificial DNA sequence

<400> 164
catatgcatc accaccacca ccacgagaac ctgtacttcc agggcatggg taaaagcatt 60

ccgcgtgacc gtggcatgga taacaccgtt aaggcgctga agaaaggtta cacctatatt 120

ccggagctgc gtgaacgtct gaacagcaac gatatcgttg cgacccgtgc gctgggtggc 180

aagaaaaccg tgatcattag cggcaaagac gcggcggagc tgttttacga caacgataag 240

atcgaacgtc aaggtaccct gccgccgcgt gtggttaaga ccctgttcgg caaaggtgcg 300

attcacacca ccagcggcaa gacccacatc gatcgtaaag cgctgtttat gagcctgatg 360

accgaggaaa acctgagcta tctgcgtgag ctgacccgtc tggaatggta ctataacacc 420

acccgtatga gccacatgga cgaggtgaac gtttacaaag aagcgattta tgtgctgacc 480

aaggttggca tcaaatgggc gggtctgcac gcgagcgagg atgaaattaa ccagtacgcg 540

gaggacatgg ataccatgat cgacagcttc aaagcgattg gcaccatgtt taagggttat 600

aaagaagcgc gtaaggcgcg tgatcgtgtt gaaacctggc tggaagcgca gattcaaggc 660

gtgcgtgatg gtgaaattac cccgccggaa ggtaccgcgc tgtacgagtt cgcgcactgg 720

aaagactatg aaggtaaccc gatggatgcg cgtctggcgg cgatcgacct gatgaacgtg 780

attcgtccgc tggttgcgat caaccgtttc gttgcgtttg gtaccctggc gatgtacgag 840

ttcccgaagg aacgtaccaa agtggcgcag aacgaggaag attacgtgta taagtttatc 900

caagaggttc gtcgtttcta cccgtttgtg ccgtatctgc cgggcaaggc gggtaaagac 960

attgagttcc gtggctacga aatcaagaaa gacaccttta tggtgctgga tgtttatggt 1020

accctgcacc gtgaggacct gtgggaaaac gcggatcagt tcattccgga gcgttttgaa 1080

gactgggatg gcagcccgtt cgatctgatc ccgcaaggtg gcggtgacta ctataccaac 1140

caccgttgcg cgggcgagtg gatgaccgtg attgttatgg aggaaagcat gaaatacttt 1200

gcgcaggcga tcaagtatga agttccggcg caagacctga ccgtggatct gaacaagctg 1260

ccgggccgtg ttaaaagcgg tttcatcatt aagaacgtgg aacgtgacat caaccgtttt 1320

taactcgag 1329

<210> 165

<211> 1320
<212> DNA
<213> Artificial Sequence

<220>
<223> Codon optimized artificial DNA sequence

<400> 165
catatgcatc accaccacca ccacgaaaac ctgtactttc agggcatggc gaagattaaa 60

cgtgacaagg gtctggataa caccctgaag gttttcaaac agggctacct gtataccacc 120

aaccaacgtg agcgtctggg tgcggaagtg tttgaaaccc gtgcgctggg tggcaaaagc 180

tacgtggttc tgagcggcaa ggaaggtgct gagctgttct atgacaacga taaaatcgaa 240

cgtgagggtg gcctgccgaa gcgtgtggtt aacaccctgt tcggcaaagg tgcgattcac 300

accaccaccg gcaaacaaca catcgatcgt aaggcgctgt ttatgagcct gatgaccgaa 360

ggtaacctgg agtacgttcg taaactgacc cgtaactatt ggaaggcgaa caccgcgcgt 420

atggaagcga tgggcgacgt gaacgtttac cacgagagca ttgttctgct gacccgtatt 480

ggtatgcgtt gggcgggtgt gaccgcgccg gaggaagaga ttgaacgtat cgcggaggac 540

atggatatta tgatcgatag ctttaagggc ctgggtaacg cgttcaaggg ttataaaagc 600

agcaaggacg cgcgtaaacg tgtggaagac tggctggagg atcagatcat taagacccgt 660

aacggcgaaa ttaacccgcc gaaaggtagc agcctgtacg aactggcgca ctggcgtgac 720

tatgagggca accaaatgga tagccgtctg gcgggtatcg acctgatgaa cacctttcgt 780

ccgctgattg cgatcaaccg tttcgtgagc tacggtgttc tggcgctgta tgaacacccg 840

gaggcgatcc gtaaaatgag caccgttgaa gactacccgt atatgttcgc gcaggaagtg 900

cgtcgtttct acccgtttgt gccgttcctg ccgggcaaga ccaaagtgga agttgagcac 960

aaaggtgtga ccattccgaa ggaccagcgt ctggtgatcg atgtttatgg taccctgcac 1020

agcgaagagc tgtggaacca accgaacaaa tttattccgg aacgtttcaa ggactgggat 1080

ggcagcccgt tcgatatgat cccgcagggt ggcggtgact actggaccaa ccaccgttgc 1140

gcgggcgagt ggattaccat catcatcatg gaagaaacca tgaagtactt cgcgaacgaa 1200

attacctggg atgttccgga gcaagacctg accatcgatc tgaacagcat tccgggctac 1260

atcaacagcg gtatggtgat caacaacgtt cgtgagaaag tggaccgttt ttaactcgag 1320

<210> 166
<211> 1296
<212> DNA
<213> Artificial Sequence

<220>
<223> Codon optimized artificial DNA sequence

<400> 166
catatgcatc accaccacca ccacgagaac ctgtacttcc agggcatgaa cgtgaacgtt 60

gcgaaagacc gtctggatca aaccatcagc ctggttcgtg agggttacgc gtttattccg 120

aaccgtctgc acagccagca cctggacgtg tatgaaacca aactgctggg ccaacgtatc 180

gcgctgattg gtggcgagga aggtgcgaac ctgttctatg atagcagccg tatgaagcgt 240

aacggtgcgc tgccgaaacc ggttctgaag accctgttcg gcgagggtgg cgtgcatacc 300

aaagatggtg cggcgcatgc gcaccgtaag cagctgttta tgagcctgat gaccccggat 360

gcgctgcgtc gtctgagcaa cctgaccacc gcgtactggg aggaatatgc gatgaagtgg 420

gaaaaacagc gtaaggtggt tctgtacgac gagagccgtg aactgctgtg ccaagtggcg 480

tgcgattggg cgggtatccc gctgggtaag aaagaggtgg aacaacgtac cgaggacctg 540

agcaaaatga tcgatggctt cagcgcgctg ggtccgaagc acattgaaag ccgtcgtgcg 600

cgtaaccgta gcgagaaatg gattcgtagc atcattgaag acgttcgtgc gaacaagctg 660

gaaacccgtg agggcagcgc gatccacgag atggcgtttc acctggatga aaacggtgac 720

cgtctggata ccgagattgc ggcggtggaa ctgctgaacg ttatccgtcc gctggttgcg 780

attagcaaat tcctggcgtt tggcgcgaag gcgtacgcgg actatccgga aacccgtgag 840

aagaccaaag agagcgatga atacatcctg cgtttcgcgc aggaagttcg tcgttactat 900

ccgttcgcgc cgtttctggg cgcgaaggtg aaacacgact tcacctggca gggttatacc 960

tttcgtgaga accaactggt tatgatcgac ctgttcggta ccaaccacga tccgcgtctg 1020

tgggaaaacc cggaccagtt cgacccggat cgttttaaag actggaaggg tggcctgttt 1080

gatctgattc cgcaaggtgg cggtgactac tatgatggtc atcgttgccc gggcgagtgg 1140

ccgaccatcg aagttctgaa agcgagcttt ggctacctga gccgtaagat tgactataaa 1200

gtgccgaagc aagatcacag ctacagcctg agcaaaatgc cggcgctgcc gaagagcggt 1260

atgatcctga agaaagtgcg tcgtaagtaa ctcgag 1296

<210> 167
<211> 1314
<212> DNA
<213> Artificial Sequence

<220>
<223> Codon optimized artificial DNA sequence

<400> 167
catatgcatc accaccacca ccacgagaac ctgtactttc agggcatgag caaactgaag 60

gaaagcggtt tcgatcacac cctgaccatg ctgaaagagg gctacaccta tattccgaac 120

cgtaccgaaa agtaccaaag cccgatcttc tttaaccgtg ttctgggtgg caaaccgtgc 180

gcgatcatta gcggcaaaga ggcggcggaa ctgttttata acaacgacct gattgagcgt 240

aaaggtacca tgccgaaacg tatcgttaag accctgtttg gcaagggtgc gattcacacc 300

accaccggta aagtgcacat cgatcgtaag gcgctgttca tgagcctgat gaccgacgaa 360

aacctgagcc tgctgcgtca gattaccaaa gacgcgtggg agaacgaagt tgatagcctg 420

atcaaggtga acgaggtgaa cgtttacaag aaaagcagcg aagtgctgac caaagttggc 480

ctgaagtggg cgggtattga ggacaccccg aaaaacattg ataaaatcgc gaaggaaatg 540

gacctgatga tcgatagctt cggccagctg ggtggcgcgt acaaaggtta tcgtgcggcg 600

caaaaggcgc gtgcgaacgt tgaggcgtac ctgcagaacc aaattaagaa agtgcgtaaa 660

ggcaagctga aaccgcgtga aaacagcgcg ctgtatgcgt ttagccactg gaaagacatg 720

aacaacaagc cgatggatct ggcgctgtgc gcggtggacc tgatgaacgt tattcgtccg 780

ctggttgcga tcaaccgttt cgtgacctac ggtgttctgg cgatccacga gttcaacggc 840

gaaaaggata aactgaagtt tggtaccgac gattacgttt atcagttcgt gcaagaggtt 900

cgtcgtttct acccgtttgt gccgtatctg ccgggcaaag cgaagaaaaa gctgaccttt 960

gacaaccaga agattaaaaa ggaccaaatg atcctgctgg atgtgtacgg taccctgcac 1020

aacgagaaac tgtgggaata tccgaaccgt ttcgatccga cccgttttga aaactgggac 1080

ggcagcccgt tcgatctgat cccgcagggt ggcggtgact atcacaccaa ccaccgttgc 1140

gcgggcgagt ggatgaccat cattatcatg gaggaaacca tgaagttctt tacccgtacc 1200

attagcttcg aagttccgcc gcaagacttt accgtggatc tgaccaaact gccgggcaag 1260

gttgcgagcg gtatgaacat ccgtcaggtg caagcgctga acaactaact cgag 1314

<210> 168
<211> 1308
<212> DNA
<213> Artificial Sequence

<220>
<223> Codon optimized artificial DNA sequence

<400> 168
catatgcatc accaccacca ccacgctaaa aaattaccaa aagtaaaggg gctggacaac 60

accgttgata tcattaaagg tggctacacc tatgtgccgg gcaagctgga ggagttcgac 120

agcaaagcgt ttgaagtgcg tgcgctgggt ggcaagaaaa tcgcggttat gagcggcaaa 180

gaggcggcgg aaattttcta cgataacgag aagatggaac gtcagggcac cctgccgaaa 240

cgtatcgtga acaccctgtt cggcaagggt gcgattcaca ccaccgcggg caagaaacac 300

gttgaccgta aagcgctgtt tatgagcctg atgaccgatg agaacctgaa ctacctgcgt 360

gaactgaccc gtaactattg gttcatgaac accgagcgta tgcaaagcat ggacaaagtg 420

aacgtttaca acgaaagcat ctatatgctg accaagattg gctttcgttg ggcgggtatc 480

attcagaccc cggaggaagc ggagcaaaac gcgaaagaca tggataccat gatcaacagc 540

ttcgtgagcc tgggcagcgc gtacaagggt tataagaaag cgaagaaagc gcgtaagcgt 600

gtggaggact ttctggaaaa acagatcatt gatgttcgta agggcaaact gcacccggag 660

gaaggcaccg cgctgtacga gttcgcgcac tgggaagacc tgaacgataa cccgatggac 720

agccacctgt gcgcggttga tctgatgaac gtggttcgtc cgctggcggc gatcaaccgt 780

ttcattagct atggtgtgaa agttctgatt gaatttgacc aggagaagga aaaactgcgt 840

ctggagaaca acgaagatta cgcgtataag tttgcgcaag aggtgcgtcg tatcttcccg 900

tttgttccgt acctgccggg tcgtgcggcg gtggacctgg aatacgatgg ctataaaatt 960

ccggcgggta tgatgaccgc gctggacgtt tatggcacca cccacgacga ggatctgtgg 1020

gaaaacccgg atcagttcaa cccgaaccgt tttgacaact gggatggcag cccgttcgac 1080

ctgatcccgc aaggtggcgg tgatttttac accaaccacc gttgcgcggg cgagtggatc 1140

accgtgatca ttatggagga aaccatgaag tacttcgcga acaagatcga atttgacgtg 1200

ccgagccaag atctgagcgt taagctggac aaactgccgg gcaacgttac cagcggcacc 1260

atcattagca acgtgcgtcc gcgtgttgcg cgtaagtaat gaaagctt 1308

<210> 169
<211> 1329
<212> DNA
<213> Artificial Sequence

<220>
<223> Codon optimized artificial DNA sequence

<400> 169
catatgcatc accaccacca ccacgaaaac ctgtacttcc aaggcatggc gaagcgtatg 60

ccgaaagacc gtggtctgga taacaccctg accattatga aagagggtta cgaatatatc 120

cagaaccgta ccaagaaatt caacagcaac gtgtttgaaa cccgtgtgct gggtggcaag 180

caagcggtgg ttattagcgg caaacaggcg gcggaactgt tttatgacaa cgataaaatt 240

gagcgtagcg gtaccctgcc gccgcgtgtg gttaagaccc tgttcggcaa aggtgcgatt 300

cacaccacca ccggtaagaa acacatcgat cgtaaggcgc tgtttatgag cctgatgacc 360

gacgaaaacc tgacctacct gcgtaaactg acccgtaact attggttcca gaacacccaa 420

cgtatgcagc tgatggatga agtgaacgtt tacaaggaga gcatcattct gctgaccaaa 480

attggctttc gttgggcggg tatcattgaa accccggaaa acatcgagca acacgcgatg 540

gacatggata agatgattga tagcttcggc gcgatcggta gcccgtacaa aggctatcgt 600

gaagcgaaga aagcgcgtac ccgtgttgag gactttctgg aggaacaaat cattaagacc 660

cgtaagggca aaattcatcc ggagccgggt agcgcgctgt acgaattcag ccactgggag 720

gacatgaacg gtcagccgat ggatagccgt ctgtgcagcg tggacctgat gaacgttatt 780

cgtccgctgg ttgcgatcaa ccgtttcgtg gcgtatggcg ttctggcgat gcacgaattt 840

ccgggcgagc gtacccgtgt tgcgcgtgac gatgacgatt acgcgtataa gttcgtgcaa 900

gaagttcgtc gttactatcc gttcgtgccg tttctgccgg gcaaggcggc gaagaacatc 960

gagttcgatg gttacaagat caagaaagac accatgatca ttctggatgt gttcggtacc 1020

ctgcaccgtg aagacacctt tagcgagccg gaacgtttca acccgtaccg ttttgacaac 1080

tgggatggca gcccgttcga tctgatcccg cagggtggcg gtgactatca caccaaccac 1140

cgttgcgcgg gtgaatggat gaccatcatt atcatggagg aaaccatgaa gtacttcgcg 1200

caagagattg actatgatgt tccgccgcag gactttaccg tggatcgtac caagctgccg 1260

ggccgtgtta aaagcggtat gattatcaac aacgtgcgtc tgaacatcga ccgtaccaaa 1320

taactcgag 1329

<210> 170
<211> 1329
<212> DNA
<213> Artificial Sequence

<220>
<223> Codon optimized artificial DNA sequence

<400> 170
catatgcatc accaccacca ccacgaaaac ctgtacttcc agggcatgag caagccgatt 60

ccggtggaca agggtatcga taaaaccctg accgttatga aagagggtta cgaatatgtg 120

agcaaccgta ccaagaaatt caacagcgat atttttgaaa cccgtgttct gggtggcaag 180

cgtgcgatgg tgatcagcgg caaagaggcg gcggaactgt tctatgacaa cgataagatc 240

gagcgtaaag gtaccctgcc gccgcgtgtg gttaagaccc tgtttggcaa aggtgcgatt 300

cacaccacca ccggcaaggt tcacatcgac cgtaaaagcc tgttcatgag cctgatgacc 360

gaaaacaacc tggagtacct gcgtaagctg acccgtaact tttggtatca gaacaccgaa 420

cgtatgcaac tgatgggcaa ggtgaacgtt tacaaagaga gcatcattct gctgaccaaa 480

gttggcttcc gttgggcggg tgtgaccgag aagccggaaa aaattgagca gcacgcgatg 540

gacatggata agatgatcga tagcttcggc agcgtgggta ccgcgtacaa aggctatcgt 600

gaagcgaaga aagcgcgtgc gcgtgttgaa gactttctgg agaagcagat cattgcgacc 660

cgtaagggca aaattcaccc ggaggaaggt accgcgctgc acagcttcag ccactggcgt 720

gactttcagg gtaactacat ggatagccgt ctggcggcgg tggacctgat gaacgttatt 780

cgtccgctgg ttgcgatcaa ccgtttcgtg gcgtatggcg ttctggcgct gcacgagttt 840

ccgggcgagc gtgaacgtgt gagcgcggat gaaaacaact acgcgtataa atttacccaa 900

gagctgcgtc gttacttccc gtttgttccg ttcctgccgg cgaaggcgaa agttgacatt 960

gattttcagg gccacgtgat cccgcaagac accatgattg ttctggatat cttcggtacc 1020

ctgcaccgtg aagacctgtg ggaagatccg gagcgtttct acccggatcg ttttgagaac 1080

tgggacggca gcccgtttga tctgatcccg cagggtggcg gtgactatca caccaaccac 1140

cgttgcgcgg gtgaatggat gaccatcatt atcatggagg aaaccatgaa gtacttcgcg 1200

cgtgagatta cctatgacgt tccggcgcaa gactttaccg tggatcgtac caagctgccg 1260

ggccgtgtga aaagcggtat ggatatcgag aacgttcgtc tgaacttcga ccgtaccctg 1320

taactcgag 1329

<210> 171
<211> 1323
<212> DNA
<213> Artificial Sequence

<220>
<223> Codon optimized artificial DNA sequence

<400> 171
catatgcatc accaccacca ccacgagaac ctgtactttc agggcatgag caccatcaag 60

cgtgacaaag gtctggataa caccctgaag gtgatgaaag agggctacct gtataccacc 120

aaccaacgta accgtctggg cgcggataac atctttgaaa cccgtggtct gggtggcaag 180

cgtgtgctgg ttctgagcgg caagaaagcg gcggaaacct tctatgacaa cgataagatt 240

gaacgtaaag gtaccctgcc gaagcgtgtg gttaacaccc tgtttggcaa aggtgcgatc 300

cacaccacca ccggcaagaa acacattgat cgtaaggcgc tgttcatgag cctgatgacc 360

gagggtaacc tgaaacacct gcgtgaactg acccgtaacc actggtacat gaacacccac 420

cgtatggagc agatggacca aatcaacatt tatcgtgaaa gcatcattct gctgaccaaa 480

gtgggcacca aatgggcggg tgttcaggcg ccggaggaaa aaatcgagga aattgcgacc 540

gacatggata tcatgattga cagctttcgt ggcctgggta ccgcgttcaa gggttaccgt 600

agcagcgtga aagcgcgtcg tcgtgttgag gactggctgg aagatcagat cattcaaacc 660

cgtaagggca aaatctttcc gccggaaggt accgcgctgt acgagttcgc gcactgggaa 720

gactatctgg gcaacccgat ggatagccgt ctgtgcgcga ttgacctgat gaacaccttc 780

cgtccgctga tcgcgattaa ccgtttcgtg agctttggtc tgctggcgat gcacgagttt 840

ccggttagcc gtgagaagat caacaacgaa gacgattacg cgtatatgtt cagccaagaa 900

gtgcgtcgtt tctacccgtt tgttccgttc ctgccggcga aggcgaaaac cgacttccac 960

tttgatggcc acaaggtgga caaagatacc atgctgctgc tggatatcta tggtaccatg 1020

caccgtgagg acgtttttga aaacccgaac gagtttcgtc cggaacgttt cctggactgg 1080

gatggtagcc cgttcgatct gattccgcag ggtggcggtg actactatac caaccaccgt 1140

tgcgcgggcg agtggatgac cgtgatcatt atggaggaaa ccatgaaata ctttgcgggt 1200

cgtatcacct atgatgtgcc ggaacaagac ctgcgtgttg atctgaacag catcccgggc 1260

tacgttaaga gcggtttcgt gattgagaac gttaaagaaa acctggaccg tcgttaactc 1320

gag 1323

<210> 172
<211> 1323
<212> DNA
<213> Artificial Sequence

<220>
<223> Codon optimized artificial DNA sequence

<400> 172
catatgcatc accaccacca ccacgaaaac ctgtacttcc aaggcatgag caccattaag 60

aaagacaagg gtctggataa caccctgaag gcgatcaaac agggctacaa gtataccgaa 120

aaccaacgta aacgtctgga cagcaacaac atttttgaaa cccgtggtct gggtggtaaa 180

cgtatcattt tcatcagcgg taaagaggcg gcggaaacct tttatgacaa cgataagatc 240

gagcgtgagg gtatgctgcc gaaacgtgtg gttagcaccc tgttcggcaa gggtgcgatt 300

cacaccacca ccggcaaggt gcacatcgat cgtaaagcgc tgtttatgag cctgatgacc 360

gagggtaacc tgcagtacct gcgtgaactg acccgtaacc aatggcgtag caacaccatt 420

aacatggaac gtcaggacac cgtgaacgtt tatcgtgagg cgattgtgca actgacccac 480

atcggcaccc gttgggcggg tgttcaggcg ccggcggagg aaattgatcg tatcgcgacc 540

gacatggatc tgatgatcga cagcttcaaa ggcctgggtc tgagcttcaa gggttttcgt 600

gaaagcaaag cggcgcgtcg tcgtgtggag gactggctgg aagatcagat tcacaagacc 660

cgtaaaggcg ttattcaccc gccggagggt accgcgctgt acgaattcgc gcactgggag 720

gactttgaag gcaacccgat ggatagccgt ctgtgcgcga ttgacctgat gaacaccttc 780

cgtccgctga ttgcgatcaa ccgtttcgtg acctttggtg ttctggcgct gcaccaatat 840

ccggaaaccc gtgagaagat caaaaacgac ccggattacg cgtatatgtt tagccaggaa 900

gtgcgtcgtt actatccgtt cgttccgttt ctgccggcga aggcgaaagt ggatttcgag 960

tttgaaggcc acaagattga caaagatgtg atgatcctgc tggatgttta cggtaccatg 1020

caccgtgacg atgttttcga caacccgaac gagttctatc cggaacgttt taaggactgg 1080

gatggcagcc cgtttgatct gatcccgcaa ggtggcggtg actaccacac caaccaccgt 1140

tgcgcgggtg aatggatgac catcattatc atggaggaaa ccatgaaata cttcgcggag 1200

cgtattacct atgacgtgcc ggaacaggac ctgaccgttg atctggagaa gctgccgggc 1260

agcgttgcga gcggtatgaa catcaccaac gtgcgtgaga acgttaaccg taaataactc 1320

gag 1323

<210> 173
<211> 1326

```
<212>  DNA
<213>  Artificial Sequence

<220>
<223>  Codon optimized artificial DNA sequence

<400>  173
catatgcatc accaccacca ccacgagaac ctgtacttcc agggcatggc gaccatcaag      60

cgtgataaag gtctggacaa cagcgtgaag gttatgaaac agggctacct gtataccacc     120

aaccaacgtg agcgtctggg cgtgaccgac ggtgttttcg aaacccgtgc gctgggtggc     180

aagcgtatca ttgttctgag cggcaaagat ggtgcggagc tgttttatga taacgacaag     240

attgaacgta gcggtaccct gccgaaacgt gtggttaaca ccctgttcgg caagggtgcg     300

atccacacca ccaccggcaa ggtgcacatt gaccgtaaag cgctgtttat gagcctgatg     360

accgagggta acctgaaata cctgcgtgaa ctgacccgta acctgtggtt tgcgaacacc     420

cagcgtatgg agagcatgga tcaagtgaac gtttatcgtg aaagcatcat tgtgctgacc     480

aaagttggta cccgttgggc gggtgttcaa gcgccggaga aggaaatcga gaacattgcg     540

accgatatgg acatcatgat tgacagcttc aaagcgatcg gcagcgcgtt taagggttac     600

cgtgaggcga aagcggcgcg tcgtcgtgtg gaagattggc tggaggacca gatcattcaa     660

acccgtaagg gcaaaatcca cccgccgaag ggtaccgcgc tgtacgagtt cgcgcactgg     720

aaggattata aaggcgaacc gatggacagc cgtctgtgcg gtattgatct gatgaacacc     780

tttcgtccgc tgatcgcgat taaccgtttc gttgcgtttg gtgcgctggc gatgtacgag     840

aacccggtgg cgcgtgaaaa gatcaaacag gacgatgact acgcgtatat gttcgcgcaa     900

gaagttcgtc gtttctaccc gtttgtgccg tatctgccgg cgaaggcgaa agttgacttt     960

cagtacaagg gctatgaaat cgagaaagat accatgctgg cgctggacat ttacggtacc    1020

atgcacgatc cgaacgtgtg ggaagacccg aacgagttct atccggaacg ttttaaggat    1080

tgggatggca gcccgtttga cctgatcccg caaggtggcg gtgattacca caccaaccac    1140

cgttgcgcgg gcgagtggat gaccgttatc attatggagg aaaccatgaa atacttcgcg    1200

agccgtatta actatgacgt tccggaacag gatctgaccg tggacctgaa cagcctgccg    1260

ggctatatca agagcggttt tgtgattgag aacgttcaag aaaacgtgga tcgtacctaa    1320

ctcgag                                                                1326
```